# EUROPEAN PATENT APPLICATION

(11) **EP 3 034 089 A1**
(43) Date of publication of application: **22.06.2016**
(21) Application number: 15189979.6
(22) Date of filing: 02.11.2005
(51) Int. Cl.: A61K 38/17, C07K 14/715, A61K 31/7048, A61K 31/7072, A61K 31/704, A61K 31/4745, A61K 31/427, A61K 31/337, A61K 31/522, C12N 15/11, C12N 15/115

(54) **STABILIZED APTAMERS TO PLATELET DERIVED GROWTH FACTOR AND THEIR USE AS ONCOLOGY THERAPEUTICS**

(30) Priority: 02.11.2004 US 980211; 30.11.2004 US 632358 P; 01.12.2004 US 632609 P; 10.02.2005 US 652496 P; 11.02.2005 US 652494 P; 01.04.2005 US 667866 P; 15.04.2005 US 672200 P
(62) Divisional of application: 11180217.9
(71) Applicant: Archemix LLC, San Francisco, CA 94111 (US)
(72) Inventor: PREISS, Jeffrey, Somerville, MA 02145 (US); WILSON, Charles, Concord, MA 01742 (US); STANTON, Martin, Boulder, CO 80301 (US); DIENER, John, Cambridge, MA 02139 (US); EPSTEIN, David, Belmont, MA 02478 (US); MCCAULEY, Thomas, Cambridge, MA 02141 (US); MCCAULEY, Dilara, Cambridge, MA 02141 (US)
(74) Representative: Clegg, Richard Ian

(57) **Abstract**

Materials and methods are provided for producing and using aptamers as oncology therapeutics capable of binding to PDGF, PDGF isoforms, PDGF receptor, VEGF, and/or VEGF receptor or any combination thereof with affinity and specificity. The compositions of the present invention are particularly useful in solid tumor therapy and can be used alone or in combination with known cytotoxic agents for the treatment of solid tumors. Also disclosed are aptamers having one or more CpG motifs embedded therein or appended thereto.

## Description

### FIELD OF THE INVENTION

The invention relates generally to the field of nucleic acids and more particularly to aptamers capable of binding to platelet derived growth factor ("PDGF") useful as therapeutics in oncology and/or other diseases or disorders in which PDGF has been implicated. The invention further relates to materials and methods for the administration of aptamers capable of binding to platelet derived growth factor.

### BACKGROUND OF THE INVENTION

Aptamers are nucleic acid molecules having specific binding affinity to molecules through interactions other than classic Watson-Crick base pairing.

Aptamers, like peptides generated by phage display or monoclonal antibodies ('mAbs"), are capable of specifically binding to selected targets and modulating the target's activity or binding interactions, *e.g*., through binding aptamers may, block their target's ability to function. Discovered by an *in vitro* selection process from pools of random sequence oligonucleotides, aptamers have been generated for over 130 proteins including growth factors, transcription factors, enzymes, immunoglobulins, and receptors. A typical aptamer is 10-15 kDa in size (20-45 nucleotides), binds its target with nanomolar to sub-nanomolar affinity, and discriminates against closely related targets (*e.g*., aptamers will typically not bind other proteins from the same gene family). A series of structural studies have shown that aptamers are capable of using the same types of binding interactions (*e.g*., hydrogen bonding, electrostatic complementarities, hydrophobic contacts, steric exclusion) that drive affinity and specificity in antibody-antigen complexes.

Aptamers have a number of desirable characteristics for use as therapeutics (and diagnostics) including high specificity and affinity, biological efficacy, and excellent pharmacokinetic properties. In addition, they offer specific competitive advantages over antibodies and other protein biologics, for example:
1) Speed and control. Aptamers are produced by an entirely *in vitro* process, allowing for the rapid generation of initial leads, including therapeutic leads. *In vitro* selection allows the specificity and affinity of the aptamer to be tightly controlled and allows the generation of leads, including leads against both toxic and non-immunogenic targets. 2) Toxicity and Immunogenicity. Aptamers as a class have demonstrated therapeutically acceptable toxicity and lack of immunogenicity. Whereas the efficacy of many monoclonal antibodies can be severely limited by immune response to antibodies themselves, it is extremely difficult to elicit antibodies to aptamers most likely because aptamers cannot be presented by T-cells via the MHC and the immune response is generally trained not to recognize nucleic acid fragments.
3) Administration. Whereas most currently approved antibody therapeutics are administered by intravenous infusion (typically over 2-4 hours), aptamers can be administered by subcutaneous injection (aptamer bioavailability via subcutaneous administration is >80% in monkey studies (Tucker et al., J. Chromatography B. 732: 203-212, 1999)). With good solubility (>150 mg/mL) and comparatively low molecular weight (aptamer: 10-50 kDa; antibody: 150 kDa), a weekly dose of aptamer may be delivered by injection in a volume of less than 0.5 mL. In addition, the small size of aptamers allows them to penetrate into areas of conformational constrictions that do not allow for antibodies or antibody fragments to penetrate, presenting yet another advantage of aptamer-based therapeutics or prophylaxis.
4) Scalability and cost. Therapeutic aptamers are chemically synthesized and consequently can be readily scaled as needed to meet production demand. Whereas difficulties in scaling production are currently limiting the availability of some biologics and the capital cost of a large-scale protein production plant is enormous, a single large-scale oligonucleotide synthesizer can produce upwards of 100 kg/year and requires a relatively modest initial investment. The current cost of goods for aptamer synthesis at the kilogram scale is estimated at $500/g, comparable to that for highly optimized antibodies. Continuing improvements in process development are expected to lower the cost of goods to < $100/g in five years.
5) Stability. Therapeutic aptamers are chemically robust. They are intrinsically adapted to regain activity following exposure to factors such as heat and, denaturants and can be stored for extended periods (>1 yr) at room temperature as lyophilized powders.

### Interstitial Fluid Pressure

The three most common types of cancer treatment are surgical removal of cancerous tissue, radiotherapy to obliterate cancerous tissue, and chemotherapy. These treatments are aimed at removing the cancerous tissues or cells or destroying them in the body with therapeutics or other agents. Chemotherapy remains a major treatment modality for solid tumors. To potentially reduce toxic side effects and to achieve higher efficacy of chemotherapeutic drugs, strategies to improve distribution of drugs between normal tissues and tumors are highly desirable.

A major obstacle in the treatment of solid tumors is the limited uptake of therapeutic agents into tumor tissue. Elevated interstitial fluid pressure ("IFP") is one of the physiologically distinctive properties of solid tumors that differ from healthy connective tissue and is considered to be the main obstacle limiting free diffusion of therapeutics into solid tumors. PDGF receptors, particularly PDGF-β, have been implicated in the regulation of IFP. As a tumor enters a hyperproliferative state, blood supplying oxygen and other nutrients cannot keep up with the tumor's demands and a state of hypoxia results. Hypoxia triggers an "angiogenic switch" which up-regulates the expression of several factors, including VEGF and PDGF, which in turn serve to initiate angiogenesis. However, the angiogenesis that results forms an abnormal tumor vasculature. The tumor vasculature becomes impaired to the point of being unable to adequately drain excess fluid from the interstitium and fluid accumulation distends the elastic interstitial matrix causing an increase in pressure. When pressure exceeds capillary wall resistance, compression occurs and blood flow resistance increases.

This property of most solid tumors, tumor interstitial hypertension or increased IFP, has been suggested as a potential target for efforts to increase tumor drug uptake (Jain et al., (1987) Cancer Res., 47:3039-3051). Increased IFP acts as a barrier for tumor transvascular transport (Jain et al. (1997), Adv. Drug Deliv. Rev. 26:71-90). Lessening of tumor IFP, or modulation of microvascular pressure, has been shown to increase transvascular transport of tumor-targeting antibodies or low-molecular weight tracer compounds (Pietras et al., (2001), Cancer Res., 61, 2929-2934). The etiology of interstitial hypertension in tumors is poorly understood. One proposed theory is that the lack of lymphatic vessels in tumors is a contributing factor to the increased tumor IFP (Jain et al., (1987), Cancer Res., 47:3039-3051). Another proposed theory is that the microvasculature and the supporting stroma compartment are likely to be important determinants for tumor IFP (Pietras et al., (2002) Cancer Res., 62: 5476-5484). Accumulating evidence points toward the transmembrane PDGF β-preceptor tyrosine kinase as a potential target for pharmacological therapeutics to modulate tumor interstitial hypertension. Among other potential targets are growth factors that bind to the PDGF β-receptor.

### PDGF Mediated Cancer

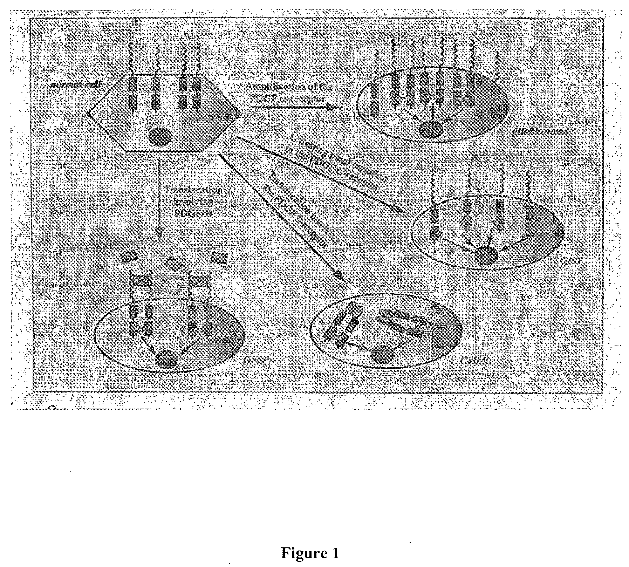

In addition to IFP and the difficulty of penetrating tumors with therapeutics, another obstacle in cancer treatment are mutations in certain forms of PDGF mediated cancer leading to constitutive expression of PDGF. These mutations drive abnormal proliferation of cells which results in the various forms of cancer as shown in Figure 1 (Pietras et al., (2001), Cancer Res., 61, 2929-2934). A gene mutation results in amplification of PDGF α-receptors in high grade glioblastomas. In chronic myelomonocytic leukemia (CMML), constitutive activation of PDGF-β receptors results from a mutation which causes the fusion of β-preceptors with proteins other than PDGF (Golub et al., (1994) Cell 77, 307-316, Magnusson et al., (2001) Blood 100, 623-626). Constitutive activation of PDGF-α -receptor due to activating point mutations has also been identified in patients with gastrointestinal stromal tumors (GIST) (Heinreich et al., (2003) Science 299, 708-710). Dermatofibrosarcoma protuberans (DFSP) is associated with constitutive production of fusion proteins which are processed to PDGF-BB (O'Brian et al., (1998) Gene Chrom. Cancer 23. 187-193; Shimiziu et al. (1999) Cancer Res. 59. 3719-3723; Simon et al. (1997) Nat. Genet., 15. 95-98). In addition to the constitutive activation of PDGF ligand and/or receptor due to mutations, up regulation has been shown in soft tissue sarcomas and gliomas (Ostman and Heldin, (2001), Adv. Cancer Res. 80, 1-38).

### PDGF

Growth factors are substances that have a cell-proliferative effect on cells or tissues. Any given growth factor may have more than one receptor or induce cell proliferation in more than one cell line or tissue. PDGF belongs to the cysteine-knot growth factor family and was originally isolated from platelets for promoting cellular mitogenic and migratory activity. PDGF is a strong mitogen and has a pivotal role in regulation of normal cell proliferation such as fibroblasts, smooth muscle cells, neuroglial cells and connective-tissue cells. In addition, PDGF mediates pathological cell growth such as in proliferative disorders, and also plays a role in angiogenesis. Another growth factor involved in tumor angiogenesis is vascular endothelial growth factor (VEGF).

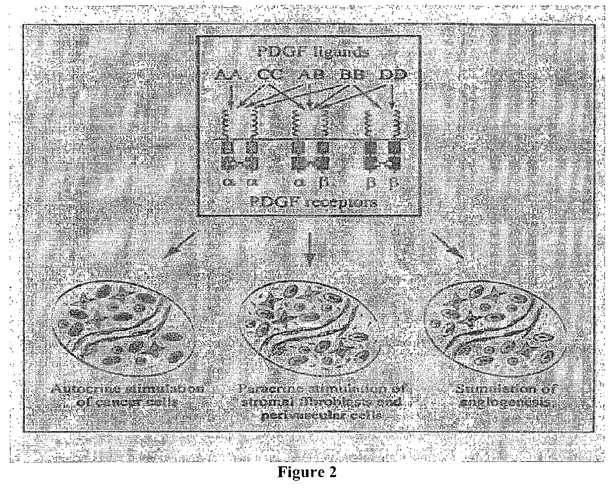

Four PDGF polypeptide chains have been identified which are currently known to make up five dimeric PDGF isoforms: PDGF-AA, -BB, -CC, -DD, and -AB. The most abundant species are PDGF AB and BB. PDGF isoforms bind to α and β tyrosine kinase receptors. PDGF receptors are expressed by many different cell types within tumors. The binding of PDGF isoforms to their cognate receptors induces the dimerization and subsequent phosphorylation of specific residues in the intracellular tyrosine kinase domain of the receptors and activation of the signaling pathway. PDGF isoforms -AA, -BB, -CC, and -AB induce PDGF α-receptor dimerization. PDGF-BB and PDGF-DD activate PDGF β receptor dimerization. All isoforms of PDGF except PDGF-AA activate both α and β receptors in cells which co-express both receptor types (Figure 2). Because they are potent mitogens, PDGF isoforms have been targeted for proliferative disease therapeutics development, such as cancer, diabetic retinopathy, glomerulonephritis, and restenosis.

PDGF, which is secreted by endothelial cells, acts as direct mitogen for fibroblasts, recruits pericytes and stimulates vascular smooth muscle cells. Many solid tumors display paracrine signaling of PDGF in the tumor stroma. PDGF is known to up-regulate synthesis of collagen and to mediate interactions of anchor proteins such as integrins with extracellular matrix (ECM) components. PDGF interactions between connective tissue, ECM and intracellular actin filament systems cause increased tensile strength which contributes to high IFP. High IFP is localized to the site of tumor and is associated with poor prognosis in human cancers as it increases with tumor size and severity and the grade of malignancy. The role of PDGF signaling in control of IFP and the up-regulated expression in various solid tumors has prompted investigation into whether the inhibition of PDGF signal ing can decrease IFP and thereby increase drug uptake into solid tumors. Previous work has demonstrated that inhibition of PDGF signaling with small molecule receptor antagonists and a PDGF specific aptamer decreases interstitial fluid pressure and increases the uptake of chemotherapeutics into solid tumors (Pietras et al., (2001), Cancer Res., 61: 2929-2934).

Accordingly, it would be benefcial to have novel materials and methods in oncology therapy to reduce tumor IFP, decrease tumor angiogenesis, and reduce the deleterious effects of mutation by the constitutive expression of PDGF. The present invention provides materials and methods to meet these and other needs.

### BRIEF DESCRIPTION OF THE DRAWINGS

Figure 1 is a schematic of gene mutations that give rise to constitutive PDGF receptor signaling in cancer cells found in glioblastomas, chronic myelomonocytic leukemia (CMML), dermatofibrosarcoma protuberans (DFSP), gastrointestinal stromal tumors (GIST), and other soft tissue sarcomas.
Figure 2 is a schematic of isoforms AA, BB, CC, DD, and AB of PDGF and cognate receptors.
Figure 3 is a schematic representation of the *in vitro* aptamer selection (SELEX™) process from pools of random sequence oligonucleotides.
Figure 4 is a schematic of the transport of cytotoxins across tumor vasculature with and without PDGF antagonists by the methods of the present invention.
Figure 5 is an illustration of a branched PEG reagent for coupling to a biomolecule.
Figure 6 is an illustration of a branched PEG conjugated to the 5'end of an aptamer.
Figure 7 is an illustration depicting various PEGylation strategies representing standard mono-PEGylation, multiple PEGylation, and dimerization via PEGylation.
Figure 8A is a plot of an ion-exchange HPLC analysis of ARC127 (5'-[40K PEG]-(SEQ ID NO:1)-HEG-(SEQ ID NO:2)-HEG-(SEQ ID NO:3)-3'-dT-3') freshly synthesized and stored at -20 °C for two years; Figure 8B is a bar-graph of 3T3 cell proliferation assay results for ARC126 (5'-(SEQ ID NO:1)-HEG-(SEQ ID NO:2)-HEG-(SEQ ID NO:3)-3'-dT-3') and ARC127 newly synthesized (Archemix) and after storage at -20 °C for 2 years (Proligo).

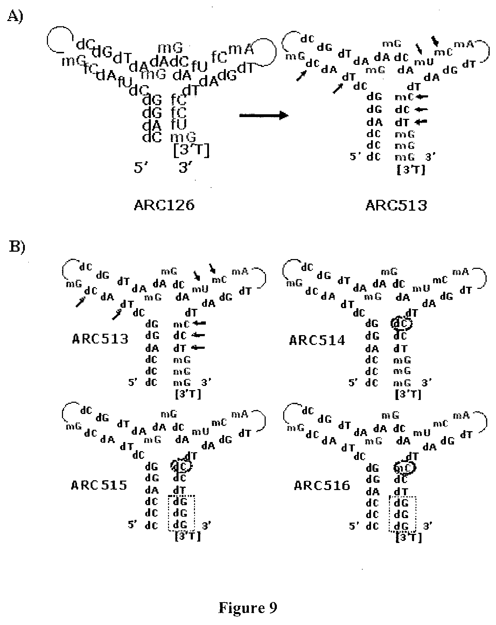

Figure 9A, left panel, is a schematic of the sequence and secondary structure of 2'-fluoro-containing PDGF aptamer composition of ARC126; Figure 9A, right panel, is a schematic of the sequence and secondary structure of ARC 126 variant ARC513 (5'-(SEQ ID NO: 69)- PEG -(SEQ ID NO: 40)- PEG -(SEQ ID NO 70) -3'-dT-3'); Figure 9B is a schematic of the sequence and secondary structure of ARC 126 variants ARC513 (5' -(SEQ ID NO: 69)- PEG -(SEQ ID NO: 40)- PEG -(SEQ ID NO 70) -3'-dT-3'), ARC514 (5'-(SEQ ID NO: 69)- PEG -(SEQ ID NO: 40)- PEG -(SEQ ID NO: 71) -3'-dT-3'), A RC515 (5'-(SEQ ID NO: 69)- PEG -(SEQ ID NO: 40)- PEG -(SEQ 1D NO: 72) -3'-dT-3'), and ARC516 (5'-(SEQ ID NO: 69)- PEG -(SEQ ID NO: 40)- PEG -(SEQ ID NO: 73) -3'-dT-3').
Figure 10A is a plot of competition binding assay results for ARC 126 and composition variants ARC128 (5'-(SEQ ID NO:4)-HEG-(SEQ ID NO:5)-HEG-(SEQ ID NO:6) -3'), ARC513, ARC514, ARC515, ARC516: Figure 10B is a plot of *in vitro* 3T3 cell-based proliferation assay data showing the activity of some composition variants of ARC126.
Figure 11A is a plot of a competition binding assay for ARC126 and two variants that are 5' conjugated to 30 kDa (ARC308, 5'-[30K PEG]-(SEQ ID NO:1)-HEG-(SEQ ID NO:2)-HEG-(SEQ ID NO:3)- 3'-dT-3') o 40 kDa (ARC127) PEG groups; Figure 11B is a plot of *in vitro* 3T3 cell-based proliferation assay data for ARC126 as a function of 5' PEG group conjugation (ARC126+30 kDa = ARC308, and ARC126+40 kDa PEG = ARC127).
Figure 12A is a plot of a pharmacokinetic profile of 5' conjugates of ARC 126 after IV administration at 10 mg/kg in mice; Figure 12B is a plot of the *in vivo* pharmacokinetic profile of ARC 127 (ARC 126 + 40 kDa PEG) after intravenous (IV), intraperitoneal (IP), and subcutaneous (SC) administration at a dose level of 10 mg/kg in mice; Figure 12C is a plot of the bioactivity profile of ARC126+ 40 k.Da PEG (*i.e.,* ARC 127) after intravenous (IV) administration at a dose level of 10 mg/kg in mice.
Figure 13A is a plot of a competition binding assay showing that ARC126 binds to PDGF-BB with a K_{d} of approximately 100 pM, and PDGF-AB with a K_{d} of approximately 100 pM, but does not bind to PDGF-AA; Fig. 13B is a plot of a competition binding assay showing that ARC 126 binds to human, rat and mouse PDGF-BB with an equal affinity of approximately 100 pM.
Figure 14A is a plot of the results of a 3T3 cell proliferation assay showing that ARC127 inhibits 3T3 cell proliferation better than PDGF Neutralizing antibody; Figure 14B is a plot of the results of a 3T3 cell proliferation assay showing that ARC127 inhibits 3T3 cell proliferation better than known tyrosine kinase inhibitors tested (AG-1295 in the left panel and AG-1433 in the right panel).
Figure 15 is a plot of cell viability assay results showing that ARC127 alone does not have any toxic effect on 3T3 cells after incubation for 24 or 48 hours.
Figure 16 is a plot of cell migration assay data performed in 96 well format using QCM Chemotaxis 96 Well Migration Assay (#ECM 510) (Chemicon, Temecula, CA); Figure 16A shows that the migration signal observed increases as a function of the number of cells plated, and Figure 16B shows migration signal as a function of increasing PDGF concentration.
Figure 17 is a plot of the results of a PDGF-driven Elk Luciferase assay showing that ARC 127 displays an IC₅₀ of 2 nM.
Figure 18A is a plot of tumor diameter versus time in Nu/Nu nude mice under a GLEEVEC™/irinotecan dose optimization study in HT29 colon cancer xenograft model; Figure 18B is a plot of tumor diameter versus time in Nu/Nu nude mice under ARC127/irinotecan study in LS174T colon cancer xenograft model. Figure 18C is a plot of tumor volume versus time in Nu/Nu nude mice under ARC127/irinotecan study in LS 174T colon cancer xenograft model.
Figure 19A is a plot of tumor diameter versus time in Nu/Nu nude mice under ARC308/irinotecan, and ARC308/GLEEVEC dosing regimens in an efficacy study in LS174T colon cancer xenotransplant model. Figure 19B is a plot of tumor volume versus time in Nu/Nu nude mice under ARC308/irinotecan dosing regimens in an efficacy study in LS 174T colon cancer xenotransplant model.
Figure 20A is a schematic of the sequence and secondary structure of an aptamer that binds to VEGF but not PDGF - referred to herein as ARC245 (SEQ ID NO:7); Figure 20B is a schematic of the sequence and secondary structure of an aptamer that binds to PDGF but not VEGF - referred to herein as ARC126 (5'-SEQ ID NO. 1-HEG-SEQ ID NO.2-HEG-SEQ ID NO. 3, wherein HEG represents hexaethylene glycol amidite.
Figure 21 A is a schematic of the sequence and secondary structure of a bivalent aptamer that binds to PDGF and VEGF (sequence TK. 131.012.A, SEQ ID NO:9); Figure 21B is a schematic of the sequence and secondary structure of a second bivalent aptamer that binds to PDGF and VEGF (sequence TK.131.012B, SEQ ID NO: 10).
Figure 22 is a plot of dot-blot assay binding data for the constituent aptamers and the multivalent aptamers to PDGF BB (Panel 1) and VEGF (Panel 2).
Figure 23 is a schematic of the sequence and secondary structures (absent an indication of the phosphorothioate bonds) of PDGF aptamers having CpG islands or motifs incorporated or embedded therein.

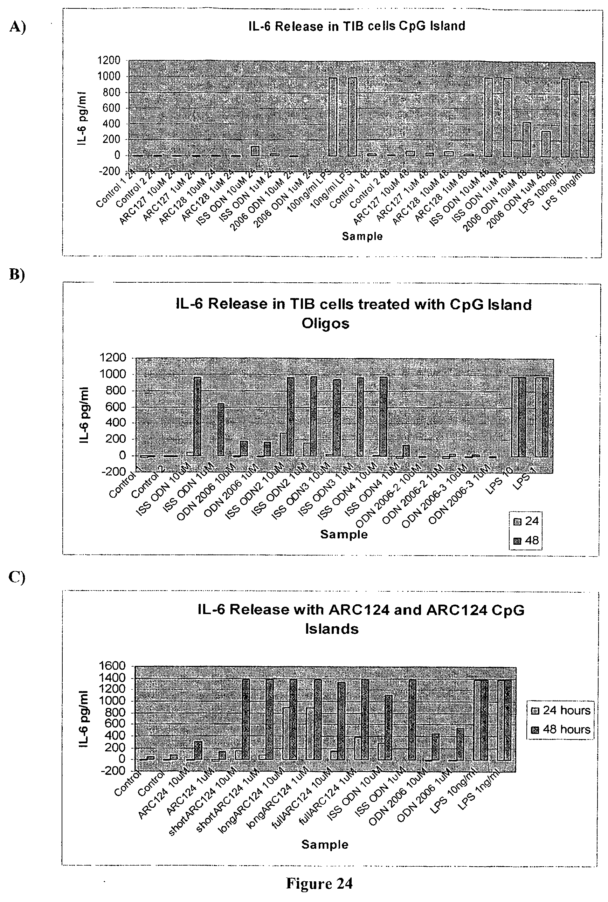

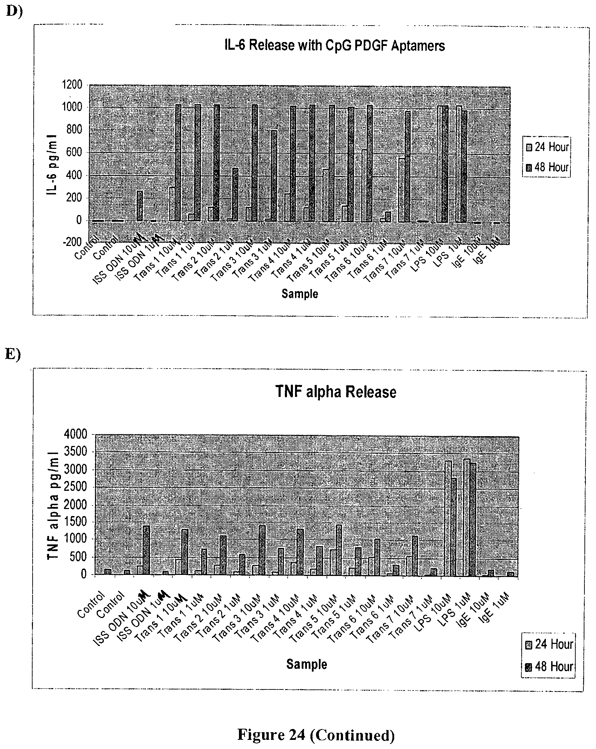

Figure 24A is a plot of the results of an IL-6 ELISA assay measuring IL-6 release in TIB cells using known immunostimulatory ODNs as positive controls, and aptamers which contain no CpG islands as negative controls in the assay; Figure 24B is a plot of the results of an IL-6 ELISA assay measuring IL-6 release in TIB cells using the ISS ODN and shortened versions of the ISS ODN; Figure 24C is a plot of the results of an IL-6 ELISA assay measuring IL-6 release in TIB cells using PDGF aptamers in which CpG motifs have been incorporated; Figure 24D is a plot of the results of an IL-6 ELISA assay measuring IL-6 release in TIB cells using additional PDGF aptamers in which CpG motifs have been incorporated. Figure 24E is a plot of the results of an TNFa ELISA assay measuring TNFa release in TIB cells using the same PDGF aptamers as in Figure 24D in which CpG motifs have been incorporated.
Figures 25A-25B are graphs depicting the dot blot binding analysis for clone RNA transcripts.
Figure 26 is a graph depicting the inhibitory effect of various PDGF-AA aptamers of the invention (ARX33P1.D2 (SEQ ID NO:78), ARX33P1.E5 (SEQ ID NO:79), and ARX33P1.E10 (SEQ ID NO:80)) on PDGF-AA induced 3T3 cell proliferation.
Figure 27 is a graph depicting the effect of PDGF-AA aptamers (ARX33P1.D2 (SEQ ID NO:78), ARX33P1.E5 (SEQ ID NO:79), and ARX33P1.E10 (SEQ ID NO:80) on PDGF-BB induced 3T3 cell proliferation.
Figure 28 is a plot of tumor volume versus time (post dosing period) in Nu/Nu nude mice under ARC127/irinotecan study in LS174T colon cancer xenograft model.
Figure 29 is a Kaplan-Meier representation of the data shown in Figure 28 wherein the percentage of mice in a treatment group exhibiting tumors less than 500 mm³ [as calculated from digital caliper measurements of length and width of the tumors, using the following formula: volume = (length x width²)/2] is depicted.
Figure 30 is graph showing group mean tumor volume (vertical axis) versus days after tumor implantation (horizontal axis) for LS174T tumors that have been treated with vehicle or ARC593.
Figure 31 is a graph showing the mean values for area density for CD31 stained blood vessels and α-SMA immunoreactive pericytes from a mouse Lewis lung carcinoma model in which the mice had been treated with vehicle, the aptamer ARC308 or the aptamer ARC594.
Figure 32 is is a graph showing area density (expressed as a percentage of vehicle control) of CD31 antibody stained and type IV collagen antibody stained Lewis lung carcinoma tissue that has been treated with vehicle, ARC308 or ARC594. In the graph: * *= P* < 0.05 vs. Vehicle, † = *P* < 0.05 vs. Aptamer 308.
Figure 33 is a graph showing area density (expressed as a percentage of vehicle control) of CD31 antibody stained, PDGFR-beta antibody stained, α-SMA antibody stained, NG2 antibody stained or desmin antibody stained Lewis lung carcinoma tissue from mice that have been treated with vehicle, ARC308 (denoted as AX101) or ARC594 (denoted as AX102). In the graph: * = *P* < 0.05 vs. Vehicle, † = *P* < 0.05 vs. Aptamer 308.
Figure 34 is a graph showing percent colocalization of CD31 and PDGFR-beta, CD31 and α-S1V1A, CD31 and NG2 and CD31 and desmin markers in Lewis lung carcinoma that had been treated with vehicle or ARC594 (denoted AX102). In the graph: *=<0.05 vs. Vehicle.
Figure 35 is a graph showing the number of individual vessels (vertical axis) in Lewis Lung carcinoma that had been treated with vehicle or ARC594 versus the range of colocalization of CD31 and NG2 (horizontal axis) on the vessels.
Figure 36 is is a graph showing area density (expressed as a percentage of vehicle control) of CD31 antibody stained (left bar) and type IV collagen antibody stained (right bar) Lewis lung carcinoma tissue that has been treated with vehicle, 2mg/kg, 10 mg/kg or 50 mg/kg of ARC594, where * *= P* < 0.05 vs. Vehicle, † = *P* < 0.05 vs. 2mg/kg ARC594, ‡ = *P <* 0.05 vs. 10mg/kg ARC594, and § = *P* < 0.05 vs. 50mg/kg ARC594.
Figure 37 is a graph showing area density (expressed as a percentage of vehicle control) of CD31 antibody stained and α-SMA antibody stained Lewis lung carcinoma tissue that has been treated with vehicle, 2mg/kg, 10 mg/kg or 50 mg/kg of ARC594, where * *= P* < 0.05 vs. Vehicle, † = *P* < 0.05 vs. 2mg/kg ARC594, ‡ = *P* < 0.05 vs. 10mg/kg ARC594, and § = *P* < 0.05 vs. 50mg/kg ARC594.
Figure 38 is a graph showing percent colocalization of CD31 antibody stained and α-SMA antibody stained or CD32 antibody stained and NG2 antibody stained Lewis lung carcinoma tissue that has been treated with vehicle, 2mg/kg, 10 mg/kg or 50 mg/kg of ARC594 where * = *P* < 0.05 vs. Vehicle, and $ = *P* < 0.05 vs. 50mg/kg ARC594.
Figure 39 is a graph showing area density (expressed as a percentage of vehicle control) of CD31 antibody stained, α-SMA antibody stained and NG2 antibody stained Lewis lung carcinoma tissue that has been treated with ARC594 for 1, 2, 4 or 7 days where * *= P* < 0.05 vs. Vehicle, † = *P* < 0.05 vs. 1 day treatment, ‡ = *P* < 0.05 vs. 2 day treatment, and § = *P* < 0.05 vs. 4 day treatment.
Figure 40 is a graph showing area density (express as a percentage of vehicle control) of α-SMA, NG2, PDGFR-beta and desmin antibody stained Lewis lung carcinoma tissue that has been treated with vehicle or ARC594 for one or seven days.
Figure 41 is a graph depicting tumor volume as a function of day of treatment with either vehicle or ARC594 (denoted as AX102).
Figure 42 is a graph showing tumor weight as function treatment time with either vehicle or ARC594 (denoted as AX102).
Figure 43 is a graph showing mouse body weight as a function of treatment time with either vehicle or ARC594 (denoted as AX102).
Figure 44 is a graph showing area density (expressed as a percentage of vehicle control) of CD31 antibody stained (left bar) and NG2 antibody stained (right bar) Lewis lung carcinoma tissue that has been treated with vehicle or ARC594 (denoted as AX102) for up to 28 days where * *= P* < 0.05 vs. Vehicle, † = *P* < 0.05 vs. CD31.
Figure 45 is a graph showing area density (expressed as a percentage of vehicle control) on the vertical axis oF CD31 antibody stained and PDGFR-beta antibody stained Lewis lung carcinoma tissue that has been treated with vehicle or ARC594 for various times, up to 28 days.
Figure 46 is a graph showing percent colocalization (vertical axis) of CD31 antibody stained and PDGFR-β antibody stained Lewis lung carcinoma tissue that has been treated with vehicle (first bar from left), with ARC594 for 7 days (second bar) or with ARC594 for 28 days (third bar).
Figure 47 is a set of three bar graphs showing the percentage of vessels (vertical axis) versus the percentage of pericyte coverage (horizontal axis) for Lewis lung carcinoma that has been treated with vehicle (top panel), ARC594 (denoted AX102) for 7 days (middle panel) or ARC594 (denoted AX102) for 28 days (bottom panel).
Figure 48 is a graph showing Vessel diameter in µm (vetical axis) versus treatment of Lewis lung carcinoma with vehicle or ARC594 for 4, 7, 14, 21 or 28 days (duration of treatment on horizontal axis).
Figure 49 is a graph showing area density (expressed as a percentage of vehicle control) on the vertical axis of Type IV collagen (denoted as Coll IV), CD31 or PDGFR-beta antibody stained Lewis lung carcinoma tissue that has been treated with vehicle, or ARC594 for 1, 2, 4, 7, 14, 21 or 28 days (duration of treatment on horizontal axis).
Figure 50 is a graph showing area density (expressed as a percentage of vehicle control) on the vertical axis of Type IV collagen (denoted as Coll IV), laminin or nidogen antibody stained Lewis lung carcinoma tissue that has been treated with vehicle, or ARC594 for 7 or 28 days (duration of treatment on horizontal axis).
Figure 51 is a graph showing area density (expressed as a percentage of vehicle control) of α-SMA antibody stained Lewis lung carcinoma tissue that has been treated with vehicle, scrambled aptamer (AX104), or 2mg/kg, 10 mg/kg or 50 mg/kg of ARC594 where * *= P* < 0.05 vs. Vehicle.
Figure 52 is a graph showing area density (expressed as a percentage of vehicle control) of α-SMA antibody stained Lewis lung carcinoma tissue that has been treated with vehicle or ARC594 for 1, 2, 4 or 7 days where * *= P* < 0.05 vs. Vehicle.
Figure 53 is a graph showing area density (expressed as a percentage of vehicle control) of CD31 antibody stained (left bar) and NG2 (right bar) antibody stained Rip-Tag2 tumor tissue that has been treated with vehicle, 2mg/kg, 10 mg/kg or 50 mg/kg of ARC594 (denoted as AX102) where * *= P* < 0.05 vs. Vehicle, † = *P <* 0.05 vs. 2mg/kg ARC594.
Figure 54 is a graph showing area density (expressed as a percentage of vehicle control) on vertical axis of α-SMA antibody-stained and CD31 antibody stained Rip-Tag2 tumor tissue that has been treated with vehicle or ARC594 for 7, 14 or 28 days (treatment duration on horizontal axis) .
Figure 55 is a plot of a competition binding assay showing that ARC594 binds to PDGF-BB with a K_{d} of approximately 100 pM, and PDGF-AB with a K_{d} of approximately 100 pM, but does not bind to PDGF AA.
Figure 56A is a graph depicting the plasma concentration of ARC594 in mice as a function of time; Figure 56B is a table listing pharmacokinetic properties of ARC594 (denoted as AX102) in mice.
Figure 57 is a bioactivity profile of ARC594 (denoted as AX102) after intravenous (IV) administration at a dose level of 10 mg/kg in mice, showing the aptamer pharmacodynamic profile is in accordance with its pharmacokinetic data.

### SUMMARY OF THE INVENTION

The present invention provides materials and methods for the treatment of cancer, solid tumor cancers in particular, by the administration to patients of therapeutically effective amounts of aptamers or aptamer compositions capable of binding with high affinity and specificity to platelet derived growth factor, vascular endothelial growth factor, their isoforms, their receptors, or any combination thereof, thus altering, e.g., inhibiting the bound ligand's biological role in cancer etiology. The aptamers of the present invention may be used in combination with known chemotherapeutic cytotoxic agents and/or other cancer treatments. Further, the aptamers of the invention may include one or more CpG motifs embedded therein or appended thereto.

The present invention provides aptamers that bind to PDGF. In one embodiment, the aptamers that bind to PDGF include a nucleic acid sequence selected from SEQ ID NO:1 to SEQ ID NO:3, SEQ ID NO:9 to SEQ ID NO:35, SEQ ID NO:36 to SEQ ID NO:73, SEQ ID NO:85, SEQ ID NO:77 to SEQ ID NO:81 and SEQ ID NOs 86-89, 91, 93-95 and 102. In one embodiment, the oligonucleotide sequence of the aptamer contains less than seven nucleotides having a 2' fluoro substituent.

In another embodiment, the aptamers of the invention comprises at least one further chemical modification. In one embodiment, the modification is selected from the group consisting of a chemical substitution at a sugar position; a chemical substitution at a phosphate position; and a chemical substitution at a base position, of the nucleic acid. In a particular embodiment, the modification is selected from the group consisting of: incorporation of a modified nucleotide, 3' capping, conjugation to a high molecular weight, non-immunogenic compound, and conjugation to a lipophilic compound. In one particular embodiment, the immunogenic, high molecular weight compound is polyalkylene glycol, particularly polyethylene glycol. For example, the immunogenic, high molecular weight compound is hexaethylene glycol amidite (HEG).

In a particular embodiment, the PDGF specific aptamer of the invention comprises the following structure: 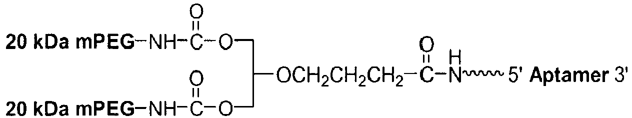 Where,
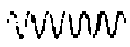 indicates a linker
Aptamer = 5'dCdCdCdAdGdGdCTdAdCmG (SEQ ID NO:69)- PEG-dCdGTdAmGdAmGdCdAmUmCmA (SEQ ID NO:40) -PEG - TdGdATmCdCTmGmGmG-3T-3' (SEQ ID NO:70); wherein d denotes a deoxy nucleotide, m denotes a 2'-OMe nucleotide, PEG denotes a PEG spacer and 3T denotes an inverted deoxy thymidine cap.

In another embodiment, the PDGF specific aptamer of the invention comprises the following structure: 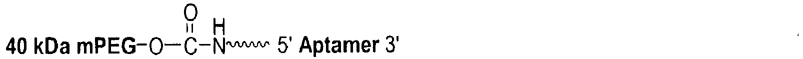 Where,
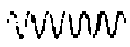 indicates a linker
Aptamer = 5'dCdCdCdAdGdGdCdTdAdCmG (SEQ ID NO:69) - PEG - dCdGdTdAmGdAmGdCdAmUmCmA (SEQ ID NO:40) -PEG - dTdGdAdTmCdCdTmGmGmG-3T-3' (SEQ ID NO:70); wherein d denotes a deoxy nucleotide, m denotes a 2'-OMe nucleotide, PEG denotes a PEG spacer, and 3T denotes an inverted deoxy thymidine cap.

In another embodiment, the PDGF specific aptamer of the invention comprises the following structure: 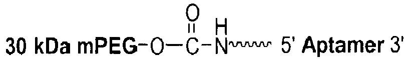 Where,
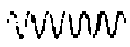 indicates a linker
Aptamer = 5'dCdCdCdAdGdGdCdTdAdCmG (SEQ ID NO:69) - PEG - dCdGdTdAmGdAmGdCdAmUmCmA (SEQ ID NO:40) -PEG - dTdGdAdTmCdCdTmGmGmG -3T-3' (SEQ ID NO:70); wherein d denotes a deoxy nucleotide, m denotes a 2'-OMe nucleotide, PEG denotes a PEG spacer, and 3T denotes an inverted deoxy thymidine cap.

In another embodiment, the PDGF specific aptamer of the invention comprises the following structure: 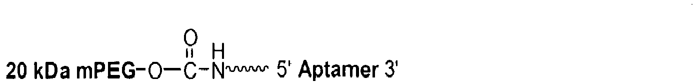 Where,
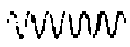 indicates a linker
Aptamer = 5'dCdCdCdAdGdGdCdTdAdCmG (SEQ ID NO:69) - PEG - dCdGdTdAmGdAmGdCdAmUmCmA (SEQ ID NO:40) -PEG - dTdGdAdTmCdCdTmGmGmG-3T-3' (SEQ ID NO:70); wherein d denotes a deoxy nucleotide, m denotes a 2'-OMe nucleotide, PEG denotes a PEG spacer and 3T denotes an inverted deoxy thymidine cap.

In another particular embodiment, the PDGF specific aptamer of the invention comprises the following structure: 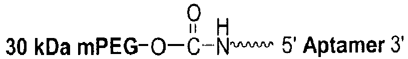 Where,
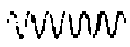 indicates a linker
Aptamer= 5'dCdAdGdGdCfUdAfCmG (SEQ ID NO: 1))-HEG-dCdGTdAmGdAmGdCdAfUfCmA (SEQ ID NO: 2)-HEG-TdGdATfCfCfUmG-3T-3' (SEQ ID NO:3); wherein d denotes a deoxy nucleotide, m denotes a 2'-OMe nucleotide, HEG denotes a hexaethylene glycol amidite (HEG) spacer, and 3T denotes an inverted deoxy thymidine.

In another embodiment, the PDGF specific aptamer of the invention comprises one of the following two structures: 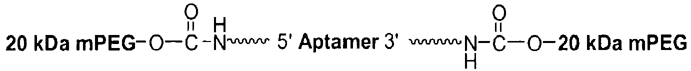 where,
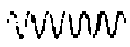 indicates a linker
**Aptamer** = 5'dCdCdCdAdGdGdCdTdAdCmG (SEQ ID NO:69) - PEG - dCdGdTdAmGdAmGdCdAmUmCmA (SEQ ID NO:40) -PEG - dTdGdAdTmCdCdTmGmGmG 3' (SEQ ID NO:70); wherein d denotes a deoxy nucleotide, m denotes a 2'-OMe nucleotide, and PEG denotes a PEG spacer; or 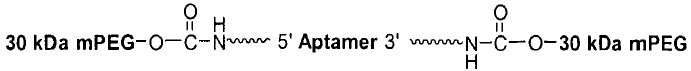 where,
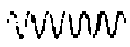 indicates a linker
**Aptamer** = 5'dCdCdCdAdGdGdCdTdAdCmG (SEQ ID NO:69)- PEG - dCdGdTdAmGdAmGdCdAmUmCmA (SEQ ID NO:40)- PEG-dTdGdAdTmCdCdTmGmGmG 3' (SEQ ID NO:70); wherein d denotes a deoxy nucleotide, m denotes a 2'-OMe nucleotide, and PEG denotes a PEG spacer.

In some embodiments of this aspect of the invention the linker is an alkyl linker. In particular embodiments, the alkyl linker comprises 2 to 18 consecutive CH₂ groups. In preferred embodiments, the alkyl linker comprises 2 to 12 consecutive CH₂ groups. In particularly preferred embodiments the alkyl linker comprises 3 to 6 consecutive CH₂ groups.

In particular embodiments, the aptamer of the invention comprises one of the following structures: 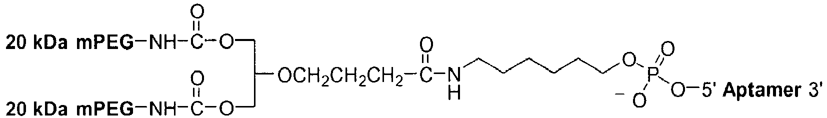 Wherein Aptamer = 5'dCdCdCdAdGdGdCTdAdCmG (SEQ ID NO:69)-PEG - dCdGTdAmGdAmGdCdAmUmCmA (SEQ ID NO:40) -PEG - TdGdATmCdCTmGmGmG-3T-3' (SEQ ID NO:70); wherein d denotes a deoxy nucleotide, m denotes a 2'-OMe nucleotide, PEG denotes a PEG spacer and 3T denotes an inverted deoxy thymidine cap (herein the above structure is referred to as ARC594 or AX102); 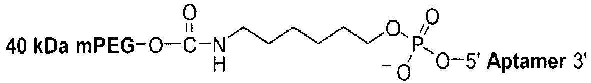 Wherein Aptamer = 5'dCdCdCdAdGdGdCTdAdCmG (SEQ ID NO:69)-PEG - dCdGTdAmGdAmGdCdAmUmCmA (SEQ ID NO:40) -PEG - TdGdATmCdCTmGmGmG-3T-3' (SEQ ID NO:70); wherein d denotes a deoxy nucleotide, m denotes a 2'-OMe nucleotide, PEG denotes a PEG spacer and 3T denotes an inverted deoxy thymidine cap (herein the above structure is referred to as ARC 1472); 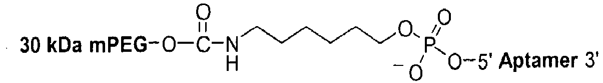 Wherein Aptamer = 5'dCdCdCdAdGdGdCTdAdCmG (SEQ ID NO:69)-PEG - dCdGTdAmGdAmGdCdAmUmCmA (SEQ ID NO:40) -PEG - TdGdATmCdCTmGmGmG-3T-3' (SEQ ID NO:70); wherein d denotes a deoxy nucleotide, m denotes a 2'-OMe nucleotide, PEG denotes a PEG spacer and 3T denotes an inverted deoxy thymidine cap (herein the above structure is referred to as ARC593); 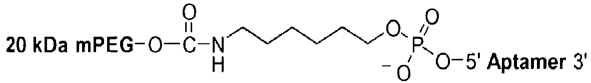 Wherein Aptamer = 5'dCdCdCdAdGdGdCTdAdCmG (SEQ ID NO:69)-PEG - dCdGTdAmGdAmGdCdAmUmCmA (SEQ ID NO:40) -PEG - TdGdATmCdCTmGmGmG-3T-3' (SEQ ID NO:70); wherein d denotes a deoxy nucleotide, m denotes a 2'-OMe nucleotide, PEG denotes a PEG spacer and 3T denotes an inverted deoxy thymidine cap (herein the above structure is referred to as ARC592).

In another particular embodiment, the aptamer of the invention comprises the following structure: 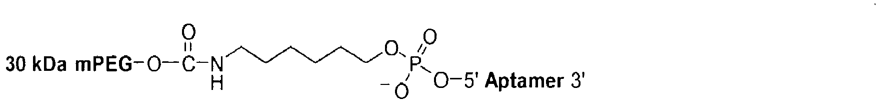 Wherein Aptamer = 5'dCdAdGdGdCfUdAfCmG (SEQ ID NO: 1)-HEG-dCdGTdAmGdAmGdCdAfUfCmA (SEQ ID NO: 2)-HEG-TdGdATfCfCtUmG-3T-3' (SEQ ID NO:3); wherein d denotes a deoxy nucleotide, m denotes a 2'-OMe nucleotide, HEG denotes a hexaethylene glycol amidite (HEG) spacer, and 3T denotes an inverted deoxy thymidine (herein the above structure is referred to as ARC308);

In some embodiments, the aptamer of the invention comprises one of the structures set forth below: 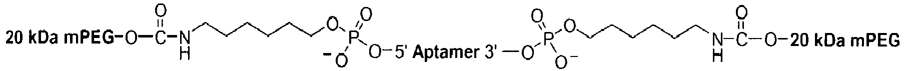 Wherein aptamer = 5'dCdCdCdAdGdGdCdTdAdCmG (SEQ ID NO:69) -PEG-dCdGdTdAmGdAmGdCdAmUmCmA (SEQ ID NO:40)-PEG-dTdGdAdTmCdCdTmGmGmG 3' (SEQ ID NO:70); wherein d denotes a deoxy nucleotide, m denotes a 2'-OMe nucleotide, and PEG denotes a PEG spacer (herein the above structure is referred to as ARC1473); or 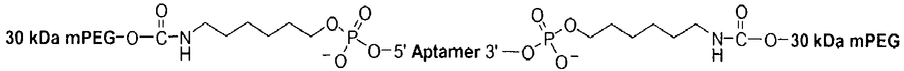 Wherein aptamer = 5'dCdCdCdAdGdGdCdTdAdCmG (SEQ ID NO:69) -PEG-dCdGdTdAmGdAmGdCdAmUmCmA (SEQ ID NO:40)-PEG-dTdGdAdTmCdCdTmGmGmG 3' (SEQ ID NO:70); wherein d denotes a deoxy nucleotide, m denotes a 2'-OMe nucleotide, and PEG denotes a PEG spacer (herein the above structure is referred to as ARC 1474).

The invention also provides aptamers that include a first sequence that binds to a first target and a second sequence that binds to a second target. In one embodiment, the first target is PDGF, PDGF-isoforms, or a PDGF receptor, and the second target is VEGF or a VEGF receptor. The PDGF isoforms are, for example, PDGF BB, PDGF AB, PDGF CC, and PDGF DD. The aptamers bind, for example, to one or more of PDGF-BB, PDGF-AB, PDGF-CC, PDGF-DD, PDGF-AA, all five isoforms, four isoforms, three isoforms or two isoforms. In one embodiment, the aptamers that bind to PDGF include a nucleic acid sequence selected from SEQ ID NO:1 to SEQ ID NO:3, SEQ ID NO:9 to SEQ ID NO:35, SEQ ID NO:36 to SEQ ID NO:73, SEQ ID NO:85, and SEQ ID NO:77 to SEQ ID NO:81 and SEQ ID NO:86-90,91, 93-95 and 102.

In one embodiment, the first target does not, upon binding of the aptamer, stimulate an immune response, and moreover, the second target does, upon binding of the aptamer, stimulate an immune response. In one embodiment, the second target is a toll-like receptor. In another embodiment, the second sequence is an immunostimulatory sequence. In one embodiment, the immunostimulatory sequence is a CpG motif.

In one embodiment, the first sequence is capable of binding to one of the following targets: PDGF, IgE, IgE Fcε R1, PSMA, CD22, TNF-α, CTLA4, PD-1, PD-L1, PD-L2, FcRIIB, BTLA, TIM-3, CD11c, BAFF, B7-X, CD19, CD20, CD25, or CD33. In one embodiment, the first sequence is capable of binding to PDGF.

The present invention also provides compositions that contain a PDGF-binding aptamer of the invention and a pharmaceutically acceptable carrier. In particular, embodiments of this aspect of the invention, the composition comprises a PDGF binding aptamer selected from ARC308, ARC404, ARC513, ARC592, ARC593, ARC594, ARC 1472, ARC1473 and ARC 1474.

In one embodiment, the compositions of the invention include a PDGF-binding aptamer of the invention, a cytotoxic agent and a pharmaceutically acceptable carrier. In one embodiment, the cytotoxic agent belongs to a class of cytotoxic agents such as tubulin stabilizers, tubulin destabilizers, anti-metabolites, purine synthesis inhibitors, nucleoside analogs, DNA alkylating agents, DNA modifying agents, and vascular disrupting agents. In one embodiment, the cytotoxic agent is used alone or in combinations with one or more cytotoxic agents selected from the group consisting of calicheamycin, doxorubicin, taxol, methotrexate, gemcitabine, cytarabine, vinblastin, daunorubicin, docetaxel, irinotecan, epothilone B, epothilone D, cisplatin, carboplatin, and 5-fluoro-U.

The invention also provides compositions that include a PDGF-binding aptamer of the invention, a VEGF-binding aptamer and a pharmaceutically acceptable carrier. In one embodiment, these compositions also include a cytotoxic agent. Suitable cytotoxic agents include agents belonging to a class of cytotoxic agents selected from the group consisting of tubulin stabilizers, tubulin destabilizers, anti-metabolites, purine synthesis inhibitors, nucleoside analogs, DNA alkylating agents, DNA modifying agents, and vascular disrupting agents. In one embodiment the cytotoxic agent is used alone or in combinations of one or more cytotoxic agents selected from the group consisting of calicheamycin, doxorubicin, taxol, methotrexate, gemcitabine, cytarabine, vinblastin, daunorubicin, docetaxel, irinotecan, epothilone B, epothilone D, cisplatin, carboplatin, and 5-fluoro-U.

The invention also provides methods of treating cancer by administering a therapeutically effective amount of a PDGF-binding aptamer of the invention. The invention also provides methods of treating cancer by administering a therapeutically effective amount of a composition of the invention.

In one embodiment, the cancer or tumor is a PDGF mediated cancer or tumor. In one embodiment, the PDGF mediated cancer or tumor is a glioblastoma, chronic myelomonocytic leukemia, a dermafibrosarcoma protuberan, a gastrointestinal stromal tumor or a soft tissue sarcoma.

In one embodiment, the composition includes a cytotoxic agent that belongs to a class of cytotoxic agents selected from the group consisting of tubulin stabilizers, tubulin destabilizers, anti-metabolites, purine synthesis inhibitors, nucleoside analogs, DNA alkylating agents, DNA modifying agents, and vascular disrupting agents. In one embodiment, the cytotoxic agent is used alone or in combinations of one or more cytotoxic agents such as calicheamycin, doxorubicin, taxol, methotrexate, gemcitabine, cytarabine, vinblastin, daunorubicin, docetaxel, irinotecan, epothilone B, epothilone D, cisplatin, carboplatin, and 5-fluoro-U.

The invention also provides methods of inhibiting growth of a solid tumor by administering a therapeutically effective amount of a PDGF-binding aptamer of the invention. The invention also provides methods of inhibiting growth of a solid tumor by administering a therapeutically effective amount of a composition of the invention.

In another aspect, the invention provides a method of reducing the number of pericytes in a tumor comprising treating the tumor with an aptamer or composition of the invention. In a particular embodiment, the aptamer is selected from ARC308, ARC404, ARC513, ARC592, ARC593, ARC594, and ARC1472 to ARC1474.

In one embodiment, the cancer or tumor is a PDGF mediated cancer or tumor. In one embodiment, the PDGF mediated cancer or tumor is a glioblastoma, chronic myelomonocytic leukemia, a dermafibrosarcoma protuberan, a gastrointestinal stromal tumor or a soft tissue sarcoma.

In one embodiment, the invention provides a method of treating a patient having a tumor, wherein the tumor does not express an elevated level of VEGF, the method comprising administering a therapeutically effective amount of a PDGF aptamer or composition of the invention to the patient. Tumors which express low levels of VEGF are known in the literature and are analogously described as weakly VEGF-driven. One of the tumor models used in the current invention, the Lewis Lung Carcinoma tumor model is weakly VEGF-driven. Strong VEGF-driven tumors, such as the RipTag2 model used to evaluate the PDGF aptamer of the present invention, express higher levels of VEGF. Human tumor cell lines used in mouse xenograft studies to profile the activity of anti-VEGF agents or other anti-angiogenic agents are known in the literature. Such cell lines are derived from human tumors of the brain, colon, lung, breast, prostate, and kidney, and are described as more or less VEGF-dependent according to level of VEGF expression. LS174t is one example of a strongly VEGF-driven xenograft tumor. In a particular embodiment, the PDGF antagonist is an anti-PDGF aptamer, particularly an aptamer selected from the group consisting of ARC308, ARC404, ARC513, ARC592, ARC593, ARC594, and ARC1472 to ARC1474.

In one embodiment, the composition includes a cytotoxic agent belonging to a class of cytotoxic agents selected from tubulin stabilizers, tubulin destabilizers, anti-metabolites, purine synthesis inhibitors, nucleoside analogs, DNA alkylating agents, DNA modifying agents, and vascular disrupting agents. In one embodiment, the cytotoxic agent is used alone or in combinations of one or more cytotoxic agents such as calicheamycin, doxorubicin, taxol, methotrexate, gemcitabine, cytarabine, vinblastin, daunorubicin, docetaxel, irinotecan, epothilone B, epothilone D, cisplatin, carboplatin, and 5-fluoro-U.

The invention also provides methods of reducing IFP in a solid tumor by administering a therapeutically effective amount of a PDGF-binding aptamer of the invention. The invention also provides methods of reducing IFP in a solid tumor by administering a therapeutically effective amount of a composition of the invention.

In one embodiment, the cancer or tumor is a PDGF mediated cancer or tumor. In one embodiment, the PDGF mediated cancer or tumor is a glioblastoma, chronic myelomonocytic leukemia, a dermafibrosarcoma protuberan, a gastrointestinal stromal tumor or a soft tissue sarcoma.

In one embodiment, the composition includes a cytotoxic agent belonging to a class of cytotoxic agents selected from the group consisting of tubulin stabilizers, tubulin destabilizers, anti-metabolites, purine synthesis inhibitors, nucleoside analogs, DNA alkylating agents, DNA modifying agents, and vascular disrupting agents. In one embodiment, the cytotoxic agent is used alone or in combinations of one or more cytotoxic agents such as calicheamycin, doxorubicin, taxol, methotrexate, gemcitabine, cytarabine, vinblastin, daunorubicin, docetaxel, irinotecan, epothilone B, epothilone D, cisplatin, carboplatin, and 5-fluoro-U.

The invention also provides methods of increasing the permeability of a solid tumor to cytotoxic agents by administering a therapeutically effective amount of a PDGF-binding aptamer of the invention. The invention also provides methods of increasing the permeability of a solid tumor to cytotoxic agents by administering a therapeutically effective amount of a composition of the invention.

In one embodiment, the cancer or tumor is a PDGF mediated cancer or tumor. In one embodiment, the PDGF mediated cancer or tumor is a glioblastoma, chronic myelomonocytic leukemia, a dermafibrosarcoma protuberan, a gastrointestinal stromal tumor or a soft tissue sarcoma.

In one embodiment, the composition includes a cytotoxic agent belonging to a class of cytotoxic agents selected from the group consisting of tubulin stabilizers, tubulin destabilizers, anti-metabolites, purine synthesis inhibitors, nucleoside analogs, DNA alkylating agents, DNA modifying agents, and vascular disrupting agents. In one embodiment, the cytotoxic agent is used alone or in combinations of one or more cytotoxic agents such as calicheamycin, doxorubicin, taxol, methotrexate, gemcitabine, cytarabine, vinblastin, daunorubicin, docetaxel, irinotecan, epothilone B, epothilone D, cisplatin, carboplatin, and 5-fluoro-U. In one embodiment of the invention the composition includes an anti-vascular agent or an anti-angiogenic agent belonging to the class of anti-VEGF agents selected from anti-VEGF or anti-VEGF-receptor RI and RII antisense drugs, anti-VEGF or anti-VEGF-receptor RI and RII siRNA drugs, anti-VEGF or anti-VEGF-receptor RI and RII aptamer drugs, anti-VEGF or anti-VEGF-receptor RI and RII antibody based drugs, anti-VEGF or anti-VEGF-receptor RI and RII monobody or adnectin based drugs, or anti-VEGF or anti-VEGF-receptor RI and RII chimeric protein based drugs, anti-Ang2, anti-Ang1 or anti-Tie 1 or anti-Tie 2 antisense, siRNA, aptamer, antibody, monobody, adnectin, or protein chimera derived drugs.

The invention also provides methods of reducing constitutive expression of platelet derived growth factor in a tumor by administering a therapeutically effective amount of a PDGF-binding aptamer of the invention. The invention also provides methods of reducing constitutive expression of platelet derived growth factor in a tumor by administering a therapeutically effective amount of a composition of the invention.

In one embodiment, the cancer or tumor is a PDGF mediated cancer or tumor. In one embodiment, the PDGF mediated cancer or tumor is a glioblastoma, chronic myelomonocytic leukemia, a dermafibrosarcoma protuberan, a gastrointestinal stromal tumor or a soft tissue sarcoma.

In one embodiment, the composition includes a cytotoxic agent belonging to a class of cytotoxic agents selected from the group consisting of tubulin stabilizers, tubulin destabilizers, anti-metabolites, purine synthesis inhibitors, nucleoside analogs, DNA alkylating agents, DNA modifying agents, and vascular disrupting agents. In one embodiment, the cytotoxic agent is used alone or in combinations of one or more cytotoxic agents such as calicheamycin, doxorubicin, taxol, methotrexate, gemcitabine, cytarabine, vinblastin, daunorubicin, docetaxel, irinotecan, epothilone B, epothilone D, cisplatin, carboplatin, and 5-fluoro-U.

The invention also provides methods of reducing angiogenesis, neovascularization or both angiogenesis and neovascularization in a tumor by administering a therapeutically effective amount of a PDGF-binding aptamer of the invention. The invention also provides methods of reducing angiogenesis, neovascularization or both angiogenesis and neovascularization in a tumor by administering a therapeutically effective amount of a composition of the invention. In a particular embodiment of the invention the aptamer is administered from 1 to 28 days prior to administration of an anti-VEGF or other anti-vascular directed agent. In another embodiment of the invention the aptamer is administered concurrently with an anti-VEGF or other anti-angiogenic of anti-vascular agent. In another embodiment of the invention the PDGF aptamer is administered for from 1 to 14 days prior to administration of the anti-angiogenic or other anti-vascular agent, other known cancer therapeutics, prior to surgical treatment or any other therapeutic or diagnostic procedure (e.g., radiation therapy). In a particular embodiment of the invention the anti-PDGF agent is administered for up to 14 days and then discontinued while anti-VEGF or anti-angiogenic therapy is continued. In another embodiment of the invention the PDGF agent is administered for from 1 to 28 days and the size of the tumor neovasculature, and the extent of tumor vascular growth is monitored by intravital microscopy to guide a physician or nurse in the timing of administration of anti-VEGF, anti-angiogenic, or anti-vascular agent. In another embodiment of the invention, the PDGF aptamer is administered from 1-28 days to remove VEGF-producing pericyte and mural cells from the tumor microenvironment as a means of altering the VEGF level of a solid tumor.

In one embodiment, the cancer or tumor is a PDGF mediated cancer or tumor. In one embodiment, the PDGF mediated cancer or tumor is a glioblastoma, chronic myelomonocytic leukemia, a dermafibrosarcoma protuberan, a gastrointestinal stromal tumor or a soft tissue sarcoma.

In one embodiment, the composition includes a cytotoxic agent belonging to a class of cytotoxic agents selected from the group consisting of tubulin stabilizers, tubulin destabilizers, anti-metabolites, purine synthesis inhibitors, nucleoside analogs, DNA alkylating agents, DNA modifying agents, and vascular disrupting agents. In one embodiment, the cytotoxic agent is used alone or in combinations of one or more cytotoxic agents such as calicheamycin, doxorubicin, taxol, methotrexate, gemcitabine, cytarabine, vinblastin, daunorubicin, docetaxel, irinotecan, epothilone B, epothilone D, cisplatin, carboplatin, and 5-fluoro-U.

The invention also provides methods of treating a PDGF mediated tumor, e.g., a solid tumor, in a subject comprising administering a PDGF binding aptamer of the invention to the subject and treating the subject with radiotherapy. In some embodiments, the PDGF binding aptamer of the invention increases edematous fluid content of the tumor, relative to an untreated tumor, prior to and/or during radiotherapy. In some embodiments, the PDGF binding aptamer of the invention increases the oxygen content of the tumor, relative to an untreated tumor, prior to and/or during radiotherapy. In a particular embodiment, the PDGF specific aptamer for use in radiotherapy is selected from the group consisting of: ARC308, ARC404, ARC513, 592, 593, ARC594, and ARC 1472 to ARC1474, particularly from the group consisting of ARC308, ARC 593 and ARC594. In one embodiment, the tumor to be treated with the aptamer of the invention and radiotherapy is a colorectal tumor.

The invention also provides methods of enhancing tumor imaging comprising administering a PDGF binding aptamer and a photosensitizer to a subject having a PDGF mediated tumor, activating the photosensitizer and detecting the tumor. In some embodiments, the PDGF binding aptamer of the invention increases the quantity of photosensitizer contained in the tumor, relative to a similar tumor not treated with the PDGF binding aptamer of the invention. In some embodiments, the PDGF binding aptamer and/or photosensitizer is administered by perfusing the tumor with, or allowing the tumor to be perfused with, an edematous fluid comprising the PDGF binding aptamer and/or photosensitizer. In a particular embodiment, the PDGF specific aptamer for use in tumor imaging is selected from the group consisting of: ARC308, ARC404, ARC513, ARC592, ARC593, ARC594, and ARC1472 to ARC 1474, particularly from the group consisting of ARC308, ARC593 and ARC594. In one embodiment, the tumor to be detected with the aptamer of the invention and photsensitizer is a colorectal tumor.

### DETAILED DESCRIPTION OF THE INVENTION

The details of one or more embodiments of the invention are set forth in the accompanying description below. Although any methods and materials similar or equivalent to those described herein can be used in the practice or testing of the present invention, the preferred methods and materials are now described. Other features, objects, and advantages of the invention will be apparent from the description. In the specification, the singular forms also include the plural unless the context clearly dictates otherwise. Unless defined otherwise, all technical and scientific terms used herein have the same meaning as commonly understood by one of ordinary skill in the art to which this invention belongs. In the case of conflict, the present Specification will control.

### THE SELEX™ METHOD

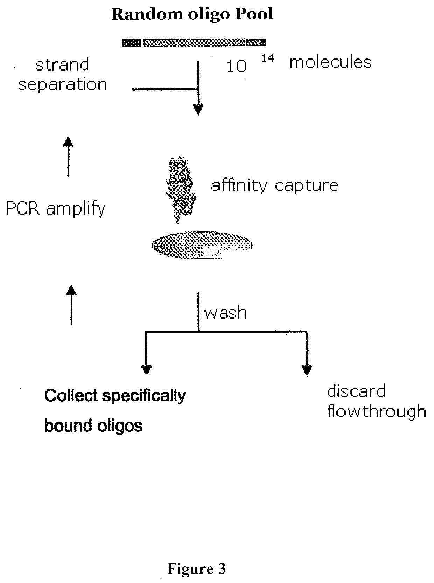

A suitable method for generating an aptamer is with the process entitled `'Systematic Evolution of Ligands by Exponential Enrichment'' ("SELEX™") generally depicted in Figure 3. The SELEX™ process is a method for the *in vitro* evolution of nucleic acid molecules with highly specific binding to target molecules and is described in, *e.g.,* U.S. patent application Ser. No. 07/536,428, filed Jun. 11, 1990, now abandoned, U.S. Pat. No. 5,475,096 entitled "Nucleic Acid Ligands", and U.S. Pat. No. 5,270,163 (*see* also WO 91/19813) entitled "Nucleic Acid Ligands". Each SELEX™-identified nucleic acid ligand, *i.e.,* each aptamer, is a specific ligand of a given target compound or molecule. The SELEX™ process is based on the unique insight that nucleic acids have sufficient capacity for forming a variety of two- and three-dimensional structures and sufficient chemical versatility available within their monomers to act as ligands (*i.e.,* form specific binding pairs) with virtually any chemical compound, whether monomeric or polymeric. Molecules of any size or composition can serve as targets.

SELEX™ relies as a starting point upon a large library or pool of single stranded oligonucleotides comprising randomized sequences. The oligonucleotides can be modified or unmodified DNA, RNA, or DNA/RNA hybrids. In some examples, the pool comprises 100% random or partially random oligonucleotides. In other examples, the pool comprises random or partially random oligonucleotides containing at least one fixed sequence and/or conserved sequence incorporated within randomized sequence. In other examples, the pool comprises random or partially random oligonucleotides containing at least one fixed and/or conserved sequence at its 5' and/or 3' end which may comprise a sequence shared by all the molecules of the oligonucleotide pool. Fixed sequences are sequences common to oligonucleotides in the pool which are incorporated for a preselected purpose, such as CpG motifs described further below, hybridization sites for PCR primers, promoter sequences for RNA polymerases (*e*.*g*., T3, T4, T7, and SP6), restriction sites, or homopolymeric sequences, such as poly A or poly T tracts, catalytic cores, sites for selective binding to affinity columns, and other sequences to facilitate cloning and/or sequencing of an oligonucleotide of interest. Conserved sequences are sequences, other than the previously described fixed sequences, shared by a number of aptamers that bind to the same target.

The oligonucleotides of the pool preferably include a randomized sequence portion as well as fixed sequences necessary for efficient amplification. Typically the oligonucleotides of the starting pool contain fixed 5' and 3' terminal sequences which flank an internal region of 30-50 random nucleotides. The randomized nucleotides can be produced in a number of ways including chemical synthesis and size selection from randomly cleaved cellular nucleic acids. Sequence variation in test nucleic acids can also be introduced or increased by mutagenesis before or during the selection/amplification iterations.

The random sequence portion of the oligonucleotide can be of any length and can comprise ribonucleotides and/or deoxyribonucleotides and can include modified or non-natural nucleotides or nucleotide analogs. *See, e.g.,* U.S. Patent No. 5,958,691; U.S. Patent No. 5,660,985; U.S. Patent No. 5,958,691; U.S. Patent No. 5,698,687; U.S. Patent No. 5,817,635; U.S. Patent No. 5,672,695, and PCT Publication WO 92/07065. Random oligonucleotides can be synthesized from phosphodiester-linked nucleotides using solid phase oligonucleotide synthesis techniques well known in the art. *See, e.g.,* Froehler et al., Nucl. Acid Res. 14:5399-5467 (1986) and Froehler et al., Tet. Lett. 27:5575-5578 (1986). Random oligonucleotides can also be synthesized using solution phase methods such as triester synthesis methods. *See, e.g.,* Sood et al., Nucl. Acid Res. 4:2557 (1977) and Hirose et al., Tet. Lett., 28:2449 (1978). Typical syntheses carried out on automated DNA synthesis equipment yield 10¹⁴-10¹⁶ individual molecules, a number sufficient for most SELEX™ experiments. Sufficiently large regions of random sequence in the sequence design increases the likelihood that each synthesized molecule is likely to represent a unique sequence.

The starting library of oligonucleotides may be generated by automated chemical synthesis on a DNA synthesizer. To synthesize randomized sequences, mixtures of all four nucleotides are added at each nucleotide addition step during the synthesis process, allowing for random incorporation of nucleotides. As stated above, in one embodiment, random oligonucleotides comprise entirely random sequences; however, in other embodiments, random oligonucleotides can comprise stretches of nonrandom or partially random sequences. Partially random sequences can be created by adding the four nucleotides in different molar ratios at each addition step.

The starting library of oligonucleotides may be either RNA or DNA, or substituted RNA or DNA. In those instances where an RNA library is to be used as the starting library it is typically generated by synthesizing a DNA library, optionally PCR amplifying, then transcribing the DNA library *in vitro* using T7 RNA polymerase or modified T7 RNA polymerases, and purifying the transcribed library. The RNA or DNA library is then mixed with the target under conditions favorable for binding and subjected to step-wise iterations of binding, partitioning and amplification, using the same general selection scheme, to achieve virtually any desired criterion of binding affinity and selectivity. More specifically, starting with a mixture containing the starting pool of nucleic acids, the SELEX™ method includes steps of: (a) contacting the mixture with the target under conditions favorable for binding; (b) partitioning unbound nucleic acids from those nucleic acids which have bound specifically to target molecules; (c) dissociating the nucleic acid-target complexes; (d) amplifying the nucleic acids dissociated from the nucleic acid-target complexes to yield a ligand-enriched mixture of nucleic acids; and (e) reiterating the steps of binding, partitioning, dissociating and amplifying through as many cycles as desired to yield highly specific, high affinity nucleic acid ligands to the target molecule. In those instances where RNA aptamers are being selected, the SELEX™ method further comprises the steps of: (i) reverse transcribing the nucleic acids dissociated from the nucleic acid-target complexes before amplification in step (d); and (ii) transcribing the amplified nucleic acids from step (d) before restarting the process.

Within a nucleic acid mixture containing a large number of possible sequences and structures, there is a wide range of binding affinities for a given target. A nucleic acid mixture comprising, for example, a 20 nucleotide randomized segment can have 4²⁰ candidate possibilities. Those which have the higher affinity (lower dissociation constants) for the target are most likely to bind to the target. After partitioning, dissociation and amplification, a second nucleic acid mixture is generated, enriched for the higher binding affinity candidates. Additional rounds of selection progressively favor the best ligands until the resulting nucleic acid mixture is predominantly composed of only one or a few sequences. These can then be cloned, sequenced and individually tested for binding affinity as pure ligands or aptamers.

Cycles of selection and amplification are repeated until a desired goal is achieved. In the most general case, selection/amplification is continued until no significant improvement in binding strength is achieved on repetition of the cycle. The method is typically used to sample approximately 10¹⁴ different nucleic acid species but may be used to sample as many as about 10¹⁸ different nucleic acid species. Generally, nucleic acid aptamer molecules are selected in a 5 to 20 cycle procedure. In one embodiment, heterogeneity is introduced only in the initial selection stages and does not occur throughout the replicating process.

In one embodiment of SELEX™, the selection process is so efficient at isolating those nucleic acid ligands that bind most strongly to the selected target, that only one cycle of selection and amplification is required. Such an efficient selection may occur, for example, in a chromatographic-type process wherein the ability of nucleic acids to associate with targets bound on a column operates in such a manner that the column is sufficiently able to allow separation and isolation of the highest affinity nucleic acid ligands.

In many cases, it is not necessarily desirable to perform the iterative steps of SELEX™ until a single nucleic acid ligand is identified. The target-specific nucleic acid ligand solution may include a family of nucleic acid structures or motifs that have a number of conserved sequences and a number of sequences which can be substituted or added without significantly affecting the affinity of the nucleic acid ligands to the target. By terminating the SELEX™ process prior to completion, it is possible to determine the sequence of a number of members of the nucleic acid ligand solution family.

A variety of nucleic acid primary, secondary and tertiary structures are known to exist. The structures or motifs that have been shown most commonly to be involved in non-Watson-Crick type interactions are referred to as hairpin loops, symmetric and asymmetric bulges, pseudoknots and myriad combinations of the same. Almost all known cases of such motifs suggest that they can be formed in a nucleic acid sequence of no more than 30 nucleotides. For this reason, it is often preferred that SELEX™ procedures with contiguous randomized segments be initiated with nucleic acid sequences containing a randomized segment of between about 20 to about 50 nucleotides, and of about 30 to about 40 nucleotides in some embodiments. In one example, the 5'-fixed:random:3'-fixed sequence comprises a random sequence of about 30 to about 50 nucleotides.

The core SELEX™ method has been modified to achieve a number of specific objectives. For example, U.S. Patent No. 5,707,796 describes the use of SELEX™ in conjunction with gel electrophoresis to select nucleic acid molecules with specific structural characteristics, such as bent DNA. U.S. Patent No. 5,763,177 describes SELEX™ based methods for selecting nucleic acid ligands containing photoreactive groups capable of binding and/or photocrosslinking to and/or photoinactivating a target molecule. U.S. Patent No. 5,567,588 and U.S. Patent No. 5,861,254 describe SELEX™ based methods which achieve highly efficient partitioning between oligonucleotides having high and low affinity for a target molecule. U.S. Patent No. 5,496,938 describes methods for obtaining improved nucleic acid ligands after the SELEX™ process has been performed. U.S. Patent No. 5,705,337 describes methods for covalently linking a ligand to its target.

SELEX™ can also be used to obtain nucleic acid ligands that bind to more than one site on the target molecule, and to obtain nucleic acid ligands that include non-nucleic acid species that bind to specific sites on the target. SELEX™ provides means for isolating and identifying nucleic acid ligands which bind to any envisionable target, including large and small biomolecules such as nucleic acid-binding proteins and proteins not known to bind nucleic acids as part of their biological function as well as cofactors and other small molecules. For example, U.S. Patent No. 5,580,737 discloses nucleic acid sequences identified through SELEX™ which are capable of binding with high affinity to caffeine and the closely related analog, theophylline.

Counter-SELEX™ is a method for improving the specificity of nucleic acid ligands to a target molecule by eliminating nucleic acid ligand sequences with cross-reactivity to one or more non-target molecules. Counter- SELEX™ is comprised of the steps of: (a) preparing a candidate mixture of nucleic acids; (b) contacting the candidate mixture with the target, wherein nucleic acids having an increased affinity to the target relative to the candidate mixture may be partitioned from the remainder of the candidate mixture; (c) partitioning the increased affinity nucleic acids from the remainder of the candidate mixture; (d) dissociating the increased affinity nucleic acids from the target; (e) contacting the increased affinity nucleic acids with one or more non-target molecules such that nucleic acid ligands with specific affinity for the non-target molecule(s) are removed; and (f) amplifying the nucleic acids with specific affinity only to the target molecule to yield a mixture of nucleic acids enriched for nucleic acid sequences with a relatively higher affinity and specificity for binding to the target molecule. As described above for SELEX™ cycles of selection and amplification are repeated as necessary until a desired goal is achieved.

One potential problem encountered in the use of nucleic acids as therapeutics and vaccines is that oligonucleotides in their phosphodiester form may be quickly degraded in body fluids by intracellular and extracellular enzymes such as endonucleases and exonucleases before the desired effect is manifest. The SELEX™ method thus encompasses the identification of high-affinity nucleic acid ligands containing modified nucleotides conferring improved characteristics on the ligand, such as improved *in vivo* stability or improved delivery characteristics. Examples of such modifcations include chemical substitutions at the sugar and/or phosphate and/or base positions. SELEX™-identified nucleic acid ligands containing modified nucleotides are described, *e.g.,* in U.S. Patent No. 5,660,985, which describes oligonucleotides containing nucleotide derivatives chemically modified at the 2' position of ribose, position of pyrimidines, and 8 position of purines, U.S. Patent No. 5,756,703 which describes oligonucleotides containing various 2'-modified pyrimidines, and U.S. Patent No. 5,580,737 which describes highly specific nucleic acid ligands containing one or more nucleotides modified with 2'-amino (2'-NH₂), 2'-fluoro (2'-F), and/or 2'-OMe substituents.

Modifications of the nucleic acid ligands contemplated in this invention include, but are not limited to, those which provide other chemical groups that incorporate additional charge, polarizability, hydrophobicity, hydrogen bonding, electrostatic interaction, and fluxionality to the nucleic acid ligand bases or to the nucleic acid ligand as a whole. Modifications to generate oligonucleotide populations which are resistant to nucleases can also include one or more substitute internucleotide linkages, altered sugars, altered bases, or combinations thereof. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil, backbone modifications, phosphorothioate or alkyl phosphate modifications, methylations, and unusual base-pairing combinations such as the isobases isocytidine and isoguanidine. Modifcations can also include 3' and 5' modifications such as capping.

In one embodiment, oligonucleotides are provided in which the P(O)O group is replaced by P(O)S ("thioate"), P(S)S ("dithioate"), P(O)NR₂ ("amidite"), P(O)R, P(O)OR', CO or CH₂ ("formacetal") or 3'-amine (-NH-CH2-CH₂-), wherein each R or R' is independently H or substituted or unsubstituted alkyl. Linkage groups can be attached to adjacent nucleotides through an -O-, -N-, or -S- linkage. Not all linkages in the oligonucleotide are required to be identical. As used herein, the term phosphorothioate encompasses one or more non-bridging oxygen atoms in a phosphodiester bond replaced by one or more sulfur atoms

In further embodiments, the oligonucleotides comprise modified sugar groups, for example, one or more of the hydroxyl groups is replaced with halogen, aliphatic groups, or functionalized as ethers or amines. In one embodiment, the 2'-position of the furanose residue is substituted by any of an O-methyl, O-alkyl, O-allyl, S-alkyl, S-allyl, or halo group. Methods of synthesis of 2'-modified sugars are described, *e.g.,* in Sproat, et al., Nucl. Acid Res. 19:733-738 (1991); Cotten, et al., Nucl. Acid Res. 19:2629-2635 (1991); and Hobbs, et al., Biochemistry 12:5138-5145 (1973). Other modifications are known to one of ordinary skill in the art. Such modifications may be pre-SELEX™ process modifications or post-SELEX™ process modifications (modification of previously identified unmodified ligands) or may be made by incorporation into the SELEX™ process.

Pre-SELEX™ process modifications or those made by incorporation into the SELEX™ process yield nucleic acid ligands with both specificity for their SELEX™ target and improved stability, *e.g., in vivo* stability. Post-SELEX™ process modifications made to nucleic acid ligands may result in improved stability, *e.g., in vivo* stability without adversely affecting the binding capacity of the nucleic acid ligand.

The SELEX™ method encompasses combining selected oligonucleotides with other selected oligonucleotides and non-oligonucleotide functional units as described in U.S. Patent No. 5,637,459 and U.S. Patent No. 5,683,867. The SELEX™ method further encompasses combining selected nucleic acid ligands with lipophilic or non-immunogenic high molecular weight compounds in a diagnostic or therapeutic complex, as described, *e.g.,* in U.S. Patent No. 6,011,020, U.S. Patent No. 6,051,698, and PCT Publication No. WO 98/18480. These patents and applications teach the combination of a broad array of shapes and other properties, with the efficient amplification and replication properties of oligonucleotides, and with the desirable properties of other molecules.

The identification of nucleic acid ligands to small, flexible peptides via the SELEX™ method has also been explored. Small peptides have flexible structures and usually exist in solution in an equilibrium of multiple conformers, and thus it was initially thought that binding affinities may be limited by the conformational entropy lost upon binding a flexible peptide. However, the feasibility of identifying nucleic acid ligands to small peptides in solution was demonstrated in U.S. Patent No. 5,648,214 in which high affinity RNA nucleic acid ligands to substance P, an 11 amino acid peptide, were identified.

The aptamers with specificity and binding affinity to the target(s) of the present invention are typically selected by the SELEX™ process as described herein. As part of the SELEX™ process, the sequences selected to bind to the target are then optionally minimized to determine the minimal sequence having the desired binding affinity. The selected aptamer sequences and/or the minimized aptamer sequences are optionally optimized by performing random or directed mutagenesis of the sequence to increase binding affinity or alternatively to determine which positions in the sequence are essential for binding activity. For example, a "doped reselections" may be used to explore the sequence requirements within an aptamer. During doped reselection, selections are carried out with a synthetic, degenerate pool that has been designed based on a single sequence. The level of degeneracy usually varies from 70% to 85% wild type nucleotide. In general, neutral mutations are observed following doped reselection but in some cases sequence changes can result in improvements in affinity. Additionally, selections can be performed with sequences incorporating modified sequences to stabilize the aptamer molecules against degradation *in vivo.*

### 2'MODIFIED SELEX™

In order for an aptamer to be suitable for use as a therapeutic, it is preferably inexpensive to synthesize, safe and stable *in vivo.* Wild-type RNA and DNA aptamers are typically not stable *in vivo* because of their susceptibility to degradation by nucleases. Resistance to nuclease degradation can be greatly increased, if desired, by the incorporation of modifying groups at the 2'-position.

2'-fluoro and 2'-amino groups have been successfully incorporated into oligonucleotide libraries from which aptamers have been subsequently selected. However, these modifications greatly increase the cost of synthesis of the resultant aptamer, and may introduce safety concerns in some cases because of the possibility that the modified nucleotides could be recycled into host DNA by degradation of the modified oligonucleotides and subsequent use of the nucleotides as substrates for DNA synthesis.

Aptamers that contain 2'-0-methyl ("2'-OMe") nucleotides, as provided in some embodiments herein, overcome many of these drawbacks. Oligonucleotides containing 2'-OMe nucleotides are nuclease-resistant and inexpensive to synthesize. Although 2'-OMe nucleotides are ubiquitous in biological systems, natural polymerases do not accept 2'-OMe NTPs as substrates under physiological conditions, thus there are no safety concerns over the recycling of 2'-OMe nucleotides into host DNA. The SELEX™ methods used to generate 2'-modified aptamers is described, *e.g*., in U.S. Provisional Patent Application Serial No. 60/430,761, filed December 3, 2002, U.S. Provisional Patent Application Serial No. 60/487,474, filed July 15, 2003, U.S. Provisional Patent Application Serial No. 60/517,039, filed November 4, 2003, U.S. Patent Application No. 10/729,581, filed December 3, 2003, U.S. Patent Application No. 10/873,856 filed June 21, 2004, entitled "Method for *in vitro* Selection of 2'-OMe Substituted Nucleic Acids", and U.S. Provisional Patent Application Serial No. 60/696,295, filed June 30, 2005, entitled "Improved Materials and Methods for the Generation of Fully 2'-Modified Containing Nucleic Acid Transcripts", each of which is herein incorporated by reference in its entirety.

The present invention includes aptamers that bind to and modulate the function of PDGF which contain modified nucleotides (*e.g*., nucleotides which have a modification at the 2'position) to make the oligonucleotide more stable than the unmodified oligonucleotide to enzymatic and chemical degradation as well as thermal and physical degradation. Although there are several examples of 2'-OMe containing aptamers in the literature (*see, e.g.,* Ruckman et al., J.Biol.Chem, (1998) 273, 20556-20567-695) these were generated by the *in vitro* selection of libraries of modified transcripts in which the C and U residues were 2'-fluoro (2'-F) substituted and the A and G residues were 2'-OH. Once functional sequences were identified then each A and G residue was tested for tolerance to 2'-OMe substitution, and the aptamer was re-synthesized having all A and G residues which tolerated 2'-OMe substitution as 2'-OMe residues. Most of the A and G residues of aptamers generated in this two-step fashion tolerate substitution with 2'-OMe residues, although, on average, approximately 20% do not. Consequently, aptamers generated using this method tend to contain from two to four 2'-OH residues, and stability and cost of synthesis are compromised as a result. By incorporating modified nucleotides into the transcription reaction which generate stabilized oligonucleotides used in oligonucleotide libraries from which aptamers are selected and enriched by SELEX™ (and/or any of its variations and improvements, including those described herein), the methods of the present invention eliminate the need for stabilizing the selected aptamer oligonucleotides (*e.g*., by resynthesizing the aptamer oligonucleotides with modified nucleotides).

In one embodiment, the present invention provides aptamers comprising combinations of 2'-OH, 2'-F, 2'-deoxy, and 2'-OMe modifications of the ATP, GTP, CTP, TTP, and UTP nucleotides. In another embodiment, the present invention provides aptamers comprising combinations of 2'-OH, 2'-F, 2'-deoxy, 2'-OMe, 2'-NH₂, and 2'-methoxyethyl modifications of the ATP, GTP, CTP, TTP, and UTP nucleotides. In another embodiment, the present invention provides aptamers comprising 56 combinations of 2'-OH, 2'-F, 2'-deoxy, 2'-OMe, 2'-NH₂, and 2'-methoxyethyl modifications of the ATP, GTP, CTP, TTP, and UTP nucleotides.

2'- modified aptamers of some embodiments of the invention are created using modified polymerases, *e.g*., a modified T7 polymerase, having a rate of incorporation of modified nucleotides having bulky substituents at the furanose 2' position that is higher than that of wild-type polymerases. For example, a mutant T7 polymerase (Y639F) in which the tyrosine residue at position 639 has been changed to phenylalanine readily utilizes 2'deoxy, 2'amino-, and 2'fluoro- nucleotide triphosphates (NTPs) as substrates and has been widely used to synthesize modified RNAs for a variety of applications. However, this mutant T7 polymerase reportedly can not readily utilize (*i.e*., incorporate) NTPs with bulky 2'-substituents such as 2'-OMe or 2'-azido (2'-N₃) substituents. For incorporation of bulky 2' substituents, a T7 polymerase mutant (Y639F/H784A) having the histidine at position 784 changed to an alanine residue in addition to the Y639F mutation has been described and has been used in limited circumstances to incorporate modified pyrimidine NTPs. *See* Padilla, R. and Sousa, R., Nucleic Acids Res., 2002, 30(24): 138. A Y639F/H784A/K378R mutant T7 RNA polymerase has been used in limited circumstances to incorporate modified purine and pyrimidine NTPs, *e.g*., 2'-OMe NPTs, but requires a spike of 2'-OH GTP for transcription. *See* Burmeister et.al., Chemistry and Biology, 2005, 12: 25-33. A mutant T7 polymerase (H784A) having the histidine at position 784 changed to an alanine residue has also been described. Padilla et al., Nucleic Acids Research, 2002, 30: 138. In both the Y639F/H784A mutant and H784A mutant T7 polymerases, the change to a smaller amino acid residue such as alanine allows for the incorporation of bulkier nucleotide substrates, *e.g*., 2'-O methyl substituted nucleotides. *See* Chelliserry, K. and Ellington, A.D., Nature Biotech, 2004, 9:1155-60. Additional T7 RNA polymerase have been described with mutations in the active site of the T7 RNA polymerase which more readily incorporate bulky 2'-modified substrates, *e.g.* a T7 mutant RNA polymerase having the tyrosine residue at position 639 changed to a leucine (Y639L). However activity is often sacrificed for increased substrate specificity conferred by such mutations, leading to low transcript yields. *See* Padilla R and Sousa, R., Nucleic Acids Res., 1999,27(6): 1561.

Generally, it has been found that under the conditions disclosed herein, the Y693F mutant can be used for the incorporation of all 2'-OMe substituted NTPs except GTP and the Y639F/H784A, Y639F/H784A/K378R, Y639L/H784A, and YG39L/H784A/K378R mutant T7 RNA polymerases can be used for the incorporation of all 2'-OMe substituted NTPs including GTP. It is expected that the H784A mutant possesses properties similar to the Y639F and the Y639F/H784A mutants when used under the conditions disclosed herein.

2'-modified oligonucleotides may be synthesized entirely of modified nucleotides, or with a subset of modified nucleotides. All nucleotides may be modified, and all may contain the same modification. All nucleotides may be modified, but contain different modifications, *e.g*., all nucleotides containing the same base may have one type of modification, while nucleotides containing other bases may have different types of modification. All purine nucleotides may have one type of modification (or are unmodified), while all pyrimidine nucleotides have another, different type of modification (or are unmodified). In this way, transcripts, or libraries of transcripts are generated using any combination of modifications, including for example, ribonucleotides (2'-OH), deoxyribonucleotides (2'-deoxy), 2'-F, and 2'-OMe nucleotides. A transcription mixture containing 2'-OMe C and U and 2'-OH A and G is referred to as a "rRmY" mixture and aptamers selected therefrom are referred to as "rRmY" aptamers. A transcription mixture containing deoxy A and G and 2'-OMe U and C is referred to as a "dRmY" mixture and aptamers selected therefrom are referred to as "dRmY" aptamers. A transcription mixture containing 2'-OMe A, C, and U, and 2'-OH G is referred to as a "rGmH" mixture and aptamers selected therefrom are referred to as "rGmH" aptamers. A transcription mixture alternately containing 2'-OMe A, C, U and G and 2'-OMe A, U and C and 2'-F G is referred to as a "alternating mixture" and aptamers selected therefrom are referred to as "alternating mixture" aptamers. A transcription mixture containing 2'-OMe A, U, C, and G, where up to 10% of the G's are ribonucleotides is referred to as a "r/mGmH" mixture and aptamers selected therefrom are referred to as "r/mGmH" aptamers. A transcription mixture containing 2'-OMe A, U, and C, and 2'-F G is referred to as a "fGmH" mixture and aptamers selected therefrom are referred to as "fGmH" aptamers. A transcription mixture containing 2'-OH A and G, and 2'-F C and U is referred to as an "rRfY" mixture and aptamers selected therefrom are referred to as "rRfY" aptamers. A transcription mixture containing 2'-OMe A, U, and C, and deoxy G is referred to as a "rGmH" mixture and aptamers selected therefrom are referred to as "dGmH" aptamers. A transcription mixture containing deoxy A, and 2'-OMe C, G and U is referred to as a "dAmB" mixture and aptamers selected therefrom are referred to as "dAmB" aptamers, and a transcription mixture containing all 2'-OH nucleotides is referred to as a "rN" mixture and aptamers selected therefrom are referred to as "rN", "rRrY", or "RNA" aptamers. A transcription mixture containing 2'-OH adenosine triphosphate and guanosine triphosphate and deoxy cytidine triphosphate and thymidine hiphosphate is referred to as a rRdY mixture and aptamers selected therefrom are referred to as "rRdY" aptamers. A "mRmY" aptamer is one containing only 2'-OMe nucleotides except for the starting nucleotide which is 2'-hydroxy.

A preferred embodiment includes any combination of 2'-OH, 2'-deoxy and 2'-OMe nucleotides. Another embodiment includes any combination of 2'-deoxy and 2'-OMe nucleotides. Yet another embodiment includes any combination of 2'-deoxy and 2'-OMe nucleotides in which the pyrimidines are 2'-OMe (such as dRmY, mRmY or dGmH).

Incorporation of modified nucleotides into the aptamers of the invention may be accomplished before (pre-) the selection process (*e.g*., a pre-SELEX™ process modification). Optionally, aptamers of the invention in which modified nucleotides have been incorporated by pre-SELEX™ process modification can be further modified by a post-SELEX™ modification process (*i.e.,* a post-SELEX™ process modification after a pre-SELEX™ modification). Pre-SELEX™ process modifications yield modified nucleic acid ligands with specificity for the SELEX™ target and also improved *in vivo* stability. Post-SELEX™ process modifications, *i.e.,* modification (*e.g.,* truncation, deletion, substitution or additional nucleotide modifications of previously identified ligands having nucleotides incorporated by pre-SELEX™ process modification) can result in a further improvement of *in vivo* stability without adversely affecting the binding capacity of the nucleic acid ligand having nucleotides incorporated by pre-SELEX™ process modification.

To generate pools of 2'-modified (*e.g*., 2'-OMe) RNA transcripts in conditions under which a polymerase accepts 2'-modified NTPs the Y693F, Y693F/ K378R, Y693F/H784A, Y693F/H784A/K378R, Y693L/H784A, Y693L/H784A/K378R, Y639L, or the Y639L/K378R mutant T7 RNA polymerases can be used. A preferred polymerase is the Y639L/H784A mutant T7 RNA polymerase. Another preferred polymerase is the Y639L/H784A/K378R mutant T7 RNA polymerase. Other T7 RNA polymerases, particularly those that exhibit a high tolerance for bulky 2'-substituents, may also be used in the present invention. When used in a template-directed polymerization using the conditions disclosed herein, the Y639L/H784A or the Y639L/H784A/K378R mutant T7 RNA polymerase can be used for the incorporation of all 2'-OMe NTPs, including GTP, with higher transcript yields than achieved by using the Y639F, Y639F/K378R, Y639F/H784A, Y639F/H784A/K378R, Y639L, or the Y639L/K378R mutant T7 RNA polymerases. When used under the 2'modified SELEX™ transcription conditions disclosed herein, the Y639L/H784A and Y639L/H784A/K378R mutant T7 RNA polymerases do not require 2'-OH GTP to achieve high yields of 2'-modified, *e.g*., 2'-OMe containing oligonucleotides.

A number of factors have been determined to be important for the transcription conditions useful in the methods disclosed herein. For example, increases in the yields of modified transcript are observed when a leader sequence is incorporated into the 5' end of the DNA transcription template. The leader sequence is typically 6-15 nucleotides long, and may be composed of all purines, or a mixture of purine and pyrimidine nucleotides.

Transcription can be divided into two phases: the first phase is initiation, during which an NTP is added to the 3'-hydroxyl end of GTP (or another substituted guanosine) to yield a dinucleotide which is then extended by about 10-12 nucleotides; the second phase is elongation, during which transcription proceeds beyond the addition of the first about 10-12 nucleotides. It has been found that small amounts of 2'-OH GTP added to a transcription mixture containing an excess of 2'-OMe GTP are sufficient to enable the polymerase to initiate transcription using 2'-OH GTP, but once transcription enters the elongation phase the reduced discrimination between 2'-OMe and 2'-OH GTP, and the excess of 2'-OMe GTP over 2'-OH GTP allows the incorporation of principally the 2'-OMe GTP.

Another important factor in the incorporation of2'-OMe substituted nucleotides into transcripts is the use of both divalent magnesium and manganese in the transcription mixture. Different combinations of concentrations of magnesium chloride and manganese chloride have been found to affect yields of 2'-O-methylated transcripts, the optimum concentration of the magnesium and manganese chloride being dependent on the concentration in the transcription reaction mixture of NTPs which complex divalent metal ions. To obtain the greatest yields of maximally 2'- O-methylated transcripts (*i.e*., all 2'-OMe A, C, and U and about 90% of G nucleotides), concentrations of approximately 5 mM magnesium chloride and 1.5 mM manganese chloride are preferred when each NTP is present at a concentration of 0.5 mM. When the concentration of each NTP is 1.0 mM, concentrations of approximately 6.5 mM magnesium chloride and 2.0 mM manganese chloride are preferred. When the concentration of each NTP is 2.0 mM, concentrations of approximately 9.5 mM magnesium chloride and 3.0 mM manganese chloride are preferred. In any case, departures from these concentrations of up to two-fold still give significant amounts of modified transcripts.

Priming transcription with GMP or guanosine, or another non-2'-OMe non-triphosphate is also important. This effect results from the specificity of the polymerase for the initiating nucleotide. As a result, the 5'-terminal nucleotide of any transcript generated in this fashion is likely to be 2'-OH G. The preferred concentration of GMP (or guanosine) is 0.5 mM and even more preferably 1 mM. It has also been found that including PEG, preferably PEG-8000, in the transcription reaction is useful to maximize incorporation of modified nucleotides.

For maximum incorporation of 2'-OMe ATP (100%), UTP (100%), CTP(100%) and GTP (∼90%) ("r/mGmH") into transcripts the following conditions are preferred: HEPES buffer 200 mM, DTT 40 mM, spermidine 2 mM, PEG-8000 10% (w/v), Triton X-100 0.01% (w/v), MgCl₂ 5 mM (6.5 mM where the concentration of each 2'-OMe NTP is 1.0 mM), MnCl₂ 1.5 mM (2.0 mM where the concentration of each 2'-OMe NTP is 1.0 mM), 2'-OMe NTP (each) 500 µM (more preferably, 1.0 mM). 2'-OH GTP 30 µM, 2'-OH GMP 500 µM, pH 7.5, Y639F/H784A T7 RNA Polymerase 200 nM, inorganic pyrophosphatase 5 units/ml, and an all-purine leader sequence of at least 8 nucleotides long. As used herein, one unit of the Y639F/H784A mutant T7 RNA polymerase (or any other mutant T7 RNA polymerase specified herein) is defined as the amount of enzyme required to incorporate 1 nmole of 2'-OMe NTPs into transcripts under the r/mGmH conditions. As used herein, one unit of inorganic pyrophosphatase is defined as the amount of enzyme that will liberate 1.0 mole of inorganic orthophosphate per minute at pH 7.2 and 25°C.

For maximum incorporation (100%) of 2'-OMe ATP, UTP and CTP ("rGmH") into transcripts the following conditions are preferred: HEPES buffer 200 mM, DTT 40 mM, spermidine 2 mM, PEG-8000 10% (w/v), Triton X-100 0.01% (w/v), MgCl₂ 5 mM (9.5 mM where the concentration of each 2'-OMe NTP is 2.0 mM), MnCl₂ 1.5 mM (3.0 mM where the concentration of each 2'-OMe NTP is 2.0 mM), 2'-OMe NTP (each) 500 µM (more preferably, 2.0 mM), pH 7.5, Y639F T7 RNA Polymerase 200 nM, inorganic pyrophosphatase 5 units/ml, and an all-purine leader sequence of at least 8 nucleotides long.

For maximum incorporation of 2'-OMe ATP (100%), 2'-OMe UTP (100%), 2'-OMe CTP (100%) and 2'-OMe GTP (100%) ("mRmY") into transcripts the following conditions are preferred: HEPES buffer 200 mM, DTT 40 mM, spermidine 2 mM, PEG-8000 10% (w/v), Triton X-100 0.01% (w/v), MgCl₂ 8 mM, MnCl₂ 2.5 mM, 2'-OMe NTP (each) 1.5 mM, 2'-OH GMP 1 mM, pH 7.5, Y639L/H784A/K378R mutant T7 RNA Polymerase 200nM, inorganic pyrophosphatase 5 units/ml, and a leader sequence that increases the transcription yield under the derived transcription conditions. In one embodiment, the leader sequence is an all purine leader sequence. In another embodiment, the leader sequence is a mixture of purines and pyrimidines. As used herein, one unit of inorganic pyrophosphatase is defined as the amount of enzyme that will liberate 1.0 mole of inorganic orthophosphate per minute at pH 7.2 and 25 °C.

For maximum incorporation (100%) of 2'-OMe UTP and CTP ("rRmY") into transcripts the following conditions are preferred: HEPES buffer 200 mM, DTT 40 mM, spermidine 2 mM, PEG-8000 10% (w/v), Triton X-100 0.01% (w/v), MgCl₂ 5 mM (9.5 mM where the concentration of each 2'-OMe NTP is 2.0 mM), MnCl₂ 1.5 mM (3.0 mM where the concentration of each 2'-OMe NTP is 2.0 mM), 2'-OMe NTP (each) 500µM (more preferably, 2.0 mM), pH 7.5, Y639F/H784A T7 RNA Polymerase 200 nM, inorganic pyrophosphatase 5 units/ml, and an all-purine leader sequence of at least 8 nucleotides long.

For maximum incorporation (100%) of deoxy ATP and GTP and 2'-OMe UTP and CTP ("dRmY") into transcripts the following conditions are preferred: HEPES buffer 200 mM, DTT 40 mM, spermine 2 mM, spermidine 2 mM, PEG-8000 10% (w/v), Triton X-100 0.01% (w/v), MgCl₂ 9.5 mM, MnCl₂ 3.0 mM, 2'-OMe NTP (each) 2.0 mM, pH 7.5, Y639F T7 RNA Polymerase 200 nM, inorganic pyrophosphatase 5 units/ml, and an all-purine leader sequence of at least 8 nucleotides long.

For maximum incorporation (100%) of 2'-OMe ATP, UTP and CTP and 2'-F GTP ("fGmH") into transcripts the following conditions are preferred: HEPES buffer 200 mM, DTT 40 mM, spermidine 2 mM, PEG-8000 10% (w/v), Triton X-100 0.01% (w/v), MgCl₂ 9.5 mM, MnCl₂ 3.0 mM, 2'-OMe NTP (each) 2.0 mM, pH 7.5, Y639F T7 RNA Polymerase 200 nM, inorganic pyrophosphatase 5 units/ml, and an all-purine leader sequence of at least 8 nucleotides long.

For maximum incorporation (100%) of deoxy ATP and 2'-OMe UTP, GTP and CTP ("dAmB") into transcripts the following conditions are preferred: HEPES buffer 200 mM, DTT 40 mM, spermidine 2 mM, PEG-8000 10% (w/v), Triton X-100 0.01% (w/v), MgCl₂ 9.5 mM, MnCl₂ 3.0 mM, 2'-OMe NTP (each) 2.0 mM, pH 7.5, Y639F T7 RNA Polymerase 200 nM, inorganic pyrophosphatase 5 units/ml, and an all-purine leader sequence of at least 8 nucleotides long.

For each of the above (a) transcription is preferably performed at a temperature of from about 20 °C to about 50 °C, preferably from about 30 °C to 45 °C, and more preferably at about 37 °C for a period of at least two hours and (b) 50-300 nM of a double stranded DNA transcription template is used (200 nM template is used in round 1 to increase diversity (300 nM template is used in dRmY transcriptions)), and for subsequent rounds approximately 50 nM, a 1/10 dilution of an optimized PCR reaction, using conditions described herein, is used). The preferred DNA transcription templates are described below (where ARC254 and ARC256 transcribe under all 2'-OMe conditions and ARC255 transcribes under rRmY conditions).
SEQ ID NO: 82 
5'-CATCGATGCTAGTCGTAACGATCCNNNNNNNNNNNNNNNNNNNNNNNNNNNNNNNNNNNNNNNNCGAGAACGTTCTCTCCTCTCCCT
ATAGTGAGTCGTATTA-3'
SEQ ID NO: 83 
5'-CATGCATCGCGACTGACTAGCCGNNNNNNNNNNNNNNNNNNNNNNNNNNNNNNNNNNNNGTAGAACGTTCTCTCCTCTCCCTA
TAGTGAGTCGTATTA-3'
SEQ ID NO: 84

5'-CATCGATCGATCGATCGACAGCGNNNNNNNNNNNNNNNNNNNNNNNNNNNNNNGTAGAACGTTCTCTCCTCTCCCTA
TAGTGAGTCGTATTA-3'

Under rN transcription conditions of the present invention, the transcription reaction mixture comprises 2'-OH adenosine triphosphates (ATP), 2'-OH guanosine triphosphates (GTP), 2'-OH cytidine triphosphates (CTP), and 2'-OH uridine triphosphates (UTP). The modified oligonucleotides produced using the rN transcription mixtures of the present invention comprise substantially all 2'-OH adenosine, 2'-OH guanosine, 2'-OH cytidine, and 2'-OH uridine. In a preferred embodiment of rN transcription, the resulting modified oligonucleotides comprise a sequence where at least 80% of all adenosine nucleotides are 2'-OH adenosine, at least 80% of all guanosine nucleotides are 2'-OH guanosine, at least 80% of all cytidine nucleotides are 2'-OH cytidine, and at least 80% of all uridine nucleotides are 2'-OH uridine. In a more preferred embodiment of rN transcription, the resulting modified oligonucleotides of the present invention comprise a sequence where at least 90% of all adenosine nucleotides are 2'-OH adenosine, at least 90% of all guanosine nucleotides are 2'-OH guanosine, at least 90% of all cytidine nucleotides are 2'-OH cytidine, and at least 90% of all uridine nucleotides are 2'-OH uridine. In a most preferred embodiment of rN transcription, the modified oligonucleotides of the present invention comprise a sequence where 100% of all adenosine nucleotides are 2'-OH adenosine, 100% of all guanosine nucleotides are 2'-OH guanosine, 100% of all cytidine nucleotides are 2'-OH cytidine, and 100% of all uridine nucleotides are 2'-OH uridine.

Under rRmY transcription conditions of the present invention, the transcription reaction mixture comprises 2'-OH adenosine triphosphates, 2'-OH guanosine triphosphates, 2'-OMe cytidine triphosphates, and 2'-OMe uridine triphosphates. The modified oligonucleotides produced using the rRmY transcription mixtures of the present invention comprise substantially all 2'-OH adenosine, 2'-OH guanosine, 2'-OMe cytidine and 2'-OMe uridine. In a preferred embodiment, the resulting modified oligonucleotides comprise a sequence where at least 80% of all adenosine nucleotides are 2'-OH adenosine, at least 80% of all guanosine nucleotides are 2'-OH guanosine, at least 80% of all cytidine nucleotides are 2'-OMe cytidine and at least 80% of all uridine nucleotides are 2'-OMe uridine. In a more preferred embodiment, the resulting modified oligonucleotides comprise a sequence where at least 90% of all adenosine nucleotides are 2'-OH adenosine, at least 90% of all guanosine nucleotides are 2'-OH guanosine, at least 90% of all cytidine nucleotides are 2'-OMe cytidine and at least 90% of all uridine nucleotides are 2'-OMe uridine In a most preferred embodiment, the resulting modified oligonucleotides comprise a sequence where 100% of all adenosine nucleotides are 2'-OH adenosine, 100% of all guanosine nucleotides are 2'-OH guanosine, 100% of all cytidine nucleotides are 2'-OMe cytidine and 100% of all uridine nucleotides are 2'-OMe uridine.

Under dRmY transcription conditions of the present invention, the transcription reaction mixture comprises 2'-deoxy adenosine triphosphates, 2'-deoxy guanosine triphosphates, 2'-O-methyl cytidine triphosphates, and 2'-O-methyl uridine triphosphates. The modified oligonucleotides produced using the dRmY transcription conditions of the present invention comprise substantially all 2'-deoxy adenosine, 2'-deoxy guanosine, 2'-O-methyl cytidine, and 2'-0-methyl uridine. In a preferred embodiment, the resulting modified oligonucleotides of the present invention comprise a sequence where at least 80% of all adenosine nucleotides are 2'-deoxy adenosine, at least 80% of all guanosine nucleotides are 2'-deoxy guanosine, at least 80% of all cytidine nucleotides are 2'-O-methyl cytidine, and at least 80% of all uridine nucleotides are 2'-O-methyl uridine. In a more preferred embodiment, the resulting modified oligonucleotides of the present invention comprise a sequence where at least 90% of all adenosine nucleotides are 2'-deoxy adenosine, at least 90 % of all guanosine nucleotides are 2'-deoxy guanosine, at least 90% of all cytidine nucleotides are 2'-0-methyl cytidine, and at least 90% of all uridine nucleotides are 2'-0-methyl uridine. In a most preferred embodiment, the resulting modified oligonucleotides of the present invention comprise a sequence where 100% of all adenosine nucleotides are 2'-deoxy adenosine, 100% of all guanosine nucleotides are 2'-deoxy guanosine, 100% of all cytidine nucleotides are 2'-O-methyl cytidine, and 100% of all uridine nucleotides are 2'-O-methyl uridine.

Under rGmH transcription conditions of the present invention, the transcription reaction mixture comprises 2'-OH guanosine triphosphates, 2'-O-methyl cytidine triphosphates, 2'-O-methyl uridine triphosphates, and 2'-O-methyl adenosine triphosphates. The modified oligonucleotides produced using the rGmH transcription mixtures of the present invention comprise substantially all 2'-OH guanosine, 2'-0-methyl cytidine, 2'-O-methyl uridine, and 2'-O-methyl adenosine. In a preferred embodiment, the resulting modified oligonucleotides comprise a sequence where at least 80% of all guanosine nucleotides are 2'-OH guanosine, at least 80% of all cytidine nucleotides are 2'-O-methyl cytidine, at least 80% of all uridine nucleotides are 2'-O-methyl uridine, and at least 80% of all adenosine nucleotides are 2'-0-methyl adenosine. In a more preferred embodiment, the resulting modified oligonucleotides comprise a sequence where at least 90% of all guanosine nucleotides are 2'-OH guanosine, at least 90% of all cytidine nucleotides are 2'-O-methyl cytidine, at least 90% of all uridine nucleotides are 2'-O-methyl uridine, and at least 90% of all adenosine nucleotides are 2'-O-methyl adenosine. In a most preferred embodiment, the resulting modified oligonucleotides comprise a sequence where 100% of all guanosine nucleotides are 2'-OH guanosine, 100% of all cytidine nucleotides are 2'-O-methyl cytidine, 100% of all uridine nucleotide are 2'-O-methyl uridine, and 100% of all adenosine nucleotides are 2'-0-methyl adenosine.

Under r/mGmH transcription conditions of the present invention, the transcription reaction mixture comprises 2'-O-methyl adenosine triphosphate, 2'-O-methyl cytidine triphosphate, 2'-O-methyl guanosine triphosphate, 2'-O-methyl uridine triphosphate and 2'-OH guanosine triphosphate. The resulting modified oligonucleotides produced using the r/mGmH transcription mixtures of the present invention comprise substantially all 2'-O-methyl adenosine, 2'-O-methyl cytidine, 2'-O-methyl guanosine, and 2'-O-methyl uridine, wherein the population of guanosine nucleotides has a maximum of about 10% 2'-OH guanosine. In a preferred embodiment, the resulting r/mGmH modified oligonucleotides of the present invention comprise a sequence where at least 80% of all adenosine nucleotides are 2'-O-methyl adenosine, at least 80% of all cytidine nucleotides are 2'-0-methyl cytidine, at least 80% of all guanosine nucleotides are 2'-O-methyl guanosine, at least 80% of all uridine nucleotides are 2'-O-methyl uridine, and no more than about 10% of all guanosine nucleotides are 2'-OH guanosine. In a more preferred embodiment, the resulting modified oligonucleotides comprise a sequence where at least 90% of all adenosine nucleotides are 2'-0-methyl adenosine, at least 90% of all cytidine nucleotides are 2'-O-methyl cytidine, at least 90% of all guanosine nucleotides are 2'-O-methyl guanosine, at least 90% of all uridine nucleotides are 2'-O-methyl uridine, and no more than about 10% of all guanosine nucleotides are 2'-OH guanosine. In a most preferred embodiment, the resulting modified oligonucleotides comprise a sequence where 100% of all adenosine nucleotides are 2'-0-methyl adenosine, 100% of all cytidine nucleotides are 2'-0-methyl cytidine, 90% of all guanosine nucleotides are 2'-O-methyl guanosine, and 100% of all uridine nucleotides are 2'-O-methyl uridine, and no more than about 10% of all guanosine nucleotides are 2'-OH guanosine.

Under mRmY transcription conditions of the present invention, the transcription mixture comprises only 2'-0-methyl adenosine triphosphate, 2'-0-methyl cytidine triphosphate, 2'-O-methyl guanosine triphosphate, 2'-O-methyl uridine triphosphate. The resulting modified oligonucleotides produced using the mRmY transcription mixture of the present invention comprise a sequence where 100% of all adenosine nucleotides are 2'-O-methyl adenosine, 100% of all cytidine nucleotides are 2'-O-methyl cytidine, 100% of all guanosine nucleotides are 2'-O-methyl guanosine, and 100% of all uridine nucleotides are 2'-O-methyl uridine.

Under fGmH transcription conditions of the present invention, the transcription reaction mixture comprises 2'-0-methyl adenosine triphosphates, 2'-0-methyl uridine triphosphates, 2'-0-methyl cytidine triphosphates, and 2'-F guanosine triphosphates. The modified oligonucleotides produced using the fGmH transcription conditions of the present invention comprise substantially all 2'-O-methyl adenosine, 2'-O-methyl uridine, 2'-O-methyl cytidine, and 2'-F guanosine. In a preferred embodiment, the resulting modified oligonucleotides comprise a sequence where at least 80% of all adenosine nucleotides are 2'-O-methyl adenosine, at least 80% of all uridine nucleotides are 2'-O-methyl uridine, at least 80% of all cytidine nucleotides are 2'-O-methyl cytidine, and at least 80% of all guanosine nucleotides are 2'-F guanosine. In a more preferred embodiment, the resulting modified oligonucleotides comprise a sequence where at least 90% of all adenosine nucleotides are 2'-O-methyl adenosine, at least 90% of all uridine nucleotides are 2'-O-methyl uridine, at least 90% of all cytidine nucleotides are 2'-O-methyl cytidine, and at least 90% of all guanosine nucleotides are 2'-F guanosine. In a most preferred embodiment, the resulting modified oligonucleotides comprise a sequence where 100% of all adenosine nucleotides are 2'-O-methyl adenosine, 100% of all uridine nucleotides are 2'-O-methyl uridine, 100% of all cytidine nucleotides are 2'-O-methyl cytidine, and 100% of all guanosine nucleotides are 2'-F guanosine.

Under dAmB transcription conditions of the present invention, the transcription reaction mixture comprises 2'-deoxy adenosine triphosphates, 2'-O-methyl cytidine triphosphates, 2'-O-methyl guanosine triphosphates, and 2'-O-methyl uridine triphosphates. The modified oligonucleotides produced using the dAmB transcription mixtures of the present invention comprise substantially all 2'-deoxy adenosine, 2'-O-methyl cytidine, 2'-O-methyl guanosine, and 2'-O-methyl uridine. In a preferred embodiment, the resulting modified oligonucleotides comprise a sequence where at least 80% of all adenosine nucleotides are 2'-deoxy adenosine, at least 80% of all cytidine nucleotides are 2'-O-methyl cytidine, at least 80% of all guanosine nucleotides are 2'-O-methyl guanosine, and at least 80% of all uridine nucleotides are 2'-O-methyl uridine. In a more preferred embodiment, the resulting modified oligonucleotides comprise a sequence where at least 90% of all adenosine nucleotides are 2'-deoxy adenosine, at least 90% of all cytidine nucleotides are 2'-O-methyl cytidine, at least 90% of all guanosine nucleotides are 2'-O-methyl guanosine, and at least 90% of all uridine nucleotides are 2'-O-methyl uridine. In a most preferred embodiment, the resulting modified oligonucleotides of the present invention comprise a sequence where 100% of all adenosine nucleotides are 2'-deoxy adenosine, 100% of all cytidine nucleotides are 2'-O-methyl cytidine, 100% of all guanosine nucleotides are 2'-O-methyl guanosine, and 100% of all uridine nucleotides are 2'-O-methyl uridine.

In each case, the transcription products can then be used for input into the SELEX™ process to identify aptamers and/or to determine a conserved sequences that has binding specificity to a given target. The resulting sequences are already partially stabilized, eliminating this step from the post-SELEX™ process to arrive at an optimized aptamer sequence and giving a more highly stabilized aptamer as a result. Another advantage of the 2'-OMe SELEX™ process is that the resulting sequences are likely to have fewer 2'-OH nucleotides required in the sequence, possibly none. To the extent 2'OH nucleotides remain they may be removed by performing post-SELEX™ modifications.

As described below, lower but still useful yields of transcripts fully incorporating 2' substituted nucleotides can be obtained under conditions other than the optimized conditions described above. For example, variations to the above transcription conditions include:

The HEPES buffer concentration can range from 0 to 1 M. The present invention also contemplates the use of other buffering agents having a pKa between 5 and 10 including, for example, Tris-hydroxymethyl-aminomethane.

The DTT concentration can range from 0 to 400 mM. The methods of the present invention also provide for the use of other reducing agents including, for example, mercaptoethanol.

The spermidine and/or spermine concentration can range from 0 to 20 mM.

The PEG-8000 concentration can range from 0 to 50 % (w/v). The methods of the present invention also provide for the use of other hydrophilic polymer including, for example, other molecular weight PEG or other polyalkylene glycols.

The Triton X-100 concentration can range from 0 to 0.1 % (w/v). The methods of the present invention also provide for the use of other non-ionic detergents including, for example, other detergents, including other Triton-X detergents.

The MgCl₂ concentration can range from 0.5 mM to 50 mM. The MnCl₂ concentration can range from 0.15 mM to 15 mM. Both MgCl₂ and MnCl2 must be present within the ranges described and in a preferred embodiment are present in about a 10 to about 3 ratio of MgCl₂:MnCl₂, preferably, the ratio is about 3-5:1, more preferably, the ratio is about 3-4:1.

The 2'-OMe NTP concentration (each NTP) can range from 5 µM to 5 mM.

The 2'-OH GTP concentration can range from 0 µM to 300 µM.

The 2'-OH GMP concentration can range from 0 to 5 mM.

The pH can range from pH 6 to pH 9. The methods of the present invention can be practiced within the pH range of activity of most polymerases that incorporate modified nucleotides. In addition, the methods of the present invention provide for the optional use of chelating agents in the transcription reaction condition including, for example, EDTA, EGTA, and DTT.

### APTAMER MEDICINAL CHEMISTRY

Aptamer Medicinal Chemistry is an aptamer improvement technique in which sets of variant aptamers are chemically synthesized. These sets of variants typically differ from the parent aptamer by the introduction of a single substituent, and differ from each other by the location of this substituent. These variants are then compared to each other and to the parent. Improvements in characteristics may be profound enough that the inclusion of a single substituent may be all that is necessary to achieve a particular therapeutic criterion.

Alternatively the information gleaned from the set of single variants may be used to design further sets of variants in which more than one substituent is introduced simultaneously. In one design strategy, all of the single substituent variants are ranked, the top 4 are chosen and all possible double (6), triple (4) and quadruple (1) combinations of these 4 single substituent variants are synthesized and assayed. In a second design strategy, the best single substituent variant is considered to be the new parent and all possible double substituent variants that include this highest-ranked single substituent variant are synthesized and assayed. Other strategies may be used, and these strategies may be applied repeatedly such that the number of substituents is gradually increased while continuing to identify further-improved variants.

Aptamer Medicinal Chemistry may be used particularly as a method to explore the local, rather than the global, introduction of substituents. Because aptamers are discovered within libraries that are generated by transcription, any substituents that are introduced during the SELEX™ process must be introduced globally. For example, if it is desired to introduce phosphorothioate linkages between nucleotides then they can only be introduced at every A (or every G, C, T, U etc.) (globally substituted). Aptamers which require phosphorothioates at some As (or some G, C, T, U etc.) (locally substituted) but cannot tolerate it at other As cannot be readily discovered by this process.

The kinds of substituent that can be utilized by the Aptamer Medicinal Chemistry process are only limited by the ability to generate them as solid-phase synthesis reagents and introduce them into an oligomer synthesis scheme. The process is certainly not limited to nucleotides alone. Aptamer Medicinal Chemistry schemes may include substituents that introduce steric bulk, hydrophobicity, hydrophilicity, lipophilicity, lipophobicity, positive charge, negative charge, neutral charge, zwitterions, polarizability, nuclease-resistance, conformational rigidity, conformational flexibility, protein-binding characteristics, mass etc. Aptamer Medicinal Chemistry schemes may include base-modifications, sugar-modifications or phosphodiester linkage-modifications.

When considering the kinds of substituents that are likely to be beneficial within the context of a therapeutic aptamer, it may be desirable to introduce substitutions that fall into one or more of the following categories:
(1) Substituents already present in the body, e.g., 2'-deoxy, 2'-ribo, 2'-O-methyl purines or pyrimidines or 5-methyl cytosine.
(2) Substituents already part of an approved therapeutic, e.g., phosphorothioate-linked oligonucleotides.
(3) Substituents that hydrolyze or degrade to one of the above two categories, e.g., methylphosphonate-linked oligonucleotides.

The PDGF aptamers of the invention include aptamers developed through aptamer medicinal chemistry as described herein.

### PDGF AND PDGF-VEGF SPECIFIC BINDING APTAMERS AS ONCOLOGY THERAPEUTICS

Aptamers specifically capable of binding and inhibiting different PDGF isoforms are set forth herein. These aptamers, which include aptamers that bind only to PDGF, aptamers that bind to both PDGF and VEGF, and either of the above aptamers having a CpG motif incorporated therein, provide a low-toxicity, safe, and effective modality of inhibiting most PDGF-mediated tumor progression, including without limitation, glioblastomas, chronic myelomonocytic leukemia (CMML), dermatofibrosarcoma protuberans (DFSP), gastrointestinal stromal tumors, (GIST), and other soft tissue sarcomas.

Examples of PDGF and PDGF-VEGF specific binding aptamers for use as oncology therapeutics include the following sequences:

### PDGF-binding aptamers:

**ARC126:** 5'-(5'-NH₂-dC-dA-dG-dG-dC-fU-dA-fC-mG- 3', SEQ ID NO. 1)-HEG-(5'-dC-dG-T-dA-mG-dA-mG-dC-dA-fU-fC-mA-3', SEQ ID NO.2)-HEG-(5'-T-dG-dA-T-fC-fC-fU-mG-3'dT-3', SEQ ID NO. 3)-3', wherein HEG = hexaethylene glycol amidite.

**ARC127:** 5'-[40K PEG]-(5'-NH₂-dC-dA-dG-dG-dC-fU-dA-fC-mG- 3', SEQ ID NO. 1)-HEG-(5'-dC-dG-T-dA-mG-dA-mG-dC-dA-fU-fC-mA-3', SEQ ID NO.2)-HEG-(5'-T-dG-dA-T-fC-fC-fU-mG-3'dT-3', SEQ ID NO. 3)-3', wherein HEG = hexaethylene glycol amidite.

**ARC240:** 5'-[20K PEG]-(5'-NH₂-dC-dA-dG-dG-dC-fU-dA-fC-mG- 3', SEQ ID NO. 1)-HEG-(5'-dC-dG-T-dA-mG-dA-mG-dC-dA-fU-fC-mA-3', SEQ ID NO.2)-HEG-(5'-T-dG-dA-T-fC-fC-fU-mG-3'dT-3', SEQ ID NO. 3)-3' , wherein HEG = hexaethylene glycol amidite.

**ARC308 :** 5'-[30K PEG]-(5'-NH₂-dC-dA-dG-dG-dC-fU-dA-fC-mG- 3', SEQ ID NO. 1)-HEG-(5'-dC-dG-T-dA-mG-dA-mG-dC-dA-fU-fC-mA-3', SEQ ID NO.2)-HEG-(5'-T-dG-dA-T-fC-fC-tU-mG-3'dT-3', SEQ ID NO. 3)-3' , wherein HEG = hexaethylene glycol amidite.

**deoxyARC126:** 5'-dCdAdGdGdCdTdAdCdGdCdGdTdAdGdAdGdCdAdTdCdAdTdGdAdTdCdCdTdG-[3T]-3' (SEQ ID NO:8), wherein "d" indicates unmodified deoxynucleotides and "[3T]" refers to an inverted thymidine nucleotide attached to the 3' end of the oligonucleotide.

ARC 124: 5' CACAGGCTACGGCACGTAGAGCATCACCATGATCCTGTG-3'InvdT (SEQ ID NO:11) where 3'InvdT refers to an inverted thymidine nucleotide attached to the 3' end of the oligonucleotide.

### Scrambled control aptamer:

**ARC128:** (Scrambled ARC126): 5'-(5'-NH₂-dC-dA-dG-fC-mG-fU-dA-fC-mG -3', SEQ ID NO. 4)-HEG-(5'- dC-dG-dT-dA-dC-dC-mG-dA-dT-fU-fC-mA-3', SEQ ID NO. 5)-HEG-(5'- dT-dG-dA-dA-dG-fC-fU-mG-3'dT-3', SEQ ID NO. 6)-3', wherein HEG = hexaethylene glycol amidite.

### VEGF-binding aptamer:

ARC245: **5**'-mAmUmGmCmAmGmUmUmUmGmAmGmAmAmGmUmCmGmCmGmCmAmU-[3T]-3' (SEQ ID NO: 7), wherein "m" indicates 2'-OMe nucleotides and "[3T]" refers to an inverted thymidine nucleotide that is attached to the 3'end of the oligonucleotide at the 3' position on the ribose sugar, thus the oligonucleotide has two 5'ends and is thus resistant to nucleases acting on the 3' hydroxyl end.

### PDGF/VEGF binding multivalent aptamers:

TK.131.012.A: (SEQ ID NO:9): 5'dCdAdGdGdCdTdAdCdGmAmUmGmCmAmGmUmUmUmGmAmGmAmAmGmUm CmGmCmGmCmAmUdCdGdTdAdGdAdGdCdAdTdCdAdGdAdAdAdTdGdAdTdCdCd TdG-[3T]-3', wherein "m" indicates 2'-OMe nucleotides, "d" and "[3T]"are as defined above.

TK.131.012.B: (SEQ ID NO: 10): 5'dCdAdGdGdCdTdAdCdGmUmGmCmAmGmUmUmUmGmAmGmAmAmGmUmCm GmCmGmCmAdCdGdTdAdGdAdGdCdAdTdCdAdGdAdAdAdTdGdAdTdCdCdTdG-[3T]-3', wherein "m" and "[3T]" are as defined above.

Other aptamers that bind PDGF and/or VEGF are described below, *e.g*., in Table 2 and in Example 12.

It has been demonstrated that inhibition of PDGF signaling with small molecule receptor antagonists decreases interstitial fluid pressure and increases the uptake of chemotherapeutics into solid tumors. Pietras *et al.* validated the hypothesis that PDGF-B is involved in IFP and that blocking PDGF-B function could lead to increased uptake of chemotherapeutics into tumors. (Pietras et al., (2003), Cancer Cell vol. 3 p.439-443). Using the KAT-4 thyroid carcinoma model, which has known PDGF paracrine signaling properties, Pietras *et al.* demonstrated that KAT-4 tumors expressed PDGF β-receptors in the stroma and that PDGF-B bound KAT-4 cells *in vitro.* Next, Pietras *et al*. used the tyrosine kinase inhibiting drug STI571 (GLEEVEC™) to block PDGF-B signaling in KAT-4 tumors and showed that this treatment significantly decreased tumor IFP *in vivo* leading to increased uptake of taxol. However, since STI571 targets both PDGF α and β receptors, as well as Kit, Abl, and Arg tyrosine kinases, it was impossible to know if the effect of ST1571 was due to PDGF-B blockage alone. This ambiguity was solved by using a highly specific aptamer to block PDGF-B in similar experiments. The aptamer has an affinity of 100 pM for PDGF-B and no appreciable affinity for the PDGF-A sequence. As with STI571, treatment of KAT-4 xenograft mice with PEG-conjugated PDGF-B aptamer lowered IFP and dramatically increased tumor uptake of taxol. Most importantly, aptamer treatment strongly enhanced taxol's ability to inhibit tumor growth. In addition, a currently marketed cancer therapeutic, the PDGF-receptor antagonist GLEEVEC™ has been shown to be effective at reducing tumor IFP and increasing the tumor uptake of cytotoxins when used in combination with a cytotoxin such as taxol. The methods and materials of the present are used to inhibit the biological activity of PDGF-B and its etiology in tumor development and growth by enhancing the uptake and efficacy of chemotherapeutics.

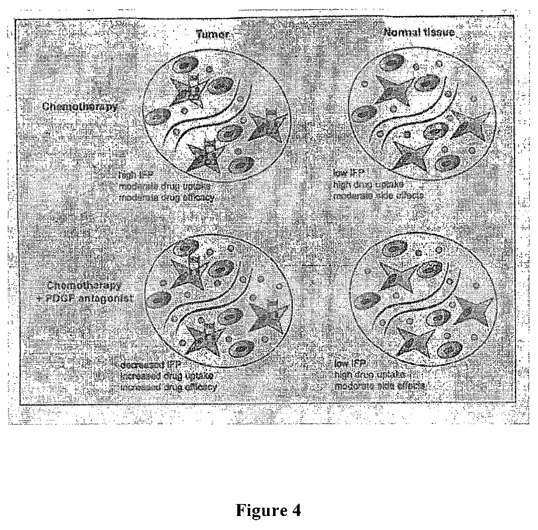

Combination therapy methods of the present invention include combining PDGF-specific aptamers of the present invention with cytotoxic agents. Without wishing to be bound by theory, combining PDGF-specific aptamers with other known cytotoxins, may provide an effective method of delivering drug specifically at the site of tumors, for example, by the selective lowering of IFP in tumor vessels. Such, in turn allows the increased uptake of cytotoxins in tumors through the tumor vasculature. Figure 4 shows a schematic of the transport to cytotoxins across tumor vasculature with and without PDGF antagonists by the methods of the present invention (Pietras et al.. (2003), Cancer Cell vol. 3 p.439-443).

The PDGF aptamers of the present invention can be used in combination with a variety of known cytotoxic or cytostatic (collectively, "cytotoxic") agents to lower tumor IFP, and thereby increase delivery and tumor uptake of cytotoxic agents to all solid tumors. Suitable cytotoxic or cytostatic agents include tubulin stabilizers/destabilizers, anti-metabolites, purine synthesis inhibitors, nucleoside analogs, and DNA alkylating or other DNA modifying agents, including but not limited to paclitaxel (TAXOL™), docetaxel, irinotecan, topotecan, gemcitabine, epothilone, cisplatinum, carboplatin, 5-fluoro-U, calicheamycin, doxorubicin, methotrexate, AraC (cytarabine), vinblastin, daunorubicin, oxaliplatinum, cyclophosphamide, iflosamide, pharmarubicin, epirubicin, vinflunine, oblimersen sodium, permetrexed, kinase inhibitors, including but not limited to EGF receptor kinase, VEGF receptor kinase, aurora kinase, either alone or in any combination thereof. The PDGF aptamers of the present invention can be used in combination with a variety of known vascular targeting agents wherein "vascular targeting agent" means a small molecule therapeutic *(e.g.,* irinotecan), a protein therapeutic *(e.g.,* bevicizumab), and/or an oligonucleotide therapeutic (*e.g*., antisense molecules, siRNA molecules) which modifies the existing vasculature and neovasculature network that supports blood and lymphatic flow to the tumor. The PDGF aptamers of the present invention can be used in combination with a conjugate therapeutic comprising a binding or targeting moiety, and a cytotoxic moiety, wherein the binding or targeting moiety is, but is not limited to, an aptamer, antibody, including, but not limited to trastuzumab, rituximab, cetuximab, panitumumab, gemtuzumab, bevicizumab, and tositumomab, peptide, a vascular targeting agent or folate compound, and wherein the cytotoxic moiety belongs to a class of compounds including but not limited to, tubulin stabilizers/destabilizers, anti-metabolites, purine synthesis inhibitors, nucleoside analogs, and DNA alkylating agents, other DNA modifying agents, or vascular disruptive agents (e.g., flavonoids) alone or in any combination thereof.

The materials and methods of the present invention provide a more effective way of administering known chemotherapeutic agents to inhibit solid tumor formation which results in a variety of cancers including but not limited to colorectal, pancreatic, breast, lung, prostate, and ovarian cancer.

In addition, PDGF aptamer compositions of the present invention can be used as anti-angiogenic agents to inhibit the formation of tumor vasculature by targeting pericytes. In one example, a PDGF aptamer composition of the present invention can be used in combination with a VEGF/VEGFR antagonist, such as an aptamer specific for VEGF. Such combinations may provide a more effective way of inhibiting tumor angiogenesis than either a PDGF aptamer therapeutic or VEGF/VEGFR antagonist therapeutic alone. One advantage of the PDGF-B- and VEGF-targeting agents of the present invention is that the therapeutic compositions of the present invention are exquisitely specific to their target ligand and hence do not appear to exhibit any off-target tyrosine kinase activity; and are not designed to enter cells to elicit their biological function.

Currently marketed small molecule kinase inhibitor therapeutics, for example GLEEVEC™, exhibit high off-target activity. GLEEVEC™ targets both the α and β tyrosine kinase receptors in addition to BCR-Abl, C-kit and Arg tyrosine kinases. Off-target activity is a major impediment to the development of small molecule kinase inhibitors in general. Additionally, small molecule kinase inhibitors, such as GLEEVEC™, are administered at or near their maximum tolerated dose when used in the treatment of solid tumors. The toxicity of GLEEVEC™ and other tyrosine kinase inhibitors (TKIs) is likely limited by both mechanism-related side-effects and non-mechanism (off-target) related side-effects. Based on *in vivo* experiments with ARC 127 (5'-[40K PEG]-(SEQ ID NO.1)-HEG-(SEQ ID NO:2)-HEG-(SEQ ID NO:3)-3'-dT-3') and ARC308 (5'-[30K PEG]-(SEQ ID NO.1)-HEG-(SEQ ID NO:2)-HEG-(SEQ ID NO:3)-3'-dT-3') dose-limiting side effects are not evident. Additional advantages are that the aptamer therapeutic compositions of the present invention can be administered via intravenous, subcutaneous, or intra-peritoneal routes. Unlike monoclonal antibody cancer therapeutics, which are immunogenic, aptamers are non-immunogenic, thus reaction to the drug and/or resistance to the drug are not an issue.

### APTAMERS TO PDGF AND PDGF ISOFORMS

The materials of the present invention comprise a series of nucleic acid aptamers of 31-35 nucleotides in length (SEQ ID NO:1 to SEQ ID NO:3, SEQ ID NO:9 to SEQ ID NO:35, SEQ ID NO:36 to SEQ ID NO:73, SEQ ID NO:85, and SEQ ID NO:77 to SEQ ID NO:81, and SEQ ID NO: 86-89, 91, 93-95 and 102) which bind specifically to PDGF-B protein *in vitro* and which functionally block the activity of PDGF-BB in *in vivo* and cell-based assays. The anti-PDGF-B aptamers sequences of the present invention are derived from a parent molecule ARC 126 (5'-(SEQ ID NO.1)-HEG-(SEQ ID NO:2)-HEG-(SEQ ID NO:3)-3'-dT-3') which contains seven individual 2'F containing residues (Figure 9A). 2'F containing residues were incorporated into ARC126 to increase the *in vitro* serum and *in vivo* stability of the therapeutic aptamer by blocking its degradation by serum endonucleases and/or exonucleases. In an effort to replace potentially toxic 2'F containing nucleotide residues in the ARC126 anti-PDGF-B aptamer, a new series of fully 2'F-free aptamers has been identified. The new aptamers of the present invention retain potent *in vitro* binding and anti-proliferative activity, and contain naturally occurring 2'deoxy or 2'OMe substituted nucleotides. These new aptamers of the present invention also retain substantial serum stability as determined through resistance to nuclease degradation in an *in vitro* stability assay, with no degradation detected for up to 48 hours.

The aptamer therapeutics of the present invention have great affinity and specificity to PDGF, PDGF isoforms, and/or PDGF receptor while reducing the deleterious side effects from non-naturally occurring nucleotide substitutions when the aptamer therapeutics break down in the body of patients or subjects. The therapeutic compositions containing the aptamer therapeutics of the present invention are free of or have a reduced amount of fluorinated nucleotides.

### MATERIALS AND METHODS TO INCREASE THE EFFICACY OF ANTI-TUMOR AGENTS

The materials and methods of the present invention further comprise methods to increase the efficacy of antitumor agents by dual therapy with the aptamers of the present invention, such as ARC 127 and ARC308. In addition, the experiments described herein have demonstrated that PDGF-B specific aptamers, ARC127 *(i.e.,* ARC126+40K PEG) and ARC308 (*i.e.,* ARC 126+30K PEG) are active anti-tumor agents when co-administered with irinotecan to nude mice bearing the colorectal LS174t tumor xenograft. The cancer therapeutic compounds of the present invention (*e.g*., both ARC 127 and ARC308) are safe non-cytotoxic agents when administered alone, but in combination with other cytotoxic agents, and that ARC 127 and ARC308 potentiate their anti-tumor effects through a novel mechanism of action. Furthermore, the serum-derived ARC 127 aptamer, when administered to mice parenterally, *i.e.,* intravenously, subcutaneously, or by intraperitoneal injection, retains full biological activity.

### APTAMER-CHIMERA SPECIFIC FOR PDGF-B AND VEGF

The invention further provides a bi-functional aptamer-chimera that targets both PDGF-B and VEGF. The PDGF-B-VEGF aptamer chimera TK.131.12.A (SEQ ID NO:9) and TK.131.12.B (SEQ ID NO: 10) of the present invention allow for the simultaneous targeting of PDGF-B and VEGF, *e.g*., for the treatment of cancer. The PDGF-B aptamer used in the chimeric molecule is derived from the ARC127 aptamer sequence. The VEGF aptamer that was used in the chimeric molecule is derived from the ARC245 (SEQ ID NO:7) aptamer sequence. The aptamer-chimera of the present invention can be constructed from any set of PDGF-B and VEGF binding aptamers. The PDGF-B-VEGF chimera of the present invention is useful in the treatment of VEGF-dependent solid tumors that also show a high degree of neovascularization as well as pericyte recruitment to support the nascent vasculature.

Recent anti-tumor data from the RipTag pancreatic mouse tumor model suggests that there is a greater inhibition in tumor growth conferred when anti-VEGF and anti-PDGFR therapy are undertaken simultaneously, than when either the anti-VEGF agent or the anti-PDGFR agent is added alone (Bergers et al., (2003), J. Clin. Invest., 111:9, p. 1287-1295). Since anti-PDGFR therapy blocks all receptor-mediated signaling events, the effects of this therapy can be expected to be non-specific and therefore suffer from side effects associated with non-specific treatments. In contrast, the PDGF-B-VEGF chimera in this invention provide for precise PDGF-B and VEGF targeting in tumors.

The aptamer therapeutics of the present invention have great affinity and specificity to VEGF and/or VEGF receptor, while reducing the deleterious side effects from non-naturally occurring nucleotide substitutions which may result when the aptamer therapeutics break down in the body of a patient or subject. The therapeutic compositions of the aptamer therapeutics of the present invention are free of or have a reduced amount of fluorinated nucleotides.

### APTAMERS HAVING IMMUNOSTIMULATORY MOTIFS

Recognition of bacterial DNA by the vertebrate immune system is based on the recognition of unmethylated CG dinucleotides in particular sequence contexts ("CpG motifs"). One receptor that recognizes such a motif is Toll-like receptor 9 ("TLR 9"), a member of a family of Toll-like receptors (∼10 members) that participate in the innate immune response by recognizing distinct microbial components. TLR 9 binds unmethylated oligodeoxynucleotide (ODN) CpG sequences in a sequence-specific manner. The recognition of CpG motifs triggers defense mechanisms leading to innate and ultimately acquired immune responses. For example, activation of TLR 9 in mice induces activation of antigen presenting cells, up regulation of MHC class I and II molecules and expression of important costimulatory molecules and cytokines including IL-12 and IL-23. This activation both directly and indirectly enhances B and T cell responses, including robust up regulation of the TH1 cytokine IFN-gamma. Collectively, the response to CpG sequences leads to: protection against infectious diseases, improved immune response to vaccines, an effective response against asthma, and improved antibody-dependent cell-mediated cytotoxicity. Thus, CpG ODN's can provide protection against infectious diseases, function as immuno-adjuvants or cancer therapeutics (monotherapy or in combination with mAb or other therapies), and can decrease asthma and allergic response.

Aptamers comprising one or more CpG motifs can be identified or generated by a variety of strategies using, *e.g*., the SELEX™ process described herein. In general the strategies can be divided into two groups. In the first group, the strategies are directed to identifying or generating aptamers comprising both a binding site for targets other than those recognizing CpG motifs and a CpG motif. These strategies are as follows: (a) performing SELEX™ to obtain an aptamer to a specific target (other than a target known to bind to CpG motifs and upon binding stimulate an immune response), preferably a target where a repressed immune response is relevant to disease development, using an oligonucleotide pool wherein a CpG motif has been incorporated into each member of the pool as, or as part of, a fixed region, *e.g*., in the randomized region of the pool members; (b) performing SELEX to obtain an aptamer to a specific target (other than a target known to bind to CpG motifs and upon binding stimulate an immune response), preferably a target where a repressed immune response is relevant to disease development, and then appending a CpG motif to the 5' and/or 3' end or engineering a CpG motif into a region, preferably a non-essential region, of the aptamer; (c) performing SELEX™ to obtain an aptamer to a specific target (other than a target known to bind to CpG motifs and upon binding stimulate an immune response), preferably a target where a repressed immune response is relevant to disease development, wherein during synthesis of the pool the molar ratio of the various nucleotides is biased in one or more nucleotide addition steps so that the randomized region of each member of the pool is enriched in CpG motifs; and (d) performing SELEX™ to obtain an aptamer to a specific target (other than a target known to bind to CpG motifs and upon binding stimulate an immune response), preferably a target where a repressed immune response is relevant to disease development, and identifying those aptamers comprising a CpG motif.

In the second group, the strategies are directed to identifying or generating aptamers comprising a CpG motif and/or other sequences that are bound by the receptors for the CpG motifs (*e.g*., TLR9 or the other toll-like receptors) and upon binding stimulate an immune response. These strategies are as follows: (i) performing SELEX™ to obtain an aptamer to a target known to bind to CpG motifs and upon binding stimulate an immune response using an oligonucleotide pool wherein a CpG motif has been incorporated into each member of the pool as, or as part of, a fixed region, *e.g*., in the randomized region of the pool members; (ii) performing SELEX to obtain an aptamer to a target known to bind to CpG motifs and upon binding stimulate an immune response and then appending a CpG motif to the 5' and/or 3' end or engineering a CpG motif into a region, preferably a non-essential region, of the aptamer; (iii) performing SELEX to obtain an aptamer to a target known to bind to CpG motifs and upon binding stimulate an immune response wherein during synthesis of the pool, the molar ratio of the various nucleotides is biased in one or more nucleotide addition steps so that the randomized region of each member of the pool is enriched in CpG motifs ; (iv) performing SELEX to obtain an aptamer to a target known to bind to CpG motifs and upon binding stimulate an immune response and identifying those aptamers comprising a CpG motif; and (v) performing SELEX to obtain an aptamer to a target known to bind to CpG motifs and identifying those aptamers which, upon binding, stimulate an immune response not comprising a CpG motif.

A variety of different classes of CpG motifs have been identified, each resulting upon recognition in a different cascade of events, release of cytokines and other molecules, and activation of certain cell types. *See, e.g.,* CpG Motifs in Bacterial DNA and Their Immune Effects, Annu. Rev. Immunol. 2002, 20:709-760, incorporated herein by reference. Additional immunostimulatory motifs are disclosed in the following U.S. Patents, each of which is incorporated herein by reference: 6,207,646; 6,239,116; 6,429,199; 6,214,806; 6,653,292; 6,426,434; 6,514,948 and 6,498,148. Any of these CpG or other immunostimulatory motifs can be incorporated into an aptamer. The choice of aptamers is dependent on the disease or disorder to be treated. Preferred immunostimulatory motifs are as follows (shown the 5' to 3' left to right) wherein "r" designates a purine, "y" designates a pyrimidine, and "X" designates any nucleotide: AACGTTCGAG (SEQ ID NO: 85); AACGTT (SEQ ID NO:90); ACGT (SEQ ID NO:92), rCGy (SEQ ID NO:96); rrCGyy (SEQ ID NO:98), XCGX (SEQ ID NO:99), XXCGXX (SEQ ID NO: 100), and X₁X₂CGY₁Y₂ (SEQ ID NO:101) wherein X₁ is G or A, X₂ is not C, Y₁ is not G and Y₂ is preferably T.

In those instances where a CpG motif is incorporated into an aptamer that binds to a specific target other than a target known to bind to CpG motifs and upon binding stimulate an immune response (a "non-CpG target"), the CpG is preferably located in a non-essential region of the aptamer. Non-essential regions of aptamers can be identified by site-directed mutagenesis, deletion analyses and/or substitution analyses. However, any location that does not significantly interfere with the ability of the aptamer to bind to the non-CpG target may be used. In addition to being embedded within the aptamer sequence, the CpG motif may be appended to either or both of the 5' and 3' ends or otherwise attached to the aptamer. Any location or means of attachment may be used so long as the ability of the aptamer to bind to the non-CpG target is not significantly interfered with.

As used herein, "stimulation of an immune response" can mean either (1) the induction of a specific response (*e.g*., induction of a Th1 response) or of the production of certain molecules or (2) the inhibition or suppression of a specific response (*e.g*., inhibition or suppression of the Th2 response) or of certain molecules.

CpG motifs can be incorporated or appended to an aptamer against any target including, but not limited to: PDGF, IgE, IgE Fcε R1, TNFa, PSMA, CTLA4, PD-1, PD-L1, PD-L2, FcRIIB, BTLA, TIM-3, CD11b, CD-11c, BAFF, B7-X, CD19, CD20, CD25 and/or CD33.

By incorporating CpG motifs into aptamers specifically targeting solid tumors these aptamers can be used to activate the immune system through the recruitment of antigen presenting cells that have taken up tumor derived material, enhance their maturation and migration to local lymph nodes and increase priming of tumor specific T-cells. This is especially relevant where aptamers deliver cytotoxic payload and result in cell death (such as a PSMA aptamer containing a CpG motif). Such CpG motif containing aptamers can also induce tumor-specific memory response (*e.g*., a prophylactic use). In addition, the IFP lowering and pericyte recruitment blocking effects of a PDGF-B aptamer combined with the increased immune response observed upon CpG administration represents a potent therapeutic for cancer. Thus, aptamers with incorporated, appended or embedded CpG motifs represent a novel class of anti-cancer compounds. When administered these compounds can lead to a significant de-bulking of the tumor through two mechanisms: first, through activation of tumor specific T-cells within the tumor bed and second, through the intended mechanism-based action of the aptamer pharmacophore.

### PHARMACEUTICAL COMPOSITIONS

The invention also includes pharmaceutical compositions containing aptamer molecules. In some embodiments, the compositions are suitable for internal use and include an effective amount of a pharmacologically active compound of the invention, alone or in combination, with one or more pharmaceutically acceptable carriers. The compounds are especially useful in that they have very low, if any toxicity.

Compositions of the invention can be used to treat or prevent a pathology, such as a disease or disorder, or alleviate the symptoms of such disease or disorder in a patient. Compositions of the invention are useful for administration to a subject suffering from, or predisposed to, a disease or disorder which is related to or derived from a target to which the aptamers of the invention specifically bind. For example, the target is a protein involved with a pathology, for example, the target protein causes the pathology. For example, the target is PDGF involvement in the development and progression of solid tumors.

Compositions of the invention can be used in a method for treating a patient or subject having a pathology. The method involves administering to the patient or subject a composition comprising aptamers that bind a target (*e.g.,* a protein) involved with the pathology, so that binding of the composition to the target *al*ters the biological function of the target, thereby treating the pathology.

The patient or subject having a pathology, *e.g*. the patient or subject treated by the methods of this invention can be a mammal, or more particularly, a human.

In practice, the aptamers or their pharmaceutically acceptable salts, are administered in amounts which will be sufficient to exert their desired biological activity, *e.g*., inhibiting the binding of a cytokine to its receptor.

One aspect of the invention comprises an aptamer composition of the invention in combination with other treatments for cancer or cancer related disorders. The aptamer composition of the invention may contain, for example, more than one aptamer. In some examples, an aptamer composition of the invention, containing one or more compounds of the invention, is administered in combination with another useful composition such as an anti-inflammatory agent, an immunosuppressant, an antiviral agent, or the like. Furthermore, the compounds of the invention may be administered in combination with a cytotoxic, cytostatic, or chemotherapeutic agent such as an alkylating agent, antimetabolite, mitotic inhibitor or cytotoxic antibiotic, as described above. In general, the currently available dosage forms of the known therapeutic agents for use in such combinations will be suitable.

"Combination therapy" (or "co-therapy") includes the administration of an aptamer composition of the invention and at least a second agent as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected).

"Combination therapy" may, but generally is not, intended to encompass the administration of two or more of these therapeutic agents as part of separate monotherapy regimens that incidentally and arbitrarily result in the combinations of the present invention. "Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents.

Sequential or substantially simultaneous administration of each therapeutic agent can be effected by any appropriate route including, but not limited to, topical routes, oral routes, intravenous routes, intramuscular routes, and direct absorption through mucous membrane tissues. The therapeutic agents can be administered by the same route or by different routes. For example, a first therapeutic agent of the combination selected may be administered by injection while the other therapeutic agents of the combination may be administered topically.

Alternatively, for example, all therapeutic agents may be administered topically or all therapeutic agents may be administered by injection. The sequence in which the therapeutic agents are administered is not narrowly critical. "Combination therapy" also can embrace the administration of the therapeutic agents as described above in further combination with other biologically active ingredients. Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

The compounds of the invention and the other pharmacologically active agent may be administered to a patient simultaneously, sequentially or in combination. It will be appreciated that when using a combination of the invention, the compound of the invention and the other pharmacologically active agent may be in the same pharmaceutically acceptable carrier and therefore administered simultaneously. They may be in separate pharmaceutical carriers such as conventional oral dosage forms which are taken simultaneously. The term "combination" further refers to the case where the compounds are provided in separate dosage forms and are administered sequentially.

Therapeutic or pharmacological compositions of the present invention will generally comprise an effective amount of the active component(s) of the therapy, dissolved or dispersed in a pharmaceutically acceptable medium. Pharmaceutically acceptable media or carriers include any and all solvents, dispersion media, coatings, antibacterial and antifungal agents, isotonic and absorption delaying agents and the like. The use of such media and agents for pharmaceutical active substances is well known in the art. Supplementary active ingredients can also be incorporated into the therapeutic compositions of the present invention.

The preparation of pharmaceutical or pharmacological compositions will be known to those of skill in the art in light of the present disclosure. Typically, such compositions may be prepared as injectables, either as liquid solutions or suspensions; solid forms suitable for solution in, or suspension in, liquid prior to injection; as tablets or other solids for oral administration; as time release capsules; or in any other form currently used, including eye drops, creams, lotions, salves, inhalants and the like. The use of sterile formulations, such as saline-based washes, by surgeons, physicians or health care workers to treat a particular area in the operating field may also be particularly useful. Compositions may also be delivered via microdevice, microparticle or sponge.

Upon formulation, therapeutics will be administered in a manner compatible with the dosage formulation, and in such amount as is pharmacologically effective. The formulations are easily administered in a variety of dosage forms. In a preferred embodiment the aptamer of the invention is formulated as an injectable solution described above, but drug release capsules and the like can also be employed.

In this context, the quantity of active ingredient and volume of composition to be administered depends on the host animal to be treated. Precise amounts of active compound required for administration depend on the judgment of the practitioner and are peculiar to each individual.

A minimal volume of a composition required to disperse the active compounds is typically utilized. Suitable regimes for administration are also variable, but would be typified by initially administering the compound and monitoring the results and then giving further controlled doses at further intervals.

For instance, for oral administration in the form of a tablet or capsule (*e.g.,* a gelatin capsule), the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, magnesium aluminum silicate, starch paste, gelatin, methylcellulose, sodium carboxymethylcellulose and/or polyvinylpyrrolidone, natural sugars such as glucose or beta-lactose, corn sweeteners, natural and synthetic gums such as acacia, tragacanth or sodium alginate, polyethylene glycol, waxes and the like. Lubricants used in these dosage forms include sodium oleate, sodium stearate, magnesium stearate, sodium benzoate, sodium acetate, sodium chloride, silica, talcum, stearic acid, its magnesium or calcium salt and/or polyethyleneglycol and the like. Disintegrators include, without limitation, starch, methyl cellulose, agar, bentonite, xanthan gum starches, agar, alginic acid or its sodium salt, or effervescent mixtures, and the like. Diluents, include, *e.g*., lactose, dextrose, sucrose, mannitol, sorbitol, cellulose and/or glycine.

Injectable compositions are preferably aqueous isotonic solutions or suspensions, and suppositories are advantageously prepared from fatty emulsions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. The compositions are prepared according to conventional mixing, granulating or coating methods, respectively, and contain about 0.1 1 to 75%, preferably about 1 to 50%, of the active ingredient.

The compounds of the invention can also be administered in such oral dosage forms as timed release and sustained release tablets or capsules, pills, powders, granules, elixirs, tinctures, suspensions, syrups and emulsions.

Liquid, particularly injectable compositions can, for example, be prepared by dissolving, dispersing, etc. The active compound is dissolved in or mixed with a pharmaceutically pure solvent such as, for example, water, saline, aqueous dextrose, glycerol, ethanol, and the like, to thereby form the injectable solution or suspension. Additionally, solid forms suitable for dissolving in liquid prior to injection can be formulated. Injectable compositions are preferably aqueous isotonic solutions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances.

The compounds of the present invention can be administered in intravenous (both bolus and infusion), intraperitoneal, subcutaneous or intramuscular form, all using forms well known to those of ordinary skill in the pharmaceutical arts. Injectables can be prepared in conventional forms, either as liquid solutions or suspensions.

Parenteral injectable administration is generally used for subcutaneous, intramuscular or intravenous injections and infusions. Additionally, one approach for parenteral administration employs the implantation of a slow-release or sustained-released systems, which assures that a constant level of dosage is maintained, according to U.S. Pat. No. 3,710,795, incorporated herein by reference.

Furthermore, preferred compounds for the present invention can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen. Other preferred topical preparations include creams, ointments, lotions, aerosol sprays and gels, wherein the concentration of active ingredient would range from 0.01% to 15%, w/w or w/v.

For solid compositions, excipients include pharmaceutical grades of mannitol, lactose, starch, magnesium stearate, sodium saccharin, talcum, cellulose, glucose, sucrose, magnesium carbonate, and the like may be used. The active compound defined above, may be also formulated as suppositories using for example, polyalkylene glycols, for example, propylene glycol, as the carrier. In some embodiments, suppositories are advantageously prepared from fatty emulsions or suspensions.

The compounds of the present invention can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in U.S. Pat. No. 5,262,564. For example, the aptamer molecules described herein can be provided as a complex with a lipophilic compound or non-immunogenic, high molecular weight compound constructed using methods known in the art. An example of nucleic-acid associated complexes is provided in U.S. Patent No. 6,011,020.

The compounds of the present invention may also be coupled with soluble polymers as targetable drug carriers. Such polymers can include polyvinylpyrrolidone, pyran copolymer, polyhydroxypropyl-methacrylamide-phenol, polyhydroxyethylaspanamidephenol, or polyethyleneoxidepolylysine substituted with palmitoyl residues. Furthermore, the compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.

If desired, the pharmaceutical composition to be administered may also contain minor amounts of non-toxic auxiliary substances such as wetting or emulsifying agents, pH buffering agents, and other substances such as for example, sodium acetate, and triethanolamine oleate.

The dosage regimen utilizing the aptamers is selected in accordance with a variety of factors including type, species, age, weight, sex and medical condition of the patient; the severity of the condition to be treated; the route of administration; the renal and hepatic function of the patient; and the particular aptamer or salt thereof employed. An ordinarily skilled physician or veterinarian can readily determine and prescribe the effective amount of the drug required to prevent, counter or arrest the progress of the condition.

Oral dosages of the present invention, when used for the indicated effects, will range between about 0.05 to 7500 mg/day orally. The compositions are preferably provided in the form of scored tablets containing 0.5, 1.0, 2.5, 5.0, 10.0, 15.0, 25.0, 50.0, 100.0, 250.0, 500.0 and 1000.0 mg of active ingredient. Infused dosages, intranasal dosages and transdermal dosages will range between 0.05 to 7500 mg/day. Subcutaneous, intravenous and intraperitoneal dosages will range between 0.05 to 3800 mg/day.

Compounds of the present invention may be administered in a single daily dose, or the total daily dosage may be administered in divided doses of two, three or four times daily.

Effective plasma levels of the compounds of the present invention range from 0.002 mg/mL to 50 mg/mL. In the dosages of the present invention, mass refers only to the molecular weight of the oligonucleotide portion of the aptamer, irrespective of the mass conferred by PEG conjugation.

### MODULATION OF PHARMACOKINETICS AND BIODISTRIBUTION OF APTAMER THERAPEUTICS

It is important that the pharmacokinetic properties for all oligonucleotide-based therapeutics, including aptamers, be tailored to match the desired pharmaceutical application. While aptamers directed against extracellular targets do not suffer from difficulties associated with intracellular delivery (as is the case with antisense and RNAi-based therapeutics), such aptamers must still be able to be distributed to target organs and tissues, and remain in the body (unmodified) for a period of time consistent with the desired dosing regimen.

Thus, the present invention provides materials and methods to affect the pharmacokinetics of aptamer compositions, and, in particular, the ability to tune aptamer pharmacokinetics. The tunability of *(i.e.,* the ability to modulate) aptamer pharmacokinetics is achieved through conjugation of modifying moieties (*e.g*., PEG polymers) to the aptamer and/or the incorporation of modified nucleotides (*e.g*., 2'-fluoro or 2'-O-methyl) to alter the chemical composition of the nucleic acid. The ability to tune aptamer pharmacokinetics is used in the improvement of existing therapeutic applications, or alternatively, in the development of new therapeutic applications. For example, in some therapeutic applications, *e.g*., in anti-neoplastic or acute care settings where rapid drug clearance or turn-off may be desired, it is desirable to decrease the residence times of aptamers in the circulation. Alternatively, in other therapeutic applications, *e.g*., maintenance therapies where systemic circulation of a therapeutic is desired, it may be desirable to increase the residence times of aptamers in circulation.

In addition, the tunability of aptamer pharmacokinetics is used to modify the biodistribution of an aptamer therapeutic in a subject. For example, in some therapeutic applications, it may be desirable to alter the biodistribution of an aptamer therapeutic in an effort to target a particular type of tissue or a specific organ (or set of organs). In these applications, the aptamer therapeutic preferentially accumulates in a specific tissue or organ(s). In other therapeutic applications, it may be desirable to target tissues displaying a cellular marker or a symptom associated with a given disease, cellular injury or other abnormal pathology, such that the aptamer therapeutic preferentially accumulates in the affected tissue. For example, as described in provisional application United States Serial No. 60/550790, filed on March 5, 2004, and entitled "Controlled Modulation of the Pharmacokinetics and Biodistribution of Aptamer Therapeutics), PEGylation of an aptamer therapeutic (*e.g*., PEGylation with a 20 kDa PEG polymer) is used to target inflamed tissues, such that the PEGylated aptamer therapeutic preferentially accumulates in inflamed tissue.

To determine the pharniacokinetic and biodistribution profiles of aptamer therapeutics (*e.g*., aptamer conjugates or aptamers having altered chemistries, such as modified nucleotides) a variety of parameters are Such parameters include, for example, the half-life (t_{1/2}), the plasma clearance (CL), the volume of distribution (Vss), the area under the concentration-time curve (AUC), maximum observed serum or plasma concentration (Cₘₐₓ), and the mean residence time (MRT) of an aptamer composition. As used herein, the term "AUC" refers to the area under the plot of the plasma concentration of an aptamer therapeutic versus the time after aptamer administration. The AUC value is used to estimate the bioavailability (*i.e.,* the percentage of administered aptamer therapeutic in the circulation after aptamer administration) and/or total clearance (CL) *(i.e.,* the rate at which the aptamer therapeutic is removed from circulation) of a given aptamer therapeutic. The volume of distribution relates the plasma concentration of an aptamer therapeutic to the amount of aptamer present in the body. The larger the Vss, the more an aptamer is found outside of the plasma (*i.e.,* the more extravasation).

The present invention provides materials and methods to modulate, in a controlled manner, the pharmacokinetics and biodistribution of stabilized aptamer compositions *in vivo* by conjugating an aptamer to a modulating moiety such as a small molecule, peptide, or polymer terminal group, or by incorporating modified nucleotides into an aptamer. As described herein, conjugation of a modifying moiety and/or altering nucleotide(s) chemical composition alter fundamental aspects of aptamer residence time in circulation and distribution to tissues.

In addition to clearance by nucleases, oligonucleotide therapeutics are subject to elimination *via* renal filtration. As such, a nuclease-resistant oligonucleotide administered intravenously typically exhibits an *in vivo* half-life of <10 min, unless filtration can be blocked. This can be accomplished by either facilitating rapid distribution out of the blood stream into tissues or by increasing the apparent molecular weight of the oligonucleotide above the effective size cut-off for the glomerulus. Conjugation of small therapeutics to a PEG polymer (PEGylation), described below, can dramatically lengthen residence times of aptamers in circulation, thereby decreasing dosing frequency and enhancing effectiveness against vascular targets.

Aptamers can be conjugated to a variety of modifying moieties, such as high molecular weight polymers, *e.g.,* PEG; peptides, *e.g*., Tat (a 13-amino acid fragment of the HIV Tat protein (Vives, et al., (1997), J. Biol. Chem. 272(25): 16010-7)), Ant (a 16-amino acid sequence derived from the third helix of the Drosophila antennapedia homeotic protein (Pietersz, et al., (2001), Vaccine 19(11-12): 1397-405)) and Arg₇ (a short, positively charged cell-permeating peptides composed of polyarginine (Arg₇) (Rothbard, et al., (2000), Nat. Med. 6(11): 1253-7; Rothbard, J et al., (2002), J. Med. Chem. 45(17): 3612-8)); and small molecules, *e.g*., lipophilic compounds such as cholesterol. Among the various conjugates described herein, *in vivo* properties of aptamers are altered most profoundly by complexation with PEG groups. For example, complexation of a mixed 2'F and 2'-OMe modified aptamer therapeutic with a 20 kDa PEG polymer hinders renal filtration and promotes aptamer distribution to both healthy and inflamed tissues. Furthermore, the 20 kDa PEG polymer-aptamer conjugate proves nearly as effective as a 40 kDa PEG polymer in preventing renal filtration of aptamers. While one effect of PEGylation is on aptamer clearance, the prolonged systemic exposure afforded by presence of the 20 kDa moiety also facilitates distribution of aptamer to tissues, particularly those of highly perfused organs and those at the site of inflammation. The aptamer-20 kDa PEG polymer conjugate directs aptamer distribution to the site of inflammation, such that the PEGylated aptamer preferentially accumulates in inflamed tissue. In some instances, the 20 kDa PEGylated aptamer conjugate is able to access the interior of cells, such as, for example, kidney cells. Modified nucleotides can also be used to modulate the plasma clearance of aptamers. For example, an unconjugated aptamer which incorporates both 2'-F and 2'-OMe stabilizing chemistries, which is typical of current generation aptamers as it exhibits a high degree of nuclease stability *in vitro* and *in vivo,* displays rapid loss from plasma *(i.e.,* rapid plasma clearance) and a rapid distribution into tissues, primarily into the kidney, when compared to unmodified aptamer.

### PEG-DERIVATIZED NUCLEIC ACIDS

As described above, derivatization of nucleic acids with high molecular weight non-immunogenic polymers has the potential to alter the pharmacokinetic and pharmacodynamic properties of nucleic acids making them more effective therapeutic agents. Favorable changes in activity can include increased resistance to degradation by nucleases, decreased filtration through the kidneys, decreased exposure to the immune system, and altered distribution of the therapeutic through the body.

The aptamer compositions of the invention may be derivatized with polyalkylene glycol (PAG) moieties. Examples of PAG-derivatized nucleic acids are found in United States Patent Application Ser. No. 10/718,833, filed on November 21, 2003, which is herein incorporated by reference in its entirety. Typical polymers used in the invention include poly(ethylene glycol) (PEG), also known as poly(ethylene oxide) (PEO) and polypropylene glycol (including poly isopropylene glycol). Additionally, random or block copolymers of different alkylene oxides (*e.g*., ethylene oxide and propylene oxide) can be used in many applications. In its most common form, a polyalkylene glycol, such as PEG, is a linear polymer terminated at each end with hydroxyl groups: HO-CH₂CH₂O-(CH₂CH₂O)ₙ-CH₂CH₂-OH. This polymer, alpha-, omega-dihydroxylpoly (ethylene glycol), can also be represented as HO-PEG-OH, where it is understood that the -PEG- symbol represents the following structural unit: - CH₂CH₂O-(CH₂CH₂O)ₙ-CH₂CH₂- where n typically ranges from about 4 to about 10,000.

As shown, the PEG molecule is di-functional and is sometimes referred to as "PEG diol." The terminal portions of the PEG molecule are relatively non-reactive hydroxyl moieties, the -OH groups, that can be activated, or converted to functional moieties, for attachment of the PEG to other compounds at reactive sites on the compound. Such activated PEG diols are referred to herein as bi-activated PEGs. For example, the terminal moieties of PEG diol have been functionalized as active carbonate ester for selective reaction with amino moieties by substitution of the relatively nonreactive hydroxyl moieties, -OH, with succinimidyl active ester moieties from N-hydroxy succinimide.

In many applications, it is desirable to cap the PEG molecule on one end with an essentially non-reactive moiety so that the PEG molecule is mono-functional (or mono-activated). In the case of protein therapeutics which generally display multiple reaction sites for activated PEGs, bi-functional activated PEGs lead to extensive cross-linking, yielding poorly functional aggregates. To generate mono-activated PEGs, one hydroxyl moiety on the terminus of the PEG diol molecule typically is substituted with non-reactive methoxy end moiety, -OCH₃. The other, un-capped terminus of the PEG molecule typically is converted to a reactive end moiety that can be activated for attachment at a reactive site on a surface or a molecule such as a protein.

PAGs are polymers which typically have the properties of solubility in water and in many organic solvents, lack of toxicity, and lack of immunogenicity. One use of PAGs is to covalently attach the polymer to insoluble molecules to make the resulting PAG-molecule "conjugate" soluble. For example, it has been shown that the water-insoluble drug paclitaxel, when coupled to PEG, becomes water-soluble. Greenwald, et al., J. Org. Chem., 60:331-336 (1995). PAG conjugates are often used not only to enhance solubility and stability but also to prolong the blood circulation half-life of molecules.

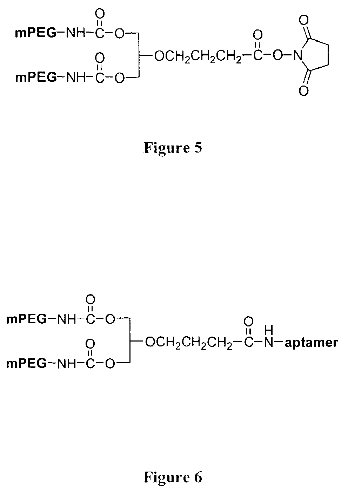

Polyalkylated compounds of the invention are typically between 5 and 80 kDa in size however any size can be used, the choice dependent on the aptamer and application. Other PAG compounds of the invention are between 10 and 80 kDa in size. Still other PAG compounds of the invention are between 10 and 60 kDa in size. For example, a PAG polymer may be at least 10, 20, 30, 40, 50, 60, or 80 kDa in size. Such polymers can be linear or branched. In some embodiments the polymers are PEG. In some embodiment the polymers are branched PEG. In still other embodiments the polymers are 40kDa branched PEG as depicted in Figure 5. In some embodiments the 40 kDa branched PEG is attached to the 5' end of the aptamer as depicted in Figure 6.

In contrast to biologically-expressed protein therapeutics, nucleic acid therapeutics are typically chemically synthesized from activated monomer nucleotides. PEG-nucleic acid conjugates may be prepared by incorporating the PEG using the same iterative monomer synthesis. For example, PEGs activated by conversion to a phosphoramidite form can be incorporated into solid-phase oligonucleotide synthesis. Alternatively, oligonucleotide synthesis can be completed with site-specific incorporation of a reactive PEG attachment site. Most commonly this has been accomplished by addition of a free primary amine at the 5'-terminus (incorporated using a modifier phosphoramidite in the last coupling step of solid phase synthesis). Using this approach, a reactive PEG (*e.g.*, one which is activated so that it will react and form a bond with an amine) is combined with the purified oligonucleotide and the coupling reaction is carried out in solution.

The ability of PEG conjugation to alter the biodistribution of a therapeutic is related to a number of factors including the apparent size (*e.g.,* as measured in terms of hydrodynamic radius) of the conjugate. Larger conjugates (>10kDa) are known to more effectively block filtration via the kidney and to consequently increase the serum half-life of small macromolecules (*e.g.*, peptides, antisense oligonucleotides). The ability of PEG conjugates to block filtration has been shown to increase with PEG size up to approximately 50 kDa (further increases have minimal benefcial effect as half life becomes defined by macrophage-mediated metabolism rather than elimination via the kidneys).

Production of high molecular weight PEGs (>10 kDa) can be difficult, inefficient, and expensive. As a route towards the synthesis of high molecular weight PEG-nucleic acid conjugates, previous work has been focused towards the generation of higher molecular weight activated PEGs. One method for generating such molecules involves the formation of a branched activated PEG in which two or more PEGs are attached to a central core carrying the activated group. The terminal portions of these higher molecular weight PEG molecules, *i.e.,* the relatively non-reactive hydroxyl (-OH) moieties, can be activated, or converted to functional moieties, for attachment of one or more of the PEGs to other compounds at reactive sites on the compound. Branched activated PEGs will have more than two termini, and in cases where two or more termini have been activated, such activated higher molecular weight PEG molecules are referred to herein as, multi-activated PEGs. In some cases, not all termini in a branch PEG molecule are activated. In cases where any two termini of a branch PEG molecule are activated, such PEG molecules are referred to as bi-activated PEGs. In some cases where only one terminus in a branch PEG molecule is activated, such PEG molecules are referred to as mono-activated. As an example of this approach, activated PEG prepared by the attachment of two monomethoxy PEGs to a lysine core which is subsequently activated for reaction has been described (Harris el al., Nature, vol.2: 214-221, 2003).

The present invention provides another cost effective route to the synthesis of high molecular weight PEG-nucleic acid (preferably, aptamer) conjugates including multiply PEGylated nucleic acids. The present invention also encompasses PEG-linked multimeric oligonucleotides, *e.g.*, dimerized aptamers. The present invention also relates to high molecular weight compositions where a PEG stabilizing moiety is a linker which separates different portions of an aptamer, *e.g.*, the PEG is conjugated within a single aptamer sequence, such that the linear arrangement of the high molecular weight aptamer composition is, *e.g.*, nucleic acid nucleic acid - PEG - nucleic acid (- PEG - nucleic acid)ₙ where n is greater than or equal to 1.

High molecular weight compositions of the invention include those having a molecular weight of at least 10 kDa. Compositions typically have a molecular weight between 10 and 80 kDa in size. High molecular weight compositions of the invention are at least 10, 20, 30, 40, 50, 60, or 80 kDa in size.

A stabilizing moiety is a molecule, or portion of a molecule, which improves pharmacokinetic and pharmacodynamic properties of the high molecular weight aptamer compositions of the invention. In some cases, a stabilizing moiety is a molecule or portion of a molecule which brings two or more aptamers, or aptamer domains, into proximity, or provides decreased overall rotational freedom of the high molecular weight aptamer compositions of the invention. A stabilizing moiety can be a polyalkylene glycol, such a polyethylene glycol, which can be linear or branched, a homopolymer or a heteropolymer. Other stabilizing moieties include polymers such as peptide nucleic acids (PNA). Oligonucleotides can also be stabilizing moieties; such oligonucleotides can include modified nucleotides, and/or modified linkages, such as phosphorothioates. A stabilizing moiety can be an integral part of an aptamer composition, *i.e.,* it is covalently bonded to the aptamer.

Compositions of the invention include high molecular weight aptamer compositions in which two or more nucleic acid moieties are covalently conjugated to at least one polyalkylene glycol moiety. The polyalkylene glycol moieties serve as stabilizing moieties. In compositions where a polyalkylene glycol moiety is covalently bound at either end to an aptamer, such that the polyalkylene glycol joins the nucleic acid moieties together in one molecule, the polyalkylene glycol is said to be a linking moiety. In such compositions, the primary structure of the covalent molecule includes the linear arrangement nucleic acid-PAG-nucleic acid. One example is a composition having the primary structure nucleic acid-PEG-nucleic acid. Another example is a linear arrangement of: nucleic acid - PEG - nucleic acid - PEG - nucleic acid.

To produce the nucleic acid-PEG-nucleic acid conjugate, the nucleic acid is originally synthesized such that it bears a single reactive site (*e.g.,* it is mono-activated). In a preferred embodiment, this reactive site is an amino group introduced at the 5'-terminus by addition of a modifier phosphoramidite as the last step in solid phase synthesis of the oligonucleotide. Following deprotection and purification of the modified oligonucleotide, it is reconstituted at high concentration in a solution that minimizes spontaneous hydrolysis of the activated PEG. In a preferred embodiment, the concentration of oligonucleotide is 1 mM and the reconstituted solution contains 200 mM NaHCO₃-buffer, pH 8.3. Synthesis of the conjugate is initiated by slow, step-wise addition of highly purified bi-functional PEG. In a preferred embodiment, the PEG diol is activated at both ends (bi-activated) by derivatization with succinimidyl propionate. Following reaction, the PEG-nucleic acid conjugate is purified by gel electrophoresis or liquid chromatography to separate fully-, partially-, and un-conjugated species. Multiple PAG molecules concatenated (*e.g.*, as random or block copolymers) or smaller PAG chains can be linked to achieve various lengths (or molecular weights). Non-PAG linkers can be used between PAG chains of varying lengths.

The 2'-O-methyl, 2'-fluoro and other modified nucleotide modifications stabilize the aptamer against nucleases and increase its half life *in vivo.* The 3'-3'-dT cap also increases exonuclease resistance. *See, e.g.,* U.S. Patents 5,674,685; 5,668,264; 6,207,816; and 6,229,002, each of which is incorporated by reference herein in its entirety.

### PAG-DERIVATIZATION OF A REACTIVE NUCLEIC ACID

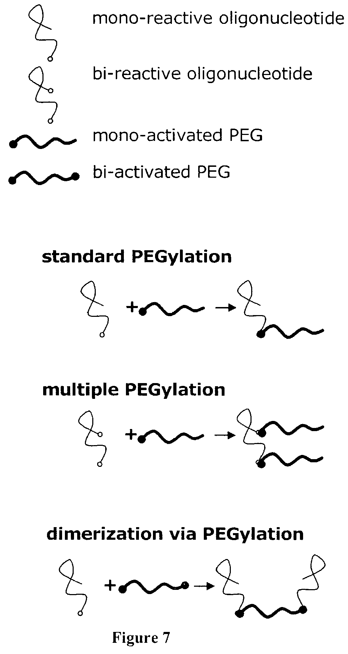

High molecular weight PAG-nucleic acid-PAG conjugates can be prepared by reaction of a mono-functional activated PEG with a nucleic acid containing more than one reactive site. In one embodiment, the nucleic acid is bi-reactive, or bi-activated, and contains two reactive sites: a 5'-amino group and a 3'-amino group introduced into the oligonucleotide through conventional phosphoramidite synthesis, for example: 3'-5'-di-PEGylation as illustrated in Figure 7. In alternative embodiments, reactive sites can be introduced at internal positions, using for example, the 5-position of pyrimidines, the 8-position of purines, or the 2'-position of ribose as sites for attachment of primary amines. In such embodiments, the nucleic acid can have several activated or reactive sites and is said to be multiply activated. Following synthesis and purification, the modified oligonucleotide is combined with the mono-activated PEG under conditions that promote selective reaction with the oligonucleotide reactive sites while minimizing spontaneous hydrolysis. In the preferred embodiment, monomethoxy-PEG is activated with succinimidyl propionate and the coupled reaction is carried out at pH 8.3. To drive synthesis of the bi-substituted PEG, stoichiometric excess PEG is provided relative to the oligonucleotide. Following reaction, the PEG-nucleic acid conjugate is purified by gel electrophoresis or liquid chromatography to separate fully-, partially-, and un-conjugated species.

The linking domains can also have one or more polyalkylene glycol moieties attached thereto. Such PAGs can be of varying lengths and may be used in appropriate combinations to achieve the desired molecular weight of the composition.

The effect of a particular linker can be influenced by both its chemical composition and length. A linker that is too long, too short, or forms unfavorable steric and/or ionic interactions with the target will preclude the formation of complex between aptamer and target. A linker, which is longer than necessary to span the distance between nucleic acids, may reduce binding stability by diminishing the effective concentration of the ligand. Thus, it is often necessary to optimize linker compositions and lengths in order to maximize the affinity of an aptamer to a target.

All publications and patent documents cited herein are incorporated herein by reference as if each such publication or document was specifically and individually indicated to be incorporated herein by reference. Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.

### EXAMPLES

### EXAMPLE 1: Large Scale Aptamer Synthesis and Conjugation

ARC 126 (5'-(SEQ ID NO:1)-HEG-(SEQ ID NO:2)-HEG-(SEQ ID NO:3)-3'-dT-3') is a 29 nucleotide aptamer (excluding an inverted T at the 3' end) specific for PDGF which contains a 3' inverted-dT cap for enhanced stability against nuclease attack. PEG moieties can be conjugated to ARC 126 by using a 5'-amino terminus modifier for subsequent conjugation reactions. Syntheses were performed using standard solid-phase phosphoramidite chemistry. The oligonucleotide was deprotected with ammonium hydroxide/methylamine (1:1) at room temperature for 12 hours and purified by ion exchange HPLC. ARC 128 (5'-(SEQ ID NO.4)-HEG-(SEQ ID NO:5)-HEG-(SEQ ID NO:6)-3'), an inactive variant which no longer binds PDGF, was synthesized using the same method.

ARC126 was conjugated to several different PEG moieties: 20 kDa PEG (ARC240, (5'-[20K PEG]-(SEQ ID NO:1)-HEG-(SEQ ID NO:2)-HEG-(SEQ ID NO:3)-3'-dT-3')); 30 kDa PEG (ARC308, (5'-[30K PEG]-(SEQ ID NO:1)-HEG-(SEQ ID NO:2)-HEG-(SEQ ID NO:3)-3' -dT-3')); 40 kDa PEG (ARC 127, (5'-[40K PEG]-(SEQ ID NO: 1)-HEG-(SEQ ID NO:2)-HEG-(SEQ ID NO:3)-3'-dT-3')). ARC 126 was dissolved to 2 mM in 100 mM sodium carbonate buffer, pH 8.5, and was reacted for 1 hour with a 2.5 molar excess of mPEG-SPA (MW 20 kDa) or mPEG₂-NHS ester (MW 40 kDa) (Shearwater Corp., Huntsville, AL), and 3.5 molar excess (for 24 hours) of mPEG-nPNC (MW 30kDa) (NOF Corporation, Tokyo, Japan) in equal volumes of acetonitrile. The resulting products were then purified by ion exchange HPLC on a 50 ml Super Q 5PW column (Tosoh Bioscience, Montgomeryville, PA) using aqueous NaCl as eluent. The conjugated oligonucleotides were then desalted on a 100 ml Amberchrom CG300S (Tosoh) column using a water/acetonitrile gradient. Aptamer conjugates were lyophilized for storage.

In order to use ARC 127 in animal models, the quality of the material synthesized was tested for endotoxin levels. Endotoxin content of synthesized ARC127 was determined using the LAL test (performed by Nelson Labs, AZ). Results for endotoxin testing are shown in Table 1 below. The detected quantities of endotoxin were below the ISO standard for sterile irrigation solutions (0.5 EU/mL), *i.e.* lower than levels allowed for IV administration. This indicated that ARC126 and ARC 127 preparations were sterile and that it was possible to proceed to animal efficacy models.

**Table 1. Endotoxin levels in large scale synthesis of therapeutic aptamers.**

| Sample | Dilution | Endotoxin detected | Spike recovery |
|---|---|---|---|
| **ARC126** | 1 :10 | 2.5EU/ml&0.52EU/mg | 73% |
| | 1 :100 | 2.8EU/ml&.58EU/mg | 103% |
| | 1 :200 | 2.5EU/ml&0.52EU/mg | 104% |
| **ARC127** | 1 :1 | 0.19EU/ml&0.17EU/mg | 51% |
| | 1 :10 | 0.33EU/ml&0.30EU/mg | 97% |
| | 1 :100 | 0.45EU/ml&0.41EU/mg | 115% |
| **ARC128** | 1 :1 | 0.52EU/ml&0.76EU/mg | 105% |
| | 1 :10 | 0.57EU/ml&0.82EU/mg | 127% |
| | 1 :100 | 0.43EU/ml&0.62EU/mg | 136% |

Small scale syntheses of the de-fluorinated ARC-126 aptamer variants were done on Applied Biosystems' Expedite 8909 DNA (Foster City, CA) synthesizer using standard solid-phase phosphoramidite chemistry and vendor's recommended coupling protocols. The aptamers were cleaved and deprotected by adding 250 µL ammonium hydroxide/40% aqueous methylamine (1:1) to column support and placed in a 65°C heating block for 30 minutes. Aptamers were dried down in a Speed Vac (Savant), then resuspended in 200µL diH₂O. HPLC purification was performed on a Transgenomic WAVE HPLC (Omaha, NE). The columns used for ion- exchange are the DNAPAC (Dionex, Sunnyvale, CA) and Resource (Amersham Biosciences, Piscataway, NJ). Buffer A: 25mM sodium phosphate/25% acetonitrile; Buffer B: 25mM sodium phosphate/400mM sodium perchlorate/25% acetonitrile, a gradient from 0-80% B was used. Purified fractions were then pooled and dried down in a Speed Vac and resuspended in 200 µL diH₂O.

### EXAMPLE 2: Stability Studies with Fluorinated Aptamers

ARC126 and ARC 127 freshly synthesized in house were compared to legacy ARC126 and **ARC127** that were synthesized by Proligo (Boulder, CO), and had been stored lyophilized for 2 years at -20°C. The freshly synthesized aptamers are referred to as "ARC126 (Archemix)" and "ARC127 (Archemix)", while the legacy aptamers are referred to as "ARC 126 (Proligo)" and "ARC 127 (Proligo)".

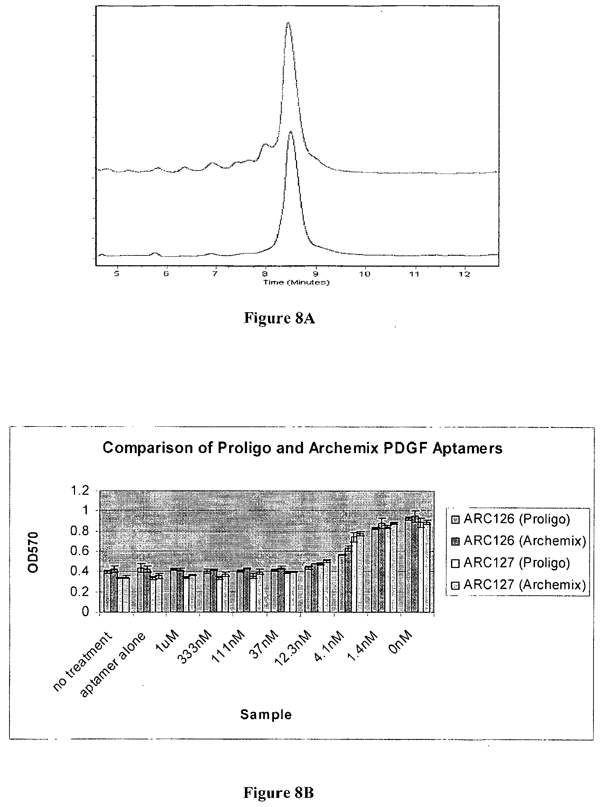

**ARC127** synthesized in house and legacy ARC127 were passed over an ion-exchange HPLC column for analysis. Figure 8A is a trace of an ion-exchange HPLC analysis of freshly synthesized (bottom trace) and legacy ARC127 (top trace) showing that after 2 years storage lyophilized at-20 °C, relatively no degradation of the legacy ARC127 was detected.

The legacy ARC126 and ARC 127 aptamers stored at-20 °C for two years were also tested for potency, and compared to freshly synthesized ARC126 and ARC127 synthesized in house using the 3T3 cell proliferation assay (Example 3). Figure 8B shows cell-based assay results for potency demonstrating that even after lyophilization and storage at -20 degrees celsius for 2 years, the legacy aptamers were just as potent as ARC 126 and ARC127 newly synthesized in house.

### EXAMPLE 3A: Composition and Sequence Optimization of ARC126 Variants

The sequence and secondary structure of the anti-PDGF aptamer designated ARC126 is shown in Figure 9A. The sequence and secondary structure of derivatives of ARC 126 in which the nucleotides with 2'-fluoro-substituents have been replaced are shown in Figure 9B. The loops shown at the termini of the two internal stems are polyethylene glycol-6 (PEG-6) spacers and modified nucleotides are represented by dA = deoxy adenosine; dG = deoxy guanosine; mA = 2'-O-methyl adenosine; dT = deoxy thymidine; dC = deoxy cytosine; mG = 2'-O-methyl guanosine; mC = 2'-O-methyl cytosine; [3'T] = inverted deoxy thymidine; fC = 2'-fluoro cytosine; and fU = 2'-fluoro uridine.

As shown in Figure 9A (left panel), the 29-nucleotide composition of ARC126 contains seven 2'-fluoro residues (three 2'-fluoro uridines and four 2'-fluoro cytosines). Due to considerations including genotoxicity of breakdown products, ARC 126 was optimized to modify the sequence composition by removing all or most of 2'-fluoro residues without compromising the potency or stability of the existing molecule. One avenue for removal of2'-fluoro residues consisted of simple substitution of all seven 2'-fluoro residues with deoxy residues. Such an all-DNA variant of ARC126, designated ARC299 (SEQ ID NO: 42-PEG-SEQ ID NO: 43-PEG-SEQ ID NO: 44, wherein PEG = PEG-6 spacer), was synthesized and tested in *in vitro* biochemical binding and cell-based proliferation assays. These experiments showed that simple substitution of all 2'-fluoro residues for deoxy residues in the 29-mer composition of ARC126 induced instability in the central and upper stems, leading to significantly reduced activity/potency.

The second approach taken to effect removal of 2'-fluoro residues from ARC 126 was the substitution, either singly or in blocks, of 2'-O-methyl residues for 2'-fluoro residues to ameliorate the relative base-pairing instability in the central and upper stems observed with the all-deoxy composition. A number of composition variants were synthesized, representing single point-substitutions of 2'-O-methyl or deoxy residues for 2'-fluoro residues (ARC277, 5'-(SEQ ID NO: 39)-PEG-(SEQ ID NO: 40)-PEG-(SEQ ID NO: 41)-[3'T]-3'), as well as block substitutions of 2'-O-methyl residues for 2'-fluoro residues (ARC337, 5'-(SEQ ID NO: 42)-PEG-(SEQ ID NO: 43)-PEG-(SEQ ID NO: 41)-[3'T]-3'; ARC338, 5'-(SEQ ID NO: 52)-PEG-(SEQ ID NO: 43)-PEG-(SEQ ID NO: 44)-[3'T]-3'; ARC339, 5'-(SEQ ID NO: 48)-PEG-(SEQ ID NO: 43)-PEG-(SEQ ID NO: 53)-[3'T]-3'; ARC340, 5'-(SEQ ID NO: 50)-PEG-(SEQ ID NO: 43)-PEG-(SEQ ID NO: 54)-[3'T]-3'; combinations of single and block substitutions (ARC341, 5'-(SEQ ID NO: 36)-PEG-(SEQ ID NO: 37)-PEG-(SEQ ID NO: 41)-[3'T]-3'; ARC342, 5'-(SEQ ID NO: 55)-PEG-(SEQ ID NO: 37)-PEG-(SEQ ID NO: 41)-[3'T]-3'; ARC344, 5'-(SEQ ID NO: 56)-PEG-(SEQ ID NO: 37)-PEG-(SEQ ID NO: 41)-[3'T]-3'; ARC345, 5'-(SEQ ID NO: 36)-PEG-(SEQ ID NO: 57)-PEG-(SEQ ID NO: 41)-[3'T]-3'; ARC346, 5'-(SEQ ID NO: 36)-PEG-(SEQ ID NO: 58)-PEG-(SEQ ID NO: 41)-[3'T]-3'; ARC347, 5'-(SEQ ID NO: 59)-PEG-(SEQ ID NO: 60)-PEG-(SEQ ID NO: 61)-[3'T]-3'; ARC362, 5'-(SEQ ID NO: 36)-PEG-(SEQ ID NO: 37)-PEG-(SEQ ID NO: 62)-[3'T]-3'; ARC363, 5'-(SEQ ID NO: 63)-PEG-(SEQ ID NO: 37)-PEG-(SEQ ID NO: 64)-[3'T]-3'; ARC364, 5'-(SEQ ID NO: 50)-PEG-(SEQ ID NO: 43)-PEG-(SEQ ID NO: 65)-(3'T]-3'; ARC365, 5'-NH₂-(SEQ ID NO: 39)-PEG-(SEQ ID NO: 40)-PEG-(SEQ ID NO: 41)-[3'T]-3'; ARC366, 5'-(SEQ ID NO: 66)-PEG-(SEQ ID NO: 40)-PEG-(SEQ ID NO: 65)-[3'T]-3'; ARC404, 5'-(SEQ ID NO: 55)-PEG-(SEQ ID NO: 40)-PEG-(SEQ ID NO: 41)-[3'T]-3'; ARC405, 5'-(SEQ ID NO: 56)-PEG-(SEQ ID NO: 40)-PEG-(SEQ ID NO: 41)-[3'T]-3'; ARC406, 5'-(SEQ ID NO: 39)-PEG-(SEQ ID NO: 57)-PEG-(SEQ ID NO: 41)-[3'T]-3'; ARC407, 5'-(SEQ ID NO: 39)-PEG-(SEQ ID NO: 58)-PEG-(SEQ ID NO: 41)-[3'T]-3'; ARC408, 5'-(SEQ ID NO: 39)-PEG-(SEQ ID NO: 40)-PEG-(SEQ ID NO: 62)-[3'T]-3'; ARC409, 5'-(SEQ ID NO: 39)-PEG-(SEQ ID NO: 40)-PEG-(SEQ ID NO: 67)-[3'T]-3'; ARC410, 5'-(SEQ ID NO: 39)-PEG-(SEQ ID NO: 40)-PEG-(SEQ ID NO: 68)-[3'T]-3'; ARC513 (5'-(SEQ ID NO: 69)- PEG -(SEQ ID NO: 40)- PEG -(SEQ ID NO: 70)-[3'T]-3'), ARC514 (5'-(SEQ ID NO:69)- PEG -(SEQ ID NO: 40)- PEG -(SEQ ID NO: 71)-[3'T]-3'), ARC515 (5'-(SEQ ID NO: 69)- PEG -(SEQ ID NO: 40)- PEG -(SEQ ID NO: 72)-[3'T]-3'), and ARC516 (5'-(SEQ ID NO: 69)- PEG -(SEQ ID NO: 40)- PEG -(SEQ ID NO: 73)-[3'T]-3'), and finally an all-2'-O-methyl composition (ARC300 (or 300B), 5'-(SEQ ID NO: 45)-PEG-(SEQ ID NO: 46)-PEG-(SEQ ID NO: 47)-[3'T]-3'). Other composition variants include: ARC276, 5'-(SEQ ID NO: 36)-PEG-(SEQ ID NO: 37)-PEG-(SEQ ID NO: 38)-[3'T]-3'; ARC335, 5'-(SEQ ID NO: 48)-PEG-(SEQ ID NO: 43)-PEG-(SEQ ID NO: 49)-[3'T]-3'; ARC336, 5'-(SEQ ID NO: 50)-PEG-(SEQ ID NO: 43)-PEG-(SEQ ID NO: 51)-[3'T]-3'; ARC343, 5'-(SEQ ID NO: 52)-PEG-(SEQ ID NO: 43)-PEG-(SEQ ID NO: 41)-[3'1']-3'.

Table 2 below summarizes the sequence and composition of all ARC 126 variants synthesized and tested. Sequences shown are listed 5'→ 3' from left to right in the table. Table 2 also summarizes the composition identity, mean affinity, and activity of all ARC 126 variants synthesized and tested in *in vitro* assays (competitive binding assay and 3T3 proliferation assay) described below in Example 5. In the table, d= deoxy residue; f = 2'-fluoro residue; m = 2'-O-methyl residue; PEG = polyethylene glycol (PEG-6) spacer; 3T = inverted deoxythymidine.

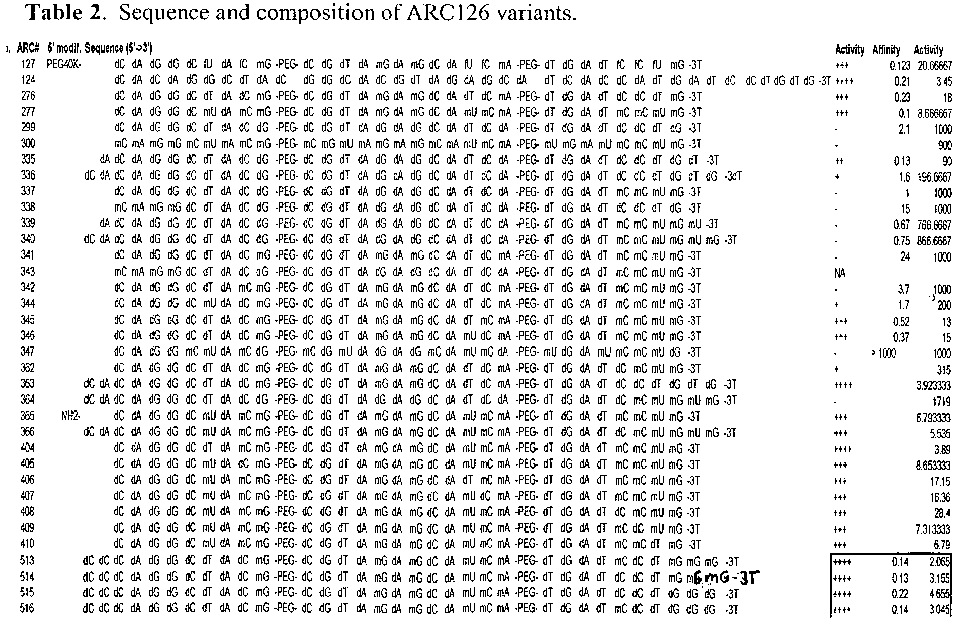

Following synthesis, these composition variants were tested in *in vitro* biochemical binding and cell-based proliferation assays described herein. These experiments showed a wide range of affinities in *in vitro* competition binding assays and a similarly wide range of activities in cell-based proliferation assays (results are described below). The composition variants that showed the highest levels of binding affinity and cell-based assay activity are exemplified by the series ARC513-516, shown in Figure 9B. Figure 9B shows the optimal composition variants of ARC 126: ARC513 (5'-(SEQ ID NO: 69)- PEG -(SEQ ID NO: 40)- PEG -(SEQ ID NO: 70)-[3'T]-3'), ARC514 (5'-(SEQ ID NO: 69)- PEG -(SEQ ID NO: 40)- PEG -(SEQ ID NO: 71)-[3'T]-3'), ARC515 (5'-(SEQ ID NO: 69)- PEG -(SEQ ID NO: 40)- PEG -(SEQ ID NO: 72)-[3'T]-3'), and ARC516 (5'-(SEQ ID NO: 69)- PEG -(SEQ ID NO: 40)- PEG -(SEQ ID NO: 73)-[3'T]-3'). From Figure 9B, all of the optimal composition variants are comprised of an extended stem, relative to ARC 126, by two base pairs, and in the upper stems, a common set of point substitutions of 2'-O-methyl for 2'-fluoro residues as well as the pre-existing 2'-O-methyl residues from ARC126. The composition variants ARC513-516 differ only in two aspects: (1) the deoxy or 2'-fluoro identity of the three 3' terminal guanosine residues in the central stem; and (2) the deoxy or 2'-O-methyl identity of the cytosine residue at the top of the central stem on the 3' side.

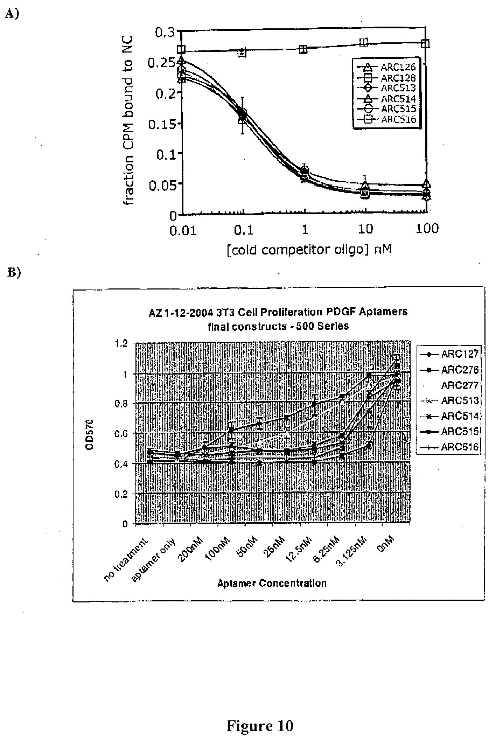

The *in vitro* binding affinity of the optimal composition variants for PDGF is shown in Figure 10A. The data shown in the figure was derived from a competitive binding assay in which the indicated concentration of unlabeled, competitor aptamer was titrated in separate reactions against a fixed (<0.1 nM) amount of ³²P-radiolabeled ARC 126 aptamer in buffer containing a fixed amount (0.1 nM) of the aptamer's cognate target (PDGF-BB; PeproTech, Rocky Hill, NJ). ARC 126 was radiolabeled at the 5' terminus by incubation with γ-³²P-ATP (MP Biomedicals, Irvine, CA) and polynucleotide kinase (New England Biolabs ("NEB"), Beverly, MA). Binding reactions were carried out in phosphate-buffered saline (Cellgro, Herndon, VA) containing 0.2 mg/mL bovine serum albumin (NEB) and 0.02 mg/mL tRNA (Sigma, St. Louis, MO). The reactions were equilibrated for a period of 15 - 30 minutes at room temperature, then filtered through a sandwich of nitrocellulose (Protran membrane, Perkin-Elmer, Boston, MA) and nylon (Hybond membrane, Amersham, Piscataway, NJ) membranes to separate target bound aptamer from free aptamer. Subsequent auto-radiographic analysis of the filter membranes corresponding to each concentration of unlabeled aptamer revealed the extent of competitive displacement of ³²P-ARC126 by unlabeled aptamer. The data is shown in Figure 10A wherein ARC128 ((5'-(SEQ ID NO:4)-HEG-(SEQ ID NO:5)-HEG-(SEQ ID NO:6) -3')) represents a sequence-scrambled and therefore inactive variant of ARC126.

Figure 10B shows *in vitro* 3T3 cell-based proliferation assay data showing the activity of some composition variants of ARC126. 3T3 cells, a rat fibroblast cell line (ATCC, Manassas, VA), were plated 3,000 cells/well in a 96 well plate one day prior to the start of the assay in 100ul DMEM/ 10%FCS. The following day, cells were washed once with straight DMEM and then 75ul DMEM/ 0.8% FCS was added to each well. Then 25ul of PDGF-BB (PeproTech, Rocky Hill, NJ) at a final concentration of 50 ng/ml +/- ARC 126 variants (6 points, final concentration 0-200 nM) were added to each well. Cells were incubated for 3 days. Following incubation 10ul MTT (Promega, Madison, WI) was added to each well and incubated for an additional 1.5 hours. Media was then removed, 200ul Isopropanol (2-propanol) was added to each well, cells were re-suspended thoroughly and absorbance at 570nm was read on a 96 well plate reader. As shown in Figure 10B, it is clear that (1) all composition variants shown are active; and (2) the relative activity ranking of ARC513 (5'-(SEQ ID NO: 69)- PEG -(SEQ ID NO: 40)- PEG -(SEQ ID NO: 70)-[3'T]-3'), ARC514 (5'-(SEQ ID NO: 69)- PEG -(SEQ ID NO: 40)-PEG -(SEQ ID NO: 71)-[3'T]-3'), ARC515 (5'-(SEQ ID NO: 69)- PEG -(SEQ ID NO: 40)- PEG -(SEQ ID NO: 72)-[3'T]-3'), and ARC516 (5'-(SEQ ID NO: 69)- PEG -(SEQ ID NO: 40)- PEG -(SEQ ID NO: 73)-[3'T]-3') is similar.

### Example 3B: ARC513 Modifications

### mU substitutions

ARC513 was further modified to replace the 3' or 5' PEG spacers with 2'-OMe tri or 2'-OMe tetra loops, resulting in the following aptamers:

ARC1012 NH₂-(dCdCdCdAdGdGdCTdAdCmGmUmUmUdCdGTdAmGdAmGdCdAmUmCmA) (SEQ ID NO:86)-PEG-(TdGdATmCdCTmGmGmG) (SEQ ID NO:87)-3T

ARC1013 NH₂-(dCdCdCdAdGdGdCTdAdCmGmUmUmUmUdCdGTdAmGdAmGdCdAmUmCmA) (SEQ ID NO:88)-PEG- (TdGdATmCdCTmGmGmG) (SEQ ID NO:87)-3T

ARC1014 NH₂-(dCdCdCdAdGdGdCTdAdCmG) (SEQ ID NO:89)-PEG-dCdGTdAmGdAmGdCdAmUmCmAmUmUmUTdGdATmCdCTmGmGmG (SEQ ID NO:91)-3T

ARC1015 NH₂-(dCdCdCdAdGdGdCTdAdCmG) (SEQ ID NO: 89)-PEG-(dCdGTdAmGdAmGdCdAmUmCmAmUmUmUmUTdGdATmCdCTmGmGmG) (SEQ ID NO:93)-3T

ARC1016 NH2-
dCdCdCdAdGdGdCTdAdCmGmUmUmUdCdGTdAmGdAmGdCdAmUmCmAmUmUmUTd
GdATmCdCTmGmGmG-3T (SEQ ID NO: 94)

ARC1017 NH2-
dCdCdCdAdGdGdCTdAdCmGmUmUmUmUdCdGTdAmGdAmGdCdAmUmCmAmUmUmU
mUTdGdATmCdCTmGmGmG-3T (SEQ ID NO: 95)

### 3T3 Proliferation Assay with Modified ARC513 Aptamers

3T3 cells, which are derived from a rat fibroblast cell line (ATCC, Manassas, VA), were used in a proliferation assay to test potency of modified ARC513 aptamers. 3T3 fibroblast cells have PDGF receptors on their cell-surface and respond to mitogen, *e.g*., PDGF stimulation by proliferation. The assay was performed as follows: 3T3 cells were plated 3,000 cells/well in a 96 well plate one day prior to the start of the assay in 100ul DMEM/ 10%FCS. The following day, cells were washed once with straight DMEM and then 75ul DMEM/ 0.8% FCS was added to each well. Then 25ul of PDGF-BB (PeproTech, Rocky Hill, NJ) at a 50 ng/ml final concentration was added to each well +/- aptamer condition to be tested. Each plate included the following conditions in triplicate: no PDGF which corresponds to growth without mitogen (negative control), a scrambled aptamer control (ARC 128) where no effect on growth rate is observed (negative control), a positive control where maximal growth is observed in the absence of PDGF aptamer, and a series of functional PDGF aptamer dilutions from which a good IC50 curve could be calculated. The functional PDGF aptamer dilutions usually consisted of 6 points in 2-fold serial dilutions.

Cells were incubated for 3 days. Following incubation 10ul MTT (Promega, Madison, WI) was added to each well and incubated for an additional 1.5 hours. Media was removed, 200 µl Isopropanol (2-propanol) was added to each well, cells were resuspended thoroughly and absorbance at 570nm was read on a 96 well plate reader. The present example depicts the potency comparison of PDGF aptamer ARC 513 to that of ARC 513 variants.

The 3T3 cell proliferation assay was performed with ARC513 and ARC 513 derivatives, ARC1012 to ARC1017. ARC513 routinely displayed IC₅₀ values <10 nM where scrambled control ARC 128 never displayed an effect on 3T3 cell proliferation (ARC128 data not shown). Table 3A below shows the IC₅₀s of ARC513 variants in 3T3 proliferation assay.

**Table 3A: ARC513 variants in 3T3 proliferation assay.**

| **ARC#** | **Mean IC50** |
|---|---|
| 513 | 3.7 |
| 1012 | 2.79 |
| 1013 | 15.4 |
| 1014 | 10.61 |
| 1015 | 10.1 |
| 1016 | 8.5 |
| 1017 | 14.3 |

### ARC513 5' PEG Conjugation

The oligonucleotide 5'-NH₂-dCdCdCdAdGdGdCTdAdCmG (SEQ ID NO 69) PEG dCdGTdAmGdAmGdCdAmUmCmA (SEQ ID NO 40) PEG TdGdATmCdCTmGmGmG-3T-'3 (SEQ ID NO 70) was synthesized on an AKTA OligoPilot 100 synthesizer (GE Healthcare, Uppsala, Sweden) according to the recommended manufacturer's procedures using standard commercially available 2'-OMe RNA, deoxyinosine and DNA phosphoramidites (Glen Research, Sterling, VA) and a inverted deoxythymidine CPG support. A terminal amine function was attached with a 5'-amino-modifier C6-TFA (Glen Research, Sterling, VA). After deprotection, the oligonucleotide was purified by ion exchange chromatography on Super Q 5PW (30) resin (Tosoh Biosciences) and ethanol precipitated.

Aliquots of the 5'-amine-modified aptamer were conjugated to a linear 30 kDa, a linear 20 kDa and either a branched or linear 40 kDa PEG moiety post-synthetically. Aptamers were dissolved in a water/DMSO (1:1) solution to a concentration between 1.5 and 3mM. Sodium carbonate buffer, pH 8.5, was added to a final concentration of 100mM, and the oligo was reacted overnight with a 1.7 molar excess of the desired PEG reagent (*e.g*. 20 kDa Sunbright MENP-20T p-nitrophenyl carbonate ester [NOF Corp, Japan], 30 kDa Sunbright MENP-30T p-nitrophenyl carbonate ester [NOF Corp, Japan], Sunbright GL2-400NP p-nitrophenyl carbonate ester [NOF Corp, Japan], 40 kDa or 60 kDa mPEG2-NHS ester [Nektar, Huntsville A.L]) dissolved in an equal volume of acetonitrile. The resulting PEGylated products were purified by ion exchange chromatography on Super Q 5PW (30) resin (Tosoh Biosciences), and desalted using reverse phase chromatography performed on Amberchrom CG300-S resin (Rohm and Haas), and lyophilized.

The structures of the resulting PEGylated aptamers are given below: ARC594: 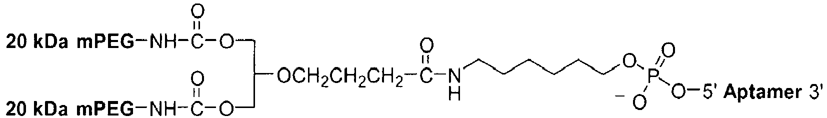 Wherein Aptamer = dCdCdCdAdGdGdCTdAdCmG (SEQ ID NO 69) PEG dCdGTdAmGdAmGdCdAmUmCmA (SEQ ID NO 40) PEG TdGdATmCdCTmGmGmG-3T-3' (SEQ ID NO 70); wherein d denotes a deoxy nucleotide, m denotes a 2'-OMe nucleotide, PEG denotes a PEG spacer and 3T denotes an inverted deoxy thymidime cap.

ARC1472: 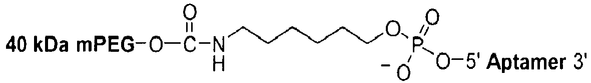 Wherein Aptamer = 5'dCdCdCdAdGdGdCTdAdCmG (SEQ ID NO 69)-PEG - dCdGTdAmGdAmGdCdAmUmCmA (SEQ ID NO 40) -PEG - TdGdATmCdCTmGmGmG-3T-3' (SEQ ID NO 70); wherein d denotes a deoxy nucleotide, m denotes a 2'-OMe nucleotide, PEG denotes a PEG spacer and 3T denotes an inverted deoxy thymidine cap.

ARC592: 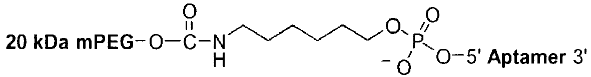 Wherein Aptamer = 5'dCdCdCdAdGdGdCTdAdCmG (SEQ ID NO 69)-PEG - dCdGTdAmGdAmGdCdAmUmCmA (SEQ ID NO 40) -PEG - TdGdATmCdCTmGmGmG-3T-3' (SEQ ID NO 70); wherein d denotes a deoxy nucleotide, m denotes a 2'-OMe nucleotide, PEG denotes a PEG spacer and 3T denotes an inverted deoxy thymidine cap.

ARC593: 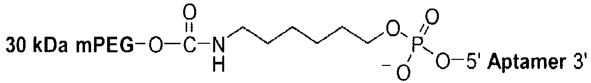 Wherein Aptamer = 5'dCdCdCdAdGdGdCTdAdCmG (SEQ ID NO:69)-PEG - dCdGTdAmGdAmGdCdAmUmCmA (SEQ ID NO:40) -PEG - TdGdATmCdCTmGmGmG-3T-3' (SEQ ID NO:70); wherein d denotes a deoxy nucleotide, m denotes a 2'-OMe nucleotide, PEG denotes a PEG spacer and 3T denotes an inverted deoxy thymidine cap.

### Aptamer-3'-5'-PEG conjugates

The oligonucleotide 5'-NH₂-dCdCdCdAdGdGdCdTdAdCmG (SEQ ID NO 69) PEG dCdGdTdAmGdAmGdCdAmUmCmA (SEQ ID NO 40) PEG dTdGdAdTmCdCdTmGmGmG -NH2-3' (SEQ ID NO 102) was synthesized on an AKTA OligoPilot 100 synthesizer (GE Healthcare Uppsala, Sweden) according to the recommended manufacturer's procedures using standard commercially available 2'-OMe RNA, deoxyinosine and DNA phosphoramidites (Glen Research, Sterling, VA) and a 3'-phthalimide-amino-modifier C6 CPG support (Glen Research, Sterling, VA). Terminal amine functions were attached with a 5'-amino-modifer C6-TFA (Glen Research, Sterling, VA). After deprotection, the oligonucleotides was purified by ion exchange chromatography on Super Q 5PW (30) resin (Tosoh Biosciences) and ethanol precipitated.

Aliquots of the 3'-5'-diamine-modified aptamer were conjugated to different PEG moieties post-synthetically. Aptamers were dissolved in a water/DMSO (1:1) solution to a concentration between 1.5 and 3mM. Sodium carbonate buffer, pH 8.5, was added to a final concentration of 100mM, and the oligo was reacted overnight with a 2.7 molar excess of the desired PEG reagent (e.g. 20 kDa Sunbright MENP-20T p-nitrophenyl carbonate ester [NOF Corp, Japan], 30 kDa Sunbright MENP-30T p-nitrophenyl carbonate ester [NOF Corp, Japan]) dissolved in an equal volume of acetonitrile. The resulting 2 x 20 kDa or 2 x 30 kDa PEGylated products were purified by ion exchange chromatography on Super Q 5PW (30) resin (Tosoh Biosciences), and desalted using reverse phase chromatography performed on Amberchrom CG300-S resin (Rohm and Haas), and lyophilized.

The structures of the resulting di-PEGylated aptamers are given below: ARC1473: 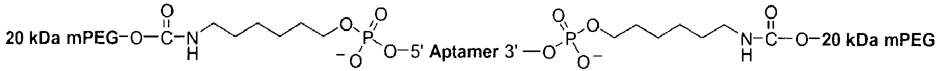 wherein the Aptamer =: Wherein Aptamer = dCdCdCdAdGdGdCTdAdCmG (SEQ ID NO 69) PEG dCdGTdAmGdAmGdCdAmUmCmA (SEQ ID NO 40) PEG TdGdATmCdCTmGmGmG-3' (SEQ ID NO 70); wherein d denotes a deoxy nucleotide, m denotes a 2'-OMe nucleotide, and PEG denotes a PEG spacer.

### ARC1474:

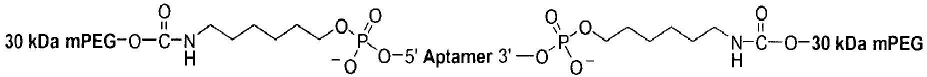

Wherein Aptamer = dCdCdCdAdGdGdCTdAdCmG (SEQ ID NO 69) PEG dCdGTdAmGdAmGdCdAmUmCmA (SEQ ID NO 40) PEG TdGdATmCdCTmGmGmG-3' (SEQ ID NO 70); wherein d denotes a deoxy nucleotide, m denotes a 2'-OMe nucleotide, and PEG denotes a PEG spacer.

### 3T3 Proliferation Assay with PEG Conjugated ARC513 variants

The 3T3 proliferation assay previously described was used to test potency of all PEG conjugated ARC513 variants described immediately above. The assay was performed as follows: 3T3 cells were plated 3,000 cells/well in a 96 well plate one day prior to the start of the assay in 100ul DMEM/ 10%FCS. The following day, cells were washed once with straight DMEM and then 75ul DMEM/ 0.8% FCS was added to each well. Then 25ul of PDGF-BB (PeproTech, Rocky Hill, NJ) at a 50 ng/ml final concentration was added to each well +/- aptamer condition to be tested. Each plate included the following conditions in triplicate: no PDGF which corresponds to growth without mitogen (negative control), a scrambled aptamer control (ARC128) where no effect on growth rate is observed (negative control), a positive control where maximal growth is observed in the absence of PDGF aptamer, and a series of functional PDGF aptamer dilutions from which a good IC50 curve could be calculated. The functional PDGF aptamer dilutions usually consisted of 6 points in 2-fold serial dilutions.

Cells were incubated for 3 days. Following incubation 10ul MTT (Promega, Madison, WI) was added to each well and incubated for an additional 1.5 hours. Media was removed, 200 µ1 Isopropanol (2-propanol) was added to each well, cells were resuspended thoroughly and absorbance at 570nm was read on a 96 well plate reader. The present example depicts the potency comparison of PDGF aptamer ARC513 to that of PEGylated ARC513 aptamers.

The 3T3 cell proliferation assay was performed with ARC513 and with all the PEG conjugated ARC513 aptamers that were synthesized as described immediately above. ARC513 routinely displayed IC₅₀ values <10 nM where scrambled control ARC128 never displayed an effect on 3T3 cell proliferation (ARC128 data not shown). Tables 3B and 3C below show the IC₅₀s of ARC513 compared to the PEG conjugated ARC513 aptamers in the 3T3 proliferation assay.

**Table 3B: ARC513 variants in 3T3 proliferation assay.**

| **ARC#** | **Mean IC50** |
|---|---|
| 513 | 3.7 |
| 594 | 1.95 |
| 1472 | 2.43 |
| 1473 | 3.42 |
| 1474 | 3.32 |

**Table 3C:ARC513 variants in 3T3 proliferation assay.**

| **ARC#** | **Mean IC50** |
|---|---|
| 513 | 3.2 |
| 592 | 0.88 |
| 593 | 1.45 |

### Example 3C: Optimization of ARC126 In Vivo Phamlacokinetic and Biodistribution Properties

In addition to optimization of the sequence composition of ARC 126 with respect to target binding affinity and *in vitro* cell-based assay activity, it is desirable to optimize the *in vivo* pharmacokinetic (PK) half-life, t_{1/2}, and biodistribution properties of the optimal sequence composition(s) for the anti-PDGF aptamers described previously. This modulation of the pharmacokinetic and biodistribution properties of an aptamer composition can be accomplished (*see* U.S. Ser No. 10/718,833, filed November 21, 2003, and U.S. Ser. No. 60/550,790, filed March 5, 2004) via conjugation of the 3' or 5' terminus, or an internal site or sites, of the molecule with a polyethylene glycol (PEG) chain or chains, *i.e.,* PEGylation (range is ∼2 kDa to 100 kDa, with typical PEGs having molecular weights of 2 kDa, 10 kDa, 20 kDa, 30 kDa, 40 kDa, 50 kDa). Note that for each pharmokinetic study described herein, all mass based concentration data refers only to the molecular weight of the oligonucleotide portion of the aptamer, irrespective of the mass conferred by PEG conjugation.

In order to establish the feasibility of using a given PEGylated aptamer sequence composition, it was necessary to confirm that the putative PEGylation does not significantly interfere with the activity of the aptamer in *in vitro* binding and cell-based proliferation assays. Competitive binding assays were performed and analyzed as described herein except that 3'-³²P-labeled NH₂-ARC126 was used rather than 5'-³²P-ARC 126 (the ARC 126 nucleotide sequence incorporates a reverse thymidine at the 3'-terminus, which is a substrate for the radiolabeling reaction catalyzed by polynucleotide kinase).

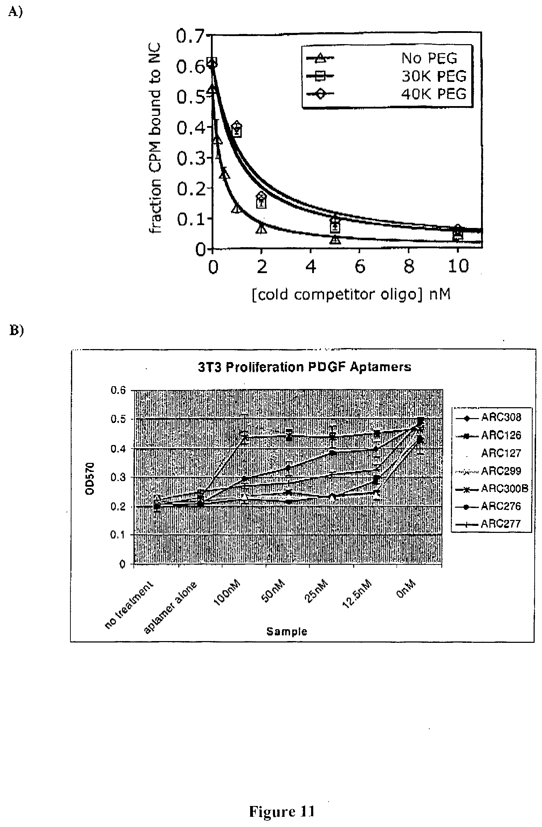

Figure 11A is a plot of competition binding assay data for ARC 126 and two variants that are 5' conjugated to 30 kDa (ARC308) and 40 kDa (ARC127) PEG groups. Figure 11B shows *in vitro* 3T3 cell-based proliferation assay data for ARC 126 as a function of 5' PEG group conjugation (ARC 126+30 kDa = ARC308, and ARC126+40 kDa PEG = ARC 127). The data shown in Figure 11B demonstrate the 5' conjugation of ARC 126 to 30kDa and 40 kDa PEG groups appears to lead to a slight decrease in the *in vitro* activity of the aptamer, the effect of the presence of the PEG groups is less than twofold relative to the unPEGylated aptamer, ARC 126. The data shown in Figure 11B also demonstrate that the 5' conjugation of ARC 126 to 30 kDa and 40 kDa PEG groups does not signifcantly reduce the *in vitro* activity in the 3T3 proliferation assay of the aptamer relative to the composition of ARC 126.

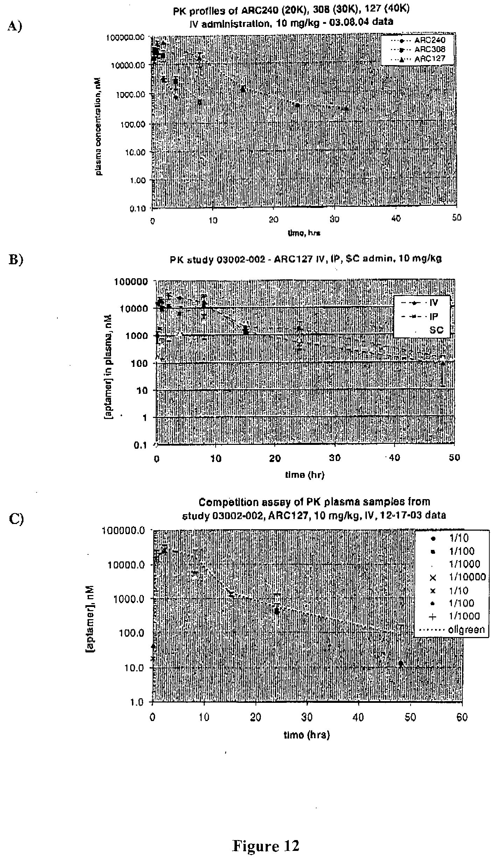

Figure 12A shows the *in vivo* pharmacokinetic profile of ARC 126 as a function of 5' PEG group conjugation. Studies were done in CD-1 mice as described in Example 10 (Charles River Labs, Wilmington, MA). From the figure, it is clear that the terminal clearance half-life, t_{1/2}, is strongly affected by the size of the 5' PEG group conjugated to the aptamer.

The pharmacokinetic (PK) data shown in Figure 12A was subjected to non-compartmental analysis (NCA) using the industry-standard software package WinNonLin™ v.4.0 (Pharsight Corp., Mountain View, CA). Table 4 below lists the primary NCA-derived *in vivo* pharmacokinetic (PK) parameters for ARC 126 + 20 kDa (ARC240), +30 kDa (ARC308), and +40 kDa (ARC 127) PEG groups after intravenous (IV) administration at 10 mg/kg in mice. The data shown in Table 4 demonstrate that the *in vivo* pharmacokinetic clearance half-life, t_{1/2}, of ARC 126 is modulated ≥ 4-fold by changing the size of the 5' PEG group conjugated to the aptamer affected by the size of the 5' PEG group conjugated to the aptamer from 20 kDa to 40 kDa. Figure 12B shows the *in vivo* pharmacokinetic profile of ARC 127 (ARC126 + 40 kDa PEG) after intravenous (IV), intraperitoneal (IP), and subcutaneous (SC) administration at a dose level of 10 mg/kg in mice. The pharmacokinetic (PK) data shown in Figure 12B was subjected to noncompartmental analysis (NCA) using the industry-standard software package WinNonLin™ v.4.0 (Pharsight Corp., Mountain View, CA).

**Table 4. Pharmacokinetic non-compartmental analysis of 5' conjugates of ARC 126.**

| | **PEG** | **Cmax,nM** | **AUC, nM·hr** | **tₘₐₓ (hr)** | **MRT (hr)** | **t_{1/2} (hr)** | **Vₛₛ(mL/Kg)** |
|---|---|---|---|---|---|---|---|
| ARC 240 | 20K | 34317 | 0.5 | 39277 | 1.16 | 0.94 | 0.3 |
| ARC 308 | 30K | 27659 | 1.0 | 64867 | 2.14 | 1.67 | 0.3 |
| ARC 127 | 40K | 60964 | 2.0 | 344356 | 5.08 | 6.84 | 0.1 |

| | | | | | | | |
|---|---|---|---|---|---|---|---|
| Pharmacokinetic parameters (noncompartmental analysis) of 5' conjugates of ARC 126: ARC240 = ARC126 + 20 kD PEG; ARC308 = ARC126 + 30 kD PEG; and ARC127 = ARC126 + 40 kD PEG after IV administration at 10 mg/kg in mice. | | | | | | | |

Table 5 below lists the primary NCA-derived *in vivo* pharmacokinetic (PK) parameters for ARC 126 + 40kDa PEG as a function of the route of administration at 10 mg/kg in mice. The pharmacokinetic (PK) data shown in Figure 12B was subjected to noncompartmental analysis (NCA) using the industry-standard software package WinNonLin™ v.4.0 (Pharsight Corp., Mountain View, CA).

**Table 5. Pharmacokinetic profile of ARC127 (ARC126 + 40 kD PEG)**

| | **Cmax, nM** | **tₘₐₓ (hr)** | **AUC (hr·nM)** | **MRT (hr)** | **t_{1/2} (hr)** | **V_{z} (L/Kg)** | **bioavailability, F** |
|---|---|---|---|---|---|---|---|
| **IV** | 29711.6 | 2 | 229686.8 | 6.573 | 8.602 | 0.053 | 1.000 |
| **IP** | 12756.0 | 8 | 143605.5 | 11.231 | 7.856 | 0.078 | 0.625 |
| **SC** | 3176.7 | 8 | 55030.91 | 16.632 | 9.176 | 0.238 | 0.240 |

| | | | | | | | |
|---|---|---|---|---|---|---|---|
| Post- intravenous (IV), intraperitoneal (IP), and subcutaneous (SC) administration at a dose level of 10 mg/kg in mice. | | | | | | | |

From data shown in Figure 12B, two primary points are clear: (1) the pharmacokinetics of ARC 127 (ARC126 + 40 kD PEG) after intravenous (IV), intraperitoneal (IP), or subcutaneous (SC) administration at a dose level of 10 mg/kg in mice a plasma concentration of ∼1 µM is present at 24 hrs post-dose; and (2) the systemic bioavailability, F, of ARC127 after intraperitoneal (IP) administration is quite high (∼63%), while for subcutaneous (SC) administration the bioavailability is still high enough (∼24%) to warrant consideration for clinical applications.

As a secondary test of both the plasma pharmacokinetics and the *in vivo* bioactivity of ARC 127 (ARC126 + 40 kD PEG), the competition binding assay described above in reference to Figure 10A was used to assay the same plasma samples used to generate the data shown in Figure 12B. Serial 1:10 dilutions of plasma sample were prepared in phosphate-buffered saline (1X PBS) and mixed with a fixed concentration of ³²P-ARC126, then added to human PDGF-BB. The final concentration of PDGF in each assay was 0.1 nM, and the final concentration of ³²P-ARC126 < 0.1 nM. In this experiment, the plasma samples were analyzed by comparison with a standard curve generated with samples of known ARC127 concentrations in 1X PBS. By comparison with the reference standards, the effective concentration of active aptamer in each plasma sample could be calculated. The effective concentration of active aptamer, as calculated using the results of the competition binding assay analysis of the plasma PK samples, is shown in Figure 12C. Figure 12C shows the bioactivity profile of ARC126+40 kD PEG after intravenous (IV) administration at a dose level of 10 mg/kg in mice. This *ex vivo* analysis thus provides verification that (1) the aptamer was present and active in the plasma of the mouse model at t=48 hrs post-dose; and (2) the plasma concentrations calculated from the fluorescence-based pharmacokinetic assay are accurate.

### EXAMPLE 4: Species Cross Reactivity of ARC126 and ARC127

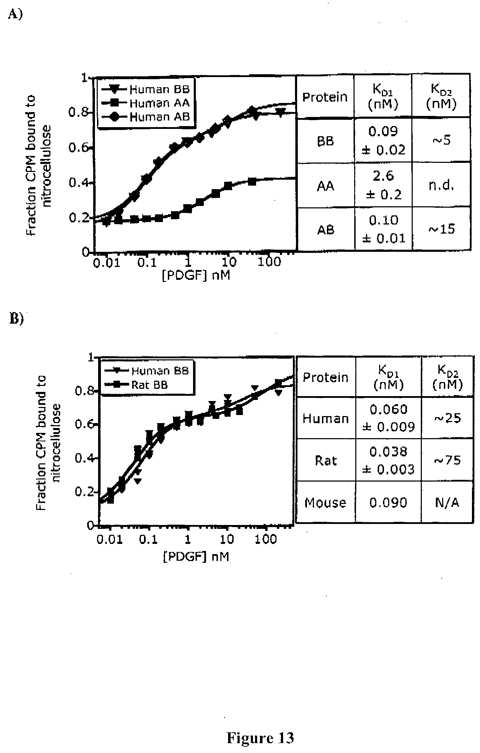

Studies were performed to determine which isoforms of PDGF ARC 126 would bind to. Competition binding assays were set up using the dot blot analysis previously described, to test ARC126 for binding to human PDGF-AA, PDGF-BB and PDGF-AB (all from PeproTech, Rocky Hill, NJ). The results of the competition binding assay show that ARC 126 (SEQ ID NO. 1-HEG-SEQ ID NO:2-HEG-SEQ ID NO:3-3T) binds to PDGF-BB with a K_{d} of approximately 100 pM, and fDGF-AB with a K_{d} of approximately 100 pM, but does not bind to PDGF-AA (Figure 13A). Next, a study was done to determine whether ARC 126 cross reacted with PDGF-BB of species other than human. Competition binding assays were set up by dot blot analysis, as previously described, using human, rat, and mouse PDGF-BB (PeproTech, Rocky Hill, NJ). The results of the competition binding assay show that ARC126 binds to human, rat and mouse PDGF-BB with equal affinity (Figure 13B).

### EXAMPLE 5: 3T3 Cell Proliferation Assay

Since ARC126 was shown to cross react with human, rat and mouse PDGF-BB, 3T3 cells, which are derived from a rat fibroblast cell line (ATCC, Manassas, VA), could be used in a proliferation assay to test potency of all PDGF aptamers, including aptamers that were obtained as part of the de-fluorination efforts described in Example 3A. 3T3 fibroblast cells have PDGF receptors on their cell-surface and respond to mitogen, e.g., PDGF stimulation by proliferation. The assay was performed as follows: 3T3 cells were plated 3,000 cells/well in a 96 well plate one day prior to the start of the assay in 100ul DMEM/ 10%FCS. The following day, cells were washed once with straight DMEM and then 75ul DMEM/ 0.8% FCS was added to each well. Then 25ul of PDGF-BB (PeproTech, Rocky Hill, NJ) at a 50 ng/ml final concentration was added to each well +/- aptamer condition to be tested. Each plate included the following conditions in triplicate: no PDGF which corresponds to growth without mitogen (negative control), a scrambled aptamer control (ARC128) where no effect on growth rate is observed (negative control), a positive control where maximal growth is observed in the absence of PDGF aptamer, and a series of functional PDGF aptamer dilutions from which a good IC50 curve could be calculated. The functional PDGF aptamer dilutions usually consisted of 6 points in 2-fold serial dilutions.

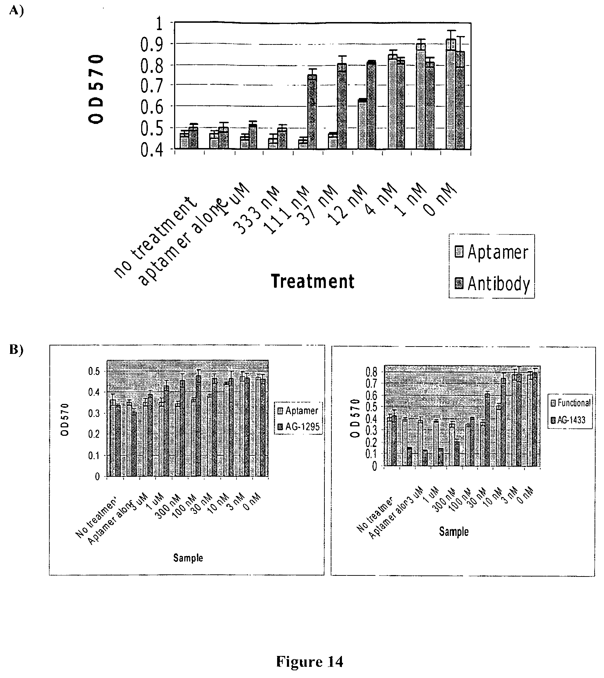

Cells were incubated for 3 days. Following incubation 10ul MTT (Promega, Madison, WI) was added to each well and incubated for an additional 1.5 hours. Media was removed, 200 µl Isopropanol (2-propanol) was added to each well, cells were resuspended thoroughly and absorbance at 570nm was read on a 96 well plate reader. The present example depicts the potency comparison of PDGF aptamer ARC 127 to that of a PDGF neutralizing polyclonal antibody as shown in Figure 14A (R&D Systems, Minneapolis, MN). The data shown in Figure 14A demonstrate that the aptamer displays better potency than the polyclonal antibody.

The 3T3 cell proliferation assay was performed with ARC126, ARC127, ARC 128 and with all the other PDGF aptamer derivatives that were obtained as described in Example 3A above. ARC126 and ARC 127 routinely display IC₅₀ values <20 nM where scrambled control ARC128 never displays an effect of 3T3 cell proliferation. Table 6 below shows the IC₅₀s of ARC126 variants in 3T3 proliferation assay.

Table 6. IC₅₀'s of defluorinated ARC126 variants in 3T3 proliferation assay.

| **ARC#** | **Mean IC₅₀** |
|---|---|
| 124 | 20.67 |
| 126 | 3.40 |
| 127 | 3.45 |
| 276 | 18.00 |
| 277 | 8.67 |
| 299 | 1000.00 |
| 300 | 900.00 |
| 335 | 90.00 |
| 336 | 196.67 |
| 337 | 1000.00 |
| 338 | 1000.00 |
| 339 | 766.67 |
| 340 | 866.67 |
| 341 | 1000.00 |
| 343 | |
| 342 | 1000.00 |
| 344 | 200.00 |
| 345 | 13.00 |
| 346 | 15.00 |
| 347 | 1000.00 |
| 362 | 315.00 |
| 363 | 3.92 |
| 364 | 1719.00 |
| 365 | 6.79 |
| 366 | 5.54 |
| 404 | 3.89 |
| 405 | 8.65 |
| 406 | 17.15 |
| 407 | 16.36 |
| 408 | 28.40 |
| 409 | 7.31 |
| 410 | 6.79 |
| 513 | 2.07 |
| 514 | 3.16 |
| 515 | 4.66 |
| 516 | 3.05 |
| 127 | 3.45 |

The ARC 127 PDGF aptamer also blocks proliferation of 3T3 cells better than known tyrosine kinase inhibitors such as AG 1295 and AG 1433 compounds (Sigma Aldrich Biochemicals, St Louis, MO). The assay conditions were exactly the same as described above. ARC 127 reduced the PDGF driven increase in proliferation to background levels at a concentration as low as 30 nM. Both of the AG compounds displayed much worse potencies compared to ARC127. AG-1433 seemed to have unspecific toxic effects at micromolar levels. This effect is visible starting from 300 nM where signal levels are lower than no treatment alone samples corresponding to loss of signal due to lethality of cells only in the presence of AG compound (Figure 14B).

### EXAMPLE 6: 3T3 Cell Viability Assay

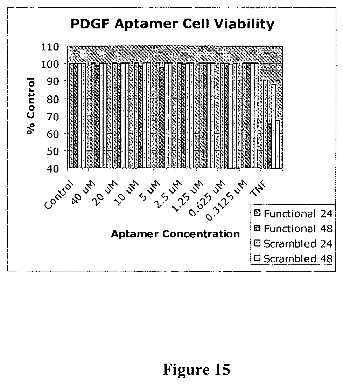

The reduction of growth in 3T3 cells observed in the cell proliferation assay described in Example 5 upon addition of ARC 126, ARC127 and other active PDGF aptamer derivatives might potentially be due to toxic effects of aptamer. To test this possibility a Calcein AM cell viability assay (Molecular Probes, Eugene, OR) was performed. 3T3 cells were plated 3,000 cells/well were treated with various concentrations of PDGF aptamer up to 40 µM were tested for 24 and 48 hours. TNF-α (100 pg/ml) was provided and used as a positive control to induce apoptosis. Following incubation cells were washed with 1X PBS. Calcein AM was prepared according to manufacturer's recommended instructions, incubated for 30 minutes and fluorescence signal intensity was determined on a 96-well plate reader. No increase in the apoptosis rate of 3T3 cells due to ARC 127 was observed (Figure 15).

### EXAMPLE 7: 3T3 or RPE Cell Migration Assay

PDGF is a strong mitogen as well as a chemoattractant. A migration assay performed both in 24 and 96-well format was chosen as an additional functional assay to test the potency of ARC 127. In the cell migration assay 80,000 cells 3T3 rat fibroblasts (ATCC, Manassas, VA) or RPE (retinal pigmented epithelial) cells (ATCC, Manassas, VA) were plated per well into a 24 well plate with 8 micron filters (BD Biosciences, Discovery Labware, Bedford, MA). 0.5ml DMEM/0.2% FCS was added to the top chamber and 0.8ml DMEM/0.2% FCS was added to the bottom chamber. The system was equilibrated by incubating for 4 hours at 37 degrees celsius. Human PDGF-BB (PeproTech, Rocky Hill, NJ) (for RPE cells) or rat PDGF-BB (PeproTech) (for 3T3 cells) was added to the bottom chamber (0 ng/ml - 100ng/ml final concentration). The system was incubated 4 hours to 12 hours. Cells were scraped off on the top of filter with a Q-tip. The cells that migrated to the bottom of the filter were fixed with a mixture of cold 50% Methanol/ 50% Acetone for 3 minutes.

Following incubation filters were washed with 1X PBS and stained with Giemsa Stain (Matheson Coleman and Bell) for 1-2 hours and migration was visualized by taking pictures on a Zeiss Axiovert 200M microscope. Specifically, migration under four different conditions was visualized: (1) background migration observed in the absence of PDGF; (2) migration observed in the presence of 5 nM PDGF BB; (3) migration observed in the presence of 5 nM PDGF BB and 100 nM functional aptamer ARC127; and (4) migration observed in the presence of 5 nM of PDGF BB and 100 nM ARC 128 scrambled control aptamer. The visualized migration results show that the presence of 100 nM ARC 127 inhibits the effects of 5 nM PDGF-BB, shown by the migration of 3T3 or RPE cells at background levels. ARC 128 scrambled control displays no activity and the migration observed at this condition is equal to the one observed with 5 nM PDGF BB alone.

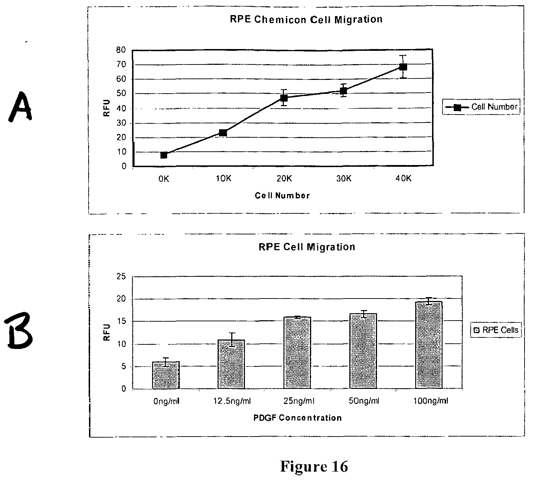

Figure 16A shows the results of a cell migration experiment performed in 96 well format using QCM Chemotaxis 96 Well Migration Assay (#ECM 510) (Chemicon, Temecula, CA). The 96 well format allowed for a more quantitative analysis of cell migration than the 24 well format, which was more qualitative. The 96 well assay starts by bringing plates and reagents to room temperature. 150ul of DMEM/ 0.2% FBS was added with or without chemoattractant to the wells of the feeder tray. 200,000 cells RPE cells in 100ul media DMEM/0.2 FBS were added into migration chamber and incubated for 1-24 hours at 37 degrees celsius.

Following incubation, the migration chamber plate was removed and the non-migratory cells were discarded. The number of migratory cells was quantitated according to manufacturer's recommended instructions. In brief, media from the top side was removed by flipping out the remaining cell suspension, and was placed onto a new feeder tray that contains 150ul of pre-warmed Cell Detachment Solution. This mixture was incubated for 30 minutes at 37 degrees celsius. CyQuant GR Dye 1:75 was diluted with 4X Lysis Buffer and 50ul of the Lysis Buffer/Dye Solution was added to each well containing 150ul of the solution containing migratory cells. This mixture was further incubated for 15 minutes at room temperature then transferred to a fresh 96 well plate and read at 480/520nm absorbance on a 96 well plate reader.

The results obtained in this 96 well format cell migration experiment (Figure 16A) confirmed the cell migration experiments done in 24 well format. ARC127 reduced migration levels to background and ARC128 scrambled aptamer did not affect migration. Figure 16B shows that the migration observed increases linearly as a function of cell number or concentration of mitogen added.

### EXAMPLE 8: Clonogenic Growth Assay

U87 glioblastoma cells (ATCC) were grown in the presence or absence of mitogen as well as aptamer as shown in the panels. U87 cells were plated in DMEM/10% FBS to 50% confluency in 100 mm dishes. Cells were incubated with PDGF-BB (PeproTech) +/- ARC 127 until cells appeared confluent (1-2 days). The addition of a final concentration of 50ng/ml PDGF BB alone caused the appearance of highly connected three dimensional cell clusters. Addition of 50 ng/ml PDGF-BB plus 10 nM to 100 nM functional aptamer ARC127 reduced the occurrence of clusters to background level. The presence of aptamer had no effect on proliferation rate of the cells determined by MTT assay. Thus, the aptamer blocks cell to cell adhesion of U87 cells which are known to have PDGF driven autocrine loops. ARC 127 seems to be blocking the cell surface displayed ligand binding to another cell's receptor as displayed by cell to cell adhesion.

### EXAMPLE 9: PDGF-drive ELK Luciferase Assay

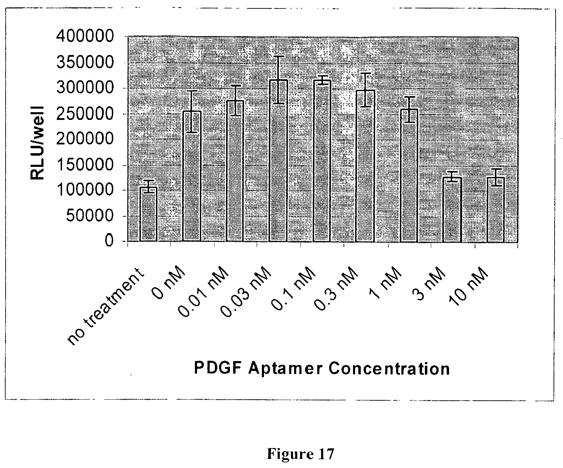

To further prove the activity of ARC 127 PDGF aptamer, a mechanistic Elk Luciferase reporter gene assays was set up. 10,000 3T3 cells/well were plated in DMEM/10% FBS in a 96 well plate. They were transfected using FuGene (Roche, Indianapolis, IN) at 3:1 ratio with 5 ng of ELK-1 (Stratagene, La Jolla, CA) and 20 ng of pFR-Luciferase plasmids (Stratagene, La Jolla, CA). When PDGF is added at a final concentration of 50 ng/ml to 3T3 cells, an increase in the Luciferase signal can be observed which corresponds to the effect of the mitogen on the reporter gene. Steady Glo Luciferase assay (Promega, Madison, WI) was used to detect luciferase. The results indicate that a concentration of ARC 127 as low as 3 nM reduces the Luciferase signal to no mitogen levels. IC50 of ARC127 deduced from this analysis is < 2 nM. (Figure 17.)

### EXAMPLE 10: In vivo Data in HT-29 LS174T and HCT-116 Colon Cancer Xenograft Models

*In vivo* efficacy studies were established to test the hypothesis that inhibition of PDGF-BB and/or its receptor with ARC127 and ARC308 increase the efficacy of cytotoxic drugs.

### HT-29 and LS147T Studies

Experimental overview. Human HT-29 or LS174T colon cancer xenotransplants were established in athymic nude mice (Nu/Nu mice, Charles River Labs, Wilmington, MA) by injecting mice with 1x10⁷ HT29 cells (ATCC, Manassas, VA), or 2x10⁶ LS174T cells (ATCC Manassas, VA) sub-cutaneously and allowing the tumors to grow for 5 days. On day 5, two dimensional measurements of the established tumors were taken using a digital caliper. Once tumor measurements were taken, the mice were randomized into groups such that the average tumor size was the same in each group. Once randomized, the mice were treated with irinotecan (Pfizer, NY, NY) which is a cytotoxic drug shown to have efficacy against colon cancer in the presence or absence of PDGF blockade using a PDGF specific aptamer or other small molecule inhibitor, to determine if PDGF blockade increases the efficacy of the cytotoxic drug.

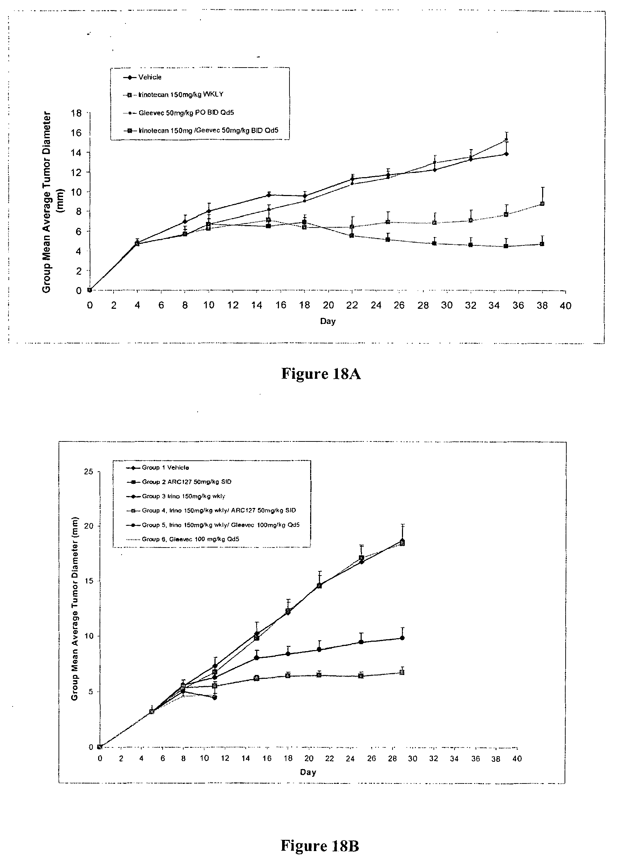

Materials and Methods. An experiment (Experiment 1) was designed to test a combination of known chemotherapeutic agents GLEEVEC™ and irinotecan in a dose optimization study in an HT29 colon cancer xenograft model. Table 7 below summarizes the experimental design of Experiment 1 using a human colon carcinoma cell line HT29 (ATCC, Manassas, VA), with a cytotoxic drug, irinotecan (Pfizer, NY, NY) administered at 150 mg/kg via intra-peritoneal injection once weekly, as well as a drug to block PDGF signaling, GLEEVEC™ (Novartis, Basel, Switzerland) dosed orally at 50 mg/kg twice daily (Monday through Friday). Though the mode of action of GLEEVEC™ is known to block the PDGF receptor function as well as other receptors for other growth factors, the effect is not necessarily PDGF specific. The results of this experiment are shown in Figure 18A showing a plot of group mean average tumor diameter in mm as a function of time for each of the treatment regimens irinotecan 150 mg/kg weekly, GLEEVEC™ 50 mg/kg orally BID Qd5, and irinotecan 150 mg/GLEEVEC™ 50mg/kg BID Qd5 combination therapies. These data show that GLEEVEC™ increases the efficacy of irinotecan alone in HT29 colon cancer xenotransplant model. GLEEVEC™-mediated PDGF blockade enhanced the efficacy of irinotecan treatment as demonstrated by the decreased rate of tumor growth as compared to animals treated with irinotecan alone. The results are statistically significant (two tailed students T-test).

**Table 7: Experiment 1: HT29 irinotecan/GLEEVEC Dose Optimization Study**

| | | | | | | | | | | | | | | |
|---|---|---|---|---|---|---|---|---|---|---|---|---|---|---|
| **Tumor Inoculation** | | | | | | **Test Article Administration** | | | | **Combination Therapy Administration (PLEASE DOSE AFTER TEST ARTICLE)** | | | | **Day of Euthanasia** |
| Group | No. of Animals | Test Material | Dose | Route | Day | Test Material | Dose (mg/kg) | Route | Day | Test Material | Dose (mg/k g) | Route | Day | |
| 1 | 10 | | | | | Diluent | DVE | | Days 7, 14, 21 | NA | NA | IP | SID Days 4-21 | |
| | | HT-29 | 1 × 10⁷ cells | SC | Day 0 | | | IP | | | | | | TBD |
| 2 | 10 | | | | | Diluent | DVE | | | ARC 127 | 50 | | | |
| 3 | 10 | | | | | Irinotecan | 150 | | | NA | NA | | | |
| 4 | 10 | | | | | Irinotecan | 50 | | | NA | NA | | | |
| 5 | 10 | | | | | Irinotecan | 150 | | | ARC 127 | 50 | | | |
| 6 | 10 | | | | | Irinotecan | 50 | | | ARC 127 | 50 | | | |
| 7 | 10 | | | | | Irinotecan | 150 | | | Gleevec | 100 | PO | | |
| 8 | 10 | | | | | Irinotecan | 50 | | | Gleevev | 100 | | | |
| 9 | | | | | | Diluent | DVE | | | Gleevec | 100 | | | |

| | | | | | | | | | | | | | | |
|---|---|---|---|---|---|---|---|---|---|---|---|---|---|---|
| 1. Cageside and clinical observations-daily 2. Body weights - biweekly 3. Tumor measurements - biweekly starting approximately on Day 7 4. Total irinotecan needed - 450 mg (for 3 doses) 5. Total Gleevec needed - 1350 mg (for 36 doses) 6. Total PDGF aptamer needed - 675 mg (for 18 doses) DVE = Dose Volume Equivalent of buffer HT29 = human colon carcinoma; ATCC #HTB-38 IP = Intraperitoneal PO = orally | | | | | | | | | | | | | | |

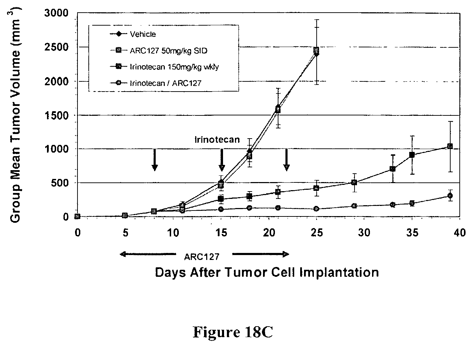

Another experiment (Experiment 2) was designed to test ARC 127 and irinotecan in a colon cancer xenograft model using human colon carcinoma cell line LS174T (ATCC, Manassas, VA). Cytotoxic drug, irinotecan (Pfizer, NY, NY) was dosed once weekly at 150 mg/kg) via intraperitoneal delivery. ARC 127 used to block PDGF signaling, was dosed at 50 mg/kg via intra-peritoneal delivery once daily, and GLEEVEC™ (Novartis, Basel, Switzerland) was dosed orally at 100 mg/kg once daily, Monday through Friday. ARC127 prevents PDGF-BB from binding the PDGF receptor; and has a 40K PEG attached. Table 8 below summarizes the experimental design for Experiment 2. The results from Experiment 2 are shown in Figure 18B and Figure 18C demonstrating that ARC 127 enhanced the efficacy of irinotecan treatment as demonstrated by the decreased rate of tumor growth as compared to animals treated with irinotecan alone. The results are statistically significant (two tailed students T-test). The data clearly show that ARC 127 increases the efficacy of irinotecan better than both GLEEVEC™/irinotecan combination treatment and irinotecan alone in LS174T colon cancer xenotransplant model. The GLEEVEC™ dosing regimen (100 mg/kg once daily, Monday through Friday, P.O.) made the animals moribund and these groups were terminated early in the experiment (Fig. 18B).

**Table 8. Experiment 2: ARC127/irinotecan dosing study in LS174T colon cancer xenograft model Experimental Design:**

| | | | | | | | | | | | | | | |
|---|---|---|---|---|---|---|---|---|---|---|---|---|---|---|
| **Tumor Inoculation** | | | | | | **Test Article Administration** | | | | **Combunation therapy Administration (PLEASE DOSE AFTER TEST** | | | | **Day of Euthanasia** |
| Group | No. of Animals | Test Material | Dose | Route | Day | Test Material | Dose (mg/kg) | Route | Day | Test Material | Dose (mg/kg) | Route | Day | |
| 1 | 10 | | | | | Diluent | DVE | | | NA | NA | IP | | |
| | | LS174T | 2×10⁶ cells | SC | Day 0 | | | IP | Days 8,15,22 | | | | SID Days 4-22 | TBD |
| 2 | 10 | | | | | Diluent | DVE | | | ARC 127 | 50 | | | |
| 3 | 10 | | | | | Irinotecan | 150 | | | NA | NA | | | |
| 4 | 10 | | | | | Irinotecan | 150 | | | ARC 127 | 50 | | | |
| 5 | 10 | | | | | Irinotecan | 150 | | | Gleevec | 100 | PO | | |
| 6 | 10 | | | | | Diluent | DVE | | | Gleevec | 100 | | | |

| | | | | | | | | | | | | | | |
|---|---|---|---|---|---|---|---|---|---|---|---|---|---|---|
| 1. Cageside and clinical observations - daily 2. Body weights - biweekly 3. Tumor measurements - biweekly starting approximately on Day 4 4. Total Irinotecan needed -337.5 mg (for 3 doses) 5. Total Gleevec needed -360 mg (for 18 doses) 6. Total PDGF aptamer needed -450 mg (for 18 doses) DVE = Dose Volume Equivalent of buffer LS174T = human colon carcinoma IP = Intraperitonea | | | | | | | | | | | | | | |

l

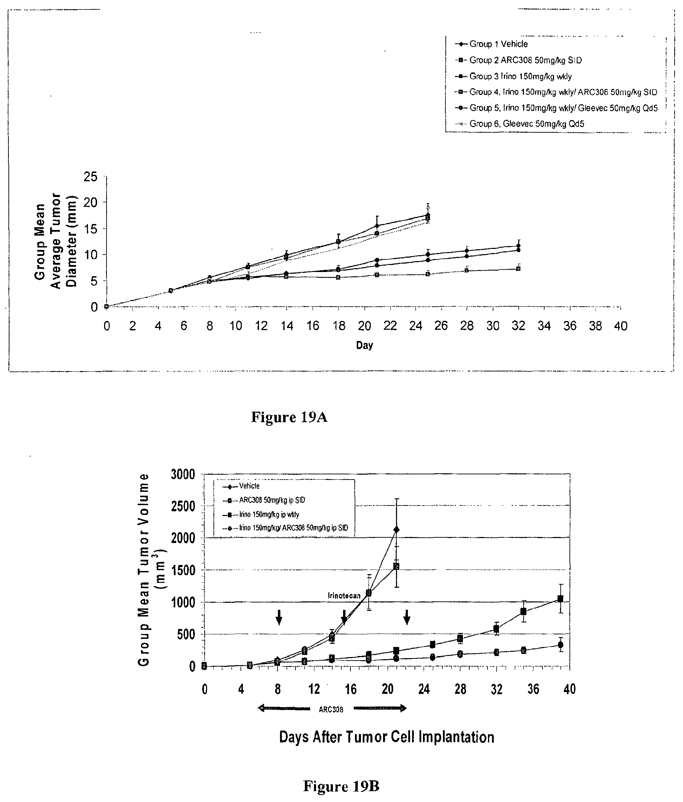

A third experiment (Experiment 3) was designed to test ARC308/irinotecan and GLEEVEC™/irinotecan dosing regimens in a colon cancer xenotransplant model using human colon carcinoma cell line LS174T (ATCC). Table 9 summarizes the experimental design for Experiment 3. Cytotoxic drug irinotecan (Pfizer, NY, NY) was dosed via intraperitoneal delivery at 150 mg/kg once weekly. ARC308 was dosed via intraperitoneal delivery at 50 mg/kg once daily, and GLEEVEC™ was dosed at 50 mg/kg once daily, Monday through Friday. ARC308 prevents PDGF-BB from binding the PDGF receptor; this molecule has a 30K PEG attached. The results from Experiment 3 are shown in Figures 19A and 19B which show that ARC308 enhanced the efficacy of irinotecan treatment as demonstrated by the decreased rate of tumor growth as compared to animals treated with irinotecan alone. In contrast the GLEEVEC™ dosing regimen (50 mg/kg once daily, Monday through Friday, P.O) did not enhance the efficacy of Irinotecan. The results are statistically significant (two tailed students T-test).

**Table 9. Experiment 3: ARC308/irinotecan and GLEEVEC/irinotecan study LS174T colon cancer xenograft model.**

| | | | | | | | | | | | | | | |
|---|---|---|---|---|---|---|---|---|---|---|---|---|---|---|
| **Tumor Inoculation** | | | | | | **Test Article Administration** | | | | **Combination therapy Administration (PLEASE DOSE AFTER TEST ARTICLE)** | | | | **Day of Euthanasia** |
| Group | No. of Animals | Test Material | Dose | Route | Day | Test Material | Dose (mg/kg) | Route | Day | Test Material | Dose (mg/kg) | Route | Day | |
| 1 | 10 | | | | | Diluent | DVE 11:7 | | | NA | NA | IP | | |
| | | LS174T | 2 × 10⁶ cells | SC | Day 0 | | | IP | Days 8,15,22 | | | | SID Days 5 - 22 | TBD |
| 2 | 10 | | | | | Diluent | DVE | | | ARC 308 | 50 | | | |
| 3 | 10 | | | | | Irinotecan | 150 | | | NA | NA | | | |
| 4 | 10 | | | | | Irinotecan | 150 | | | ARC 308 | 50 | | | |
| 5 | 10 | | | | | Irinotecan | 150 | | | Gleevec | 5U | PO | M-F | |
| b | 10 | | | | | Diluent | DVE | | | Gleevec | 50 | | | |

| | | | | | | | | | | | | | | |
|---|---|---|---|---|---|---|---|---|---|---|---|---|---|---|
| 1. Cageside and clinical observations - daily 2. Body weights - biweekly 3. Tumor measurements - biweekly starting approximately on Day 4 4. Total irinotecan needed - 337.5 rng (for 3 doses) 5. Total needed - 360 mg (for 18 doses) 6. Total PDGF aptamer needed - 450 mg (for 18 doses) DVE = Dose Volume Equivalent of buffer, LS174T = human colon carcinoma IP = Intraperitoneal | | | | | | | | | | | | | | |

An efficacy analysis of three treatment groups in this study is shown in Table 10 in terms of log cell kill and log cell kill net. Log cell kill is the log (base 10) of the number of tumor cells before treatment divided by the number of cells after treatment. Log cell kill is calculated as (T-C)/(3.32 x D), where T = the time in days for the treated group to reach a certain volume size tumor, C = the time in days for the control group to reach the same volume size tumor, and D = the time for the tumor, treated with control, to double in volume. A log cell kill less than 0.7 indicates no activity, while a value greater than 2.8 indicates high activity. Log cell kill net takes into account the treatment duration (R), which was 18 days, using the formula [(T-C)-R]/(3.32 x D). A log cell kill net less than 0 indicates no activity of the treatment compared with control, while a value greater or equal to 0 indicates activity. The target size was 1000 mm³, the doubling time was 3.25 days, and the control group was the group to which vehicle (0.9% sodium chloride in water) was administered. ARC308 alone showed no activity. While Irinotecan showed activity, addition of concomitant treatment with ARC308 (50 mg/kg) increased the activity determined by log cell kill and log cell kill net by 1.62 and 5.29 fold. The log cell kill and log cell kill net values for combined treatment with Irinotecan and ARC308 (50 mg/kg) were obtained by extrapolating the tumor size for this group to the target size, using a growth curve with the same slope as the Irinotecan-treated alone group, since the average volume of the combination treatment group never reached 1000 mm³ in the time period of the experiment.

**Table 10: Efficacy Analysis of ARC308.**

| Treatment | Log cell kill | Log cell kill net | Activity Assessment |
|---|---|---|---|
| ARC308 | 0 | -1.67 | Inactive |
| Irinotecan | 1.95 | 0.28 | Active |
| Irinotecan + ARC308 | 3.15 | 1.48 | Active |

In summary, these *in vivo* studies in HT-29 and LS174T colon cancer xenograft models confirm that PDGF blockade affected by the therapeutic aptamers of the present invention can increase the efficacy of irinotecan treatment.

A fourth experiment (Experiment 4) was designed to further test ARC308/irinotecan dosing regimens in a colon cancer xenotransplant model using human colon carcinoma cell line LS174T (ATCC). Table 11 summarizes the experimental design for Experiment 4.

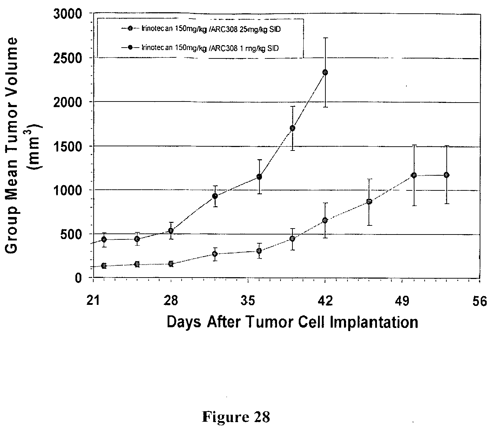

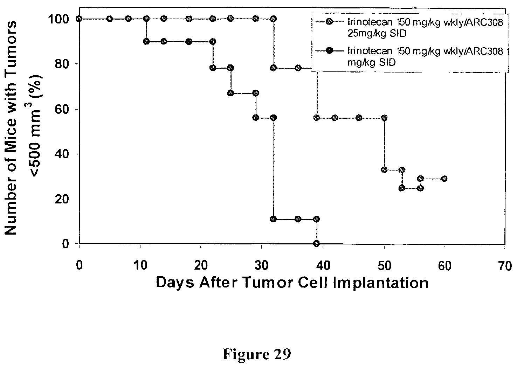

These studies again showed that ARC308 prevents PDGF-BB from binding the PDGF receptor and enhanced the efficacy of Irinotecan treatment as demonstrated by the decreased rate of tumor growth as compared to animals treated with Irinotecan alone in a dose dependent manner. As shown in Figure 28 ARC308 showed a dose dependent enhancement of the efficacy of Irinotecan treatment as demonstrated by the decreased rate of tumor growth in the group treated with Irinotecan plus ARC308 (25 mg/kg) as compared to animals treated with Irinotecan plus ARC308 (1 mg/kg). This was particularly evident in the post-dosing period (days 23-53) when the mice were not administered either Irinotecan or ARC308. Figure 29 is a Kaplan-Meier representation of the data shown in Figure 28 wherein the percentage of mice in a treatment group exhibiting tumors less than 500 mm³ [as calculated from digital caliper measurements of length and width of the tumors, using the following formula: volume = (length x width²)/2] is depicted. Once again a statistically significant difference is noted between the group treated with Irinotecan plus ARC308 (25 mg/kg) as compared to animals treated with Irinotecan plus ARC308 (1 mg/kg), supporting an ARC 308 dose dependent enhancement of the efficacy of Irinotecan. By day 38 all mice in the Irinotecan plus ARC308 (1 mg/kg) group had developed tumors greater than or equal to 500 mm³ while 56% the mice in the group treated with Irinotecan plus ARC308 at 25 mg/kg still exhibited tumors less than 500 mm³.

**Table 11: Experiment 4: ARC308/irinotecan study LS174T colon cancer xenograft model.**

| | | | | | | | | | | | | | | |
|---|---|---|---|---|---|---|---|---|---|---|---|---|---|---|
| **Tumor Inoculation** | | | | | | **Test Article Administration** | | | | **Combination Therapy Administration (PLEASE DOSE AFTER TEST ARTICLE)** | | | | **Day of Euthanasia** |
| Group | No. of Animals | Test material | Dose | Route | Day | lest Material | Dose (mg/kg) | Route | Day | Test Material | Dose (mg/k g) | Route | Day | |
| 1 | 10 | | | SC | | Diluent | DVE | | | Diluent | DVE | IP | | |
| | | LS174T | 2 x 10⁶ | | Day 0 | | | IP | Days 8,15,22 | | | | SID Days 5-22 | TBD |
| 2 | 10 | | cells | | | Diluent | DVE | | | ARC 308 | 50 | | | |
| 3 | 10 | | | | | Diluent | DVE | | | scrambled | 50 | | | |
| 4 | 10 | | | | | Irinotecan | 150 | | | Diluent | DVE | | | |
| 5 | 10 | | | | | Irinotecan | 150 | | | ARC 308 | 50 | | | |
| 6 | 10 | | | | | Irinotecan | 150 | | | ARC 308 | 25 | | | |
| 7 | 10 | | | | | irinotecan | 150 | | | ARC 308 | 10 | | | |
| 8 | 10 | | | | | Irinotecan | 150 | | | ARC 308 | 1 | | | |
| 9 | 10 | 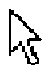 | | | | Irinotecan | 150 | | | ARC 308 | 50 | | SID days 7, 8, 14,15,21,22 | |

| | | | | | | | | | | | | | | |
|---|---|---|---|---|---|---|---|---|---|---|---|---|---|---|
| 1. Cageside and clinical observations - daily 2. Body weights - biweekly 3. Tumor measurements - biweekly starting approximately on Day 4 4. Total irinotecan needed - 562.5 mg (for 3 doses) 5. Total PDGF aptamer needed - 612 mg (for 18 doses); 75 mg for 6 doses; Grand total = 687 mg 6. Total Scrambled aptamer needed - 225 mg (for 18 doses) DVE = Dose Volume Equivalent of buffer LS174T = human colon carcinoma IP = Intraperitoneal | | | | | | | | | | | | | | |

A fifth xenograft study was performed to examine the effect of treating human tumors in athymic mice with ARC593 in comparison with vehicle. On day 0 LS 174t tumor cells from a human colorectal cancer cell line were implanted subcutaneously into the right flank of 8-9 week old female outbred athymic nude mice from Harlan (Indianapolis, Indiana). The resultant subcutaneous tumors were measured on day 6 with calipers to determine their length and width and the mice were weighed; tumor sizes were calculated using the formula (length x width²)/2 and expressed as cubic millimeters. Mice with tumors smaller than 7.2 mm³ and larger than 26.95 mm³ were excluded from the following group formation. Two groups of mice, 12 mice per group, were formed by randomization such that the group mean tumor sizes were essentially equal (mean of groups ± standard deviation of groups = 17.16 ± 0.16 mm³), with similar group standard deviations (mean of group standard deviations ± standard deviation of group standard deviations = 6.20 ± 1.0 mm³).

Each group was assigned to a treatment regimen as follows. All mice were dosed once per day beginning on day 6 with ARC593 or vehicle with the last dose administered on day 23. Doses were adjusted for changing body weight twice per week and all mice were dosed with vehicle or aptamer at 10 ml of dosing solution per kg body weight given intraperitoneally. Group 1 received normal saline as vehicle control; Group 2 received ARC593, 50 mg/kg of mouse body weight.

ARC593 dosing solutions were prepared by dissolving lyophilized aptamer into normal saline, adjusting the concentration of the dosing solution with normal saline until the correct concentration as determined by spectophotometric analysis was achieved, and sterile filtering the resultant solutions through a 0.22 µm filter into sterile sample vials which were then kept, frozen at -20°C and defrosted overnight for use the next day at 4°C. The aptamer was allowed to come to room temperature for an hour before dosing. Unused material was stored at 4°C for use the next day.

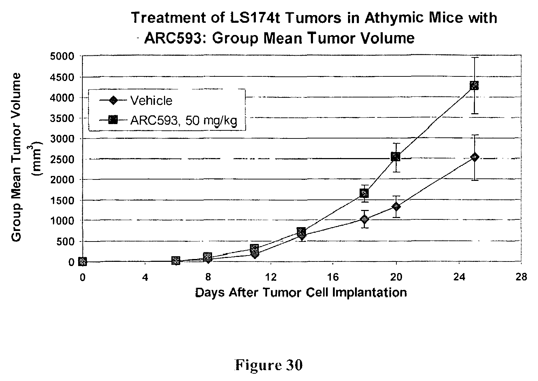

During and after the dosing period the mice were weighed and tumors measured twice per week. All results in terms of tumor volume are shown in Figure 30. Group 1 showed the expected tumor growth rate, with a doubling time of approximately 5 days. One mouse in Group 1 treated with vehicle was found dead on day 12. Death was due to intraperititoneal bleeding resulting from mechanical injury during Intraperitoneal dosing the previous day. That mouse was excluded from further data analysis. The group tumor volumes were analyzed by the Wilcoxon rank sum test for independent groups, with p<0.05 considered to be significant. On day 20, the last day that all mice were still alive in the aptamer-treated groups, the tumor volumes in Group 2 were significantly greater than those in Group 1. After day 20 three mice from Group 2 were sacrificed as their tumors exceeded IACUC mandated maximum volumes. The tumor volume enhancing effect of treatment with ARC593 was evident in a majority of the ARC593-treated mice.

As an indication of the toxicity of treatments, the change during the study in group mean corrected body weights were examined. Corrected body weight is the body weight of a mouse minus the approximate weight of the subcutaneous tumor. The approximate weight of the tumor is determined from the formula "weight (g) = volume (mm³)", with the volume determined as above. Corrected body weights thus determined reflect changes in the non-tumor body weight and are thus better indicators of toxicity-derived weight loss. All groups showed some decrease in corrected body weight as expected with the growth of this xenograft tumor. There was no significant difference between the vehicle or aptamer treated groups (Group 1, 2). Thus treatment with aptamer did not increase toxicity as measured by body weight.

A sixth xenograft study was performed to examine the effect of treating human tumors in athymic mice with ARC594 in comparison with vehicle. On day 0 US174t tumor cells from a human colorectal cancer cell line were implanted subcutaneously on the left shoulder of 8-9 week old female outbred athymic nude mice from Harlan (Indianapolis, Indiana). The resultant subcutaneous tumors were measured on day 12 with calipers to determine their length and width and the mice were weighed; tumor sizes were calculated using the formula (length x width²)/2 and expressed as cubic millimeters. Mice with tumors smaller than 49.69 mm³ and larger than 233.44 mm³ were excluded from the following group formation. Two groups of mice, 12 mice per group, were formed by randomization such that the group mean tumor sizes were essentially equal (mean of groups ± standard deviation of groups = 136.83 ± 1.2 mm³), with similar group standard deviations (mean of group standard deviations ± standard deviation of group standard deviations = 62.38 ± 5.03 mm³).

Each group was assigned to a treatment regimen as follows. All mice were dosed once per day beginning on day 12 with ARC594 or vehicle with the last dose administered on day 27. Doses were adjusted for changing body weight twice per week and all mice were dosed with vehicle or aptamer at 10 ml of dosing solution per kg body weight given intraperitoneally. Group 1 received normal saline as vehicle control; Group 2 received ARC594, 50 mg/kg of mouse body weight.

ARC594 dosing solutions were prepared by dissolving lyophilized aptamer into normal saline, adjusting the concentration of the dosing solution with normal saline until the correct concentration as determined by spectophotometric analysis was achieved, and sterile filtering the resultant solutions through a 0.22 µm filter into sterile sample vials which were then kept, frozen at -20°C and defrosted overnight for use the next day at 4°C. The aptamer was allowed to come to room temperature for an hour before dosing. Unused material was stored at 4°C for use the next day.

During and after the dosing period the mice were weighed and tumors measured twice per week. Group 1 showed the expected tumor growth rate, with a doubling time of approximately 7 days. The group tumor volumes were analyzed by the Wilcoxon rank sum test for independent groups, with p<0.05 considered to be significant. There was no significant difference between the mean tumor volumes of Group 1 and 2.

Upon further analysis of the data, it was noted that 2 mice (2-8 and 2-9) in Group 2 exhibited tumors with very fast growth rates and had to be sacrificed on day 24 as their tumors exceeded IACUC mandated maximum volumes. Their tumor doubling times were 2.3 and 1.6 days (for mouse 2-8 and 2-9, respectively), in comparison with the mean doubling time of Group 1 of 7 days and of all other members of Group 2 of 6 days. The tumors of two mice of Group 2 treated with ARC594 grew much faster than either the other members of Group 2 or Group 1.

### HCT-116 Study

A seventh xenograft study (Experiment 7) was performed to confirm the efficacy of ARC308 and ARC594 in inhibiting human tumor growth in athymic mice. On day 0 HCT-116 tumor cells from a human colorectal cancer cell line were implanted subcutaneously into the right flank of 8-9 week old female outbred athymic nude mice from Harlan (Indianapolis, Indiana) The resultant subcutaneous tumors were measured on day 8 with calipers to determine their length and width and the mice were weighed; tumor sizes were calculated using the formula (length x width²)/2 and expressed as cubic millimeters. Mice with tumors smaller than 62.65 mm³ and larger than 126.0 mm³ were excluded from the following group formation. Ten groups of mice, 12 mice per group, were formed by randomization such that the group mean tumor sizes were essentially equal (mean of groups ± standard deviation of groups = 82.67± 0.00 mm³), with similar group standard deviations (mean of group standard deviations ± standard deviation of group standard deviations = 21.34 ± 0.00 mm³). An additional group of 10 mice was also formed (mean ± standard deviation = 81.45± 24.87 mm³) as a group that received no treatment but was monitored for body eight and tumor volume (Group 1).

Each group was assigned to a treatment regimen as follows. All mice were dosed once per day beginning on day 8 with ARC308, ARC 594 or vehicle at 10 ml/kg, with the last dose administered on day 25. Doses were adjusted for changing body weight twice per week and all mice were dosed with vehicle or aptamer at 10 ml of dosing solution per kg body weight given intraperitoneally. Group 2 received normal saline as vehicle control; Groups 4 and 5 received ARC308, 50 mg/kg of mouse body weight; Groups 6 and 7 received ARC594, 50 mg/kg of mouse body weight; Group 8 received ARC594, 10 mg/kg of mouse body weight; and Group 9 received ARC594, 1 mg/kg of mouse body weight. Groups 3, 10 and 11 were dosed with vehicle intraperitinoneally once per day from days 8-25. Groups 3, 5, 7, 8, and 9 were dosed on day 11, 18 and 25 with irintoecan (100 mg/kg, intraperitoneally). Group 10 was dosed with imatinib (a small molecule drug which inhibits PDGFR as well as other tyrosine kinases) at 50 mg/kg orally twice a day at 12 hour intervals, beginning on day 8. Group 11 was dosed on day 11 and 25 with irintoecan (100 mg/kg, intraperitoneally) and twice a day with imatinib at 50 mg/kg given orally on days 8-12 and 23-25.

ARC308 and ARC594 dosing solutions were prepared by dissolving lyophilized aptamer into normal saline, adjusting the concentration of the dosing solution with normal saline until the correct concentration as determined by spectophotometric analysis was achieved, and sterile filtering the resultant solutions through a 0.22 µm filter into sterile sample vials which was then kept at 4°C for shipment, frozen at -20°C upon receipt by Piedmont, and defrosted overnight for use the next day at 4°C. The aptamer was allowed to come to room temperature for an hour before dosing. Unused material was stored at 4°C for use the next day. Normal saline for the vehicle control was sterile filtered into sample vials and stored in the same manner as the aptamer dosing solutions. Aptamer and vehicle dosing solutions were coded so that the personnel dosing were blinded from their identity. Irintoecan (Camptosar, NDC 009-7529) was prepared fresh for each dosing by dissolving the compound in 5% dextrose in water (pH 4.8). Gleevec (imantinib mesylate, NDC 0078-0373-866) was prepared fresh for each dosing by dissolving in 5% dextrose in water (pH 4.8).

During and after the dosing period the mice were weighed and tumors measured twice per week. Group 1 showed the expected tumor growth rate, with a doubling time of approximately 7 days. The group tumor volumes were analyzed by the Wilcoxon rank sum test for independent groups, with p<0.05 considered to be significant. There was no significant difference in tumor volumes between the no treatment (Group 1) and either vehicle (Group 2), ARC308 (50 mg/kg, Group 4) groups or ARC594 (50 mg/kg, Group 6). By day 17 the tumor volumes of the groups given irintoecan alone or with aptamer (Groups 3, 5, 7, 8, 9) were significantly smaller than the vehicle-treated group, and they remained so throughout the remainder of the study. The tumor volumes of the irintoecan plus ARC308 (50 mg/kg, Group 5) group were significantly smaller from those of the group treated only with irinotecan on day 21 and days 28-38, after which sufficiently few mice remained in the groups to allow comparison. The tumor volumes of the irintoecan plus ARC594 (50 mg/kg, Group 7) group were significantly smaller from those of the group treated only with irinotecan from days 28-52, after which sufficiently few mice remained in the groups to allow comparison. The tumor volumes of the irintoecan plus ARC594 (10 mg/kg, Group 8) group were significantly smaller from those of the group treated only with irinotecan from days 17-42. The tumor volumes of the irintoecan plus ARC594 (1 mg/kg, Group 9) group were not significantly smaller from those of the group treated only with irinotecan during this study. There was no significant tumor volume difference between the groups treated with irinotecan plus ARC308 (50 mg/kg, Group 5), irinotecan plus ARC594 (50 mg/kg, Group 7) or irinotecan plus ARC594 (10 mg/kg, Group 8). There was a significant difference in tumor volumes between irintoecan plus ARC594 (1 mg/kg, Group 9) and the group treated with irinotecan plus ARC594 (50 mg/kg, Group 7) on days 38-56. Thus the addition of ARC 308 or ARC594 to treatment with irinotecan did produce an increase in irinotecan efficacy as measured by reduced tumor volume in this xenpgraft study, and there was a dose response to treatment with ARC594.

There was no significant difference in tumor volumes between the vehicle (Group2) and Gleevec (Group 10) treated group. Addition of irinotecan to the Gleevec treatment produced unacceptable toxicity causing 4 of 12 mice to die by day 17. As a result treatment with both drugs was stopped for this group until day 23. The irintoecan plus Gleevec group actually showed significantly less efficacy as measured by tumor volumes form days 28-45.

As an indication of the toxicity of treatments, the change during the study in group mean corrected body weights were examined. Corrected body weight is the body weight of a mouse minus the approximate weight of the subcutaneous tumor. The approximate weight of the tumor is determined from the formula "weight (g) = volume (mm³)", with the volume determined as above. Corrected body weights thus determined reflect changes in the non-tumor body weight and are thus better indicators of toxicity-derived weight loss. All groups showed some decrease in corrected body weight as expected with the growth of this xenograft tumor. There was no significant difference between the no treatment, vehicle, either aptamer alone, or Gleevec alone treated groups

(Group 1, 2, 4, 5, 10). There was a significant difference with the addition of irintoecan to the treatment protocol noted during some timepoints in the study, but there was no difference between the groups receiving aptamer and irintoecan versus irinotecan alone, with the exception of day 17, when there was a significant decrease in body weight between the irinotecan plus ARC594 (50 mg/kg, Group 7) group that was no apparent at any other time. Thus addition of aptamer to irintoecan treatment did not increase toxicity, as measured by body weight.

### EXAMPLE 11: PDGF/VEGF Bi-Functional Aptamers as Oncology Therapeutics

Combination therapies have advantages over single-agent therapies in the treatment of solid tumors as shown in the colon cancer xenograft models with the aptamers of the present invention (Example 10). Similarly, aptamers that are able to bind to more than one of the targets that are implicated in solid tumor cancers are effective in potentiating the therapeutic effects of individual therapeutic agents. Aptamers such as these can inhibit multiple proteins (or other targets) and therefore provide a single compound that acts in a manner that is substantially equivalent to a combination of compounds. Multi-functional aptamers can be engineered, *e.g.,* from combinations of known aptamers. These multifunctional aptamers can be shown to bind to multiple targets, and can be generated either directly by solid-phase chemical synthesis, or by transcription from a corresponding DNA template. Examples of such multi-functional aptamers include aptamers capable of binding to VEGF and PDGF for cancer indications.

In order to design multifunctional aptamers from previously identifed aptamers (or regions of aptamers) it is important to conjoin the individual aptamers via regions of the individual aptamer that do not make contact with the target. This can typically be accomplished by identifying regions of the secondary structure which tolerate substitution of individual nucleotides at most or all positions. If structural units are required, such as a stem, then these can be preserved in the final design. Additionally, it is important that the structural integrity of each of the individual aptamers is preserved in the final folded structure. This can most easily be achieved by predicting the secondary structures of the original aptamer sequences using an algorithm such as mfold, and then ensuring that these predicted secondary structures are preserved, according to the same algorithm, when they are part of the conjoined structure. The general mfold algorithm for determining multiple optimal and suboptimal secondary structures is described by the author of the program, Dr. Michael Zuker (Science 244, 48-52 (1989)). A description of the folding parameters used in the algorithm is presented in Jaeger, Turner, and Zuker , Proc. Natl. Acad. Sci. USA, (1989) 86, 7706-7710 (*see* also M. Zuker, et al., Algorithms and Thermodynamics for RNA Secondary Structure Prediction: A Practical Guide in RNA Biochemistry and Biotechnology, J. Barciszewski and B.F.C. Clark, eds., NATO ASI Series, Kluwer Academic Publishers, (1999)). Other programs that can be used according to the methods of the present invention to obtain secondary structures of nucleic acid sequences include, without limitation, RNAStructure (Mathews, D.H.; Sabina, J.; Zuker, M.; and Turner, D.H., "Expanded Sequence Dependence of Thermodynamic Parameters Improves Prediction of RNA Secondary Structure," Journal of Molecular Biology, 288, 911-940(1999), and RnaViz (Nucleic Acids Res., Nov. 15; 25(22):4679-84 (1997).

Having determined the secondary structural motifs and units of the component aptamers, these are combined into a single oligonucleotide that folds in a manner that preserves the functionalities of each of the constituent aptamers. As a result, multifunctional aptamers are capable of binding to multiple targets, and can be generated either directly by solid phase chemical synthesis, or by transcription from a corresponding DNA template.

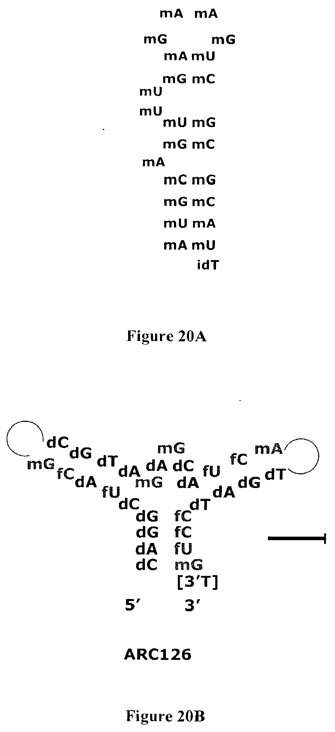

VEGF and PDGF Bifunctional Aptamers. The methods of the present invention were applied to generate an aptamer having binding specificity to PDGF BB and to VEGF. This multi-functional aptamer was generated by joining two aptamers individually identified using SELEX - one (ARC 245, SEQ ID NO. 7) which recognized VEGF but not PDGF (*see* Figure 20A) and one (ARC126, 5'-(SEQ ID NO:1)-HEG-(SEQ ID NO:2)-HEG-(SEQ ID NO:3)-3'-dT-3') which recognized PDGF but not VEGF (*see* Figure 20B). ARC245 (SEQ ID NO. 7) is a fully 2'O methylated (2'-OMe) aptamer with binding specificity to VEGF with a K_{D} of about 2 nM. The ARC126 used in the multivalent aptamer is entirely DNA with binding specificity to PDGF with a K_{D} of about 100 pM.

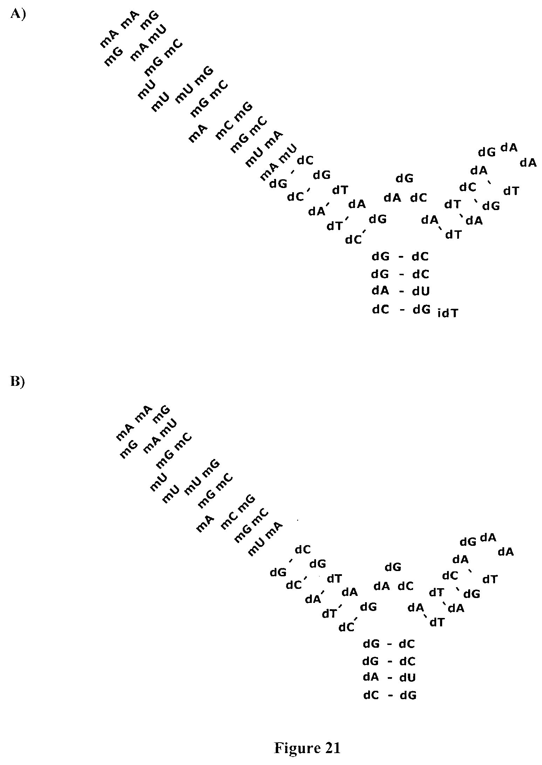

A schematic of the structure and sequence of the multivalent aptamer capable of binding to PDGF and VEGF resulting from this combination, sequence TK.131.012.A (SEQ ID NO. 9), is shown in Figure 21 A. A second multivalent aptamer capable of binding to VEGF and PDGF, sequence TK.131.012.B (SEQ ID NO. 10), was also formed by combining ARC245 (SEQ ID NO. 7) and ARC 126 (Figure 21B). As shown in Figure 21B, in this VEGF-PDGF multivalent aptamer the first mA-mU pair of the stem of ARC245 was removed before joining ARC245 and all deoxy-ARC126. In each case, as shown in Figure 21, one of the ARC 126 PEG linkers was removed for the addition of the VEGF specific aptamer and the other was substituted with an oligonucleotide linker having the sequence dGdAdAdA (SEQ ID NO:97).

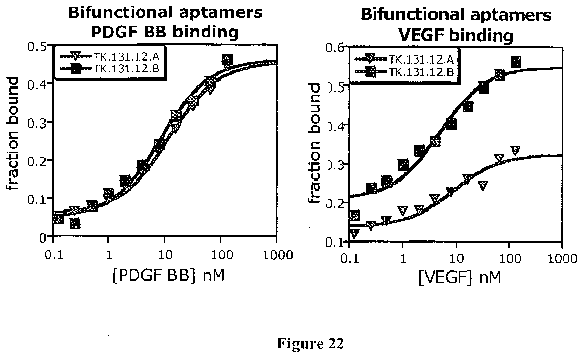

Binding data for the constituent aptamers and the multivalent aptamers were collected by dot-blot assays in which radio-labeled aptamer is incubated with protein target and then forced through a sandwich of nitrocellulose over nylon and the results shown in Figure 22. Protein-associated radio-label is captured on the nitrocellulose membrane while the balance of the radio-label is captured on the nylon membrane. The radio-label data is collected on a phosphorimaging plate. This data is then used to calculate the binding coefficients. The multivalent aptamers TK.131.012.A (SEQ ID NO. 9) and TK.131.012.B (SEQ ID NO. 10) were made synthetically.

The multivalent aptamers with the sequence TK.131.012.A (SEQ ID NO.9) shows a K_{D} of about 10 nM for PDGF and about 10 nM for VEGF. The multivalent aptamer with the sequence TK.131.012.B (SEQ ID NO. 10) shows a K_{D} of about 10 nM for PDGF and about 5 nM for VEGF.

### EXAMPLE 12: PDGF and VEGF Aptamers Containing Immunostimulatory Motifs

To test the ability of aptamers comprising CpG motif(s) to stimulate the innate immune response, a murine CpG motif was engineered into an aptamer specific for PDGF-B. These aptamers were then used in *in vitro* mouse cell-based assays to confirm functionality of the CpG motifs (*e.g*., stimulation of the release of IL-6 and TNF-α. These aptamers were also used in aptamer mechanism-based biological activity assays (PDGF-B signaling through the MAPK pathway as measured by the effect of the CpG-PDGF-B-aptamer on PDGF-B activation of ERK Phosphorylation) to confirm functionality of the CpG motif comprising aptamer upon binding of the aptamer to its target, in this case PDGF-B.

To generate the aptamers disclosed herein with an embedded or appended CpG motif, the sequence of previously identified immunostimulatory oligonucleotides comprising CpG motifs ("ISS-ODN" or "ODN") or fragments thereof were engineered into ARC124, an aptamer identified through the SELEX process that binds to PDGF AB and BB with a K_{d} of approximately 100pm. The sequence of ARC124 is shown below.
**ARC124**
5' CACAGGCTACGGCACGTAGAGCATCACCATGATCCTGTG3'InvdT (SEQ ID NO:11)

The various ODN sequences, both full length and fragments or derivatives thereof are shown below 5'→ 3' from left to right (wherein an * indicates a phosphorothioate bond and 3'InvdT indicates an inverted T at the 3' end). In addition to these ODN sequences or fragments thereof being engineered into PDGF aptamers, they were also used for controls in assaying the ability of these aptamers to stimulate the immune response.
**ISS-ODN** (SEQ ID NO: 12)
   T*G*A*C*T*G*T*G*A*A*C*G*T*T*C*G*A*G*A*T*G*A
**ISS-ODN2** (SEQ ID NO: 13)
   T*G*A* A*C*G* T*T*C* G*A*G* A*T*
**ISS-ODN3** (SEQ ID NO: 14)
   A* A*C*G* T*T*C* G*A*G* A*T*
**ISS-ODN4** (SEQ ID NO:15)
   A* A*C*G* T*T*C* G*A*G
**ISS**-**ODN5** (SEQ ID NO:16)
   G*T*G*A*A*C*G*T*T*C*G*A*G
**ODN 2006** (SEQ ID NO: 17)
   T*C*G* T*C*G* T*T*T* T*G*T* C*G*T* T*T*T* G*T*C* G*T*T
**ODN 2006.2** (SEQ ID NO: 18)
   G* T*C*G*T*T*T* T*G*T*C*G*T*T*T*T* G*T
**ODN 2006.3** (SEQ ID NO: 19)
   G*T*C*G*T*T*T*T*G*T*C*G*T*T

ISS-ODN was identified from Martin-Orzco et al., Int. Immunol., 1999. 11 (7): 1111-1118. ISS-ODN 2-5 are fragments of ISS-ODN. ODN 2006 was identified from Hartmann et al., Eur. J. Immunol. 2003. 33:1633-1641. ODN 2006.2 and 2006.3 are fragments of ODN 2006.

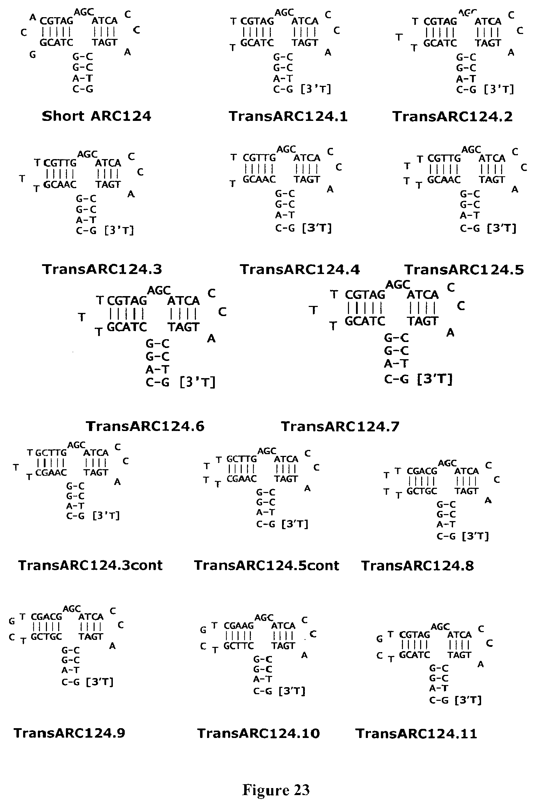

The sequences of the PDGF aptamers comprising a CpG motif are shown below 5'→ 3' from left to right (wherein an * indicates a phosphorothioate bond and 3'lnvdT indicates an inverted T at the 3' end). A schematic of the sequence and secondary structure of these aptamers is shown in Figure 23.
**CpGARC124short a**.**k**.**a**. **shortARC124** (SEQ ID **NO:20**) 
A*A*C* G*T*T* C*G*A* G* CA GGC TAC GGC ACG TAG AGC ATC ACC ATG
ATC CT*G* C/3InvdT/
**LongCpGARC124 a.k.a. longARC124** (SEQ ID NO:21) 
G*T*G* A*A*C* G*T*T* C*G*A* G* CA GGC TAC GGC ACG TAG AGC ATC
ACC ATG ATC CT*G* C/3InvdT/
**FullCpGARC124 also referred to herein as fullARC124** (SEQ ID NO:22) 
T*G*A* C*T*G* T*G*A* A*C*G* T*T*C* G*A*G* A*T*G* A* CA GGC TAC
GGC ACG TAG AGC ATC ACC ATG ATC CT*G* T*T*T* T*T*T* T/3InvdT/
**TransARC124.1** (SEQ ID NO:23)
   C*A*G*GCTAC*G*T*T*C*GTAGAGCATCACCATGATC*C*T*G*/3InvdT/
**TransARC124.2** (SEQ ID NO:24)
   C*A*G*GCTAC*G*T*T*T*C*GTAGAGCATCACCATGATC*C*T*G*/3InvdT/
**TransARC124.3** (SEQ ID NO:25)
   C*A*G*GCAAC*G*T*T*T*C*GTTGAGCATCACCATGATC*C*T*G*/3InvdT/
**TransARC124**.**4** (SEQ ID NO:26)
   C*A*G*GCAAC*G*T*T*C*GTTGAGCATCACCATGATC*C*T*G*/3InvdT/
**TransARC124.5** (SEQ ID NO:27)
   C*A*G*GCAAC*G*T*T*T*T*C*GTTGAGCATCACCATGATC*C*T*G*/3InvdT/
**TransARC124.6** (SEQ ID NO:28)
   C*A*G*GCTACGTTTCGTAGAGCATCACCATGATC*C*T*G*/3InvdT/
**TransARC124.7** (SEQ ID NO:29)
   C*A*GGCTACGTTTCGTAGAGCATCACCATGATCC*T*G*/3InvdT/
**TransARC124.8** (SEQ ID NO:30)
   C*A*G*GCGTCGTTTTCGACGAGCATCACCATGATC*C*T*G*/3InvdT/
**TransARC124.9** (SEQ ID NO:31)
   C*A*G*GCGTCGTCGTCGACGAGCATCACCATGATC*C*T*G*/3InvdT/
**TransARC124.10** (SEQ ID NO:32)
   C*A*G*GCTTCGTCGTCGAAGAGCATCACCATGATC*C*T*G*/3InvdT/
**TransARC124.11** (SEQ ID NO:33)
   C*A*G*GCTACGTCGTCGTAGAGCATCACCATGATC*C*T*G*/3invdT/

As indicated above, ISS-ODN and ODN 2006 are phosphorothioated oligonucleotides that are reported to be immunostimulatory and their sequences are derived from the literature. Truncated versions of these oligonucleotides were designed by shortening them from both their 5' and 3' ends. The resulting constructs are ISS-ODN2 through 5 and ODN 2006.2 through 3. Each of the newly designed constructs was tested in IL-6 release assay to assess the effect on immunostimulatory ability. The shortest construct that still retained the ability to induce IL-6 release was picked for the sequence appended either to the 5' or 3' end of ARC 124. Constructs that are labeled as CpGARC 124short a.k.a. shortARC124, LongCpGARC124 a.k.a. longARC124, and FullCpGARC124 a.k.a. fullARC124 correspond to constructs where ISS-ODN4 is appended to the 5'-end of ARC 124. These constructs carry phosphorothioated bonds at their 5' and 3' ends in addition to inverted T at their 3'-end to assure the adequate stability in the cell-based assay but the core middle part corresponding to ARC 124 is free of phosphorothioate bonds.

ARC124 at position 8-9 and 13-14 has two naturally occurring CpG islands. Constmcts with the nomenclature of TransARC124.1 through TransARC124.11 correspond to constructs that made use of these two naturally occurring CpG sequences to create an immunostimulatory sequence that would maximize the CpG response based on reports in the literature that CpG motifs that are embedded in varying lengths of Ts create maximal immunostimulatory effect. Thus, the changes that were performed on ARC 124 consisted of substituting the non-essential GCA bulge at position 10-12 with various T residues. The effect of varying the T bulge on the immunostimulatory effect as well as addition of phosphorothioated bonds on the stability of the construct are assessed with IL-6 release assay and phosphorylation of ERK as described herein.

For negative controls, ARC 124 was engineered to remove the CpG motifs. These control sequences are shown below.
**TransARC124.3control (CpG-Scrambled Aptamer)** (SEQ ID NO:34)
   C*A*G*GCAAG*C*T*T*T*G*CTTGAGCATCACCATGATC*C*T*G*/3InvdT/
**TransARC124.5control (CpG-Scrambled Aptamer)** (SEQ ID NO:35)
   C*A*G*GCAAG*C*T*T*T*T*G*CTTGAGCATCACCATGATC*C*T*G*/3InvdT/

The activity of PDGF aptamers containing CpG islands of the present invention were tested in cell based assays. Supernatants of J774A.1 cells (TIB cells), a mouse macrophage cell line (ATCC #TIB-67) in the presence of CpG motifs will contain more IL-6 and TNF-alpha than cells not in the presence of CpG islands. Thus, an IL-6 and TNF-alpha solid phase ELISA was used to quantify the levels of IL-6 and TNF-alpha released into the supernatants (both from R&D System, Minneapolis, MN) upon exposure to various oligonucleotides comprising CpG sequences. For the IL-6 ELISA, a monoclonal antibody specific for mouse IL-6 was pre-coated onto a 96 well microplate. An enzyme-linked polyclonal antibody specific for mouse IL-6 was used to detect any bound IL-6 from the cell supernatants. For the TNF-alpha ELISA, an affinity purified polyclonal antibody specific for mouse TNF-alpha was pre-coated onto a 96 well microplate. An enzyme-linked polyclonal antibody specific for mouse TNF-alpha was used to detect any bound TNF-alpha from the cell supernatants. Both ELISAs used a colorimetric readout quantified by spectrophotometry.

J774A.1 cells (TIB cells) were cultured in Dulbecco's Modified Eagle Media (DMEM) with 10% fetal bovine serum (FBS) (Invitrogen, Carlsbad, CA) at 37 °C, 5%CO₂. 100,000 cells were plated into an appropriate number of wells on a 24 well plate one day prior to the experiment. The CpG embedded PDGF aptamers were incubated with the cells for 24 and 48 hours at 37 °C, 5% COY. Final aptamer concentrations were 1uM and 10uM. Supernatants were collected at the indicated time-points and centrifuged for 8 minutes at 5,000 rpm at 4 °C. Centrifuged supernatants were frozen at -20 °C until use in the IL-6 or TNF-alpha ELISA. Both ELISAs were used according to manufacturer's recommendations. CpG motif containing oligonucleotides previously reported to be immunostimulatory, (ISS ODN and ODN2006) and LPS (Sigma), were used as positive controls and non-CpG containing aptamers were used as negative controls. Figure 24A shows the results of an IL-6 ELISA measuring IL-6 release in TIB cells using only the standard ODN's as positive controls, and aptamers which contain no CpG islands as negative controls in the assay. Positive and negative controls only were used in this experiment to establish whether this assay was robust enough to measure CpG induced IL-6 release. The data shown in Figure 24B demonstrate that the ISS ODN and shortened versions of the ISS ODN induce IL-6 release in TIB cells better than ODN2006 and shortened versions thereof. Figure 24C shows the short, long, and full versions of ARC 124 embedded with CpG motifs induces IL-6 release in TIB cells as well as the ISS ODN. The data shown in Figure 24D shows that TransARC124.1-TransARC124.7 (SEQ ID NO:23 - SEQ ID NO:29) induce IL-6 release in TIB cells. The data shown in Figure 24E shows TransARC124.1-TransARC124.7 induce TNF-alpha release in TIB cells.

The results of the IL-6 release and TNF-alpha release assays show that when CpG motifs are incorporated into existing aptamers, in this case ARC 124 (SEQ ID NO: 11), the aptamer is capable of eliciting a CpG response.

### EXAMPLE 13 : CpG Island Containing PDGF Aptamers in ERK phosphorylation Assay

ERK phosphorylation was used to test whether ARC 124 (SEQ ID NO:11) retained its functionality after the incorporation of CpG motifs into the aptamer sequence. One hundred and fifty thousand 3T3 cells (a mouse fibroblast cell line which contains PDGF-R) were plated into an appropriate number of wells on a 12 well plate one day prior to the experiment and serum starved in 0.5% FBS overnight. Cells were treated with 10ng/ml PDGF-BB (R&D Systems, Minneapolis, MN) in the presence or absence of CpG-island containing PDGF aptamers for 30 minutes. Cells were collected and lysed with lysis buffer containing 10mM Tris pH 7.5, 100mM NaCl, 0.125% NP-40, 0.875% Brij 97, 1.5mM sodium vanadate, and 1 mini-EDTA free protease inhibitor tablet. Protein concentration in cell lysates was determined using BIO-RAD protein assay reagent according to manufacturer's recommendations (Bio-Rad, Hercules, CA). Lysates were prepared by adding NuPage LDS Sample Buffer 4X with 0.1M DTT to a final concentration of 1X (Invitrogen, Carlsbad, CA) and incubated at 70 °C for 7 minutes. Forty micrograms of total protein was loaded into each well of a NuPage 10% Bis-Tris gel (Invitrogen, Carlsbad, CA). The gel was run at 100 mAmp for 1 hour. Gel was then transferred to a HyBond ECL nitrocellulose membrane (Amersham, Piscataway, NJ) at 250 mAmp for 3 hours. After transfer, the nitrocellulose was blocked with 5% BSA (Sigma, St. Louis, MO) in PBS for one hour at room temperature. The nitrocellulose membrane was incubated with a p44/42 MAP Kinase antibody overnight at 4°C (Cell Signaling Technology, Beverly, MA). The nitrocellulose membrane was washed 3X with PBS containing 1% Tween 20 and then incubated with an anti-rabbit IgG, HRP-conjugated antibody for 1 hour (Amersham, Piscataway, NJ). After the one hour incubation, the nitrocellulose membrane was washed 3X with PBS containing Tween-20. Membrane was developed using an ECL + Western Blotting Detection System according to manufacturer's recommendations (Amersham, Piscataway, NJ) and scanned using a STORM 860 (Amersham). 3T3 cells in the presence of PDGF-BB without aptamer was used as a positive control.

The results show that shortARC124 and longARC124, both PDGF aptamers carrying known CpG motifs, are still functionally active and can block phosphorylation of ERK upon binding to PDGF.

Collectively, the data show that when CpG motifs are incorporated into existing aptamers, the aptamer is capable of eliciting a CpG response and still maintain the ability to block non-CpG target (e.g., PDGF) driven effects (e.g., ERK-MAPK phosphorylation) with the same potency as native aptamers.

### EXAMPLE 14: PDGF-AA Selection

### PDGF-AA SELECTION SUMMARY

One selection for the short form of PDGF-AA (Roche Biomedical) was completed using a 2-fluoro pyrimidine containing pool. Round 1 of the selection began with incubation of 2x10¹⁴ molecules of 2'F pyrimidine modifed ARC 212 pool (5' GGGAAAAGCGAAUCAUACACAAGA-N40-GCUCCGCCAGAGACCAACCGAGAA 3') (SEQ ID NO: 74), including a spike of α³²P ATP body labeled pool, with 50 pmoles of PDGF-AA protein in a final volume of 100 µL for 1hr at room temperature. The selection was perfonned in 1X SHMCK buffer, pH 7.4 (20mM Hepes pH 7.4, 120mM NaCl, 5mM KC1, 1mM MgCl₂, 1mM CaCl₂). RNA:PDGF-AA complexes and free RNA molecules were separated using 0.45um nitrocellulose spin columns from Schleicher & Schuell (Keene, NH). The column was pre-washed with 1 ml 1X SHMCK, and then the RNA:protein-containing solution was added to the column and spun in a centrifuge at 1500g for 2 min. Buffer washes were performed to remove nonspecific binders from the filters (Round 1, 2 x 500 µL 1X SHMCK; in later rounds, more stringent washes (increased number of washes and volume) were used to enrich for specific binders, then the RNA:protein complexes attached to the filters were eluted with 2x200ul washes (2 x100 µL washes in later rounds) of elution buffer (7M urea, 100 mM sodium acetate, 3mM EDTA, pre-heated to 95 °C). The eluted RNA was phenol:ch(oroform extracted, then precipitated (40 µg glycogen, 1 volume isopropanol). The RNA was reverse transcribed with the ThermoScript RT-PCR™ system according to their instructions, using the primers KMT.108.38.C (5' TAATACGACTCACTATAGGGAAAAGCGAATCATACACAAGA 3') (SEQ ID NO: 75) and KMT.108.38.D (5' TTCTCGGTTGGTCTCTGGCGGAGC 3') (SEQ ID NO: 76), followed by amplification by PCR (20mM Tris pH 8.4, 50mM KCl, 2mM MgCl₂, 0.5µM KMT.108.38.C, 0.5µM KMT.108.38.D, 0.5mM each dNTP, 0.05 units/µL Taq polymerase (New England Biolabs)). The PCR template was purified using the QIAquick PCR purification kit (Qiagen). Templates were transcribed using α³²P ATP body labeling overnight at 37°C (4% PEG-8000,40 mM Tris pH 8.0, 12 mM MgCl₂, 1 mM spermidine, 0.002 % Triton x-100, 3 mM 2'OH purines, 3mM 2'F pyrimidines, 25mM DTT, inorganic pyrophosphatase, T7 Y639F single mutant RNA polymerase, 5uCi α³²P ATP). The reactions were desalted using Bio Spin columns (Bio-Rad) according to the manufacturer's instructions.

Subsequent rounds were repeated using the same method as for round 1, but with the addition of a negative selection step. Prior to incubation with protein target, the pool RNA was passed through 0.45 micron nitrocellulose to remove filter binding sequences, then the filtrate was carried on into the positive selection step. In alternating rounds, the pool RNA was gel purified. Transcription reactions were quenched with 50 mM EDTA and ethanol precipitated then purified on a 1.5 mm denaturing polyacrylamide gels (8 M urea, 10% acrylamide; 19:1 acrylamide:bisacrylamide). Pool RNA was removed from the gel by electroelution in an Elutrap® apparatus (Schleicher and Schuell, Keene, NH) at 225V for 1 hour in 1X TBE (90 mM Tris, 90 mM boric acid, 0.2 mM EDTA). The eluted material was precipitated by the addition of 300mM sodium acetate and 2.5 volumes of ethanol.

The RNA remained in excess of the protein throughout the selection (∼1 µM RNA). The protein concentration was 500 nM for the first 4 rounds, and then was dropped gradually over the successive rounds. Competitor tRNA was added to the binding reactions at 0.1 mg/mL starting at Round 2. A total of 11 rounds were completed, with binding assays performed at select rounds. Table 12 contains the selection details including pool RNA concentration, protein concentration, and tRNA concentration used for each round. Elution values (ratio of CPM values of protein-bound RNA versus total RNA flowing through the filter column) along with binding assays were used to monitor selection progress.

**Table 12. Conditions used for PDGF-AA (human short form) selection sfh-PDGFAA**

| **Round #** | **RNA pool conc (uM)** | **protein type** | **protein conc (uM)** | **tRNA conc (mg/mL)** | **neg** | **%elution** | **PCR cycle #** |
|---|---|---|---|---|---|---|---|
| 1 | 3.3 | sfhPDGFAA | 0.5 | 0 | none | 0.92 | 8 |
| 2 | ∼1 | sfhPDGFAA | 0.5 | 0.1 | NC | 0.24 | 15 |
| 3 | ∼1 | sfhPDGFAA | 0.5 | 0.1 | NC | 0.46 | 12 |
| 4 | ∼1 | sfhPDGFAA | 0.5 | 0.1 | NC | 0.1 | 15 |
| 5 | 1 | sfhPDGFAA | 0.4 | 0.1 | NC | 1.39 | 10 |
| 6 | ∼1 | sfhPDGFAA | 0.4 | 0.1 | NC | 0.5 | 8 |
| 7 | 1 | sfhPDGFAA | 0.3 | 0.1 | NC | 1.23 | 8 |
| 8 | ∼1 | sfhPDGFAA | 0.3 | 0.1 | NC | 0.44 | 10 |
| 9 | 1 | sfhPDGFAA | 0.3 | 0.1 | NC | 5.05 | 8 |
| 10 | ∼1 | sfhPDGFAA | 0.2 | 0.1 | NC | 0.83 | 10 |
| 11 | 1 | sfhPDGFAA | 0.2 | 0.1 | NC | 4.32 | 7 |

### Protein Binding Analysis

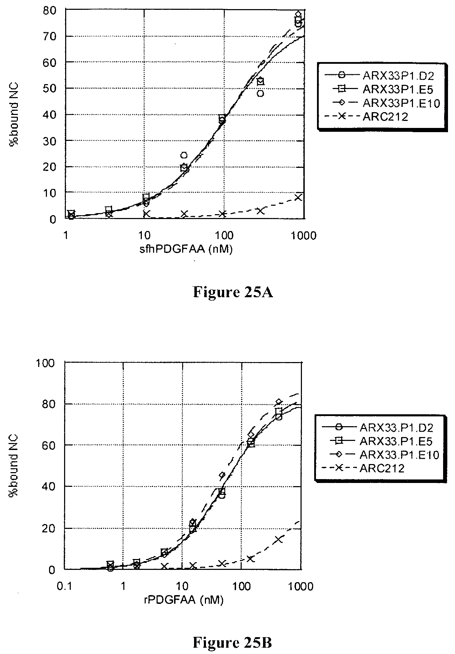

Dot blot binding assays were performed throughout the selection to monitor the protein binding affinity of the pool. Trace ³²P-labeled RNA was combined with PDGF-AA and incubated at room temperature for 30min in 1X SHMCK plus 0.1mg/ml tRNA for a final volume of 20 µl. The reaction was added to a dot blot apparatus (Schleicher and Schuell Minifold-1 Dot Blot, Acrylic), assembled (from top to bottom) with nitrocellulose, nylon, and gel blot membranes. RNA that is bound to protein is captured on the nitrocellulose filter, whereas the non-protein bound RNA is captured on the nylon filter. When a signifcant positive ratio of binding of RNA in the presence of PDGF-AA versus in the absence of PDGF-AA was seen, the pool was cloned using the TOPO TA cloning kit (lnvitrogen, Carlsbad, CA) according to the manufacturer's instructions. The Round 10 pool template was cloned, and 17 sequences were obtained. Only five different sequences were seen, with two major families and one unique sequence (not of the two families). For K_{d} determination, the clone RNA transcripts were 5'end labeled with γ-³²P ATP. K_{d} values were determined using the dot blot assay and fitting an equation describing a 1:1 RNA:protein complex to the resulting data (Kaleidagraph, Figures 25A and 25B). Results of protein binding characterization are tabulated in Table 13. Clones with high affinity to PDGF-AA were prepped and screened for functionality in cell-based assays.

**Table 13. Clone binding activity***

| **R10** | | | |
|---|---|---|---|
| **PDGF-AA Clones** | | | |
| **#** | **Clone Name** | **PDGF-AA (human short form) Kd (nM)** | **PDGF-AA (rat) Kd (nM)** |
| 1 | ARX33P1.D1 | N.B. | N.B. |
| 2 | ARX33P1.D2 | 104.5 | 51.7 |
| 3 | ARX33P1.E5 | 117.0 | 57.4 |
| 4 | ARX33P1.E10 | 132.9 | 47.1 |
| 5 | ARX33P1.E11 | N.B. | N.B. |

| | | | |
|---|---|---|---|
| N.B. = no significant binding observed *All measurements were done in the presence of 0.1 mg/mL tRNA alongside the naïve pool ARC 212 (which showed no significant binding). When tRNA was not included in the reactions, measured K_{d} values were approximately 2-3-fold lower (i.e. higher affinity). | | | |

### PDGF-AA Aptamers

The sequences for PDGF-AA short form aptamers of the invention are presented in Table 14. In the aptamers of the invention derived under the conditions of this selection, the pyrimidines (C and U) are fluorinated at the 2' position.

**Table 14: Sequence Information for PDGF-AA (short form) aptamers**

| |
|---|
| **ARX33P1.D1** (SEQ ID NO: 77) |
| GGGAAAAGCGAAUCAUACACAAGAUCGCCAGGAGCAAAGUCACGGAGGAGU GGGGGUACGAAUGCUCCGCCAGAGACCAACCGAGAA |
| |
| **ARX33P1.D2** (SEQ ID NO: 78) |
| GGGAAAAGCGAAUCAUACACAAGACCGGGAACUCGGAUUCUUCGCAUGUGG AUGCGAUCAGUAUGCUCCGCCAGAGACCAACCGAGAA |
| |
| **ARX33P1.E5** (SEQ ID NO: 79) |
| GGGAAAAGCGAAUCAUACACAAGACCGGAACUCGGAUUCUUCACAUGUGG AUGUGAUCAGUAUGCUCCGCCAGAGACCAACCGAGAA |
| |
| **ARX33P1.E10** (SEQ ID NO: 80) |
| GGGAAAAGCGAAUCAUACACAAGACCGGAAACUCGGAUUCUUCGCAUGUGG AUGCGAUCAGUAUGCUCCGCCAGAGACCAACCGAGAA |
| |
| **ARX33P1.E11** ((SEQ ID NO: 81) |
| GGGAAAAGCGAAUCAUACACAAGAGAGUGGAGGAGGUAUGUAUGGUUUGU GCGUCUGGUGCGGUGCUCCGCCAGAGACCAACCGAGAA |

### Cell Based Assays with PDGF-AA Aptamers

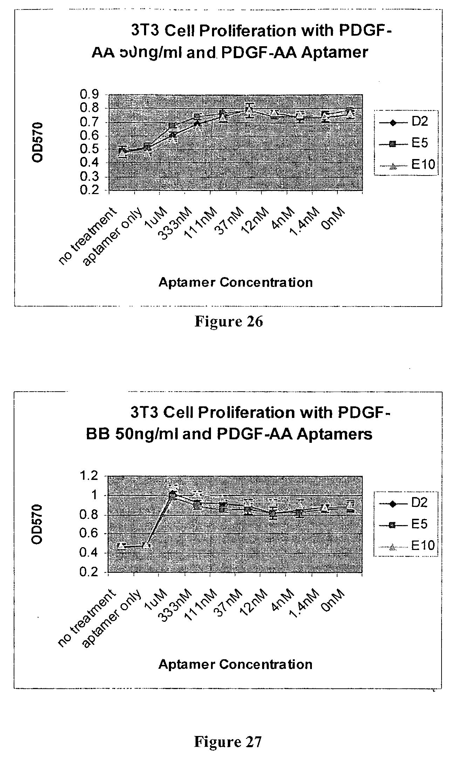

The PDGF-AA aptamers that showed *in vitro* binding were tested in the 3T3 proliferation assay for their ability to inhibit PDGF-AA induced 3T3 cell proliferation. The assay was set up as previously described, using a titration of PDGF-AA aptamer (0-1 uM) against a constant concentration (50 ng/ml) of PDGF-AA protein (R&D Systems). The results in Figure 26 show that the PDGF-AA aptamers ARX33P1.D2, ARX33P1.E5, and ARX33P1.E10 do inhibit PDGF-AA induced 3T3 cell proliferation, but are not very potent. Figure 27 shows that PDGF-AA aptamers have no effect on PDGF-BB induced 3T3 cell proliferation, indicating that the PDGF-AA aptamers are highly specific for the PDGF-AA isoform.

### EXAMPLE 15: Daily Dose study of ARC308 and ARC594 in Mouse Lewis Lung Carcinoma Model

A 1mm³ piece of Lewis lung carcinoma was implanted under the dorsal skin of wild-type C57BL/6 mice and allowed to grow. Beginning on day five after implantation, five mice were treated for seven days, intraperitoneally, as follows: five mice were treated with vehicle (saline) once daily, five mice were treated with the aptamer ARC308 at 50 mg/kg, once daily, and five mice were treated with the aptamer ARC594 at 50mg/kg, once daily. ARC308 is also referred to herein as AX101 and ARC594 is also referred to herein as AX102. At the end of the seven day period, the mice were anesthetized, and the following tissues were fixed by vascular perfusion with 1 % paraformaldehyde, removed and frozen for cryotstat sectioning: tumor (Lewis lung carcinoma), pancreas, liver, kidney, trachea, spleen, jejunum, and thyroid tissue. The resulting slides were stained with antibodies against various markers as described below.

### Anti-CD31 and anti-α-SMA antibody staining

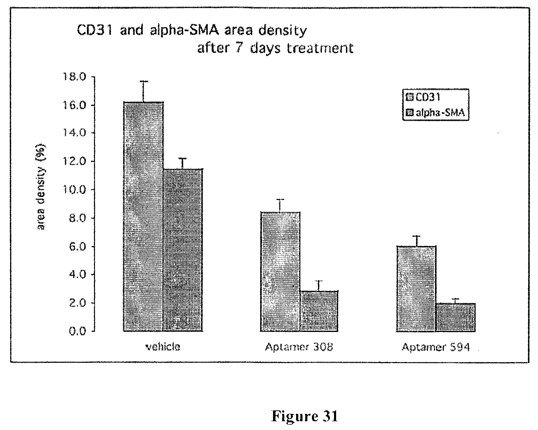

Sections of tumors were cut (80 µm) and stained with either anti-CD31 or anti-α smooth muscle actin ("α - SMA ") antibodies. Digital (fluorescence microscopic images were obtained for area density measurements by using a CoolCam low-light 3-chip RGB CCD camera. Results of this experiment are depicted in Figure 31. In addition, the tumors were imaged by confocal microscopy.

A fluorescence threshold value of 45 to 50 (fluorescence intensity = 0 <255) was determined to most accurately represent the area density of CD31 and α-SMA staining in fluorescence microscopic images of Lewis lung carcinoma (section thickness 80µm). Mean values for area density (mean % of tumor surface area ± S.E; n= 3-5 mice per group) for CD31-stained blood vessels and α-SMA immunoreactive pericytes are summarized in Table 15 below.

**Table 15: Area Densities**

| Treatment | CD31 mean | CD31 se | α-SMA | α-SMA se | count |
|---|---|---|---|---|---|
| Vehicle | 16.21 | 1.47 | 11.48 | 0.73 | 3 |
| ARC308 | 8.42 | 0.92 | 2.87 | 0.71 | 5 |
| ARC594 | 6.05 | 0.68 | 2.03 | 0.32 | 4 |

The results are statistically significant as assessed using ANOVA (analysis of variance in between groups) followed by Fisher and Bonferroni's test for multiple comparisons, *see,* Nouchedehi JM, White RJ, Dunn CD. Comput. Programs Biomed. 1982 14(2):197-205, as shown in Tables 16 and 17 below.

**Table 16: Fisher's PLSD for Area Density**

| Effect: group | | | | | |
|---|---|---|---|---|---|
| Significance level: 5% | | | | | |
| | | Mean Diff. | Crit. Diff. | P-Value | |
| ARC308-CD31 | veh-CD31 | -7.797 | 2.557 | <.0001 | S |
| ARC594-CD31 | veh-CD31 | -10.165 | 2.696 | <.0001 | S |
| ARC308-CD31 | ARC594-CD31 | 2.368 | 2.557 | 0.0676 | |
| ARC308-SMA | veh-SMA | -8.612 | 2.784 | <.0001 | S |
| ARC594-SMA | veh-SMA | -9.459 | 2.912 | <.0001 | S |
| ARC308-SMA | ARC594-SMA | 0.846 | 2.557 | 0.4969 | |

**Table 17*: Bonferroni/Dunn for Area Density**

| Effect: group | | | | | |
|---|---|---|---|---|---|
| Significance level: 5% | | | | | |
| | | Mean Diff. | Crit. Diff. | P-Value | |
| ARC308-CD31 | veh-CD31 | -7.797 | 4.098 | <.0001 | S |
| ARC594-CD31 | veh-GD31 | -10.165 | 4.32 | <.0001 | S |
| ARC308-CD31 | ARC594-CD31 | 2.368 | 4.098 | 0.0676 | |
| ARC308-SMA | veh-SMA | -8.612 | 4.462 | <.0001 | S |
| ARC594-SMA | veh-SMA | -9.459 | 4.666 | <.0001 | S |
| ARC308-SMA | ARC594-SMA | 0.846 | 4.098 | 0.4969 | |

| | | | | | |
|---|---|---|---|---|---|
| *Comparisons in this table are not significant unless the corresponding p-value is less than 0.0033. | | | | | |

These results demonstrate that both aptamers significantly reduced the area density of CD31 stained blood vessels and α-SMA positive pericytes in Lewis lung carcinoma. ARC308 reduced tumor blood vessel density by 50%, while ARC594 reduced it by 60%. The α-SMA immunoreactive pericyte area of density was reduced by about 75% with either aptamer. Moreover, confocal imaging revealed that Lewis lung carcinoma that had not been treated with aptamer had abundant vessels with abnormally loose pericytes, while treatment with either aptamer resulted in surviving vessels either having no pericytes or pericytes having the normal tight association with tumor vessels.

### Anti-CD31 and anti type IV collagen antibody staining

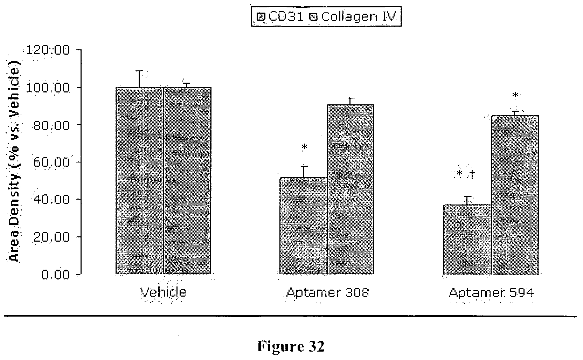

Sections of tumors were cut (80 µm) and stained with either anti-CD31 or anti-type IV collagen antibodies. Digital fluorescence microscopic images were obtained for area density measurements by using a CoolCam camera. Results of this experiment are depicted in Figure 32. In addition, the tumors were imaged by confocal microscopy.

A fluorescence threshold value of 40 to 50 (fluorescence intensity = 0 <255) was determined to most accurately represent the area density of CD31 and α-SMA staining in fluorescence microscopic images of Lewis lung carcinoma (section thickness 80µm). The results are summarized in Table 18 below. Mean values for area density (mean % of tumor surface area ± S.E; n= 3-5 mice per group) for CD31-stained blood vessels and type IV collagen stained basement membrane.

**Table 18**

| **Treatment** | **CD31 mean** | **CD31 se** | **Coll IV mean** | **Coll IV se** |
|---|---|---|---|---|
| **Vehicle** | 100.00 | 9.09 | 100.00 | 2.06 |
| **ARC308** | 51.91 | 5.70 | 90.99 | 3.47 |
| **ARC594** | 37.31 | 4.18 | 85.14 | 2.58 |

The significance of difference between groups was assessed using ANOVA followed by Fisher's post-hoc test for multiple comparisons as shown in Table 19 below.

**Table 19:**

| **Fisher's PLSD for % of** | | | | |
|---|---|---|---|---|
| **Effect:** | | | **P-Value** | |
| | | **Crit. Diff.** | | |
| **Sianificance Level:** | **Mean DifffsDiff** | | | |
| ARC308-CD31,Veh- | **-** | 15.3 | <.000 | S |
| ARC594-CD31. Veh- | - | 16.2 | <.000 | S |
| ARC308-CD31, ARC594- | 14.6 | 15.3 | 0.0 | |
| ARC308-CollIV, ARC594- | 5.8 | 15.3 | 0.4 | |
| ARC308-Co | - | 14.5 | 0.2 | |
| ARC594-CollIV, Veh- | - | 15.3 | 0.0 | |

These results indicate that inhibition of PDGF-B leads to a decrease in blood vessel density with minor effects on the vascular basement membrane. ARC308 and ARC594 reduced blood vessel area density by 48% and 63%, respectively, indicating that ARC594 was more effective than ARC 308 under the conditions of the experiment. While the effect of PDGF-B inhibition on type IV collagen was relatively small (9% and 15%, respectively, with ARC308 and 594), the reduction in type IV collagen produced by ARC594 was significant. While not wishing to be bound by theory, this effect may indicate a secondary effect on the vascular basement membrane.

### Anti-CD31, anti-PDGFR-β, anti-α-SMA, anti-NG2 and anti-desmin antibody combination staining

Sections of tumors in mice that had been treated with vehicle, ARC308 or ARC594, were cut (80 µm) and stained with anti-CD31 and anti-PDGFR-β, anti-CD31 and anti- α-SMA, anti-CD31 and anti-NG2 antibodies or anti-CD31 and anti-desmin antibodies. Pericyte expression was measured using the Cool Cam and confocal images were also taken.

A fluorescence threshold value of 40-50 was determined to most accurately represent the immunoreactivities of CD31, PDGF-β α-SMA, NG2 and desmin of Lewis lung carcinoma in the digital images of the experiment (80 µm thick sections).

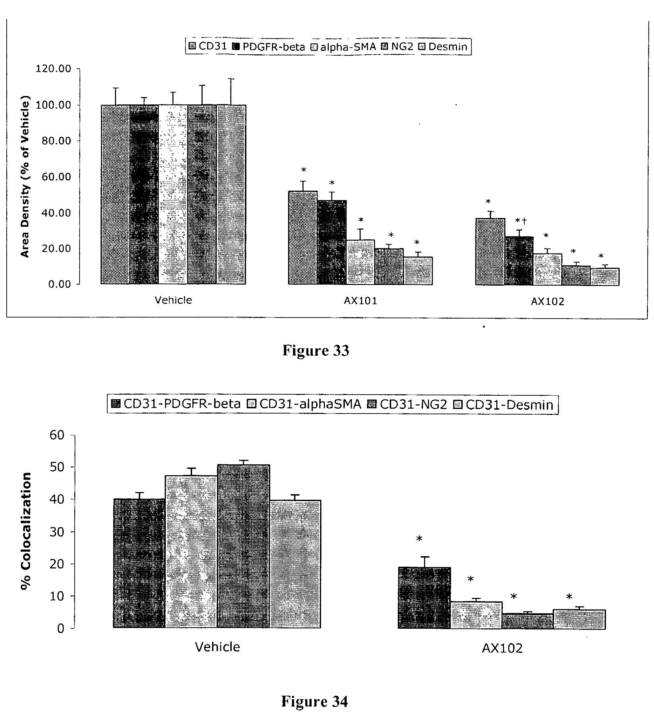

The results are presented in Figure 33 and summarized in Table 20 below. Values are mean area densities ± S.E. (n = 4-5 mice per group) expressed as % of vehicle. The significance of differences between groups was assessed using ANOVA followed by Fisher's post-hoc test for multiple comparisons.

**Table 20:**

| **Treatment** | **CD31 mean** | **CD31 se** | **PDGFR-beta mean** | **PDGFR-beta se** | **alpha-SMA mean** | **alpha-SMA se** |
|---|---|---|---|---|---|---|
| **Vehicle** | 100.00 | 9.09 | 100.00 | 3.59 | 100.00 | 6.37 |
| **ARC308** | 51.91 | 5.70 | 47.04 | 4.53 | 25.01 | 6.14 |
| **ARC594** | 37.31 | 4.18 | 27.18 | 3.75 | 17.64 | 2.81 |

| **Treatment** | **NG2 mean** | **NG2 se** | **Desmin mean** | **Desmin se** | **Count** | |
|---|---|---|---|---|---|---|
| **Vehicle** | 100.00 | 10.26 | 100.00 | 13.97 | 3 to 5 | |
| **ARC308** | 20.12 | 2.41 | 15.60 | 2.61 | 5 | |
| **ARC594** | 11.10 | 1.87 | 9.82 | 1.79 | 4 to 5 | |

The significance of differences between groups was assessed using ANOVA followed by Fisher's post-hoc test for multiple comparisons as shown in Table 21 below (AX101 refers to ARC308 and AX102 refers to ARC594 in Table 20 below as well as wherever used in the present application and figures).

**Table 21:**

| **Fisher's PLSD for % of Vehicle** | | | | |
|---|---|---|---|---|
| **Effect: Group** | | | | |
| **Significance Level: 5%** | | | | |
| | **Mean Diff.** | **Crit. Diff.** | **P-Value** | |
| AX101-CD31, Veh-CD31 | -48.09 | 15.38 | <.0001 | **S** |
| AX102-CD31, Veh-CD31 | -62.69 | 16.22 | <.0001 | **S** |
| AX101-CD31, AX102-CD31 | 14.61 | 15.38 | 0.06 | |
| AX101-APB5, Veh-APB5 | -52.96 | 15.38 | <.0001 | **S** |
| AX102-APB5, Veh-APB5 | -72.82 | 15.38 | <.0001 | **S** |
| AX101-APB5, AX102-APB5 | 19.86 | 14.50 | 0.01 | **S** |
| AX101-SMA, Veh-SMA | -74.99 | 16.75 | <.0001 | **S** |
| AX102-SMA, Veh-SMA | -82.36 | 17.52 | <.0001 | **S** |
| AX101-SMA, AX102-SMA | 7.37 | 15.38 | 0.34 | |
| AX101-NG2, Veh-NG2 | -79.88 | 15.38 | <.0001 | **S** |
| AX102-NG2,Veh-NG2 | -88.90 | 15.38 | <.0001 | **S** |
| AX101-NG2, AX102-NG2 | 9.02 | 14.50 | 0.22 | |
| AX101-Desmin, Veh-Desmin | -84.40 | 15.38 | <.0001 | **S** |
| AX102-Desmin, Veh-Desmin | -90.18 | 16.22 | <.0001 | **S** |
| AX101-Desmin, AX102-Desmin | 5.78 | 15.38 | 0.46 | |

These results indicate that treatment with PDGF-B aptamer 308 or 594 (50 mg/kg, ip, daily for 7 days) decreased the density of blood vessels by as much as 63% and caused an even greater reduction in pericytes, with decreases of 73, 82, 89 and 90% found with three immunohistochemical markers (PDGFR-β, α-smooth muscle actin, NG2, and desmin, respectively). Confocal images confirmed these findings and showed that the aptamer treatment left some tumor vessels without pericytes and others with pericytes that were more tightly associated with endothelial cells than found in untreated tumors.

### Colocalization of CID31, PDGFR-beta, α-SMA, NG2 and desmin markers

Sections of tumors treated with vehicle or ARC594 were cut (20 µm) and stained with: anti-CD31 and anti-PDGFR-beta antibodies, anti CD31 and anti-α-SMA antibodies, anti-CD31 and anti-NG2 antibodies, and anti-CD31 and anti-desmin antibodies. The extent of pericyte colocalization with endothelial cells was then measured on digital Cool Cam images.

A fluorescence threshold value of 40-50 was determined to most accurately represent the area densities of CD31 and NG2 of Lewis lung carcinoma in the digital images of the experiment (20 µm thick sections).

The results are depicted in the graph of Figure 34 and summarized in Table 22 below. Values are percent colocalization ± S.E. (n = 4 per group).

**Table 22:**

| **Colocalization** | **CD31-PDGFR mean** | **CD31-PDGFR se** | **CD31-SMA mean** | **CD31-SMA se** | |
|---|---|---|---|---|---|
| **Vehicle** | 40.01 | 2.00 | 47.27 | 2.27 | |
| **ARC594** | 19.11 | 3.34 | 8.42 | 1.13 | |
| | | | | | |

| **Colocalization** | **CD31-NG2 mean** | **CD31-NG2 se** | **CD31-Desmin mean** | **CD31-Desmin se** | **Count** |
|---|---|---|---|---|---|
| **Vehicle** | 50.68 | 1.42 | 39.67 | 1.68 | 4 |
| **ARC594** | 4.82 | 0.50 | 6.11 | 0.88 | 4 |

The significance of differences between groups was assessed using ANOVA followed by Fisher's post-hoc test for multiple comparisons, shown in Table 23 below.

**Table 23:**

| **Fisher's PLSD for Colocalization** | | | | |
|---|---|---|---|---|
| **Effect: Group** | | | | |
| **Significance Level: 5 %** | | | | |
| | **Mean Diff.** | **Crit. Diff.** | **P-Value** | |
| AX102-PDGFR, Veh-PDGFR | -20.90 | 5.43 | <.0001 | **S** |
| AX102-SMA, Veh-SMA | -38.85 | 5.43 | <.0001 | **S** |
| AX102-NG2, Veh-NG2 | -45.86 | 5.43 | <.0001 | **S** |
| AX102-Desmin, Veh-Desmin | -33.57 | 5.43 | <.0001 | **S** |

These results indicate that in Lewis lung carcinoma under baseline conditions about half of the endothelial surface of blood vessels was covered by pericytes (40-51 %). However, when the tumors were treated for 7 days with ARC594, the number of tumor vessels was reduced by ∼ 60%, and the pericyte coverage of the surviving tumor vessels was reduced to 5-19%. These findings indicate that most tumors vessels surviving PDGF-B aptamer treatment lack pericytes. However, a certain fraction of tumor vessels were lined with pericytes exhibiting a "normalized" phenotype with respect to pericyte-endothelial cell interactions. Of the surviving tumor vessels enveloped by pericytes were normalized in the sense that the pericytes are more closely associated with endothelial cells than in untreated tumors. This effect of ARC594 is very different from the effect of VEGF signaling inhibitors, where most surviving tumor vessels are covered by pericytes. The data presented herein indicate that treatment of solid tumors with anti-PDGF-B agents such as ARC594 results in the survival of at least two types of tumor vessels, 1) those with little to no pericytes attached to or lining the vessel wall, and 2) those that exhibit a more normalized phenotype with more tightly associated pericytes. These results suggest that the use of anti-PDGF-B agents, such as ARC594, may alter the tumor vasculature thereby rendering the remaining vessels more susceptible to treatment with a combination of agents such as anti-VEGF, anti-vascular agents, other anti-angiogenic agents or cytotoxic agents. Thus, combination therapy a PDGF aptamer (*e.g.,* PDGF-B) in combination with known cancer therapies is an effective method for treating a variety of solid tumors.

Images of aptamer treated Lewis Lung Carcinoma suggest that pericytes are not uniformly distributed on surviving tumor vessels. The remaining vessels appear to lack pericyte coverage (most common) or appear to have heavy pericyte coverage (less common).

### CD31 and NG2 colocalization on individual tumor vessels

20-µm sections of Lewis lung carcinomas treated with either vehicle or ARC594 were stained with anti-CD31 and anti-NG2 antibodies. The extent of pericyte colocalization with endothelial cells was measured on digital CoolCam images. Individual vessels were examined in the images to determine the distribution of vessels covered in pericytes or void of pericytes.

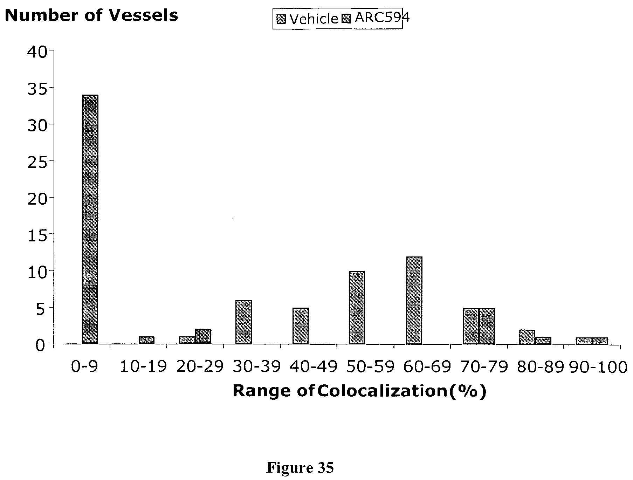

A threshold value of 40-50 was determined to most accurately represent the area densities of CD31 and NG2 in Lewis lung carcinoma (the sections were 20 µm thick). The results are depicted in the graph of Figure 35 and summarized in Table 24 below. Values are percent colocalization (n = 3 mice per group, 12-15 vessels per mouse).

**Table 24:**

| **Distribution** | **Number Vessels in that Range** | |
|---|---|---|
| **x-axis = Range of Colocaliztion (%)** | **Vehicle** | **ARC594** |
| 0-9 | 0 | 34 |
| 10-19 | 0 | 1 |
| 20-29 | 1 | 2 |
| 30-39 | 6 | 0 |
| 40-49 | 5 | 0 |
| 50-59 | 10 | 0 |
| 60-69 | 12 | 0 |
| 70-79 | 5 | 5 |
| 80-89 | 2 | 1 |
| 90-100 | 1 | 1 |
| **Total vessels examined** | **42** | **44** |

These results indicate that in Lewis lung carcinoma under baseline conditions, there is an even distribution of pericyte coverage on tumor blood vessels. Treatment with ARC594 for seven days, however, disrupts this distribution and one detects vessels covered with very few pericytes or vessels that are covered with pericytes. There is, however, virtually nothing in between.

### Example 16: Dose-Response of ARC594 in Mouse Lewis Lung Carcinoma Model

A 1mm³ piece of Lewis lung carcinoma was implanted under the dorsal skin of wild-type C57BL/6 mice and allowed to grow. Beginning on day five after implantation, five mice were treated for seven days, intraperitoneally, as follows: five mice were treated with vehicle (saline) once daily, five mice were treated with the aptamer ARC594 at 2 mg/kg, once daily, five mice were treated with ARC594 at 10 mg/kg once daily, and five mice were treated with ARC594 at 50 mg/k once daily. At the end of the seven day period, each of the mice was anesthetized, and the following tissues were fixed by vascular perfusion, removed and frozen for cryotstat sectioning: tumor (Lewis lung carcinoma), pancreas, liver, kidney, trachea, spleen, jejunum, heart, brain and thyroid. Sections of tumors were cut and stained with antibodies to markers as described below. Digital fluorescence microscopic images were obtained for area density measurements by using a CoolCam images. In addition, the tumors were imaged by confocal microscopy.

### Anti-CD31 and anti-type IV collagen antibody staining

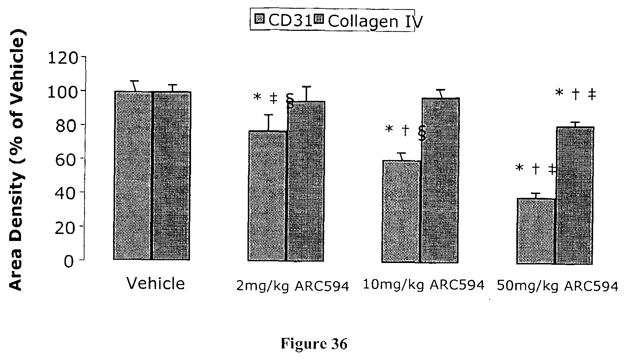

80-µm sections of Lewis lung carcinoma were stained with anti-CD31 and anti-type IV collagen antibodies. A fluorescence threshold value of 40 was determined to most accurately represent the area density of CD31 and type IV collagen immunoreactivities in Lewis lung carcinoma (section thickness 80µm). Results are shown graphically in Figure 36 and summarized in Table 25 below. Values are mean area densities ± S.E; (n= 3-5 mice per group) expressed as % of mean value for vehicle.

**Table 25:**

| **Treatment** | **CD31 mean** | **CD31 se** | **Coll IV mean** | **Coll IV se** | **Count** |
|---|---|---|---|---|---|
| **Vehicle** | 100 | 5.96 | 100 | 3.64 | 5 |
| **2mg/kgARC594** | 77.16 | 9.27 | 95.11 | 8.03 | 3 |
| **10mg/kg ARC594** | 60.67 | 4.35 | 97.68 | 4.72 | 3 |
| **50mg/kgARC594** | 39.14 | 2.65 | 81.55 | 2.58 | 5 |

The significance of differences between groups was assessed using ANOVA followed by Fisher's post-hoc test for multiple comparisons as shown in Table 26 below:

**Table 26:**

| **Fisher's PLSD for % vs vehicle** | | | | |
|---|---|---|---|---|
| **Effect: Group** | | | | |
| **Significance Level: 5 %** | | | | |
| | **Mean Diff.** | **Crit. Diff.** | **P-Value** | |
| 10mg/kg-CD31, 2mg/kg-CD31 | -16.49 | 15.75 | 0.04 | **S** |
| 10mg/kg-CD31, 50mg/kg-CD31 | 21.53 | 13.31 | 0.00 | **S** |
| 10mg/kg-CD31, Veh-CD31 | -39.33 | 13.64 | <.0001 | **S** |
| 2mg/kg-CD31, 50mg/kg-CD31 | 38.03 | 13.31 | <.0001 | **S** |
| 2mg/kg-CD31, Veh-CD31 | -22.84 | 13.64 | 0.00 | **S** |
| 10mg/kg-CollIV, 2mg/kg-CollIV | 2.56 | 15.75 | 0.74 | |
| 10mg/kg-CollIV, 50mg/kg-CollIV | 16.13 | 13.31 | 0.02 | **S** |
| 10mg/kg-CollIV, Veh-CollIV | -2.32 | 13.31 | 0.73 | |
| 2mg/kg-CollIV, 50mg/kg-CollIV | 13.57 | 13.31 | 0.05 | **S** |
| 2mg/kg-CollIV, Veh-CollIV | -4.89 | 13.31 | 0.46 | |

The results indicate that the reduction of blood vessels induced by the inhibition of PDGF-B is dose dependent. The magnitude of the decrease in CD31 immunoreactive blood vessels increased with dose of ARC594. Reduction in type IV collagen immunoreactivity was only seen at the highest dose (50 mg/kg i.p., daily for 7 days).

### Anti-CD31 and anti-α-SMA antibody staining

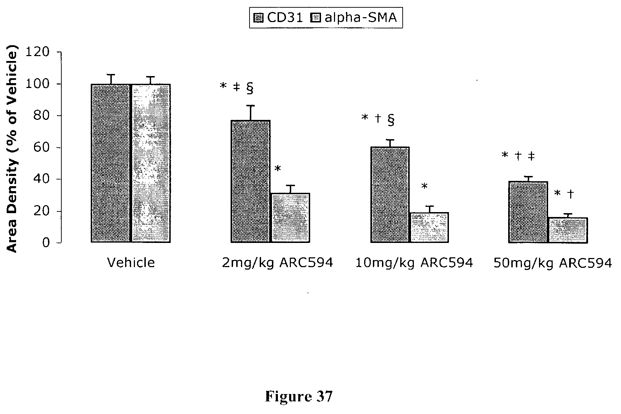

80-µm sections of Lewis lung carcinoma were stained with anti-CD31 and anti-α-SMA antibodies. A threshold value of 40 was determined to most accurately represent the area density of CD31 and α-SMA immunoreactivities in Lewis lung carcinoma (section thickness 80µm). Results are shown graphically in Figure 37 and summarized in Table 27 below. Values are mean area densities ± S.E; (n= 3-5 mice per group) expressed as % of mean value for vehicle.

**Table 27:**

| **Treatment Vehicle** | **CD31 mean** | **CD31= se** | **SMA mean** | **SMA se** | **Count** |
|---|---|---|---|---|---|
| | 100 | 5.96 | 100 | 4.44 | |
| **2mg/kg ARC594** | 77.16 | 9.27 | 31.44 | 4.52 | 3 |
| **10mg/kg ARC594** | 60.67 | 4.35 | 19.47 | 3.74 | 3 |
| **50mg/kg ARC594** | 39.14 | 2.65 | 16.57 | 2.06 | 5 |

The significance of differences between groups was assessed using ANOVA followed by Fisher's post-hoc test for multiple comparisons as shown in Table 28 below:

**Table 28:**

| **Fisher's PLSD for % vs vehicle_{;}** | | | | |
|---|---|---|---|---|
| **Effect: Group** | | | | |
| **Significance Level: 5%** | | | | |
| | **Mean Diff.** | **Crit. Diff.** | **P-Value** | |
| 10mg/kg-SMA, 2mg/kg-SMA | -11.97 | 15.75 | 0.13 | |
| 10mg/kg-SMA, 50mg/kg-SMA | 2.89 | 13.31 | 0.66 | |
| 10mg/kg-SMA, Veh-SMA | -80 53 | 14.09 | <0001 | **S** |
| 2mg/kg-SMA, 50mg/kg-SMA | 14.87 | 13.31 | 0.03 | **S** |
| 2mg/kg-SMA, Veh-SMA | -68.56 | 14.09 | <0001 | **S** |
| 50mg/kg-SWA, Veh-SMA | -83.43 | 11.30 | <.0001 | **S** |

The results indicate a significant reduction in α-SMA immunoreactive pericytes even at the lowest dose (2 mg/kg i.p., daily for 7 days). Higher concentrations of ARC594 had increased effectiveness.

### Colocalization of CD31 and multiple pericyte markers

20 µm sections of Lewis lung carcinoma from mice treated with vehicle or 2, 10 or 50 mg/kg of ARC594 as described above were stained with anti-CD31 and anti-α-SMA or anti-CD31 and anti-NG2 anti-bodies. The extent of pericyte colocalization with endothelial cells was measured on digital CoolCam images.

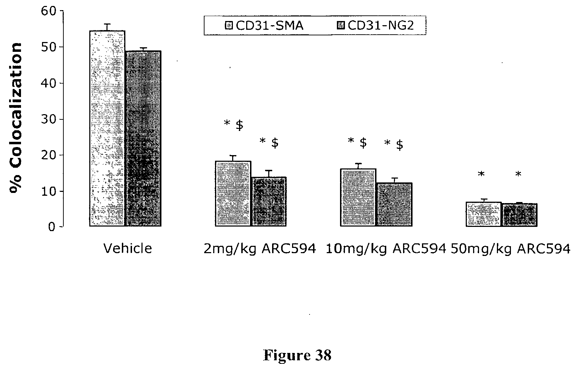

A threshold value of 40-50 most accurately represented the area densities of CD31-α-SMA and CD31-NG2 in Lewis lung carcinoma (the sections were 20 µm thick). The results are depicted in Figure 38 and summarized in Table 29 below. Values are mean colocalization ± S.E. (n = 2-5 mice per group).

**Table 29:**

| **Treatment** | **CD31-SMA mean** | **CD31-SMA se** | **CD31-NG2 mean** | **CD31-NG2 se** | **Count** |
|---|---|---|---|---|---|
| Vehicle | 54.44 | 1.67 | 48.79 | 0.72 | 2 |
| 2mg/kgARC594 | 18.40 | 1.46 | 14.01 | 1.73 | 3 |
| 10mg/kg ARC594 | 16.30 | 1.28 | 12.33 | 1.22 | 5 |
| 50mg/kg ARC594 | 7.15 | 0.68 | 6.69 | 0.20 | 4 |

The significance of differences between groups was assessed using ANOVA followed by Fisher's post-hoc test for multiple comparisons as shown in Table 30.

**Table 30:**

| **Fisher's PLSD for % Colocalization** | | | | |
|---|---|---|---|---|
| **Effect: Group** | | | | |
| **Significance Level: 5 %** | | | | |
| | **Mean Diff.** | **Crit. Diff.** | **P-Value** | |
| 2mg/kgCD31-SMA, 50mg/kgCD31-SMA | 11.253 | 3.444 | <.0001 | **S** |
| 2mg/kgCD31-SMA, VehCD31-SMA | -36.037 | 4.117 | <.0001 | **S** |
| 10mg/kgCD31-SMA, VehCD31-SMA | -38.137 | 4.117 | <.0001 | **S** |
| 10mg/kgCD31-SMA, 50mg/kgCD31-SMA | 9.153 | 3.444 | <.0001 | **S** |
| 10mg/kgCD31-SMA, 2mg/kgCD31-SMA | -2.1 | 3.682 | 0.2464 | |
| 50mg/kgCD31-SMA, VehCD31-SMA | -47.29 | 3.906 | <.0001 | **S** |
| 2mg/kgCD31-NG2, 50mg/kgCD31-NG2 | 7.318 | 3.444 | 0.0003 | **S** |
| 2mg/kgCD31-NG2, VehCD31-NG2 | -34.784 | 4.117 | <.0001 | **S** |
| 10mg/kgCD31-NG2, 2mg/kgCD31-NG2 | -1.678 | 3.293 | 0.2986 | |
| 10mg/kgCD31-NG2, 50mg/kgCD31-NG2 | 5.64 | 3.025 | 0.001 | **S** |
| 10mg/kgCD31-NG2, VehCD31-NG2 | -36.462 | 3.773 | <.0001 | **S** |
| 50mg/kgCD31-NG2, VehCD31-NG2 | -42.102 | 3.906 | <.0001 | **S** |

These results indicate that in Lewis lung carcinoma treated for 7 days with 2mg/kg of ARC594, the number of tumor vessels was reduced only by ∼ 23%, but the pericyte coverage was reduced to 14-18%. After treatment with 10mg/kg of ARC594, the number of tumor vessels was reduced by ∼ 40% and the pericyte coverage was reduced to 12-16%. While not wishing to be bound by any theory, these findings indicate that ARC594 first reduces the number of pericytes leaving the remaining blood vessels either bare or with reduced pericyte coverage and secondarily reduces the number of blood vessels, likely through the reduction of supporting pericytes and basement membrane cells stroma.

At the lowest dose, surviving tumor vessels enveloped by pericytes were normalized in the sense that the pericytes were more closely associated with endothelial cells than in untreated tumors.

The findings from the dose-response study confirm that ARC594 acts at multiple levels. While not wishing to be bound by any theory, these results provide strong evidence that the primary action of ARC594 results in reduction in number of pericytes, (whose function is to support the capillary network supplied blood and nutrients to the tumor). Again, while not wishing to be bound by any theory, the reduction in blood vessels could be indirect through the PDGF-B aptamer dependent blockade of tumor pericyte-derived and tumor stroma-derived growth factors such as VEGF.

### Example 17A: ARC594 Short Time-Course in Lewis Lung Carcinoma Model

A 1-mm³ piece of Lewis lung carcinoma was implanted under the dorsal skin of wild-type C57BL/6 mice, and then beginning on day 6, treated for 7 days with vehicle or 50mg/kg of ARC594, according to the following protocol:
n= 4 mice treated with vehicle (saline i.p., once daily) for 7 days,
n= 4 mice treated with ARC594 (50 mg/kg, i.p., once daily) for 1 day,
n= 4 mice treated with ARC594 (50 mg/kg, i.p., once daily) for 2 days,
n= 4 mice treated with ARC594 (50 mg/kg, i.p., once daily) for 4 days,
n= 4 mice treated with ARC594 (50 mg/kg, i.p., once daily) for 7 days.

At the end of each treatment, each group of mice was perfused and Lewis lung carcinoma and different organs were collected. 80 µm sections of Lewis lung carcinomas were cut and stained with antibodies to various markers as described below. Finally, the blood vessel and pericyte area density was determined using the Cool Cam.

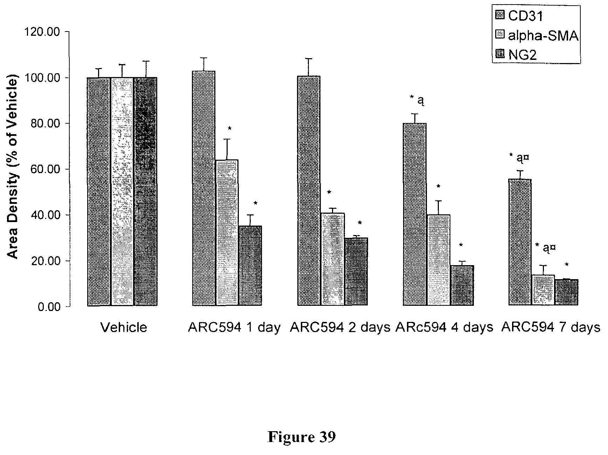

A threshold value of 30-50 was determined to most accurately represent the area density of CD31, α-SMA, and NG2 immunoreactivities in 80-µm sections of Lewis lung carcinoma. The results are presented in the graph of Figure 39 and are summarized in Table 31 below. Values are mean area densities ± S.E. (n = 3-5 mice per group) expressed as % of mean value for vehicle. The significance of differences between groups was assessed using ANOVA followed by Fisher's post-hoc test for multiple comparisons.

**Table 31:**

| **Treatment** | **CD31 Mean** | **CD31 SE** | **SMA Mean** | **SMA SE** | **NG2 Mean** | **NG2 SE** | **Count** |
|---|---|---|---|---|---|---|---|
| **Vehicle** | 100.00 | 3.85 | 100.00 | 5.34 | 100.00 | 6.86 | 4 |
| **ARC594 1 day** | 102.77 | 5.39 | 63.83 | 9.14 | 34.83 | 4.72 | 3 |
| **ARC594 2 days** | 100.41 | 7.36 | 40.64 | 1.96 | 29.92 | 0.83 | 4 |
| **ARC594 4 days** | 79.75 | 4.02 | 39.80 | 5.96 | 17.73 | 1.83 | 4 |
| **ARC594 7 days** | 55.17 | 3.55 | 13.64 | 3.90 | 11.64 | 0.27 | 3 |

These data indicate that after one day of treatment with ARC594 (50mg/kg), pericytes were reduced by 36% for α-SMA-positive and by 66% for NG2. After 7 days of treatment they are reduced even farther (86%-89% total decrease). In contrast, CD31 area density is not significantly unchanged until after 4 days of treatment with ARC594. While not wishing to be bound by any theory these results provide strong evidence that the primary effect of ARC594 is on the pericytes while secondary effects reduce endothelial cells through a reduction in pericyte-derived VEGF. Hence, under these conditions of PDGF-B treatment the net effect is to reduce the abundance of pericytes thereby directly reducing the source of host-derived VEGF which in turn causes a net reduction in the abundance of tumor endothelial cells.

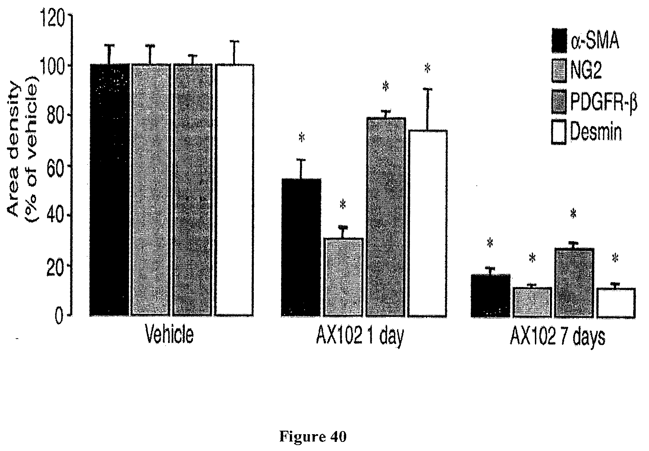

In a similar study, Lewis lung carcinomas treated with vehicle or ARC594 for seven days were also stained for detection of a total of 4 pericyte markers: α-SMA, NG2 , PDGF-β, and desmin. Under baseline conditions, pericytes expressed all 4 markers. Specifically, α-SMA area density was 10.0%, NG2 was 14.9%, PDGFR-β was 14.0%, and desmin was 9.0%. Compared to the vehicle-treated Lewis lung carcinoma, ARC594 treatment for 1 day caused a reduction in all 4 pericyte markers (Figure 40). Although there was a significant reduction in all 4 markers after one day of ARC564 treatment, reductions in α-SMA (45.4 ± 7.8%) and NG2 (69.8 ± 4.2%) were greater than PDGFR-β (31.3 ± 2.8%) and desmin (25.7 ± 16.0%: Figure 40). While not wishing to be bound by any theory, this unequal reduction in pericyte markers may indicate that while some pericytes are lost, the remaining pericytes have changed their phenotype by changing their expression markers. After 7 days of ARC594 treatment, however, all 4 pericyte markers were reduced by 70-80% (Figure 40), thus leaving tumor blood vessels bare of pericytes by 7 days. Confocal imaging confirmed these results.

### Example 17B:ARC594 Long Time-Course in Lewis Lung Carcinoma Model

A 1-mm³ piece of Lewis lung carcinoma was implanted under the dorsal skin of wild-type C57BL/6 mice (day 0). Beginning on day 6, mice were treated for 7-14-28 days with vehicle or 50mg/kg of ARC594, according to the following protocol:
n= 5 mice treated with vehicle (saline i.p., once daily) for 7 days,
n= 5 mice treated with ARC594 (50 mg/kg, i.p., once daily) for 7 days,
n= 6 mice treated with vehicle (saline i.p., once daily) for 14 days,
n= 6 mice treated with ARC594 (50 mg/kg, i.p., once daily) for 14 days,
n= 6 mice treated with vehicle (saline i.p., once daily) for 28 days,
n= 6 mice treated with ARC594 (50 mg/kg, i.p., once daily) for 28 days.

During the treatment, the mice were weighed daily and the tumor size was monitored. Tumor size was measured with calipers every other day during treatment. Tumor volume was calculated using the following formula: Tumor Volume = (0.52*Width^2)*Length. Tumors were also weighed at the end of the experiment.

### Tumor and Mouse Growth

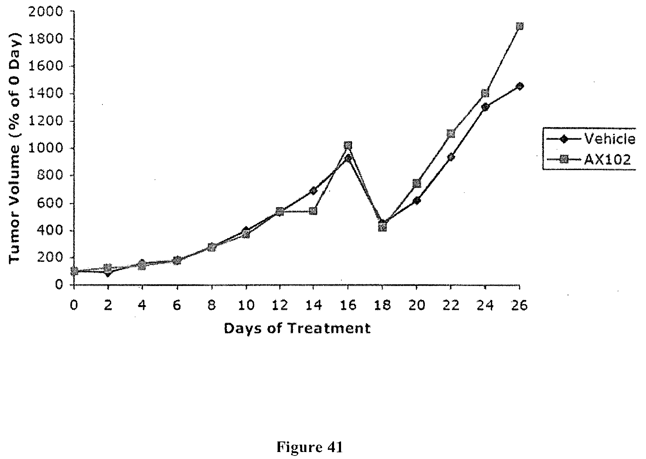

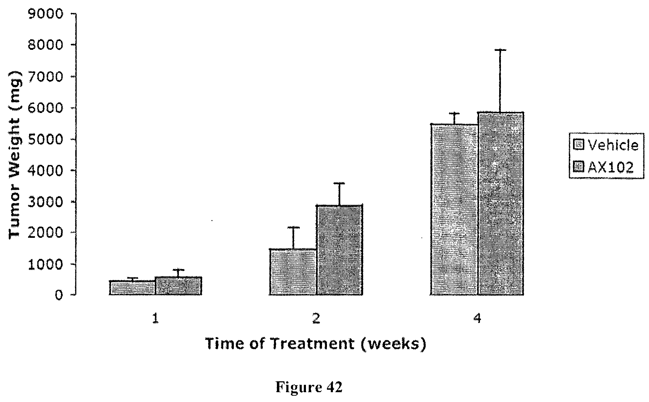

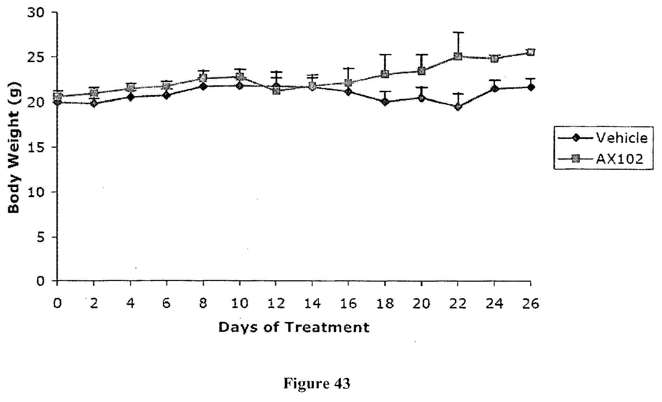

Tumor growth (as represented by tumor volume) during treatment is depicted in Figure 41. Tumor weight during treatment is depicted in Figure 42. Mouse growth during treatment is shown in Figure 43.

Treatment with ARC594 did not affect the tumor size of Lewis lung carcinoma: the growth curves for the tumors treated with ARC594 (50mg/kg) and vehicle are the same. In addition, there was no significant difference in the tumor weights measured at the end of the experiment. Mice treated with ARC594, however, appeared to gain more weight in the third and fourth week of treatment.

### Anti-CD31 and anti-NG2 antibody staining

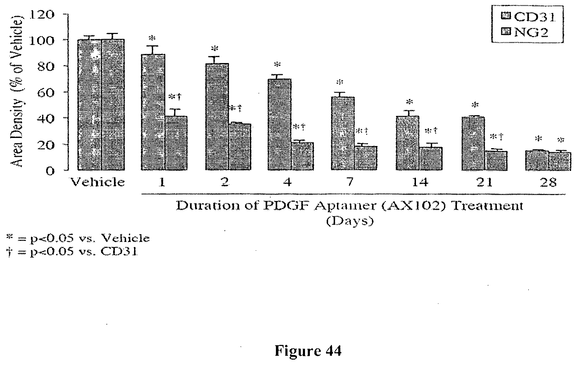

A threshold value of 40-50 was determined to most accurately represent the area density of CD31 and NG2 immunoreactivities in 80 µm sections of Lewis lung carcinoma. The results are depicted in Figure 44 and summarized in Table 32 below: Values are normalized mean area densities ± S.E. (n = 1-5 mice per group) expressed as % of mean value for vehicle. AX102 refers to ARC594. The significance of differences between groups was assessed using ANOVA followed by Fisher's post-hoc test for multiple comparisons.

**Table 32:**

| **Treatment** | **CD31 Mean** | **CD31 SE** | **NG2 Mean** | **NG2 se** | **Count** |
|---|---|---|---|---|---|
| Vehicle 1 week | 100.0 | 2.0 | 100.0 | 2.1 | 4 |
| AX102 1 week | 59.3 | 4.9 | 22.2 | 3.0 | 5 |
| Vehicle 2 weeks | 107.1 | 5.8 | 94.1 | 8.3 | 5 |
| Ax102 2 weeks | 40.5 | 4.2 | 18.2 | 3.4 | 4 |
| Vehicle 3 weeks | 101.4 | | 100.3 | | 1 |
| Ax102 3 weeks | 39.9 | 1.2 | 15.4 | 1.5 | 3 |
| Vehicle 4 weeks | 98.9 | 2.4 | 113.6 | 7.5 | 3 |
| Ax102 4 weeks | 15.0 | 0.2 | 14.9 | 1.4 | 2 |

In the Lewis lung carcinoma treated with vehicle the CD31 area density did not change. The NG2 area density of Lewis lung carcinoma treated with vehicle remained about the same after weeks 1, 2 and 3 of treatment while it increased somewhat after week 4 treatment. This difference was only statistically significant when compared to week 2 and week 4 time points.

In Lewis lung carcinoma treated with ARC594 (50mg/kg), CD31 area density decreased 40% compared to vehicle after one week, 60% after two weeks, stayed the same after three weeks and decreased 85% after four weeks. In Lewis lung carcinoma treated with ARC594, NG2 area density decreased 80% after one week and thereafter remained almost the same. These data show that ARC594 treatment reduces NG2 area density faster than CD31 area density.

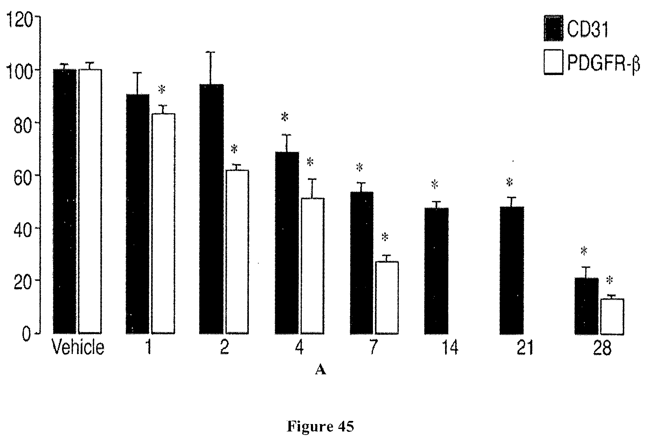

In a similar study, sections of Lewis lung carcinoma that had been treated with ARC594 for 28 days was stained with anti-CD31 and anit-PDGFR-beta antibodies. While ARC594 treatment caused an immediate reduction of pericytes after 1 day of treatment, tumor vessel area density was unchanged. Additional treatment, however, revealed that there was an observed reduction in tumor vessels at 7 days - a trend that continued for the 28 days of treatment. Quantification of the CD31 area density revealed that after 4 days of treatment, tumor blood vessels were significantly reduced by 31.3 % compared to the vehicle-treated mice (Figure 45). The tumor vessels were progressively reduced by 46.5% after 7 days of treatment and 79.9% after 28 days of treatment (Figure 45).

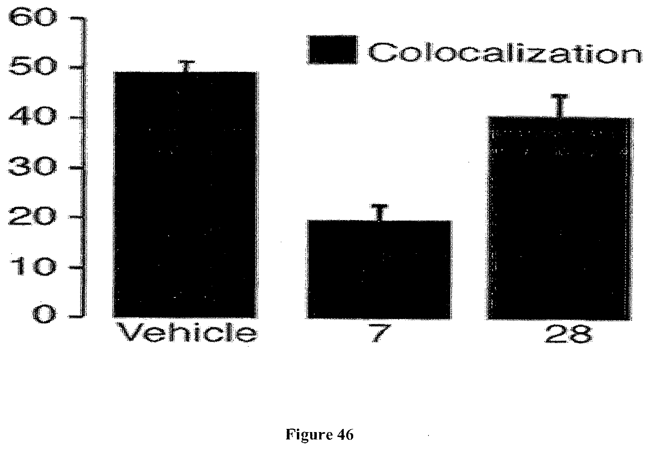

Figure 45 illustrates further reduction in tumor vessels, but not in PDGFR-beta-positive pericytes with additional ARC594 treatment for 28 days. Tumor vessels of vehicle treated mice were 48.6% covered by pericytes, while the remaining vessels after ARC594 treatment for 7 days were only 19.1% covered by pericytes (Figure 46). After ARC594 treatment for 28 days, which further reduces the tumor vessels, the remaining tumor blood vessels were 40.1% covered by pericytes (Figure 46).

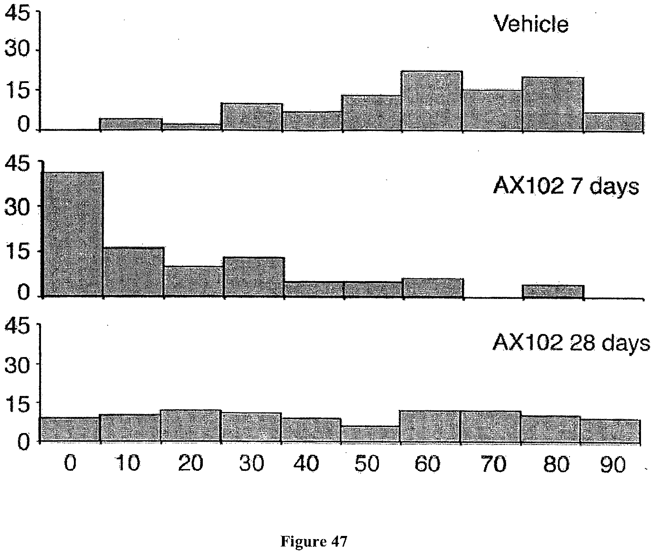

Previous results indicated that bare blood vessels were the ones affected by treatment with ARC594 for 28 days. To determine whether the vessels affected were the ones lacking pericytes, the distribution of CD31 and PDGFR-beta on individual vessels was examined. In the vehicle treated LLC, most of the vessels are covered by pericytes with the majority of the vessels falling between 40% and 90% coverage (Figure 47). After 7 days of treatment, fewer vessels survived and the remaining vessels were covered by few or no pericytes (<40% coverage; Figure 47). Additional ARC594 treatment for 28 days led to even further reductions in tumor vessels. These remaining vessels, however, lost the population of bare vessels seen after 7 days of treatment and in fact had an equal distribution of pericyte coverage (Figure 47).

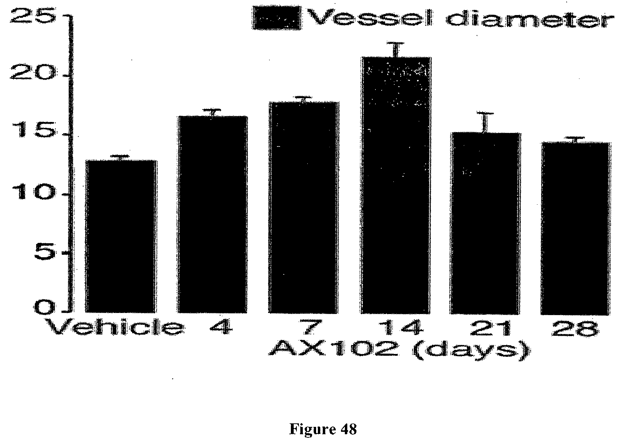

Whether the reduction in pericytes caused by ARC594 had an effect on vessel diameter was investigated. Vehicle-treated LLC had tumor blood vessels with a diameter of 12.8µm. Treatment with ARC594, however, increased the diameter at 4 days (Figure 48) and was further increased at 14 days (Figure 48). Additional treatment after 14 days, however, caused tumor vessels to decrease in diameter as shown on days 21 and 28. (Figure 48)

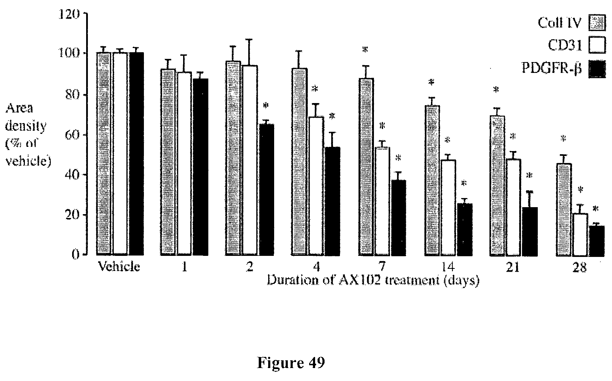

In a similar experiment, whether ARC594 treatment affected the basement membrane was investigated. In vehicle treated Lewis lung carcinoma, CD31-positive vessels were enveloped by type IV collagen. ARC594 did not change the total amount of collagen IV after 1 day of treatment, when pericyte number began to decrease; or after 4 days of treatment, when blood vessel number started to decrease. However after 7 days of treatment, the vascular basement membrane gradually started to decrease, reaching the greatest reduction after 28 days Indeed, type IV collagen area density was reduced by 20% at day 7, and by 50% at day 28 (Figure 49).

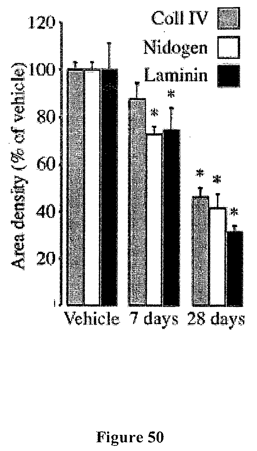

In a similar experiment, changes in vascular basement membrane were also analyzed by using two other markers for basement membrane components: nidogen and laminin. Double staining with type IV collagen, or laminin, and nidogen showed that these markers have the same immunoreactivity and the vascular basement membrane was positive for all the three of them. Analysis of the area density showed that nidogen and laminin were reduced in the same manner as type IV collagen (Figure 50).

### Example 18: ARC594 Dose-Response Long Time Course in Lewis Lung Carcinoma

A 1 mm³ piece of Lewis lung carcinoma (LLC) was implanted under the dorsal skin of 9 to 10-week old C57BL/6 mice. Treatment began 4-6 days thereafter. The aptamer ARC594 (2, 10, or 50 mg/kg) or vehicle (saline) was injected *ip* daily for up to 28 days. In addition, the scrambled aptamer ARC128 (also referred to herein is AX104) (50 mg/kg), which contains the same nucleotides as ARC594 but in a random order, was used to determine the specificity of ARC594. At the end of the treatment mice were anesthetized, prefused with fixative and tissue was collected from each mouse.

For staining the endothelial cells of tumor vessels, 80-µm thick cryostat sections of Lewis lung carcinoma were stained with anti-CD31 antibody (clone 2H8 Chemicon). For pericytes, sections were stained with one or more of four antibodies: α-smooth muscle actin (α-SMA, clone 1A4, Sigma Chemical Co.), NG2 chondroitin sulfate proteoglycan (AB5320, Chemicon), PDGFR-β (clone APB5, from Akiyoshi Uemura, Kyoto University, Japan), and desmin (DAKO).

CD31 and pericyte marker area densities (proportion of sectional area) were measured by fluorescence microscopy in images acquired at 10X. Transmission electron microscopy of 50-100 nm sections were stained with lead citrate and examined with a Zeiss EM-10 electron microscope.

Pericyte coverage was measured on 20 µm sections stained with CD31 and a pericyte marker (α-SMA, NG2, PDGFR-β, and desmin). Images of each marker were captured separately and analyzed by Image J. After determining the threshold of each channel, the images were merged and the colocalized pixels were measured. The area density was determined as the proportion of colocalized pixels to CD31 positive pixels.

### Anti-α-SMA antibody staininn

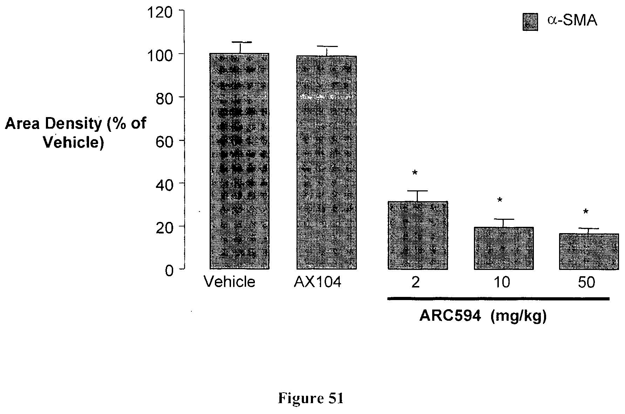

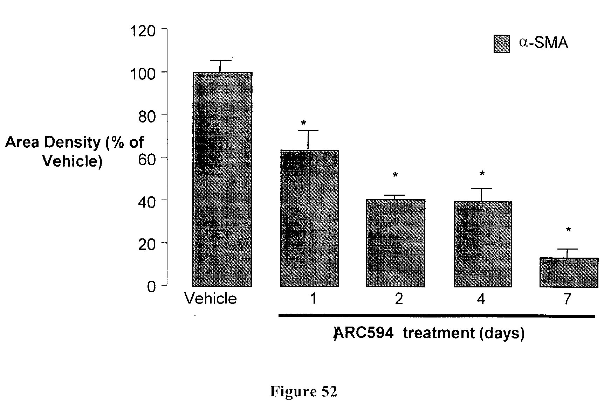

Tumors treated with ARC594 (50mg/kg), scrambled aptamer (50 mg/kg) or vehicle for up to 7 days were sectioned and stained with anti-α-SMA antibodies. As shown in Figure 51, ARC594, descreased the α-SMA immunoreactive pericytes in a dose-response fashion. Figure 52 shows that ARC594 (50 mg/kg) reduced pericytes after only one day of treatment. Confocal images show that scrambled aptamer did not affect the tumor vasculature.

### Example 19: Dose-Response of ARC594 in RIP_Tag2 Model

9 week old RIP-Tag2 positive mice were treated for 7 days with vehicle, or 2mg/kg, 10mg/kg or 50mg/kg of ARC594, according to the following protocol:
n= 4 mice treated with vehicle (saline i.p., once daily)
n= 5 mice treated with ARC594 (2 mg/kg, i.p., once daily)
n= 5 mice treated with ARC594 (10 mg/kg, i.p., once daily)
n= 5 mice treated with ARC594 (50 mg/kg, i.p., once daily)

At the end of this treatment, the mice were perfused and the liver, spleen, thyroid, trachea, kidney, jejunum, heart, brain, and pancreas were collected for each mouse. Finally, 80 µm sections were cut and stained for CD31, type IV collagen, PDGFR-beta, α-SMA, and NG2.

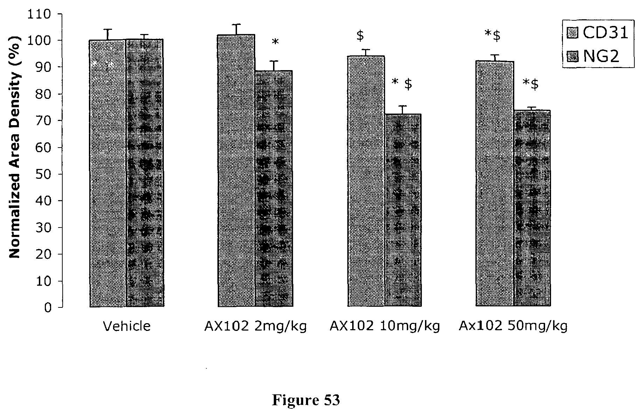

A threshold value of 40 was determined to most accurately represent the area density of CD31, and NG2 immunoreactivities in 80-µm sections of RIP-Tag2 tumor. The results are depicted in Figure 53 and summarized in Table 33 below. Values are mean area densities ± S.E. (n = 4-5 mice per group) expressed as area density and also as % of the mean value for vehicle.

**Table 33:**

| **Treatment** | **CD31 Mean** | **CD31 SE** | **NG2 Mean** | **NG2 se** | **Count** |
|---|---|---|---|---|---|
| Vehicle | 100.0 | 4.0 | 100.0 | 1.8 | 4 |
| ARC594 2mg/kg | 101.6 | 3.8 | 88.2 | 3.5 | 4 |
| ARC594 10mg/kg | 93.5 | 2.4 | 72.0 | 2.9 | 5 |
| ARC594 50mg/kg | 91.6 | 2.3 | 73.5 | 1.1 | 4 |

The significance of differences between groups was assessed using ANOVA followed by Fisher's post-hoc test for multiple comparisons as shown in Table 34.

**Table 34:**

| **Fisher's PLSD for Area density** | | | | |
|---|---|---|---|---|
| **Effect: Group** | | | | |
| **Significance Level: 5 %** | | | | |
| | **Mean Diff.** | **Crit. Diff.** | **P-Value** | |
| ARC594 -2mg/kg-CD31, veh-CD31 | 0.86 | 4.52 | 0.6989 | |
| ARC 594 -2mg/kg-CD31, ARC594-50mg/kg-CD31 | 5.509 | 4.52 | 0.0188 | **S** |
| ARC594-10mg/kg-CD31, veh-CD31 | -3.604 | 4.288 | 0.0959 | |
| ARC594-10mg/kg-CD31, ARC594-2mg/kg-CD31 | -4.464 | 4.288 | 0.0419 | **S** |
| ARC594-10mg/kg-CD31, ARC594-50mg/kg-CD31 | 1.045 | 4.288 | 0.6205 | |
| ARC594-50mg/kg-CD31, veh-CD31 | -4.649 | 4.52 | 0.0442 | **S** |
| ARC594-2mg/kg-NG2, veh-NG2 | -5.618 | 4.52 | 0.0168 | **S** |
| AARC594 -2mg/kg-NG2, Ax102-50mg/kg-NG2 | 6.99 | 4.52 | 0.0038 | **S** |
| ARC594-10mg/kg-NG2, veh-NG2 | -13.306 | 4.288 | <.0001 | **S** |
| ARC594-10mg/kg-NG2, ARC594-2mg/kg-NG2 | -7.688 | 4.288 | 0.0011 | **S** |
| ARC594-10mg/kg-NG2, ARC594-50mg/kg-NG2 | -0.698 | 4.288 | 0.7405 | |
| ARC594-50mg/kg-NG2, veh-NG2 | -12.608 | 4.52 | <.0001 | **S** |

These results indicate that in RIP-Tag2 tumors treated for 7 days with 2mg/kg of ARC594, the density of NG2 positive pericytes was reduced by 12%. At 10mg/kg dose the reduction was higher (28%). The 50mg/kg dose had the same effect than 10mg/kg dose. ARC594 at 10mg/kg and 50 mg/kg dose induced 10% reduction in RIP-Tag2 tumor vessels.

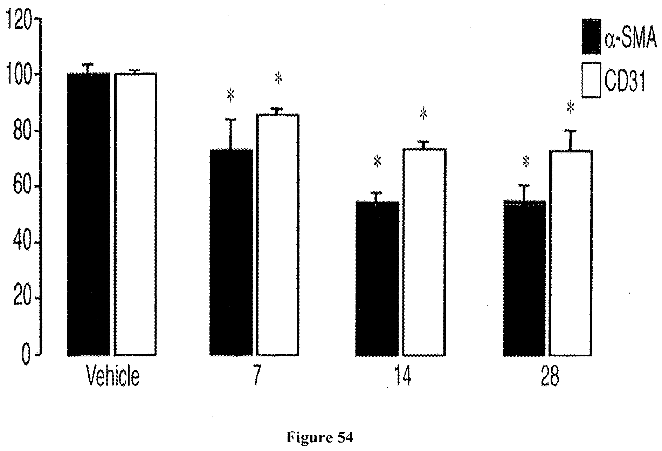

In another study, treatment of RIPTag2 tumor with ARC594 for 7 days led to a reduction in pericytes and this reduction was greater after 28 days. The untreated RIP-Tag2 tumors are highly vascularized. ARC594 treatment caused a regression of blood vessels (CD31 area density) and this decrease was time-dependent; the blood vessels started to regress after 7 days of treatment. Prolongation of treatment to 28 days resulted in a greater reduction in blood vessels. Measurements showed that 7-day treatment with ARC594 decreased α-SMA and CD31 immunoreactivity by 27.2% and 12.3%, respectively. After 14 days α-SMA and CD31 immunoreactivity were reduced by 45.8% and by 26.6%, respectively. After 28 days α-SMA and CD31 immunoreactivity were reduced by 45.5% and by 38.1 %, respectively (Figure 54).

Pericytes in RIP-Tag2 tumors were loosely associated with the endothelium. As seen in the Lewis lung carcinoma model, after ARC594 treatment the surviving pericytes were tightly associated with the blood vessels, indicating that in this tumor model PDGF-B inhibition induced phenotypic changes of pericytes.

### Example 20: Pharmacokinetic Analysis of ARC594 in Mice.

For the pharmakokinetic studies described herein, all mass based concentration data refers only to the molecular weight of the oligonucleotide portion of the aptamer, irrespective of the mass conferred by PEG conjugation.

Each dose group consisted of 3 mice that were terminally sacrificed at each time point for collection of plasma. The aptamer was formulated for injection from a lyophilized powder to a final concentration of 10 mg/mL (oligonucleotide weight) in standard 0.9% saline and sterile-filtered (0.2 µm) prior to dosing. The route of administration used was a single intravenous bolus injection via the tail vein at a dose of 10 mg/kg. At specified time points, t=pre-dose, 0.5, 1, 2, 4, 8, 16, 24, 32, and 48 hours, blood samples were obtained from the jugular vein catheters, transferred directly to EDTA-coated tubes, mixed by inversion, and placed on wet ice. Plasma was harvested by centrifugation of blood-EDTA tubes at 3000 µm for 5 minutes. Plasma samples were stored at -80°C until the time of analysis. Analysis of plasma samples for aptamer concentration was accomplished using a homogeneous assay format utilizing the direct addition of plasma aliquots to assay wells containing the commercially available fluorescent nucleic acid detection reagent Oligreen™. After a brief incubation period (5 min) at room temperature, protected from light, the assay plates were read by a fluorescence plate reader.

The fluorescence signal from each well was proportional to the concentration of aptamer in the well, and sample concentrations were calculated by interpolation of fluorescence values from a fluorescence-concentration standard curve (mean values from duplicate curves). Mean plasma concentrations were obtained at each time point from the three animals in each group. Plasma concentration versus time data appeared was subjected to noncompartmental analysis (NCA) using the industry standard pharmacokinetic modeling software WinNonLin™ v.4.0. Estimates were obtained for the following primary phannacokinetic parameters: maximum plasma concentration, Cₘₐₓ, area under the concentration-time curve, AUC; terminal half-life, t_{1/2}; terminal clearance, C1; and volume of distribution at steady state, Vₛₛ. Mean plasma concentration versus time data appeared to be monophasic over the entire timecourse studied (0-48 hrs).

The competitive binding assay previously described was used to determine the *in vitro* binding affinity and selectivity of ARC594 for different PDGF isoforms. Different concentrations of unlabeled, competitor aptamer were titrated in separate reactions against a fixed (<0.1 nM) amount of ³²P-radiolabeled ARC594 in buffer containing a fixed amount (0.1 nM) of the aptamer's cognate target (PDGF-BB; PeproTech). UnPEGylated version of ARC594 was radiolabeled at the 5' terminus by incubation with γ-³²P-ATP (ICN) and polynucleotide kinase (NEB). Binding reactions were carried out in phosphate-buffered saline (Cellgro) containing 0.2 mg/mL bovine serum albumin (NEB) and 0.02 mg/mL tRNA (Sigma). The reactions were equilibrated for a period of 15-30 minutes at room temperature, then filtered through a sandwich of nitrocellulose (Protran membrane, Perkin-Elmer) and nylon (Hybond membrane, Amersham) membranes to separate target bound aptamer from free aptamer. Subsequent autoradiographic analysis of the filter membranes corresponding to each concentration of unlabeled aptamer revealed the extent of competitive displacement of ³²P-ARC594 by unlabeled aptamer. The data was expressed as the ratio of the counts per minute (CPM) of radioactivity due to ³²P-ARC594 bound to PDGF BB on the nitrocellulose membrane (CPM_{nitrocellulose} or CPM_{nc}) over the total CPM (CPMₜₒₜₐₗ = CPM_{nitrocellulose} + CPM_{nylon}).

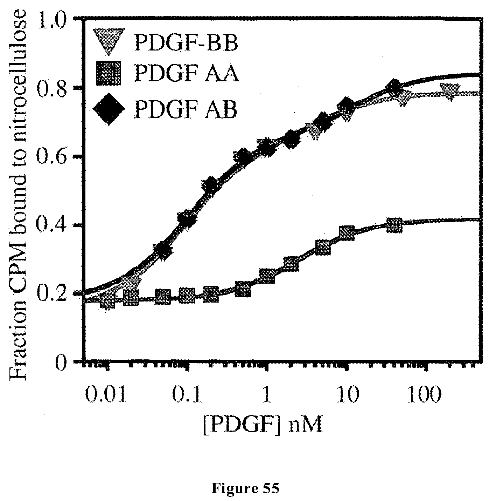

Of the different PDGF ligands, ARC594 had the highest affinity for PDGF-BB (K_{d} = 0.09 ± 0.02 nM) and PDGF-AB (K_{d} = 0.10 ± 0.01 nM), but not PDGF-AA (K_{d} = 2.6 ± 0.2 nM (Figure 55).

As a secondary test of both the plasma pharmacokinetics and the *in vivo* bioactivity of ARC594 the competition binding assay was used to assay the same plasma samples used to generate the pharmacokinetic data. Serial 1:10 dilutions of plasma sample were prepared in phosphate-buffered saline (1X PBS) and mixed with a fixed concentration of ²P-ARC594, then added to human PDGF-BB. The final concentration of PDGF in each assay was 0.1 nM, and the final concentration of ³²P-ARC594 < 0.1 nM. In this experiment, the plasma samples were analyzed by comparison with a standard curve generated with samples of known ARC594 concentrations in 1X PBS. By comparison with the reference standards, the effective concentration of active aptamer in each plasma sample could be calculated.

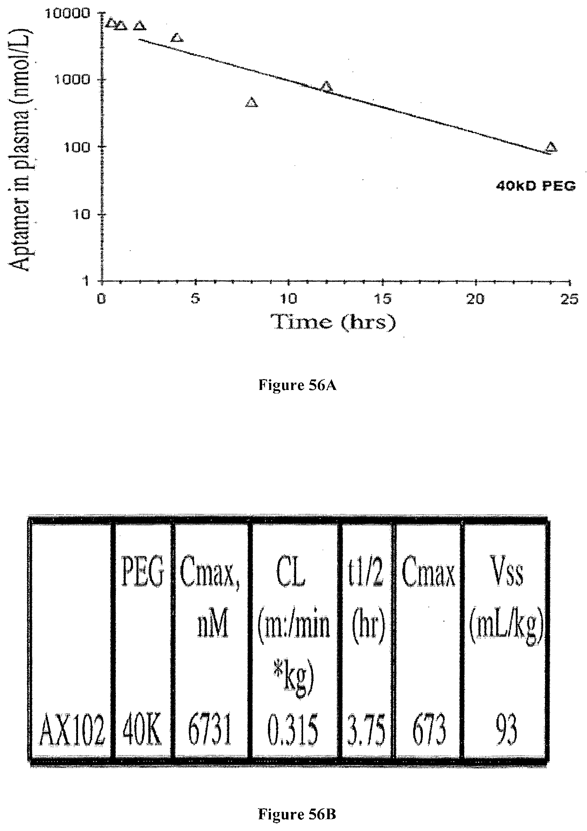

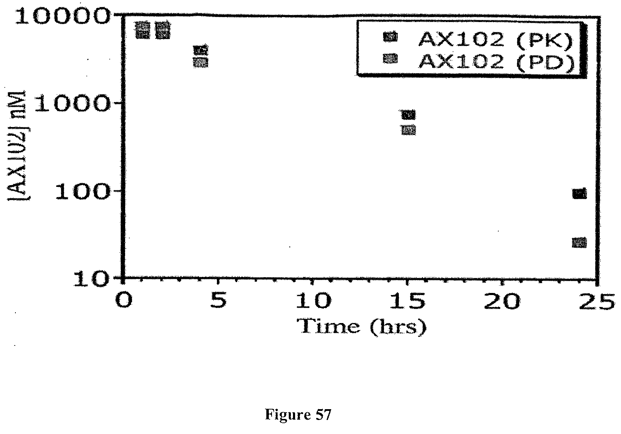

The results of this study indicate that at 10 mg/kg-dose of ARC594 has a half-life of approximately 4 hours (Figures 56A and 56B), and the pharmacodynamic profile is in accordance with its pharmacokinetic data (Figure 57). The bioactivity profile of this *ex vivo* analysis, shown in Figure 57, provides verification that (1) the aptamer was present and active in the plasma of the mouse model at t=48 hrs post-dose; and (2) the plasma concentrations calculated from the fluorescence-based pharmacokinetic assay were correct.

The invention having now been described by way of written description and example, those of skill in the art will recognize that the invention can be practiced in a variety of embodiments and that the description and examples above are for purposes of illustration and not limitation of the following claims.
1. A PDGF specific aptamer comprising the following structure: 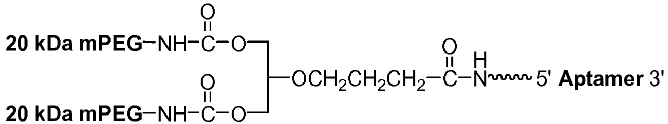 Where,
   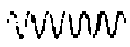 indicates a linker
   Aptamer = 5'dCdCdCdAdGdGdCdTdAdCmG (SEQ ID NO:69)- PEG-dCdGdTdAmGdAmGdCdAmUmCmA (SEQ ID NO:40) -PEG - dTdGdAdTmCdCdTmGmGmG-3T-3' (SEQ ID NO:70); wherein d denotes a deoxy nucleotide, m denotes a 2'-OMe nucleotide, PEG denotes a PEG spacer and 3T denotes an inverted deoxy thymidine cap.
2. A PDGF specific aptamer comprising the following structure: 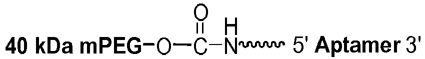 Where,
   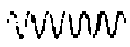 indicates a linker
   Aptamer = 5'dCdCdCdAdGdGdCdTdAdCmG (SEQ ID NO:69) -PEG-dCdGdTdAmGdAmGdCdAmUmCmA (SEQ ID NO:40) -PEG-dTdGdAdTmCdCdTmGmGmG-3T-3' (SEQ ID NO:70); wherein d denotes a deoxy nucleotide, m denotes a 2'-OMe nucleotide, PEG denotes a PEG spacer, and 3T denotes an inverted deoxy thymidine cap.
3. A PDGF specific aptamer, comprising the following structure: 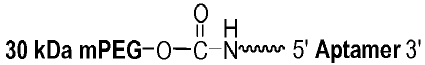 Where,
   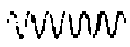 indicates a linker
   Aptamer = 5'dCdCdCdAdGdGdCdTdAdCmG (SEQ ID NO:69) -PEG-dCdGdTdAmGdAmGdCdAmUmCmA (SEQ ID NO:40) -PEG - dTdGdAdTmCdCdTmGmGmG -3T-3' (SEQ ID NO:70); wherein d denotes a deoxy nucleotide, m denotes a 2'-OMe nucleotide, PEG denotes a PEG spacer, and 3T denotes an inverted deoxy thymidine cap.
4. A PDGF specific aptamer, comprising the following structure: 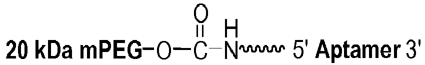 Where,
   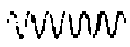 indicates a linker
   Aptamer = 5'dCdCdCdAdGdGdCdTdAdCmG (SEQ ID NO:69) -PEG-dCdGdTdAmGdAmGdCdAmUmCmA (SEQ ID NO:40) -PEG-dTdGdAdTmCdCdTmGmGmG-3T-3' (SEQ ID NO:70); wherein d denotes a deoxy nucleotide, m denotes a 2'-OMe nucleotide, PEG denotes a PEG spacer and 3T denotes an inverted deoxy thymidine cap.
5. A PDGF specific aptamer, comprising the following structure: 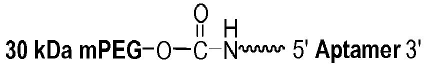 Where,
   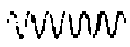 indicates a linker
   Aptamer = 5'dCdAdGdGdCfUdAfCmG (SEQ ID NO: 1))-HEG-dCdGdTdAmGdAmGdCdAfUfCmA (SEQ ID NO: 2)-HEG-dTdGdAdTfCfCfUmG-3T-3' (SEQ ID NO:3); wherein d denotes a deoxy nucleotide, m denotes a 2'-OMe nucleotide, HEG denotes a hexaethylene glycol amidite (HEG) spacer, and 3T denotes an inverted deoxy thymidine.
6. A PDGF specific aptamer, comprising the following structure: 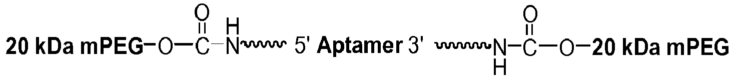 where,
   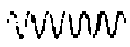 indicates a linker
   **Aptamer** = 5'dCdCdCdAdGdGdCdTdAdCmG (SEQ ID NO:69) -PEG-dCdGdTdAmGdAmGdCdAmUmCmA (SEQ ID NO:40) -PEG - dTdGdAdTmCdCdTmGmGmG 3' (SEQ ID NO:70); wherein d denotes a deoxy nucleotide, m denotes a 2'-OMe nucleotide, and PEG denotes a PEG spacer.
7. A PDGF specific aptamer, comprising the following structure: 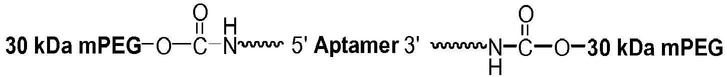 where,
   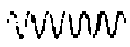 indicates a linker
   **Aptamer** = 5'dCdCdCdAdGdGdCdTdAdCmG (SEQ ID NO:69)- PEG - dCdGdTdAmGdAmGdCdAmUmCmA (SEQ ID NO:40)- PEG-dTdGdAdTmCdCdTmGmGmG 3' (SEQ ID NO:70); wherein d denotes a deoxy nucleotide, m denotes a 2'-OMe nucleotide, and PEG denotes a PEG spacer.
8. An aptamer according to any one of the preceding paragraphs, wherein the linker is an alkyl linker.
9. The aptamer of paragraph 8, wherein the alkyl linker comprises 2 to 18 consecutive CH₂ groups.
10. The aptamer of paragraph 8, wherein the alkyl linker comprises 2 to 12 consecutive CH₂ groups.
11. The aptamer of paragraph 8, wherein the alkyl linker comprises 3 to 6 consecutive CH₂ groups.
12. A PDGF specific aptamer, comprising the following structure: 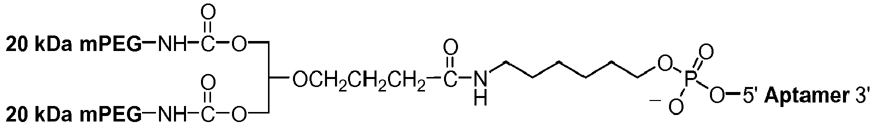 Wherein Aptamer = 5'dCdCdCdAdGdGdCdTdAdCmG (SEQ ID NO:69)-PEG -dCdGdTdAmGdAmGdCdAmUmCmA (SEQ ID NO:40) -PEG - dTdGdATmCdCdTmGmGmG-3T-3' (SEQ ID NO:70); wherein d denotes a deoxy nucleotide, m denotes a 2'-OMe nucleotide, PEG denotes a PEG spacer and 3T denotes an inverted deoxy thymidine cap.
13. An aptamer according to any one of the preceeding paragraphs, wherein the spacer is a PEG 6 spacer.
14. A PDGF specific aptamer selected from the group consisting of SEQ ID Nos 86-89, 91, 93-95 and 102.
15. A composition comprising a therapeutically effective amount of the aptamer of any of the proceeding paragraphs or a salt thereof.
16. The composition of paragraph 15, comprising a pharmaceutically acceptable carrier or diluent.
17. A method of treating a solid tumor, comprising administering the composition of paragraph 16 to a subject having the solid tumor.
18. The method of paragraph 17, further comprising administering to the subject a cytotoxic agent.
19. A method of treating a PDGF mediated tumor in a subject comprising administering a PDGF specific aptamer to the subject and treating the subject with radiotherapy.
20. A method of enhancing tumor imaging comprising administering a PDGF binding aptamer and a photosensitizer to a subject having a PDGF mediated tumor, activating the photosensitizer and detecting the tumor.
21. The method of paragraph 19 or 20, wherein the PDGF specific apatamer is selected from the group consisting of: ARC308, ARC404, ARC513, 592, 593, ARC594, and ARC1472 to ARC1474.

## Claims

1. An aptamer or pharmaceutically acceptable salt thereof that binds to PDGF, comprising the structure 5'-(SEQ ID NO: 55)-PEG-(SEQ ID NO: 40)-PEG-(SEQ ID NO: 41)-3T-3', wherein PEG is a polyethylene glycol spacer and 3T is an inverted deoxythymidine.

2. The aptamer or pharmaceutically acceptable salt of the aptamer of claim 1, wherein the aptamer is pegylated.

3. The aptamer or pharmaceutically acceptable salt of the aptamer of claim 2, wherein the aptamer is pegylated with a 40 kDa PEG moiety.

4. The aptamer or pharmaceutically acceptable salt of the aptamer of claim 3, wherein the 40 kDa PEG moiety is branched.

5. The aptamer or pharmaceutically acceptable salt of the aptamer of claim 4, wherein the aptamer comprises the following structure at its 5' end: 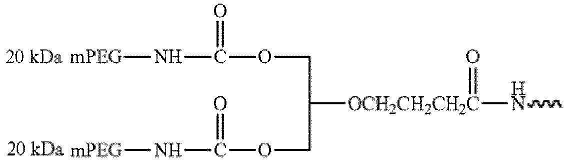 where ^{∼∼} indicates a linker.

6. The aptamer or pharmaceutically acceptable salt of the aptamer of claim 5, wherein the polyethylene glycol spacer is a PEG-6 spacer.

7. A composition comprising a therapeutically effective amount of the aptamer or pharmaceutically acceptable salt thereof of any one of claims 1 to 6 and a pharmaceutically acceptable carrier.

8. The composition of claim 7, further comprising a VEGF/VEGFR antagonist.

9. The composition of claim 7, further comprising an aptamer that binds to VEGF.


SEQUENCE LISTING

<110>  Archemix Corp., et al.

<120>  Stabilized Aptamers to Platelet Derived Growth Factor and Their
       Use as Oncology Therapeutics

<130>  23239-558A CIP3-061

<140>  PCT/US05/039975
<141>  2005-11-02

<150>  US 10/980,211
<151>  2004-11-02

<150>  US 60/632,358
<151>  2004-11-30

<150>  US 60/632,609
<151>  2004-12-01

<150>  US 60/652,496
<151>  2005-02-10

<150>  US 60/652,494
<151>  2005-02-11

<150>  US 60/667,866
<151>  2005-04-01

<150>  US 60/672,200
<151>  2005-04-015

<160>  102

<170>  PatentIn version 3.2


<210>  1
<211>  9
<212>  DNA
<213>  artificial

<220>
<223>  synthetic aptamer


<220>
<221>  modified_base
<222>  (6)..(6)
<223>  uridine is 2'-fluoro

<220>
<221>  modified_base
<222>  (8)..(8)
<223>  cytidine is 2'-fluoro

<220>
<221>  modified_base
<222>  (9)..(9)
<223>  gm

<400>  1

caggcuacg                                                              9

<210>  2
<211>  12
<212>  DNA
<213>  artificial

<220>
<223>  synthetic aptamer

<220>
<221>  modified_base
<222>  (5)..(5)
<223>  gm

<220>
<221>  modified_base
<222>  (7)..(7)
<223>  gm

<220>
<221>  modified_base
<222>  (10)..(10)
<223>  uridine is 2'-fluoro

<220>
<221>  modified_base
<222>  (11)..(11)
<223>  cytidine is 2'-fluoro

<220>
<221>  modified_base
<222>  (12)..(12)
<223>  adenosine is 2'-O-Methyl

<400>  2
cgtagagcau ca                                                         12

<210>  3
<211>  8
<212>  DNA
<213>  artificial

<220>
<223>  synthetic aptamer

<220>
<221>  modified_base
<222>  (5)..(6)
<223>  cytidine is 2'-fluoro

<220>
<221>  modified_base
<222>  (7)..(7)
<223>  uridine is 2'-fluoro

<220>
<221>  modified_base
<222>  (8)..(8)

<223>  gm

<400>  3
tgatccug                                                                 8


<210>  4
<211>  9
<212>  DNA
<213>  artificial

<220>
<223>  synthetic aptamer


<220>
<221>  modified_base
<222>  (4)..(4)
<223>  cytidine is 2'-fluoro

<220>
<221>  modified_base
<222>  (5)..(5)
<223>  gm

<220>
<221>  modified_base
<222>  (6)..(6)
<223>  uridine is 2'-fluoro

<220>
<221>  modified_base
<222>  (8)..(8)
<223>  cytidine is 2'-fluoro

<220>
<221>  modified_base
<222>  (9)..(9)
<223>  gm

<400>  4
cagcguacg                                                                9


<210>  5
<211>  12
<212>  DNA
<213>  artificial

<220>
<223>  synthetic aptamer


<220>
<221>  modified_base
<222>  (7)..(7)
<223>  gm

<220>
<221>  modified_base
<222>  (10)..(10)
<223>  uridine is 2'-fluoro

<220>
<221>  modified_base
<222>  (11)..(11)
<223>  cytidine is 2'-fluoro

<220>
<221>  modified_base
<222>  (12)..(12)
<223>  adenosine is 2'-O-Methyl

<400>  5
cgtaccgatu ca                                                          12


<210>  6
<211>  8
<212>  DNA
<213>  artificial

<220>
<223>  synthetic aptamer


<220>
<221>  modified_base
<222>  (6)..(6)
<223>  cytidine is 2'-fluoro

<220>
<221>  modified_base
<222>  (7)..(7)
<223>  uridine is 2'-fluoro

<220>
<221>  modified_base
<222>  (8)..(8)
<223>  gm

<400>  6
tgaagcug                                                                8


<210>  7
<211>  23
<212>  RNA
<213>  artificial

<220>
<223>  synthetic aptamer


<220>
<221>  modified_base
<222>  (1)..(23)
<223>  all pyrimidines and all purines are 2'-O-Methyl

<400>  7
augcaguuug agaagucgcg cau                                              23


<210>  8
<211>  29
<212>  DNA

<213>  artificial

<220>
<223>  synthetic aptamer

<400>  8
caggctacgc gtagagcatc atgatcctg                                        29


<210>  9
<211>  56
<212>  DNA
<213>  artificial

<220>
<223>  synthetic aptamer


<220>
<221>  modified_base
<222>  (10)..(32)
<223>  From nucleotides 10 to 32, all purines (A and G) and all
      pyrimdines (C and U) are 2'-O-Methyl

<400>  9
caggctacga ugcaguuuga gaagucgcgc aucgtagagc atcagaaatg atcctg          56


<210>  10
<211>  54
<212>  DNA
<213>  artificial

<220>
<223>  synthetic aptamer


<220>
<221>  modified_base
<222>  (10)..(30)
<223>  From nucleotides 10 to 30, all purines (A and G) and all
      pyrimdines (C and U) are 2'-O-Methyl

<400>  10
caggctacgu gcaguuugag aagucgcgca cgtagagcat cagaaatgat cctg            54


<210>  11
<211>  39
<212>  DNA
<213>  artificial

<220>
<223>  synthetic aptamer

<400>  11
cacaggctac ggcacgtaga gcatcaccat gatcctgtg                             39


<210>  12
<211>  22
<212>  DNA
<213>  artificial

<220>
<223>  synthetic aptamer


<220>
<221>  misc_feature
<222>  (1)..(22)
<223>  phosphorothioate backbone

<400>  12
tgactgtgaa cgttcgagat ga                                                22


<210>  13
<211>  14
<212>  DNA
<213>  artificial

<220>
<223>  synthetic aptamer


<220>
<221>  misc_feature
<222>  (1)..(14)
<223>  phosphorothioate backbone

<400>  13
tgaacgttcg agat                                                         14


<210>  14
<211>  12
<212>  DNA
<213>  artificial

<220>
<223>  synthetic aptamer


<220>
<221>  misc_feature
<222>  (1)..(12)
<223>  phosphorothioate backbone

<400>  14
aacgttcgag at                                                           12


<210>  15
<211>  10
<212>  DNA
<213>  artificial

<220>
<223>  synthetic aptamer


<220>
<221>  misc_feature
<222>  (1)..(10)
<223>  phosphorothioate backbone

<400>  15
aacgttcgag                                                              10


<210>  16
<211>  13
<212>  DNA
<213>  artificial

<220>
<223>  synthetic aptamer


<220>
<221>  misc_feature
<222>  (1)..(13)
<223>  phosphorothioate backbone

<400>  16
gtgaacgttc gag                                                          13


<210>  17
<211>  24
<212>  DNA
<213>  artificial

<220>
<223>  synthetic aptamer


<220>
<221>  misc_feature
<222>  (1)..(24)
<223>  phosphorothioate backbone

<400>  17
tcgtcgtttt gtcgttttgt cgtt                                              24


<210>  18
<211>  18
<212>  DNA
<213>  artificial

<220>
<223>  synthetic aptamer


<220>
<221>  misc_feature
<222>  (1)..(18)
<223>  phosphorothioate backbone

<400>  18
gtcgttttgt cgttttgt                                                     18


<210>  19
<211>  14
<212>  DNA
<213>  artificial

<220>
<223>  synthetic aptamer


<220>
<221>  misc_feature
<222>  (1)..(14)
<223>  phosphorothioate backbone

<400>  19
gtcgttttgt cgtt                                                          14


<210>  20
<211>  46
<212>  DNA
<213>  artificial

<220>
<223>  synthetic aptamer


<220>
<221>  misc_feature
<222>  (1)..(11)
<223>  phosphorothiate backbone between nucleotides 1-11

<220>
<221>  misc_feature
<222>  (44)..(46)
<223>  phosphorothiate backbone between nucleotides 44-46

<400>  20
aacgttcgag caggctacgg cacgtagagc atcaccatga tcctgc                       46


<210>  21
<211>  49
<212>  DNA
<213>  artificial

<220>
<223>  synthetic aptamer


<220>
<221>  misc_feature
<222>  (1)..(14)
<223>  phosphorothioate backbone between nucleotides 1-14

<220>
<221>  misc_feature
<222>  (47)..(49)
<223>  phosphorothioate backbone between nucleotides 47-49

<400>  21
gtgaacgttc gagcaggcta cggcacgtag agcatcacca tgatcctgc                    49


<210>  22
<211>  64
<212>  DNA

<213>  artificial

<220>
<223>  synthetic aptamer


<220>
<221>  misc_feature
<222>  (1)..(23)
<223>  phoshporothiate backbone between nucleotides 1-23

<220>
<221>  misc_feature
<222>  (56)..(64)
<223>  phoshporothiate backbone between nucleotides 56-64

<400>  22
tgactgtgaa cgttcgagat gacaggctac ggcacgtaga gcatcaccat gatcctgttt      60

tttt                                                                    64


<210>  23
<211>  34
<212>  DNA
<213>  artificial

<220>
<223>  synthetic aptamer


<220>
<221>  misc_feature
<222>  (1)..(4)
<223>  phosphorothioate backbone between nucleotides 1-4

<220>
<221>  misc_feature
<222>  (8)..(13)
<223>  phosphorothioate backbone between nucleotides 8-13

<220>
<221>  misc_feature
<222>  (31)..(34)
<223>  phosphorothioate backbone between nucleotides 31-34

<400>  23
caggctacgt tcgtagagca tcaccatgat cctg                                   34


<210>  24
<211>  35
<212>  DNA
<213>  artificial

<220>
<223>  synthetic aptamer


<220>
<221>  misc_feature
<222>  (1)..(4)
<223>  phosphorothioate backbone between nucleotides 1-4

<220>
<221>  misc_feature
<222>  (8)..(14)
<223>  phosphorothioate backbone between nucleotides 8-14

<220>
<221>  misc_feature
<222>  (32)..(35)
<223>  phosphorothioate backbone between nucleotides 32-35

<400>  24
caggctacgt ttcgtagagc atcaccatga tcctg                                35


<210>  25
<211>  35
<212>  DNA
<213>  artificial

<220>
<223>  synthetic aptamer


<220>
<221>  misc_feature
<222>  (1)..(4)
<223>  phosphorothioate backbone between nucleotides 1-4

<220>
<221>  misc_feature
<222>  (8)..(14)
<223>  phosphorothioate backbone between nucleotides 8-14

<220>
<221>  misc_feature
<222>  (32)..(35)
<223>  phosphorothioate backbone between nucleotides 32-35

<400>  25
caggcaacgt ttcgttgagc atcaccatga tcctg                                35


<210>  26
<211>  34
<212>  DNA
<213>  artificial

<220>
<223>  synthetic aptamer


<220>
<221>  misc_feature
<222>  (1)..(4)
<223>  phosphorothioate backbone between nucleotides 1-4

<220>
<221>  misc_feature
<222>  (8)..(13)
<223>  phosphorothioate backbone between nucleotides 8-13

<220>

<221>  misc_feature
<222>  (31)..(34)
<223>  phosphorothioate backbone between nucleotides 31-34

<400>  26
caggcaacgt tcgttgagca tcaccatgat cctg                                34


<210>  27
<211>  36
<212>  DNA
<213>  artificial

<220>
<223>  synthetic aptamer


<220>
<221>  misc_feature
<222>  (1)..(4)
<223>  phosphorothioate backbone between nucleotides 1-4

<220>
<221>  misc_feature
<222>  (8)..(15)
<223>  phosphorothioate backbone between nucleotides 8-15

<220>
<221>  misc_feature
<222>  (33)..(36)
<223>  phosphorothioate backbone between nucleotides 33-36

<400>  27
caggcaacgt tttcgttgag catcaccatg atcctg                              36


<210>  28
<211>  35
<212>  DNA
<213>  artificial

<220>
<223>  synthetic aptamer


<220>
<221>  misc_feature
<222>  (1)..(4)
<223>  phosphorothioate backbone between nucleotides 1-4

<220>
<221>  misc_feature
<222>  (32)..(35)
<223>  phosphorothioate backbone between nucleotides 32-35

<400>  28
caggctacgt ttcgtagagc atcaccatga tcctg                               35


<210>  29
<211>  35
<212>  DNA
<213>  artificial

<220>
<223>  synthetic aptamer


<220>
<221>  misc_feature
<222>  (1)..(3)
<223>  phosphorothioate backbone between nucleotides 1-3

<220>
<221>  misc_feature
<222>  (33)..(35)
<223>  phosphorothioate backbone between nucleotides 33-35

<400>  29
caggctacgt ttcgtagagc atcaccatga tcctg                                35


<210>  30
<211>  36
<212>  DNA
<213>  artificial

<220>
<223>  synthetic aptamer


<220>
<221>  misc_feature
<222>  (1)..(4)
<223>  phosporothioate backbone between nucleotides 1-4

<220>
<221>  misc_feature
<222>  (33)..(36)
<223>  phosporothioate backbone between nucleotides 33-36

<400>  30
caggcgtcgt tttcgacgag catcaccatg atcctg                               36


<210>  31
<211>  36
<212>  DNA
<213>  artificial

<220>
<223>  synthetic aptamer


<220>
<221>  misc_feature
<222>  (1)..(4)
<223>  phosphorothioate backbone between nucleotides 1-4

<220>
<221>  misc_feature
<222>  (33)..(36)
<223>  phosphorothioate backbone between nucleotides 33-36

<400>  31
caggcgtcgt cgtcgacgag catcaccatg atcctg                               36

<210>  32
<211>  36
<212>  DNA
<213>  artificial

<220>
<223>  synthetic aptamer


<220>
<221>  misc_feature
<222>  (1)..(4)
<223>  phosphorothioate backbone between nucleotides 1-4

<220>
<221>  misc_feature
<222>  (33)..(36)
<223>  phosphorothioate backbone between nucleotides 33-36

<400>  32
caggcttcgt cgtcgaagag catcaccatg atcctg                                36


<210>  33
<211>  36
<212>  DNA
<213>  artificial

<220>
<223>  synthetic aptamer


<220>
<221>  misc_feature
<222>  (1)..(4)
<223>  phosphorothioate backbone between nucleotides 1-4

<220>
<221>  misc_feature
<222>  (33)..(36)
<223>  phosphorothioate backbone between nucleotides 33-36

<400>  33
caggctacgt cgtcgtagag catcaccatg atcctg                                36


<210>  34
<211>  35
<212>  DNA
<213>  artificial

<220>
<223>  synthetic aptamer


<220>
<221>  misc_feature
<222>  (1)..(4)
<223>  phosphorothioate backbone between nucleotides 1-4

<220>

<221>  misc_feature
<222>  (8)..(14)
<223>  phosphorothioate backbone between nucleotides 8-14

<220>
<221>  misc_feature
<222>  (32)..(35)
<223>  phosphorothioate backbone between nucleotides 32-35

<400>  34
caggcaagct ttgcttgagc atcaccatga tcctg                                35


<210>  35
<211>  36
<212>  DNA
<213>  artificial

<220>
<223>  synthetic aptamer


<220>
<221>  misc_feature
<222>  (1)..(4)
<223>  phosphorothioate backbone between nucleotides 1-4

<220>
<221>  misc_feature
<222>  (8)..(15)
<223>  phosphorothioate backbone between nucleotides 8-15

<220>
<221>  misc_feature
<222>  (33)..(36)
<223>  phosphorothioate backbone between nucleotides 33-36

<400>  35
caggcaagct tttgcttgag catcaccatg atcctg                               36


<210>  36
<211>  9
<212>  DNA
<213>  artificial

<220>
<223>  synthetic aptamer


<220>
<221>  modified_base
<222>  (9)..(9)
<223>  gm

<400>  36
caggctacg                                                              9


<210>  37
<211>  12
<212>  DNA
<213>  artificial

<220>
<223>  synthetic aptamer


<220>
<221>  modified_base
<222>  (5)..(5)
<223>  gm

<220>
<221>  modified_base
<222>  (7)..(7)
<223>  gm

<220>
<221>  modified_base
<222>  (12)..(12)
<223>  adenosine is 2'-O-Methyl

<400>  37
cgtagagcat ca                                                          12


<210>  38
<211>  8
<212>  DNA
<213>  artificial

<220>
<223>  synthetic aptamer


<220>
<221>  modified_base
<222>  (8)..(8)
<223>  gm

<400>  38
tgatcctg                                                                8


<210>  39
<211>  9
<212>  DNA
<213>  artificial

<220>
<223>  synthetic aptamer


<220>
<221>  modified_base
<222>  (6)..(6)
<223>  um

<220>
<221>  modified_base
<222>  (8)..(8)
<223>  cm

<220>
<221>  modified_base

<222>  (9)..(9)
<223>  gm

<400>  39
caggcuacg                                                                9


<210>  40
<211>  12
<212>  DNA
<213>  artificial

<220>
<223>  synthetic aptamer


<220>
<221>  modified_base
<222>  (5)..(5)
<223>  gm

<220>
<221>  modified_base
<222>  (7)..(7)
<223>  gm

<220>
<221>  modified_base
<222>  (10)..(10)
<223>  um

<220>
<221>  modified_base
<222>  (11)..(11)
<223>  cm

<220>
<221>  modified_base
<222>  (12)..(12)
<223>  adenosine is 2'-O-Methyl

<400>  40
cgtagagcau ca                                                           12


<210>  41
<211>  8
<212>  DNA
<213>  artificial

<220>
<223>  synthetic aptamer


<220>
<221>  modified_base
<222>  (5)..(6)
<223>  cm

<220>
<221>  modified_base
<222>  (7)..(7)
<223>  um

<220>
<221> modified_base
<222> (8)..(8)
<223> gm

<400> 41
tgatccug 8


<210> 42
<211> 9
<212> DNA
<213> artificial

<220>
<223> synthetic aptamer

<400> 42
caggctacg 9


<210> 43
<211> 12
<212> DNA
<213> artificial

<220>
<223> synthetic aptamer

<400> 43
cgtagagcat ca 12


<210> 44
<211> 8
<212> DNA
<213> artificial

<220>
<223> synthetic aptamer

<400> 44
tgatcctg 8


<210> 45
<211> 9
<212> DNA
<213> artificial

<220>
<223> synthetic aptamer


<220>
<221> modified_base
<222> (1)..(9)
<223> All purines (A and G) and all pyrimidines (C and U) are
      2'-O-Methyl

<400> 45
caggcuacg 9

<210>  46
<211>  12
<212>  DNA
<213>  artificial

<220>
<223>  synthetic aptamer


<220>
<221>  modified_base
<222>  (1)..(12)
<223>  All purines (A and G) and all pyrimidines (C and U) are
       2'-O-Methyl

<400>  46
cguagagcau ca                                                          12


<210>  47
<211>  8
<212>  RNA
<213>  artificial

<220>
<223>  synthetic aptamer


<220>
<221>  modified_base
<222>  (1)..(8)
<223>  All purines (A and G) and all pyrimidines (C and U) are
       2'-O-Methyl

<400>  47
ugauccug                                                                8


<210>  48
<211>  10
<212>  DNA
<213>  artificial

<220>
<223>  synthetic aptamer

<400>  48
acaggctacg                                                             10


<210>  49
<211>  9
<212>  DNA
<213>  artificial

<220>
<223>  synthetic aptamer

<400>  49
tgatcctgt                                                               9

<210>  50
<211>  11
<212>  DNA
<213>  artificial

<220>
<223>  synthetic aptamer

<400>  50
cacaggctac g                                                            11


<210>  51
<211>  10
<212>  DNA
<213>  artificial

<220>
<223>  synthetic aptamer

<400>  51
tgatcctgtg                                                              10


<210>  52
<211>  9
<212>  DNA
<213>  artificial

<220>
<223>  synthetic aptamer


<220>
<221>  modified_base
<222>  (1)..(1)
<223>  cm

<220>
<221>  modified_base
<222>  (2)..(2)
<223>  adenosine is 2'-O-Methyl

<220>
<221>  modified_base
<222>  (3)..(4)
<223>  gm

<400>  52
caggctacg                                                                9


<210>  53
<211>  9
<212>  DNA
<213>  artificial

<220>
<223>  synthetic aptamer


<220>

<221>  modified_base
<222>  (5)..(6)
<223>  cm

<220>
<221>  modified_base
<222>  (7)..(7)
<223>  um

<220>
<221>  modified_base
<222>  (8)..(8)
<223>  gm

<220>
<221>  modified_base
<222>  (9)..(9)
<223>  um

<400>  53
tgatccugu                                                                9


<210>  54
<211>  10
<212>  DNA
<213>  artificial

<220>
<223>  synthetic aptamer


<220>
<221>  modified_base
<222>  (5)..(6)
<223>  cm

<220>
<221>  modified_base
<222>  (7)..(7)
<223>  um

<220>
<221>  modified_base
<222>  (8)..(8)
<223>  gm

<220>
<221>  modified_base
<222>  (9)..(9)
<223>  um

<220>
<221>  modified_base
<222>  (10)..(10)
<223>  gm

<400>  54
tgatccugug                                                               10


<210>  55
<211>  9

<212>  DNA
<213>  artificial

<220>
<223>  synthetic aptamer


<220>
<221>  modified_base
<222>  (8)..(8)
<223>  cm

<220>
<221>  modified_base
<222>  (9)..(9)
<223>  gm

<400>  55
caggctacg                                                                9


<210>  56
<211>  9
<212>  DNA
<213>  artificial

<220>
<223>  synthetic aptamer


<220>
<221>  modified_base
<222>  (6)..(6)
<223>  um

<220>
<221>  modified_base
<222>  (9)..(9)
<223>  gm

<400>  56
caggcuacg                                                                9


<210>  57
<211>  12
<212>  DNA
<213>  artificial

<220>
<223>  synthetic aptamer


<220>
<221>  modified_base
<222>  (5)..(5)
<223>  gm

<220>
<221>  modified_base
<222>  (7)..(7)
<223>  gm

<220>
<221>  modified_base
<222>  (11)..(11)
<223>  cm

<220>
<221>  modified_base
<222>  (12)..(12)
<223>  2'-O-Methyl adenosine

<400>  57
cgtagagcat ca                                                          12


<210>  58
<211>  12
<212>  DNA
<213>  artificial

<220>
<223>  synthetic aptamer


<220>
<221>  modified_base
<222>  (5)..(5)
<223>  gm

<220>
<221>  modified_base
<222>  (7)..(7)
<223>  gm

<220>
<221>  modified_base
<222>  (10)..(10)
<223>  um

<220>
<221>  modified_base
<222>  (12)..(12)
<223>  2'-O-Methyl adenosine

<400>  58
cgtagagcau ca                                                          12


<210>  59
<211>  9
<212>  DNA
<213>  artificial

<220>
<223>  synthetic aptamer


<220>
<221>  modified_base
<222>  (5)..(5)
<223>  cm

<220>
<221>  modified_base

<222>  (6)..(6)
<223>  um

<220>
<221>  modified_base
<222>  (8)..(8)
<223>  cm

<400>  59
caggcuacg                                                                9


<210>  60
<211>  12
<212>  DNA
<213>  artificial

<220>
<223>  synthetic aptamer


<220>
<221>  modified_base
<222>  (1)..(1)
<223>  cm

<220>
<221>  modified_base
<222>  (3)..(3)
<223>  um

<220>
<221>  modified_base
<222>  (8)..(8)
<223>  cm

<220>
<221>  modified_base
<222>  (10)..(10)
<223>  um

<220>
<221>  modified_base
<222>  (11)..(11)
<223>  cm

<400>  60
cguagagcau ca                                                           12


<210>  61
<211>  8
<212>  DNA
<213>  artificial

<220>
<223>  synthetic aptamer


<220>
<221>  modified_base
<222>  (1)..(1)
<223>  um

<220>
<221>  modified_base
<222>  (4)..(4)
<223>  um

<220>
<221>  modified_base
<222>  (5)..(6)
<223>  cm

<220>
<221>  modified_base
<222>  (7)..(7)
<223>  um

<400>  61
ugauccug                                                                8


<210>  62
<211>  8
<212>  DNA
<213>  artificial

<220>
<223>  synthetic aptamer


<220>
<221>  modified_base
<222>  (6)..(6)
<223>  cm

<220>
<221>  modified_base
<222>  (7)..(7)
<223>  um

<220>
<221>  modified_base
<222>  (8)..(8)
<223>  gm

<400>  62
tgatccug                                                                8


<210>  63
<211>  11
<212>  DNA
<213>  artificial

<220>
<223>  synthetic aptamer


<220>
<221>  modified_base
<222>  (11)..(11)
<223>  gm

<400>  63

cacaggctac g                                                            11


<210>  64
<211>  10
<212>  DNA
<213>  artificial

<220>
<223>  synthetic aptamer

<400>  64
tgatcctgtg                                                             10


<210>  65
<211>  10
<212>  DNA
<213>  artificial

<220>
<223>  synthetic aptamer


<220>
<221>  modified_base
<222>  (6)..(6)
<223>  cm

<220>
<221>  modified_base
<222>  (7)..(7)
<223>  um

<220>
<221>  modified_base
<222>  (8)..(8)
<223>  gm

<220>
<221>  modified_base
<222>  (9)..(9)
<223>  um

<220>
<221>  modified_base
<222>  (10)..(10)
<223>  gm

<400>  65
tgatccugug                                                             10


<210>  66
<211>  11
<212>  DNA
<213>  artificial

<220>
<223>  synthetic aptamer


<220>

<221>  modified_base
<222>  (8)..(8)
<223>  um

<220>
<221>  modified_base
<222>  (10)..(10)
<223>  cm

<220>
<221>  modified_base
<222>  (11)..(11)
<223>  gm

<400>  66
cacaggcuac g                                                            11


<210>  67
<211>  8
<212>  DNA
<213>  artificial

<220>
<223>  synthetic aptamer


<220>
<221>  modified_base
<222>  (5)..(5)
<223>  cm

<220>
<221>  modified_base
<222>  (7)..(7)
<223>  um

<220>
<221>  modified_base
<222>  (8)..(8)
<223>  gm

<400>  67
tgatccug                                                                 8


<210>  68
<211>  8
<212>  DNA
<213>  artificial

<220>
<223>  synthetic aptamer


<220>
<221>  modified_base
<222>  (5)..(6)
<223>  cm

<220>
<221>  modified_base
<222>  (8)..(8)

<223>  gm

<400>  68
tgatcctg                                                                8


<210>  69
<211>  11
<212>  DNA
<213>  artificial

<220>
<223>  synthetic aptamer


<220>
<221>  modified_base
<222>  (11)..(11)
<223>  gm

<400>  69
cccaggctac g                                                           11


<210>  70
<211>  10
<212>  DNA
<213>  artificial

<220>
<223>  synthetic aptamer


<220>
<221>  modified_base
<222>  (5)..(5)
<223>  cm

<220>
<221>  modified_base
<222>  (8)..(10)
<223>  gm

<400>  70
tgatcctggg                                                             10


<210>  71
<211>  10
<212>  DNA
<213>  artificial

<220>
<223>  synthetic aptamer


<220>
<221>  modified_base
<222>  (8)..(10)
<223>  gm

<400>  71
tgatcctggg                                                             10

<210>  72
<211>  10
<212>  DNA
<213>  artificial

<220>
<223>  synthetic aptamer

<400>  72
tgatcctggg                                                              10


<210>  73
<211>  10
<212>  DNA
<213>  artificial

<220>
<223>  synthetic aptamer


<220>
<221>  modified_base
<222>  (5)..(5)
<223>  cm

<400>  73
tgatcctggg                                                              10


<210>  74
<211>  88
<212>  RNA
<213>  artificial

<220>
<223>  synthetic template


<220>
<221>  modified_base
<222>  (1)..(88)
<223>  all pyrimidines (C and U) are 2'-fluoro

<220>
<221>  misc_feature
<222>  (25)..(64)
<223>  n is a, g, c, or u

<400>  74
gggaaaagcg aaucauacac aagannnnnn nnnnnnnnnn nnnnnnnnnn nnnnnnnnnn      60

nnnngcuccg ccagagacca accgagaa                                          88


<210>  75
<211>  41
<212>  DNA
<213>  artificial

<220>

<223>  synthetic primer

<400>  75
taatacgact cactataggg aaaagcgaat catacacaag a                41


<210>  76
<211>  24
<212>  DNA
<213>  artificial

<220>
<223>  synthetic primer

<400>  76
ttctcggttg gtctctggcg gagc                                   24


<210>  77
<211>  87
<212>  RNA
<213>  artificial

<220>
<223>  synthetic aptamer


<220>
<221>  modified_base
<222>  (1)..(87)
<223>  all pyrimidines (C and U) are 2'-fluoro

<400>  77
gggaaaagcg aaucauacac aagaucgcca ggagcaaagu cacggaggag uggggguacg      60

aaugcuccgc cagagaccaa ccgagaa                                87


<210>  78
<211>  88
<212>  RNA
<213>  artificial

<220>
<223>  synthetic aptamer


<220>
<221>  modified_base
<222>  (1)..(88)
<223>  all pyrimidines (C and U) are 2'-fluoro

<400>  78
gggaaaagcg aaucauacac aagaccggga acucggauuc uucgcaugug gaugcgauca      60

guaugcuccg ccagagacca accgagaa                               88


<210>  79
<211>  88
<212>  RNA
<213>  artificial

<220>
<223>  synthetic aptamer

<220>
<221>  modified_base
<222>  (1)..(88)
<223>  all pyrimidines (C and U)  are 2'-fluoro

<400>  79
gggaaaagcg aaucauacac aagaccggga acucggauuc uucacaugug gaugugauca     60

guaugcuccg ccagagacca accgagaa                                        88

<210>  80
<211>  88
<212>  RNA
<213>  artificial

<220>
<223>  synthetic aptamer

<220>
<221>  modified_base
<222>  (1)..(88)
<223>  all pyrimidines (C and U) are 2'-fluoro

<400>  80
gggaaaagcg aaucauacac aagaccggaa acucggauuc uucgcaugug gaugcgauca     60

guaugcuccg ccagagacca accgagaa                                        88

<210>  81
<211>  88
<212>  RNA
<213>  artificial

<220>
<223>  synthetic aptamer

<220>
<221>  modified_base
<222>  (1)..(88)
<223>  all pyrimidines (C and U) are 2'-fluoro

<400>  81
gggaaaagcg aaucauacac aagagagugg aggagguaug uaugguuugu gcgucuggug     60

cggugcuccg ccagagacca accgagaa                                        88

<210>  82
<211>  93
<212>  DNA
<213>  artificial

<220>
<223>  synthetic template

<220>
<221>  modified_base
<222>  (1)..(93)
<223>  all purines (A and G) and all pyrimidines (C and T) can be
      2'-O-Methyl

<220>
<221>  misc_feature
<222>  (25)..(54)
<223>  n is a, g, c, or t

<400>  82
catcgatgct agtcgtaacg atccnnnnnn nnnnnnnnnn nnnnnnnnnn nnnncgagaa      60

cgttctctcc tctccctata gtgagtcgta tta                                   93


<210>  83
<211>  92
<212>  DNA
<213>  artificial

<220>
<223>  synthetic template


<220>
<221>  misc_feature
<222>  (1)..(92)
<223>  all purines (A and G) and all pyrimidines (C and T) can be
      2'-O-Methyl

<220>
<221>  misc_feature
<222>  (24)..(53)
<223>  n is a, g, c, or t

<400>  83
catgcatcgc gactgactag ccgnnnnnnn nnnnnnnnnn nnnnnnnnnn nnngtagaac      60

gttctctcct ctccctatag tgagtcgtat ta                                    92


<210>  84
<211>  92
<212>  DNA
<213>  artificial

<220>
<223>  synthetic template


<220>
<221>  modified_base
<222>  (1)..(92)
<223>  all pyrimidines (C and T) can be 2'-O-Methyl

<220>
<221>  misc_feature
<222>  (24)..(53)
<223>  n is a, g, c, or t

<400>  84
catcgatcga tcgatcgaca gcgnnnnnnn nnnnnnnnnn nnnnnnnnnn nnngtagaac     60

gttctctcct ctccctatag tgagtcgtat ta                                   92


<210>  85
<211>  10
<212>  DNA
<213>  artificial

<220>
<223>  synthetic

<400>  85
aacgttcgag                                                            10



<210>  86
<211>  26
<212>  DNA
<213>  artificial

<220>
<223>  synthetic aptamer


<220>
<221>  modified_base
<222>  (11)..(14)
<223>  2'-O-Methyl

<220>
<221>  modified_base
<222>  (19)..(19)
<223>  2'-O-Methyl

<220>
<221>  modified_base
<222>  (21)..(21)
<223>  2'-O-Methyl

<220>
<221>  modified_base
<222>  (24)..(26)
<223>  2'-O-Methyl

<400>  86
cccaggctac guuucgtaga gcauca                                          26


<210>  87
<211>  10
<212>  DNA
<213>  Artificial

<220>
<223>  synthetic aptamer


<220>
<221>  modified_base

<222>  (5)..(5)
<223>  2'-O-Methyl

<220>
<221>  modified_base
<222>  (8)..(10)
<223>  2'-O-Methyl

<400>  87
tgatcctggg                                                              10


<210>  88
<211>  27
<212>  DNA
<213>  Artificial

<220>
<223>  synthetic aptamer


<220>
<221>  modified_base
<222>  (11)..(15)
<223>  2'-O-Methyl

<220>
<221>  modified_base
<222>  (20)..(20)
<223>  2'-O-Methyl

<220>
<221>  modified_base
<222>  (22)..(22)
<223>  2'-O-Methyl

<220>
<221>  modified_base
<222>  (25)..(27)
<223>  2'-O-Methyl

<400>  88
cccaggctac guuucgtag agcauca                                            27


<210>  89
<211>  11
<212>  DNA
<213>  Artificial

<220>
<223>  synthetic aptamer


<220>
<221>  modified_base
<222>  (11)..(11)
<223>  2'-O-Methyl

<400>  89
cccaggctac g                                                            11

<210>  90
<211>  6
<212>  DNA
<213>  Artificial

<220>
<223>  synthetic aptamer

<400>  90
aacgtt                                                                  6


<210>  91
<211>  25
<212>  DNA
<213>  Artificial

<220>
<223>  synthetic aptamer


<220>
<221>  modified_base
<222>  (5)..(5)
<223>  2'-O-Methyl

<220>
<221>  modified_base
<222>  (7)..(7)
<223>  2'-O-Methyl

<220>
<221>  modified_base
<222>  (10)..(15)
<223>  2'-O-Methyl

<220>
<221>  modified_base
<222>  (20)..(20)
<223>  2'-O-Methyl

<220>
<221>  modified_base
<222>  (23)..(25)
<223>  2'-O-Methyl

<400>  91
cgtagagcau cauuutgatc ctggg                                            25


<210>  92
<211>  4
<212>  DNA
<213>  Artificial

<220>
<223>  synthetic aptamer

<400>  92
acgt                                                                    4


<210>  93

<211>  26
<212>  DNA
<213>  Artificial

<220>
<223>  synthetic aptamer


<220>
<221>  modified_base
<222>  (5)..(5)
<223>  2'-O-Methyl

<220>
<221>  modified_base
<222>  (7)..(7)
<223>  2'-O-Methyl

<220>
<221>  modified_base
<222>  (10)..(16)
<223>  2'-O-Methyl

<220>
<221>  modified_base
<222>  (21)..(21)
<223>  2'-O-Methyl

<220>
<221>  modified_base
<222>  (24)..(26)
<223>  2'-O-Methyl

<400>  93
cgtagagcau cauuuutgat cctggg                                          26


<210>  94
<211>  39
<212>  DNA
<213>  Artificial

<220>
<223>  synthetic aptamer


<220>
<221>  modified_base
<222>  (11)..(14)
<223>  2'-O-Methyl

<220>
<221>  modified_base
<222>  (19)..(19)
<223>  2'-O-Methyl

<220>
<221>  modified_base
<222>  (21)..(21)
<223>  2'-O-Methyl

<220>
<221>  modified_base

<222>   (24)..(29)
<223>   2'-O-Methyl

<220>
<221>   modified_base
<222>   (34)..(34)
<223>   2'-O-Methyl

<220>
<221>   modified_base
<222>   (37)..(39)
<223>   2'-O-Methyl

<400>   94
cccaggctac guuucgtaga gcaucauuut gatcctggg                              39


<210>   95
<211>   41
<212>   DNA
<213>   Artificial

<220>
<223>   synthetic aptamer


<220>
<221>   modified_base
<222>   (11)..(15)
<223>   2'-O-Methyl

<220>
<221>   modified_base
<222>   (20)..(20)
<223>   2'-O-Methyl

<220>
<221>   modified_base
<222>   (22)..(22)
<223>   2'-O-Methyl

<220>
<221>   modified_base
<222>   (25)..(31)
<223>   2'-O-Methyl

<220>
<221>   modified_base
<222>   (36)..(36)
<223>   2'-O-Methyl

<220>
<221>   modified_base
<222>   (39)..(41)
<223>   2'-O-Methyl

<400>   95
cccaggctac guuuucgtag agcaucauuu utgatcctgg g                           41


<210>   96
<211>   4
<212>   DNA

<213>  Artificial

<220>
<223>  synthetic aptamer

<400>  96
rcgy                                                                    4


<210>  97
<211>  4
<212>  DNA
<213>  Artificial

<220>
<223>  synthetic aptamer

<400>  97
gaaa                                                                    4


<210>  98
<211>  6
<212>  DNA
<213>  Artificial

<220>
<223>  synthetic aptamer

<400>  98
rrcgyy                                                                  6


<210>  99
<211>  4
<212>  DNA
<213>  Artificial

<220>
<223>  synthetic aptamer


<220>
<221>  misc_feature
<222>  (1)..(1)

<220>
<221>  misc_feature
<222>  (1)..(1)
<223>  n is a, c, g, or t

<220>
<221>  misc_feature
<222>  (4)..(4)

<220>
<221>  misc_feature
<222>  (4)..(4)
<223>  n is a, c, g, or t

<400>  99
ncgn                                                                    4

<210>  100
<211>  6
<212>  DNA
<213>  Artificial

<220>
<223>  synthetic aptamer


<220>
<221>  misc_feature
<222>  (1)..(2)
<223>  n is a, c, g, or t

<220>
<221>  misc_feature
<222>  (5)..(6)
<223>  n is a, c, g, or t

<400>  100
nncgnn                                                                  6


<210>  101
<211>  6
<212>  DNA
<213>  Artificial

<220>
<223>  synthetic aptamer

<400>  101
rdcght                                                                  6


<210>  102
<211>  10
<212>  DNA
<213>  Artificial

<220>
<223>  synthetic aptamer


<220>
<221>  modified_base
<222>  (5)..(5)
<223>  2'-O-Methyl

<220>
<221>  modified_base
<222>  (8)..(10)
<223>  2'-O-Methyl

<400>  102
tgatcctggg                                                             10